(19) Europäisches Patentamt
European Patent Office
Office européen des brevets

(11) EP 4 242 210 A1

(12) # EUROPEAN PATENT APPLICATION

published in accordance with Art. 153(4) EPC

(43) Date of publication:
**13.09.2023 Bulletin 2023/37**

(21) Application number: **21888710.7**

(22) Date of filing: **09.11.2021**

(51) International Patent Classification (IPC):
***C07D 487/04*** (2006.01) ***C07F 9/6561*** (2006.01)
***A61K 31/5517*** (2006.01) ***A61K 31/675*** (2006.01)
***A61P 25/20*** (2006.01) ***A61P 23/00*** (2006.01)

(52) Cooperative Patent Classification (CPC):
Y02P 20/55

(86) International application number:
**PCT/CN2021/129655**

(87) International publication number:
**WO 2022/096018 (12.05.2022 Gazette 2022/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.11.2020 CN 202011237945**

(71) Applicant: **China Pharmaceutical University**
**Nanjing, Jiangsu 210009 (CN)**

(72) Inventors:
- **QIAN, Hai**
  **Nanjing, Jiangsu 210009 (CN)**
- **SHI, Wei**
  **Nanjing, Jiangsu 210009 (CN)**
- **HUANG, Wenlong**
  **Nanjing, Jiangsu 210009 (CN)**
- **LI, Qifei**
  **Nanjing, Jiangsu 210009 (CN)**
- **CAI, Yan**
  **Nanjing, Jiangsu 210009 (CN)**
- **MO, Jiaxian**
  **Nanjing, Jiangsu 210009 (CN)**
- **CHEN, Bin**
  **Nanjing, Jiangsu 210009 (CN)**
- **ZHOU, Jiaqi**
  **Nanjing, Jiangsu 210009 (CN)**
- **ZOU, Yuxing**
  **Nanjing, Jiangsu 210009 (CN)**

(74) Representative: **Gille Hrabal Partnerschaftsgesellschaft mbB**
**Patentanwälte**
**Brucknerstraße 20**
**40593 Düsseldorf (DE)**

(54) **NEW BENZODIAZEPINE COMPOUNDS, AND PREPARATION METHOD THEREFOR AND USE THEREOF**

(57) Provided are new benzodiazepine compounds, and a preparation method therefor and the use thereof. The present invention belongs to the field of pharmaceutical chemistry. Short-acting benzodiazepine derivatives have the characteristics of a fast onset, short acting time, deep strength, being quickly metabolized, fast wake-up, etc. In anesthesia experiments on mice and rats, short-acting benzodiazepine derivatives have a comparable onset time to remazolam, but have a significantly shortened acting time and wake-up time. Moreover, some of the compounds have a significantly enhanced depth of action. Thus, the compounds have the characteristics of a faster onset, shorter acting time, greater strength of action, being metabolized faster, faster wake-up, etc. In long-term infusion anesthesia experiments on rats, compared with remazolam, the short-acting benzodiazepine derivatives have a significantly shortened wake-up time and recovery time, and have the characteristics of faster wake-up, a faster recovery, etc.

EP 4 242 210 A1

## Description

The present application claims priority to the Patent Application for Invention with the application No. 202011237945.7 and entitled "NEW BENZODIAZEPINE COMPOUNDS, AND PREPARATION METHOD THEREFOR AND USE THEREOF" filed with China National Intellectual Property Administration on November 9, 2020, which is incorporated herein by reference in its entirety.

## TECHNICAL FIELD

The present disclosure relates to a benzodiazepine derivative of formula (I) as a short-acting anaesthetic, a pharmaceutical composition comprising the same, a kit comprising the same, a preparation method therefor, a method of sedation and anaesthesia using the same, and use thereof for manufacturing a medicament for sedation and anaesthesia.

## BACKGROUND

Anesthetic drugs can cause temporary and reversible loss of consciousness and pain in the body or part of the body and help patients reduce pain and other uncomfortable symptoms, and are essential auxiliary drugs for clinical surgery.

Benzodiazepines are activators of a $GABA_A$ receptor (also known as $\gamma$-aminobutyric acid type A receptor). The $GABA_A$ receptor is a gated receptor for a chloride ion channel which is composed of two $\alpha$ and two $\beta$ subunits ($\alpha 2\beta 2$). There is a GABA receptor site on the $\beta$ subunit, and when GABA binds to the GABA receptor site, the chloride ion channel is opened to allow chloride ions to flow in, so that the nerve cells are hyperpolarized, and an inhibition effect is generated. There is a benzodiazepine receptor on the $\alpha$ subunit, and when a benzodiazepine binds to the benzodiazepine receptor, the binding of GABA to the GABAA receptor can be promoted to enable an increased opening frequency of the chloride channel (rather than an increased opening time of the chloride channel or an increased chloride ion flow), thereby allowing more chloride ions to flow in. This enables benzodiazepine derivatives to enhance GABA neurotransmission function and synapse inhibitory effect, thereby exerting various therapeutic effects of anesthesia induction, hypnosis, anxiolysis, and alleviation of central nervous system disorder or epileptic spasm, and the like in clinical practice. Benzodiazepine drugs, which are drugs for sedation and anesthesia that have been rapidly developed in recent years, have good pharmaceutical effects such as oblivion, anxiolysis, and sedation, and thus are widely used in the fields of sedation and anesthesia. The first benzodiazepine drug, chlordiazepoxide (Librium), was marketed in 1960. Since then, an increasing number of novel benzodiazepine drugs have emerged, including ultrashort-acting drugs (remimazolam), short-acting drugs (triazolam/midazolam), medium-acting drugs (lorazepam/estazolam), long-acting (diazepam), and the like. There are more than 20 benzodiazepine derivatives commonly used in clinical practice, which have similar structures but different clinical indications. The reason is that the binding sites and actions of GABA and GABA receptors are heterogeneous. GABA-gated chloride channels of different nerves are composed of different kinds of subunits, and the composition of the different subunits can cause subtle changes in the interaction of these channels with allosteric modulators, resulting in different sedative and anesthetic effects. Midazolam was introduced to the market in the early 1980s as the first benzodiazepine compound with water solubility and was used as an intravenous injection to provide sedative and anesthetic means for short-term surgery or intensive care units. However, midazolam produces active metabolites *in vivo,* resulting in a relatively long time required for patients to regain consciousness from the midazolam-induced sedative and anesthetic state. In addition, since the metabolism of midazolam depends on the hepatic enzyme cytochrome $P_{450}$3A4, after it is administered to patients with impaired liver function, the problem of drug-drug interaction may occur. Remimazolam was approved for marketing in December 2019 as a novel ultrashort-acting $GABA_A$ receptor agonist by introducing a methyl propionate side chain that can be metabolized rapidly on the benzodiazepine parent nucleus structure. Since the side chain of methyl propionate can be rapidly metabolized by esterase, and the main metabolite remimazolam propionate has almost no sedative and anesthetic activity, it has a very short duration of efficacy and belongs to the ultrashort-acting benzodiazepine drugs. However, long-term infusion of remimazolam still causes problems such as wake-up delay and drug accumulation, and is only approved for sedation for gastroscopy and general anesthesia procedures.

In conclusion, benzodiazepine anaesthetic drugs are often used with dependence and addiction, and most of the drug metabolites still have certain drug effects and cannot rapidly return patients to a normal state, and long-term use of the drugs tends to result in drug accumulation, which hinders further development of the drugs. Therefore, the development of a benzodiazepine drug with fast onset of action, short recovery time, and no accumulation is a problem to be solved.

## SUMMARY

In view of the problem described above, the present disclosure provides a novel benzodiazepine compound, and a preparation method therefor and use thereof.

The technical solutions of the present disclosure are as follows: provided is a novel benzodiazepine compound, comprising a compound represented by general formula (I), and a pharmaceutically acceptable salt, a stereoisomer, a tautomer, a polymorph, a solvate, a metabolite, or a prodrug thereof:

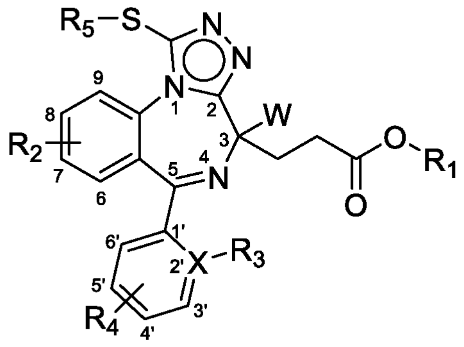

(I)

wherein:

$R_1$ is selected from hydrogen, $C_{1-20}$ alkyl, $C_{2-20}$ alkenyl, $C_{2-20}$ alkynyl, 3- to 10-membered cycloalkyl or C1-10 alkylene-3-to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more (e.g., 1, 2, 3, or 4) substituents independently selected from halogen, hydroxy, cyano, amino, nitro, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl; preferably $R_1$ is selected from hydrogen, $C_{1-6}$ alkyl, and $C_{1-10}$ alkylene-5- to 14-membered heteroaryl; more preferably $R_1$ is selected from hydrogen, methyl, ethyl, propyl, isopropyl, *n*-butyl, isobutyl, *tert*-butyl, n-pentyl, isopentyl, neopentyl, and $C_{1-6}$ alkylene-morpholinyl;

$R_2$ is located at any position of the benzene ring and is monosubstituted or polysubstituted, preferably substituted at positions selected from positions 6, 7, 8, and 9, more preferably substituted at a position selected from position 7;

$R_2$ is selected from hydrogen, halogen, hydroxy, cyano, $(R_6)(R_7)N-(CH_2)_n-$, $R_6R_7N-(CH_2)_n-$, trifluoromethyl, nitro, amino, $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more (e.g., 1, 2, 3, or 4) substituents independently selected from halogen, hydroxy, cyano, amino, nitro, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl; preferably, $R_2$ is selected from hydrogen, halogen, hydroxy, $R_6R_7N-(CH_2)_{1-10}-$, $(R_6)(R_7)N-(CH_2)_{1-10}-$, trifluoromethyl, nitro, cyano, amino, $C_{1-6}$ alkyl, $C_{1-6}$ alkoxy, $C_{2-6}$ alkenyl, and $C_{2-6}$ alkynyl; more preferably, $R_2$ is selected from hydrogen, chlorine, bromine, fluorine, nitro, cyano, amino, $(R_6)(R_7)N-(CH_2)_{1-6}-$ or $R_6R_7N-(CH_2)_{1-6}-$, and trifluoromethyl; further preferably, $R_2$ is selected from hydrogen, chlorine, bromine, fluorine, nitro, and trifluoromethyl;

X is selected from C and N, and when X is C, $R_3$ is selected from hydrogen, halogen, hydroxy, cyano, $R_6R_7N-(CH_2)_n-$,

$(R_6)(R_7)N-(CH_2)_n-$, trifluoromethyl, nitro, amino, $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more (e.g., 1, 2, 3, or 4) substituents independently selected from halogen, hydroxy, cyano, amino, nitro, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl; preferably, $R_3$ is selected from hydrogen, halogen, hydroxy, $R_6R_7N-(CH_2)_{1-10}-$, $(R_6)(R_7)N-(CH_2)_{1-10}-$, trifluoromethyl, nitro, cyano, amino, $C_{1-6}$ alkyl, $C_{1-6}$ alkoxy, $C_{2-6}$ alkenyl, and $C_{2-6}$ alkynyl; more preferably, $R_3$ is selected from hydrogen, chlorine, bromine, fluorine, nitro, cyano, amino, $R_6R_7N-(CH_2)_{1-6}-$, $(R_6)(R_7)N-(CH_2)_{1-6}-$, and trifluoromethyl; when X is N, the $R_3$ substituent is absent;

$R_4$ is located at any position of the benzene ring or pyridine ring, and is monosubstituted or polysubstituted, preferably substituted at positions selected from positions 3', 4', and 5', more preferably substituted at a position selected from position 4';

$R_4$ is selected from hydrogen, halogen, hydroxy, cyano, $R_6R_7N-(CH_2)_n-$, $(R_6)(R_7)N-(CH_2)_n-$, trifluoromethyl, nitro, amino, $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more (e.g., 1, 2, 3, or 4) substituents independently selected from halogen, hydroxy, cyano, amino, nitro, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl; preferably, $R_4$ is selected from hydrogen, halogen, hydroxy, $R_6R_7N-(CH_2)_1-_{10}-$, $(R_6)(R_7)N_-(CH_2)_{1-10}-$, trifluoromethyl, nitro, cyano, amino, $C_{1-6}$ alkyl, $C_{1-6}$ alkoxy, $C_{2-6}$ alkenyl, and $C_{2-6}$ alkynyl; more preferably, $R_4$ is selected from hydrogen, chlorine, bromine, fluorine, nitro, cyano, amino, $(R_6)(R_7)N-(CH_2)_{1-6}-$, and trifluoromethyl;

$R_5$ is selected from hydrogen, $C_{1-20}$ alkyl, $C_{1-20}$ acyl, $C_{2-20}$ alkenyl, $C_{2-20}$ alkynyl, $C_{1-10}$ alkylene-phosphate group, $P(O)(OH)_2-C_{1-10}$ alkylene, 3- to 10-membered cycloalkyl or $C_{1-10}$ alkylene-3- to 10-membered cycloalkyl, $R_6R_7N-(CH_2)_n-$, $(R_6)(R_7)N-(CH_2)_n-$, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl; it will be understood by those skilled in the art that when $R_5$ is hydrogen, -SH is capable of resonating with the triazole ring attached thereto to form an =S structure;

the $C_{1-20}$ alkyl, $C_{1-20}$ acyl, $C_{2-20}$ alkenyl, $C_{2-20}$ alkynyl, 3- to 10-membered cycloalkyl or $C_{1-10}$ alkylene-3- to 10-membered cycloalkyl, $R_6R_7N-(CH_2)_n-$, $(R_6)(R_7)N-(CH_2)_n-$, 3- to 10-membered heterocyclyl or $C_{1-10}$ alkylene-3- to 10-membered heterocyclyl, 6- to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more (e.g., 1, 2, 3, or 4) substituents independently selected from halogen, oxo (= O), $C_{1-10}$ alkylene-halogen, hydroxy, $C_{1-10}$ alkylene-hydroxy, $R_6R_7N-(CH_2)_n-$, $(R_6)(R_7)N-(CH_2)_n-$, cyano, $C_{1-10}$ alkylene-cyano, nitro, $C_{1-10}$ alkylene-nitro, amino, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_1$ $-_{10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl; preferably, $R_5$ is selected from hydrogen and $C_{1-10}$ alkyl, $C_{1-10}$ alkylene-phosphate group, $P(O)(OH)_2-C_{1-10}$ alkylene, 3- to 10-membered cycloalkyl or $C_{1-10}$ alkylene-3- to 10-membered cycloalkyl, $R_6R_7N-(CH_2)_n-$, $(R_6)(R_7)N-(CH_2)_n-$, and 3- to 10-membered heterocyclyl or $C_{1-10}$ alkylene-3- to 10-membered heterocyclyl; more preferably, $R_5$ is selected from hydrogen, methyl, ethyl, methylene-phosphate group or $P(O)(OH)_2$-methylene, $C_{1-10}$ alkylene-morpholinyl, $C_{1-10}$ alkylene-pyrrolyl, $C_{1-10}$ alkylene-imidazolyl, $C_{1-10}$ alkylene-piperidinyl, 2-(4-hydroxypiperidin-1-yl) ethyl, $C_{1-10}$ alkylene-piperidinyl, $C_{1-10}$ alkylene-piperazinyl, 2-(4-methylpiperazin-1-yl) ethyl, 3-(4-(2-hydroxyethyl)piperazin-1-yl)propyl, 2-(4-methylpiperazin-1-yl)2-oxyethyl, 2-(4-methylpiperazin)-1-carbonyl, (4-phenylpiperazin-1-yl)methyl, 2-hydroxyethyl, 2-aminoethyl, acetyl, prop-2-yn-1-yl, dimethylaminomethyl, dimethylaminoethyl, dimethylamino-*n*-propyl, dimethylamino-isopropyl, dimethylamino-*n*-butyl, dimethylamino-isobutyl, dimethylamino-*tert*-butyl, dimethylamino-*n*-pentyl, dimethylamino-isopentyl, dimethylamino-neopentyl, cyclopropylmethyl, cyclopropylethyl, cyclopropyl-n-propyl, cyclopropylisopropyl, 1-methylpiperidin-4-yl, 1-ethyl-carbamoyl, 1-methyl-carbamoyl, (dimethylamino)-3-oxopropyl, and (4,5-dihydro-1*H*-imidazol-2-yl)methyl;

$R_6$ or $R_7$ are selected from hydrogen, $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{2-20}$ alkenyl, $C_{2-20}$ alkynyl, 3- to 10-membered cycloalkyl, $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more (e.g., 1, 2, 3, or 4) substituents independently selected from halogen, $C_{1-10}$ alkylene-halogen, hydroxy, $C_{1-10}$ alkylene-hydroxy, $R_6R_7N-(CH_2)_n-$, $(R_6)(R_7)N-(CH_2)_n-$, cyano, $C_{1-10}$ alkylene-cyano, nitro, $C_{1-10}$ alkylene-nitro, amino, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl; preferably, $R_6$ or $R_7$ is selected from $C_{1-6}$ alkyl; more preferably, $R_6$ or $R_7$ is selected from methyl, ethyl, propyl, isopropyl, *n*-butyl, isobutyl, *tert*-butyl, *n*-pentyl, isopentyl, and neopentyl;

n is selected from 1 to 20, preferably n is selected from 1 to 10, and more preferably, n is selected from 1 to 6;

W is selected from hydrogen, halogen, hydroxy, cyano, $R_6R_7N-(CH_2)_n-$, $(R_6)(R_7)N-(CH_2)_n-$, trifluoromethyl, nitro, amino, $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3-10 heterocyclyl or $C_{1-10}$ alkylene-3- to 10-membered heterocyclyl, 6- to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more (e.g., 1, 2, 3, or 4) substituents independently selected from halogen, $C_{1-10}$ alkylene-halogen, hydroxy, $C_{1-10}$ alkylene-hydroxy, $(R_6)(R_7)N-(CH_2)_n-$, cyano, $C_{1-10}$ alkylene-cyano, nitro, $C_{1-10}$ alkylene-nitro, amino, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl; preferably, W is selected from hydrogen, halogen, hydroxy, $(R_6)(R_7)N-(CH_2)_{1-10}-$, trifluoromethyl, nitro, cyano, $C_{1-6}$ alkyl, $C_{1-6}$ alkoxy, $C_{2-6}$ alkenyl, and $C_{2-6}$ alkynyl; more preferably, W is selected from hydrogen, hydroxy, chlorine, bromine, or fluorine, nitro, cyano, $(R_6)(R_7)N-(CH_2)_{1-6}-$, and trifluoromethyl;

wherein the hydrogen described above and the hydrogen contained in the groups described above are independently selected from protium, deuterium, and tritium, preferably protium and deuterium.

Further, provided is a novel benzodiazepine compound, and a pharmaceutically acceptable salt, a stereoisomer, a tautomer, a polymorph, a solvate, a metabolite, or a prodrug thereof;

wherein, X is C; $R_2$ and $R_3$ are halogen; $R_5$ is selected from hydrogen, $C_{1-20}$ alkyl, $C_{1-20}$ acyl, $C_{2-20}$ alkenyl, $C_{2-20}$ alkynyl, $C_{1-10}$ alkylene-phosphate group, $P(O)(OH)_2-C_{1-10}$ alkylene, 3- to 10-membered cycloalkyl or $C_{1-10}$ alkylene-3- to 10-membered cycloalkyl, $R_6R_7N-(CH_2)_n-$, $(R_6)(R_7)N-(CH_2)_n-$, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{1-20}$ acyl, $C_{2-20}$ alkenyl, $C_{2-20}$ alkynyl, 3- to 10-membered cycloalkyl or $C_{1-10}$ alkylene-3- to 10-membered cycloalkyl, $R_6R_7N-(CH_2)_n-$, $(R_6)(R_7)N-(CH_2)_n-$, 3- to 10-membered heterocyclyl or $C_{1-10}$ alkylene-3- to 10-membered heterocyclyl, 6- to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more (e.g., 1, 2, 3, or 4) substituents independently selected from halogen, $C_{1-10}$ alkylene-halogen, hydroxy, $C_{1-10}$ alkylene-hydroxy, $R_6R_7N-(CH_2)_n-$, $(R_6)(R_7)N-(CH_2)_n-$, cyano, $C_{1-10}$ alkylene-cyano, nitro, $C_{1-10}$ alkylene-nitro, amino, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl; preferably, $R_5$ is selected from hydrogen and $C_{1-10}$ alkyl, $C_{1-10}$ alkylene-phosphate group, 3- to 10-membered cycloalkyl or $C_{1-10}$ alkylene-3- to 10-membered cycloalkyl, $R_6R_7N-(CH_2)_n-$, $(R_6)(R_7)N-(CH_2)_n-$, and 3- to 10-membered heterocyclyl or $C_{1-10}$ alkylene-3- to 10-membered heterocyclyl; more preferably, $R_5$ is selected from hydrogen, methyl, ethyl, methylene-phosphate group, $C_{1-10}$ alkylene-morpholinyl, $C_{1-10}$ alkylene-pyrrolyl, $C_{1-10}$

alkylene-imidazolyl, 2-(4-hydroxypiperidin-1-yl) ethyl, $C_{1-10}$ alkylene-piperidinyl, $C_{1-10}$ alkylene-piperazinyl, 2-(4-methylpiperazin-1-yl) ethyl, 3-(4-(2-hydroxyethyl)piperazin-1 -yl)propyl, 2-(4-methylpiperazin-1 -yl)2-oxyethyl, 2-(4-methylpiperazin)-1 -carbonyl, (4-phenylpiperazin-1-yl)methyl, 2-hydroxyethyl, 2-aminoethyl, acetyl, prop-2-yn-1-yl, dimethylaminomethyl, dimethylaminoethyl, dimethylamino-*n*-propyl, dimethylamino-isopropyl, dimethylamino-n-butyl, dimethylamino-isobutyl, dimethylamino-*tert*-butyl, dimethylatnino-*n*-pentyl, dimethylamino-isopentyl, dimethylamino-neopentyl, cyclopropylmethyl, cyclopropylethyl, cyclopropyl-*n*-propyl, cyclopropylisopropyl, 1-methylpiperidin-4-yl, 1-ethyl-carbamoyl, 1-methyl-carbamoyl, (dimethylamino)-3-oxopropyl, and (4,5-dihydro-1*H*-imidazol-2-yl)methyl.

Further, provided is a novel benzodiazepine compound, and a pharmaceutically acceptable salt, a stereoisomer, a tautomer, a polymorph, a solvate, a metabolite, or a prodrug thereof,

wherein, X is N; $R_5$ is selected from hydrogen, $C_{1-20}$ alkyl, $C_{1-20}$ acyl, $C_{2-20}$ alkenyl, $C_{2-20}$ alkynyl, $C_{1-10}$ alkylene-phosphate group, $P(O)(OH)_2$-$C_{1-10}$ alkylene, 3- to 10-membered cycloalkyl or $C_{1-10}$ alkylene-3- to 10-membered cycloalkyl, $R_6R_7N$-$(CH_2)_n$-, $(R_6)(R_7)N$-$(CH_2)_n$-, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl; the $C_{1-20}$ alkyl, $C_{1-20}$ acyl, $C_{2-20}$ alkenyl, $C_{2-20}$ alkynyl, 3- to 10-membered cycloalkyl or $C_{1-10}$ alkylene-3- to 10-membered cycloalkyl, $R_6R_7N$-$(CH_2)_n$-, $(R_6)(R_7)N$-$(CH_2)_n$-, 3- to 10-membered heterocyclyl or $C_{1-10}$ alkylene-3- to 10-membered heterocyclyl, 6- to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more (e.g., 1, 2, 3, or 4) substituents independently selected from halogen, $C_{1-10}$ alkylene-halogen, hydroxy, $C_{1-10}$ alkylene-hydroxy, $R_6R_7N$-$(CH_2)_n$-, $(R_6)(R_7)N$-$(CH_2)_n$-, cyano, $C_{1-10}$ alkylene-cyano, nitro, $C_{1-10}$ alkylene-nitro, amino, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl; preferably, $R_5$ is selected from hydrogen and $C_{1-10}$ alkyl, $C_{1-10}$ alkylene-phosphate group, 3- to 10-membered cycloalkyl or $C_{1-10}$ alkylene-3- to 10-membered cycloalkyl, $R_6R_7N$-$(CH_2)_n$-, $(R_6)(R_7)N$-$(CH_2)_n$-, and 3- to 10-membered heterocyclyl or $C_{1-10}$ alkylene-3- to 10-membered heterocyclyl; more preferably, $R_5$ is selected from hydrogen, methyl, ethyl, methylene-phosphate group, $C_{1-10}$ alkylene-morpholinyl, $C_{1-10}$ alkylene-pyrrolyl group, $C_{1-10}$ alkylene-imidazoliyl, 2-(4-hydroxypiperidin-1-yl)ethyl, $C_{1-10}$ alkylene-pyridyl, $C_{1-10}$ alkylene-piperazinyl, 2-(4-methylpiperazin-1 -yl)ethyl, 3 -(4-(2-hydroxyethyl)piperazin-1 -yl)propyl, 2-(4-methylpiperazin-1-yl)2-oxyethyl, 2-(4-methylpiperazin)-1-carbonyl, (4-phenylpiperazin-1-yl)methyl, 2-hydroxyethyl, 2-aminoethyl, acetyl, propan-2-yn-1-yl, dimethylaminomethyl, dimethylaminoethyl, dimethylamino-*n*-propyl, dimethylamino-isopropyl, dimethylamino-*n*-butyl, dimethylamino-isobutyl, dimethylamino-*tert*-butyl, dimethylamino-n-pentyl, dimethylamino-isopentyl, dimethylamino-neopentyl, cyclopropylmethyl, cyclopropylethyl, cyclopropyl *n*-propyl, cyclopropylisopropyl, 1-methylpiperidin-4-yl, 1-ethyl-carbamoyl, 1-methyl-carbamoyl, (dimethylamino)-3-oxopropyl, and (4,5-dihydro-1*H*-imidazol-2-yl)methyl;
further, wherein the compound is selected from:

methyl (*S*)-3-(8-chloro-6-(2-fluorophenyl)-1-mercapto-4*H*-benzo[*f*][1,2,4]triazolo[4,3-*a*][1,4]diazepin-4-yl)propionate (compound 1),
methyl (*S*)-3-(8-chloro-6-(2-fluorophenyl)-1-thio-2.4-dihydro-1*H*-bcnzo[*f*][1.2,4]triazolo[4,3-*a*][1,4]diazepin-4-yl)propionate (tautomer of compound 1),
methyl (*S*)-3-(8-chloro-6-(2-chlorophenyl)-1-mercapto-4*H*-benzo[*f*][1,2,4]triazolo[4,3-*a*][1,4]diazepin-4-yl)propionate (compound 2),
methyl (*S*)-3-(8-chloro-6-(2-chlorophenyl)-1-thio-2.4-dihydro-1*H*-beno[*f*][1,2,4]triazolo[4,3-*a*][1,4]diazepin-4-yl)propionate (tautomer of compound 2),
methyl (*S*)-3-(8-bromo-6-(pyridin-2-yl)-1-mecapto-4*H*-benzo[*f*][1,2,4]triazolo[4,3-*a*][1,4]diazepin-4-yl)propionate (compound 3),
methyl (*S*)-3-(8-bromo-6-(pyridin-2-yl)-1-thio-2,4-dihydro-1*H*-benzo[*f*][1,2,4]triazolo[4,3-*a*][1,4]diazepin-4-yl)propionate (tautomer of compound 3), yl)propionate (tautomer of compound 3),
methyl (*S*)-3-(8-bromo-6-(2-fluophenyl)-1-mercapto-4*H*-benzo[*f*][1,2,4]triazolo[4,3-*a*][1,4]diazepin-4-yl)propionate (compound 4),
methyl (*S*)-3-(8-bromo-6-(2-fluorophenyl)-1-thio-2,4-dihydro-1*H*-benzo[*f*][1,2,4]triazolo[4,3-*a*][1,4]diazepin-4-yl) propionate (tautomer of compound 4),
methyl (*S*)-3-(6-phenyl-1-mercapto-4*H*-benzo[*f*][1,2,4]triazolo[4,3-*a*][1,4]dlazepin-4-yl)proplonate (compound 5), methyl (*S*)-3-(6-phenyl-1-thio-2,4-dihydro-1*H*-benzo[*f*][1,2,4]triazolo[4,3-*a*][14]diazepin-4-yl)propionate (tautomer of compound 5),

methyl (*S*)-3-(6-(4-fluorophenyl)-1-mercapto-4*H*-benzo[*f*][1,2,4]triazolo[4.3-*a*][1,4]diazepin-4-yl)propionate (compound 6),

methyl (*S*)-3-(6-(4-fluorophenyl)-1-thio-2,4-dihydro-1*H*-benzo[*f*][2,4]triazolo[4,3-*a*][1,4]diazepin-4-yl)propionate (tautomer of compound 6),

methyl (*S*)-3-(8-chloro-6-phenyl-1-mercapto-4*H*-benzo[*f*][1,2,4,]triazolo[4,3-*a*][diazepin-4)propionate (compound 7),

methyl (*S*)-3-(8-chloro-6-phenyl-1-thio-2,4-dihydro-1*H*-benzo][*f*][1,2,4]triazolo[4,3-*a*][1,2,4]diazepin-4-yl)propionate (tautomer of compound 7),

methyl (*S*)-3-(8-chloro-6-(2-fluorophenyl)-1-(methylthio)-4*H*-benzo[*f*][1,2,4]triazolo[4,3-*a*][1,4]diazepin-4-yl)propionate (compound 8),

methyl (*S*)-3-(8-bomo-6-(2-fluorophenyl)-1-methylthio)-4*H*-benzo[*f*][1,2,4]triazolo[4,3-*a*][1,4]diazepin-4-yl)propionate (compound 9),

methyl (*S*)-3-(8-nitro-6-(2-fluorophenyl)-1-(methylthio)-4*H*-benzo[*f*][1,2,4]triazolo[4,3-*a*][1,4]diazepin-4-yl)propionate (compound 10),

methyl (*S*)-3-(8-chloro-6-(2-fluorophenyl)-1-(ethylthio)-4*H*-benzo[f][1,2,4]triazolo[4,3-*a*][1,4]diazepin-4-yl)propionate (compound 11),

methyl (*S*)-3-(8-bromo-6-(2-fhiorophenyl)-1-(ethylthio)-4*H*-benzo[*f*][1,2,4]triazolo[4,3-*a*][1,4]diazepin-4-yl)propionate (compound 12),

methyl (*S*)-3-(8-nitro-6-(2-fluorophenyl)-1-(ethylthio)-4*H*-benzo[*f*][1,2,4]triazolo[4,3-*a*][1,4]diazepin-4-yl)propionate (compound 13),

methyl (*S*)-3-(8-chloro-6-(2-fluorophenyl)-1-(propylthio)-4*H*-benzo[*f*][1,2,4]triazolo[4,3-*a*][1,4]diazepin-4-yl)propionate (compound 14),

methyl (*S*)-3-(8-bromo-6-(2-fluorophenyl)-1-(propylthio)-4*H*-benzo[*f*][1,2,4]triazolo[4,3-*a*][1,4]diazepin-4-yl)propionate (compound 15),

methyl (*S*)-3-(8-nitro-6-(2-fluorophenyl)-1-(propylthio)-4*H*-benzo[*f*][1,2,4]triazolo[4,3-*a*][1,4]diazepin-4-yl)propionate (compound 16),

methyl (*S*)-3-(8-chloro-6-(2-fluorophenyl)-1-((2-hydroxyethyl)thio)-4*H*-benzo[*f*][1,2,4]triazolo[4,3-*a*][1,4] dlazepin-4-yl)propionate (compound 17),

methyl (*S*)-3-(8-bromo-6-(2-fluorophenyl)-1-((2-hydroxyethyl)thio)-4*H*-benzo[*f*][1,2,4]triazolo[4,3-*a*][1,4]diazepin-4-yl)propionate (compound 18),

methyl (*S*)-3-(8-nitro-6-(2-fluorophenyl)-1-((2-hydroxyethyl)thio)-4*H*-benzo[*f*][1,2,4]triazolo[4,3-*a*][1,4]diazepin-4-yl)propionate (compound 19),

methyl (*S*)-3-(8-chloro-6-(2-fluorophenyl)-1-((2-aminoethyl)thio)-4*H*-benzo[*f*][1,2,4]triazolo[4,3-*a*][1,4]diazepin-4-yl)propionate (compound 20),

methyl (*S*)-3-(8-bromo-6-(2-fluorophenyl)-1-((2-aminoethyl)thio)-4*H*-benzo[*f*][1,2,4]triazolo[4,3-*a*][1,4]diazepin-4-yl)propionate (compound 21),

methyl (*S*)-3-(8-nitro-6-(2-fluorophenyl)-1-((2-aminoethyl)thio)-4*H*-benzo[*f*][1,2,4]triazolo[4,3-*a*][1,4]dlazepin-4-yl)propionate (compound 22),

methyl (*S*)-3-(8-chloro-6-(2-chlorophenyl)-1-(methylthio)-4*H*-benzo[*f*][1,2,4]triazolo[4,3-*a*][1,4]diazepin-4-yl)propionate (compound 23),

methyl (*S*)-3-(8-bromo-6-(2-chlorophenyl)-1-(methylthio)-4*H*-benzo[*f*][1,2,4]triazolo[4,3-*a*][1,4]diazepin-4-yl)propionate (compound 24),

methyl (*S*)-3-(8-nitro-6-(2-chlorophenyl)-1-(methylthio)-4*H*-benzo[*f*][1,2,4]triazolo[4,3-*a*][1,4]diazepin-4-yl)propionate (compound 25),

methyl (*S*)-3-(8-chloro-6-(2-chlorophenyl)-1-(ethylthio)-4*H*-benzo[*f*][1,2,4]triazolo[4,3-*a*][1,4]diazepin-4-yl)propionate (compound 26),

methyl (*S*)-3-(8-bromo-6-(2-chlorophenyl)-1-(ethylthio)-4*H*-benzo[*f*][1,2,4,triazolo[1,4]diazepin-yl)propionate (compound 27),

methyl (*S*)-3-(8-nitro-6-(2-chlorophenyl)-1-(ethylthio)-4*H*-benzo[*f*][1,2,4]triazolo[4,3-*a*][1,4]diazepin-4-yl)propionate (compound 28),

methyl (*S*)-3-(8-chloro-6-(2-chlorophenyl)-1-(propylthio)-4*H*-benzo[*f*][1,2,4]triazolo[4,3-*a*][1,4]diazepin-4-yl)propionate (compound 29),

methyl (*S*)-3-(8-bromo-6-(2-chlorophenyl)-1-(propylthio)-4*H*-benzo[*f*][1,2,4]triazolo[4,3-*a*][1,4]diazepin-4-yl)propionate (compound 30),

methyl (*S*)-3-(8-nitro-6-(2-chlorophenyl)-1-(propylthio)-4*H*-benzo[*f*][1,2,4]triazolo[4,3-*a*][1,4]diazepin-4-yl)propionate (compound 31),

methyl (*S*)-3-(8-chloro-6-(2-chlorophenyl)-1-((2-hydroxyethyl)thio)-4*H*-benzo[*f*][1,2,4]triazolo[4,3-*a*][1,4]diazepin-4-yl)propionate (compound 32),

methyl (*S*)-3-(8-bromo-6-(2-chlorophenyl)-1-((2-hydroxyethyl)thio)-4*H*-benzo[*f*][1,2,4]triazolo[4,3-*a*][1,4]dlazepin-4-yl)propionate (compound 33),
methyl (*S*)-3-(8-nitro-6-(2-chlorophenyl)-1-((2-hydroxyethyl)thio)-4*H*-benzo[*f*][1,2,4]triazolo[4,3-*a*][1,4]diazepin-4-yl)propionate (compound 34),
methyl (*S*)-3-(8-chloro-6-(2-chlorophenyl)-1-((2-aminoethyl)thio)-4*H*-benzo[*f*][1,2,4]triazolo[4,3-*a*][1,4]diazepin-4-yl)propionate (compound 35),
methyl (*S*)-3-(8-bromo-6-(2-chlorophenyl)-1-((2-aminoethyl)thio)-4*H*-benzo[*f*][1,2,4]triazolo[4,3-*a*][1,4]diazepin-4-yl)propionate (compound 36),
methyl (*S*)-3-(8-nitro-6-(2-chlorophenyl)-1-((2-aminoethyl)thio)-4*H*-benzo[*f*][1,2,4]triazolo[4,3-*a*][1,4]diazepin4-yl)propionate (compound 37),
methyl (*S*)-3-(8-chloro-6-(2-chlorophenyl)-1-(acetylthio)-4*H*-benzo[*f*][1,2,4]triazolo[4,3-*a*][1,4] diazepin-4-yl)propionate (compound 38),
methyl (*S*)-3-(8-bromo-6-(2-chlorophenyl)-1-(acetylthio)-4*H*-benzo[*f*][1,2,4]triazolo[4,3-*a*][1,4]diazepin-4-yl)propionate (compound 39),
methyl (*S*)-3-(8-nitro-6-(2-chlorophenyl)-1-(acetylthio)-4*H*-benzo[*f*][1,2,4]triazolo[4,3-*a*][1,4]diazepin-4-yl)propionate (compound 40),
ethyl (*S*)-3-(8-chloro-6-(2-chlorophenyl)-1-mercapto-4*H*-benzo[*f*][1,2,4]triazolo[4,3-*a*][1,4]diazepin-4-yl)propionate (compound 41),
ethyl (*S*)-3-(8-chloro-6-(2-chlorophenyl)-1-thio-2,4-dihydro-1*H*-benzo[*f*][1,2,4]triazolo[4,3-*a*][1,4]diazepin-4-yl)propionate (tautomer of compound 41),
ethyl (*S*)-3-(8-chloro-6-(2-chlorophenyl)-1-(methylthio)-4*H*-benzo[*f*][1,2,4]triazolo[4,3-*a*][1,4]diazepin-4-yl)propionate (compound 42),
ethyl (*S*)-3-(8-chloro-6-(2-chlorophenyl)-1-(methylthio)-4*H*-benzo[*f*][1,2,4]triazolo[4,3-*a*][1,4]diazepin-4-yl)propionate (compound 43),
ethyl (*S*)-3-(8-nitro-6-(2-chlorophenyl)-1-(methylthio)-4*H*-benzo[*f*][1,2,4]triazolo[4,3-*a*][1,4]diazepin-4-yl)propionate (compound 44),
methyl (*S*)-3-(8-chloro-6-(2-fluorophenyl)-1-((cyclopropylmethyl)thio)-4*H*-benzo[*f*][1,2,4]triazolo[4,3-*a*][1,4]diazepin-4-yl)propionate (compound 45),
methyl (*S*)-3-(8-bromo-6-(2-fluorophenyl)-1-((cyclopropylmethyl)thio)-4*H*-benzo[*f*][1,2,4]triazolo[4,3-*a*][1,4]diazepin-4-yl)propionate (compound 46),
methyl (*S*)-3-(8-nitro-6-(2-fluorophenyl)-1-((cyclopropylmethyl)thio)-4*H*-benzo[*f*][1,2,4]triazolo[4,3-*a*][1,4]diazepin-4-yl)propionate (compound 47),
methyl (*S*)-3-(8-chloro-6-(2-fluorophenyl)-1-(propargylthio)-4*H*-benzo[*f*][1,2,4]triazolo[4,3-*a*][1,4]diazepin-4-yl)propionate (compound 48),
methyl (*S*)-3-(8-bromo-6-(2-fluorphenyl)-1-(propargylthio)-4*H*-benzo[*f*][1,2,4]triazolo[4,3-*a*][1,4]diazepin-4-yl)propionate (compound 49),
methyl (*S*)-3-(8-nitro-6-(2-fluorophenyl)-1-(propargylthio)-4*H*-benzo[*f*][1,2,4]triazolo[4,3-*a*[1,4]diazepin-4-yl)propionate (compound 50),
methyl (*S*)-3-(8-chloro-6-(2-fluorophenyl)-1-((2-morpholinoethyl)thio)-4*H*-benzo[*f*][1,2,4]triazolo[4,3-a][1,4]diazepin-4-yl)propionate (compound 51),
methyl (*S*)-3-(8-bromo-6-(2-fluorophenyl)-1-((2-morpholinoethyl)thio)-4*H*-benzo[*f*][1,2,4]triazolo[4,3-a][1,4]dlazepin-4-yl)propionate (compound 52),
methyl (*S*)-3-(8-nitro-6-(2-fluorophenyl)-1-((2-morpholinoethyl)thio)-4*H*-benzo[*f*][1,2,4]triazolo[4,3-*a*][1,4]diazepin-4-yl)propionate (compound 53),
methyl (*S*)-3-(8-chloro-6-(2-fluorophenyl)-1-((2-(diethylatnino)ethyl)thio)-4*H*-benzo[*f*][1,2,4]triazolo[4,3-*a*][1,4] diazepin-4-yl)propionate (compound 54),
methyl (*S*)-3-(8-bromo-6-(2-fluorophenyl)-1-((2-(diethylatnino)ethyl)thio)-4*H*-benzo[*f*][1,2,4]triazolo[4,3-*a*][1,4] diazepin-4-yl)propionate (compound 55),
methyl (*S*)-3-(8-nitro-6-(2-fluorophenyl)-1-((2-(diethylamino)ethyl)thio)-4*H*-benzo[*f*][1,2,4]triazolo[4,3-*a*][1,4]diazepin-4-yl)propionate (compound 56),
methyl (*S*)-3-(8-chloro-6-(2-fluorophenyl)-1-((3-(dimethylamino)propyl)thio)-4*H*-benzo[*f*][1,2,4]triazolo[4,3-*a*][1,4]diazepin-4-yl)propionate (compound 57),
methyl (*S*)-3-(8-bromo-6-(2-fluorophenyl)-1-((3-(dimethylamino)propyl)thio)-4*H*-benzo[*f*][1,2,4]triazolo[4,3-*a*][1,4]diazepin-4-yl)propionate (compound 58),
methyl (*S*)-3-(8-nitro-6-(2-fluorophenyl)-1-((3-(dimethylamino)propyl)thio)-4*H*-benzo[*f*][1,2,4]triazolo[4,3-*a*][1,4] diazepin-4-yl)propionate (compound 59),
methyl (*S*)-3-(8-chloro-6-(2-fluorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 60),

methyl (*S*)-3-(8-bromo-6-(2-fluorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 61),
methyl (*S*)-3-(8-nitro-6-(2-fluorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 62),
methyl (*S*)-3-(8-chloro-6-(2-fluorophenyl)-1-((1-methylpiperidin-4-yl)thio)-4*H*-benzo[*f*][1,2,4]triazolo[4,3-*a*][1,4]diazepin-4-yl)propionate (compound 63),
methyl (*S*)-3-(8-bromo-6-(2-fluorophenyl)-1-((1-methylpiperidin-4-yl)thio)-4*H*-benzo[*f*][1,2,4]triazolo[4,3-*a*][1,4]diazepin-4-yl)propionate (compound 64),
methyl (*S*)-3-(8-nitro-6-(2-fluorophenyl)-1-((1-methylpiperidin-4-yl)thio)-4*H*-benzo[*f*][1,2,4]triazolo[4,3-*a*][1,4]diazepin-4-yl)propionate (compound 65),
methyl (*S*)-3-(8-chloro-6-(2-fluorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 66),
methyl (*S*)-3 -(8-bromo-6-(2-fluorophenyl)-1 -((3 -(4- (2-hydroxyethyl)piperazin-1-yl)propyl)thio)-4*H*-benzo[*f*][1,2,4]triazolo[4,3-*a*][1,4]diazepin-4-yl)propionate (compound 67),
methyl (*S*)-3-(8-nitro-6-(2-fluorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 68),
methyl (*S*)-3-(8-chloro-6-(2-fluorophenyl)-1-((morpholinomethyl)thio)-4*H*-benzo[*f*][1,2,4]triazolo[4,3-*a*][1,4]diazepin-4-yl)propionate (compound 69),
methyl (*S*)-3-(8-bromo-6-(2-fluorophenyl)-1-((morpholinomethyl)thio)-4*H*-benzo[*f*][1,2,4]triazolo[4,3-*a*][1,4]diazepin-4-yl)propionate (compound 70),
methyl (*S*)-3-(8-nitro-6-(2-fluorophenyl)-1-((morpholinomethyl)thio)-4*H*-benzo[*f*][1,2,4]triazolo[4,3-*a*][1,4]diazepin-4-yl)propionate (compound 71),
methyl (*S*)-3-(8-chloro-6-(2-fluorophenyl)-1-(((4-phenylpiperazin-1-yl)methyl)thio)-4*H*-benzo[*f*][1,2,4]triazolo[4,3 - *a*][1,4]diazepin-4-yl)propionate (compound 72),
methyl (*S*)-3-(8-bromo-6-(2-fluorophenyl)-1-(((4-phenylpiperazin-1-yl)methyl)thio)-4*H*-benzo[*f*][1,2,4]triazolo[4,3 - *a*][1,4]diazepin-4-yl)propionate (compound 73),
methyl (*S*)-3-(8-nitro-6-(2-fluorophenyl)-1-(((4-phenylpiperazin-1-yl)methyl)thio)-4*H*-benzo[*f*][1,2,4]triazolo[4,3 - *a*][1,4]diazepin-4-yl)propionate (compound 74),
methyl (*S*)-3-(8-chloro-6-(2-chlorophenyl)-1-((cyclopropylmethyl)thio)-4*H*-benzo[*f*][1,2,4]triazolo[4,3-*a*][1,4]diazepin-4-yl)propionate (compound 75),
methyl (*S*)-3-(8-bromo-6-(2-chlorophenyl)-1-((cyclopropylmethyl)thio)-4*H*-benzo[*f*][1,2,4]triazolo[4,3-*a*][1,4]diazepin-4-yl)propionate (compound 76),
methyl (*S*)-3-(8-nitro-6-(2-chlorophenyl)-1-((cyclopropylmethyl)thio)-4*H*-benzo[*f*][1,2,4]triazolo[4,3-*a*][1,4]diazepin-4-yl)propionate (compound 77),
methyl (*S*)-3-(8-chloro-6-(2-chlorophenyl)-1-(propargylthio)-4*H*-benzo[*f*][1,2,4]triazolo[4,3-*a*][1,4]diazepin-4-yl)propionate (compound 78),
methyl (*S*)-3-(8-bromo-6-(2-chlorophenyl)-1-(propargylthio)-4*H*-benzo[*f*][1,2,4]triazolo[4,3-*a*][1,4]diazepin-4-yl)propionate (compound 79),
methyl (*S*)-3-(8-nitro-6-(2-chlorophenyl)-1-(propargylthio)-4*H*-benzo[*f*][1,2,4]triazolo[4,3-*a*][1,4]diazepin-4-yl)propionate (compound 80),
methyl (*S*)-3-(8-chloro-6-(2-chlorophenyl)-1-((2-morpholinoethyl)thio)-4*H*-benzo[*f*][1,2,4]triazolo[4,3-*a*][1,4]diazepin-4-yl)propionate (compound 81),
methyl (*S*)-3-(8-bromo-6-(2-chlorophenyl)-1-((2-morpholinoethyl)thio)-4*H*-benzo[*f*][1,2,4]triazolo[4,3-*a*][1,4]diazepin-4-yl)propionate (compound 82),
methyl (*S*)-3-(8-nitro-6-(2-chlorophenyl)-1-((2-morpholinoethyl)thio)-4*H*-benzo[*f*][1,2,4]triazolo[4,3-*a*][1,4]diazepin-4-yl)propionate (compound 83),
methyl *a*][1,4]diazepin-4-yl)propionate (compound 84),
methyl (*S*)-3-(8-bromo-6-(2-chlorophenyl)-1-((2-(diethylamino)ethyl)thio)-4*H*-benzo[*f*][1,2,4]triazolo[4,3-*a*][1,4]diazepin-4-yl)propionate (compound 85),
methyl (*S*)-3-(8-nitro-6-(2-chlorophenyl)-1-((2-(diethylamino)ethyl)thio)-4*H*-benzo[*f*][1,2,4]triazolo[4,3-*a*][1,4]diazepin-4-yl)propionate (compound 86),
methyl *a*][1,4]diazepin-4-yl)propionate (compound 87),
methyl (*S*)-3-(8-bromo-6-(2-chlorophenyl)-1-((3-(dimethylamino)propyl)thio)-4*H*-benzo[*f*][1,2,4]triazolo[4,3-*a*][1,4]diazepin-4-yl)propionate (compound 88),
methyl (*S*)-3-(8-nitro-6-(2-chlorophenyl)-1-((3-(dimethylamino)propy)propyl)thio)-4*H*-benzo[*f*][1,2,4]triazolo[4,3- *a*][1,4]diazepin-4-yl)propionate (compound 89),
methyl (*S*)-3-(8-chloro-6-(2-chlorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 90),

methyl (*S*)-3-(8-bromo-6-(2-chlorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 91),
methyl (*S*)-3-(8-nitro-6-(2-chlorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 92),
methyl (*S*)-3-(8-chloro-6-(2-chlorophenyl)-1-((1-methylpiperidin-4-yl)thio)-4*H*-benzo[*f*][1,2,4]triazolo[4,3-*a*][1,4]diazepin-4-yl)propionate (compound 93),
methyl (*S*)-3-(8-bromo-6-(2-chlorophenyl)-1-((1-methylpiperidin-4-yl)thio)-4*H*-benzo[*f*][1,2,4]triazolo[4,3-*a*][1,4]diazepin-4-yl)propionate (compound 94),
methyl (*S*)-3-(8-nitro-6-(2-chlorophenyl)-1-((1-methylpiperidin-4-yl)thio)-4*H*-benzo[*f*][1,2,4]triazolo[4,3-*a*][1,4]diazepin-4-yl)propionate (compound 95),
methyl (*S*)-3-(8-chloro-6-(2-chlorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 96),
methyl (*S*)-3-(S-bromo-6-(2-chlorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 97),
methyl (*S*)-3-(-8-nitro-6-(2-chlorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 98),
methyl (*S*)-3-(8-chloro-6-(2-chlorophenyl)-1-((morpholinomethyl)thio)-4*H*-benzo[*f*][1,2,4]triazolo[4,3-*a*][1,4]dlazepin-4-yl)propionate (compound 99),
methyl (*S*)-3-(S-bromo-6-(2-chlorophenyl)-1-((morpholinomethyl)thio)-4*H*-benzo[*f*][1,2,4]triazolo[4,3-*a*][1,4]diazepin-4-yl)propionate (compound 100),
methyl (*S*)-3-(8-nitro-6-(2-chlorophenyl)-1-((morpholinomethyl)thio)-4*H*-benzo[*f*][1,2,4]triazolo[4,3-*a*][1,4]diazepin-4-yl)propionate (compound 101),
methyl (compound 102),
methyl (*S*)-3-(8-bromo-1-(methylthio)-6-phenyl-4*H*-benzo[*f*][1,2,4]triazolo[4,3-*a*][1,4]diazepin-4-yl)propionate (compound 103),
methyl (*S*)-3-(8-nitro-1-(methylthio)-6-phenyl-4*H*-benzo[*f*][1,2,4]triazolo[4,3-*a*][1,4]diazepin-4-yl)propionate (compound 104),
methyl (*S*)-3-(8-chloro-1-((2-morpholinoethyl)thio)-6-phenyl-4*H*-benzo[*f*][1,2,4]triazolo[4,3-*a*][1,4]diazepin-4-yl)propionate (compound 105),
methyl (*S*)-3-(S-bromo-1-((2-morpholinoethyl)thio)-6-phenyl-4*H*-benzo[*f*][1,2,4]triazolo[4,3-*a*][1,4]diazepin-4-yl)propionate (compound 106),
methyl (*S*)-3-(8-nitro-1-((2-morpholinoethyl)thio)-6-phenyl-4*H*-benzo[*f*][1,2,4]triazolo[4,3-*a*][1,4]dlazepin-4-yl)propionate (compound 107),
methyl (*S*)-3-(8-chloro-1-((3-(dimethylamino)propyl)thio)-6-phenyl-4*H*-benzo[*f*][1,2,4]triazolo[4,3-*a*][1,4]dlazepin-4-yl)propionate (compound 108),
methyl (*S*)-3-(S-bromo-1-((3-(dimethylamino)propyl)thio)-6-phenyl-4*H*-benzo[*f*][1,2,4]triazolo[4,3-*a*][1,4]diazepin-4-yl)propionate (compound 109),
methyl (*S*)-3-(8-nitro-1-((3-(dimethylamino)propyl)thio)-6-phenyl-4*H*-benzo[*f*][1,2,4]triazolo[4,3-*a*][1,4]diazepin-4-yl)propionate (compound 110),
methyl (*S*)-3-(8-chloro-6-(2-chlorophenyl)-1-(((phosphonooxy)methyl)thio)-4*H*-benzo[*f*][1,2,4]triazolo[4,3-*a*][1,4]diazepin-4-yl)propionate (compound 111),
methyl (*S*)-3-(8-bromo-6-(2-chlorophenyl)-1-(((phosphonooxy)methyl)thio)-4*H*-benzo[*f*][1,2,4]triazolo[4,3-*a*][1,4]diazepin-4-yl)propionate (compound 112),
methyl (*S*)-3-(8-nitro-6-(2-chlorophenyl)-1-(((phosphonooxy)methyl)thio)-4*H*-benzo[*f*][1,2,4]triazolo[4,3-*a*][1,4]diazepin-4-yl)propionate (compound 113),
methyl (*S*)-3-(8-bromo-6-(pyridin-2-yl)-1-methylthio-4*H*-benzo[*f*][1,2,4]triazolo[4,3-*a*][1,4]diazepin-4-yl)propionate (compound 114),
methyl (*S*)-3-(8-bromo-6-(pyridin-2-yl)-1-ethylthio-4*H*-benzo[*f*][1,2,4]triazolo[4,3-*a*][1,4]diazepin-4-yl)propionate (compound 115),
methyl (*S*)-3-(8-bromo-6-(pyridin-2-yl)-1-((cyclopropylmethyl)thio)-4*H*-benzo[*f*][1,2,4]triazolo[4,3-*a*][1,4]diazepin-4-yl)propionate (compound 116),
methyl (*S*)-3-(8-bromo-6-(pyridin-2-yl)-1-((2-morpholinoethyl)thio)-4*H*-benzo[*f*][1,2,4]triazolo[4,3-*a*][1,4]diazepin-4-yl)propionate (compound 117),
methyl (*S*)-3-(8-bromo-6-(pyridin-2-yl)-1-(3-((dimethylatnino)propyl)thio)-4*H*-benzo[*f*][1,2,4]triazolo[4,3-*a*][1,4]diazepin-4-yl)propionate (compound 118),
methyl (*S*)-3-(8-chloro-6-(2-chlorophenyl)-1-(2-((pyrrolidin-1-yl)ethyl)thio[*f*][1,2,4]triazolo[4,3-*a*][1,4]diazepin-4-yl)propionate (compound 119),
methyl (*S*)-3-(8-bromo-6-(2-fluorophenyl)-1-(2-((pyrrolidin-1-yl)ethyl)thio)-4*H*-benzo[*f*][1,2,4]triazolo[4,3-*a*][1,4]

diazepin-4-yl)propionate (compound 120),
methyl (*S*)-3-(8-bromo-6-(2-chlorophenyl)-1-((2-(pyrrolidin-1-yl)ethyl)thio)-4*H*-benzo[*f*][1,2,4]triazolo[4,3-*a*][1,4]diazepin-4-yl)propionate (compound 121),
methyl (*S*)-3-(8-chloro-6-(2-chlorophenyl)-1-(2-((piperidin-1-yl)ethyl)thio)-4*H*-benzo[*f*][1,2,4]triazolo[4,3-*a*][1,4]diazepin-4-yl)propionate (compound 122),
methyl (*S*)-3-(8-bromo-6-(2-fluorophenyl)-1-(2-((piperidin-2-yl)ethyl)thio)-4*H*-benzo[f][1,2,4]triazolo[4,3-*a*][1,4]diazepin-4-yl)propionate (compound 123),
methyl (*S*)-3-(8-chloro-6-(2-chlorophenyl)-1-(2-((pyridin-4-yl)methyl)thio)-4*H*-benzo[*f*][1,2,4]triazolo[4,3-*a*][1,4]diazepin-4-yl)propionate (compound 124),
methyl (*S*)-3-(8-bromo-6-(2-fluorophenyl)-1-(2-((pyridin-4-yl)methyl)thio)-4*H*-benzo[*f*][1,2,4]triazolo[4,3-*a*][1,4]diazepin-4-yl)propionate (compound 125),
methyl (*S*)-3-(8-bromo-6-(pyridin-2-yl)-1-(((pyridin-4-yl)methyl)thio)-4*H*-benzo[*f*][1,2,4]triazolo[4,3-*a*][1,4]diazepin-4-yl)propionate (compound 126),
methyl (*S*)-3-(8-chloro-6-(2-chlorophenyl)-1-(2-((4,4-difluoropiperidin-1-yl)ethyl)thio)-4*H*-benzo[*f*][1,2,4]triazolo[4,3-*a*][1,4]diazepin-4-yl)propionate (compound 127),
methyl (*S*)-3-(8-bromo-6-(2-fluorophenyl)-1-(2-((4,4-difluoropiperidin-1-yl)ethyl)thio))-4*H*-benzo[*f*][1,2,4]triazolo[4,3-*a*][1,4]diazepin-4-yl)propionate (compound 128),
methyl (*S*)-3-(8-chloro-6-(2-chlorophenyl)-1-((2-(4-methylpiperazin-1-yl)-2-oxyethyl)thio)-4*H*-benzo[*f*][1,2,4]triazolo[4,3-*a*][1,4]diazepin-4-yl)propionate (compound 129),
methyl (*S*)-3-(8-chloro-6-(2-chlorophenyl)-1-(2-(4-methylpiperazine-1-carbonyl)thio)-4*H*-benzo[*f*][1,2,4]triazolo[4,3-*a*][1,4]diazepin-4-yl)propionate (compound 130),
methyl (*S*)-3-(8-bromo-6-(2-fluorophenyl)-1-(2-(4-methylpiperazine-1-carbonyl)thio))-4*H*-benzo[*f*][1,2,4]triazolo[4,3-*a*][1,4]diazepin-4-yl)propionate (compound 131),
methyl (*S*)-3-(8-chloro-6-(2-chlorophenyl)-1-(3-((diethylamino)propyl)thio)-4*H*-benzo[*f*][1,2,4]triazolo[4,3-*a*][1,4]diazepin-4-yl)propionate (compound 132),
methyl *a*][1,4]diazepin-4-yl)propionate (compound 133),
methyl (*S*)-3-(8-bromo-6-(2-fluorophenyl)-1-(2-((dimethylamino)ethyl)thio)-4*H*-benzo[*f*][1,2,4]triazolo[4,3-*a*][1,4]diazepin-4-yl)propionate (compound 134),
methyl (*S*)-3-(8-chloro-6-(2-chlorophenyl)-1-(2-((4-hydroxypiperidin-1-yl)ethyl)thio)-4*H*-benzo[*f*][1,2,4]triazolo[4,3-*a*][1,4]diazepin-4-yl)propionate (compound 135),
methyl (*S*)-3-(8-chloro-6-(2-chlorophenyl)-1-(2-((4-methylpiperidin-2-carbonyl)ethyl)thio)-4*H*-benzo[*f*][1,2,4]triazolo[4,3-*a*][1,4]diazepin-4-yl)propionate (compound 136),
(*S*)-3-(8-chloro-6-(2-chlorophenyl)-1-thio-2,4-dihydro-1*H*-benzo[*f*][1,2,4]triazolo[4,3-*a*][1,4]diazepin-4-yl)propionic acid-2-morpholinoethyl ester (compound 137),
methyl (*S*)-3-(8-chloro-6-(2-chlorophenyl)-1-(2-((1*H*-imidazol-1-yl)ethyl)thio)-4*H*-benzo[*f*][1,2,4]triazolo[4,3-*a*][1,4]diazepin-4-yl)propionate (compound 138),
methyl (*S*)-3-(8-bromo-6-(2-fluorophenyl)-1-(2-((1*H*-imidazol-1-yl)ethyl)thio)-4*H*-benzo[*f*][1,2,4]triazolo[4,3-*a*][1,4]diazepin-4-yl)propionate (compound 139),
methyl (*S*)-3-(8-chloro-6-(2-chlorophenyl)-1-(2-((1*H*-pyrrol-1-yl)ethyl)thio[*f*][1,2,4]triazolo[4,3-*a*][1,4]diazepin-4-yl)propionate (compound 140),
methyl (*S*)-3-(8-chloro-6-(2-chlorophenyl)-1-((1-methyl-1-1*H*-imidazol-4-yl)thio)-4*H*-benzo[*f*][1,2,4]triazolo[4,3-*a*][1,4]diazepin-4-yl)propionate (compound 141),
methyl (*S*)-3-(8-chloro-6-(2-chlorophenyl)-1-((ethyl(methyl)carbamoyl)thio)-4*H*-benzo[*f*][1,2,4]triazolo[4,3-*a*][1,4]diazepin-4-yl)propionate (compound 142),
methyl (*S*)-3-(8-chloro-6-(2-chlorophenyl)-1-(3-((dimethylamino)-3-oxopropyl)thio)-4*H*-benzo[*f*][1,2,4]triazolo[4,3-*a*][1,4]diazepin-4-yl)propionate (compound 143), and
methyl (*S*)-3-(8-chloro-6-(2-chlorophenyl)-1-(((4,5-dihydro-1*H*-imidazol-2-yl)methyl)thio)-4*H*-benzo[*f*][1,2,4]triazolo[4,3-*a*][1,4]diazepin-4-yl)propionate (compound 144);
some of the compounds have the following structures:

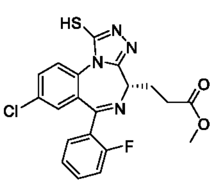
compound 1

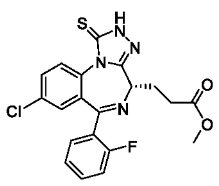
tautomer of compound 1

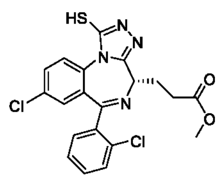
compound 2

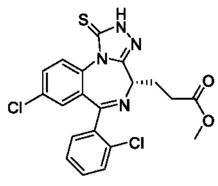
tautomer of compound 2

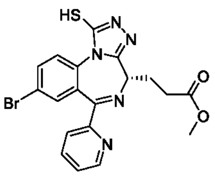

compound 3

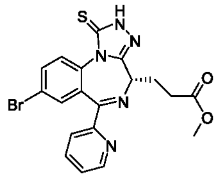

tautomer of compound 3

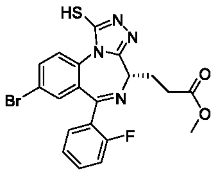

compound 4

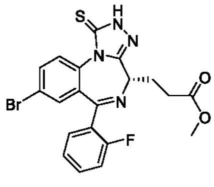

tautomer of compound 4

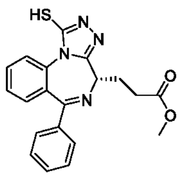

compound 5

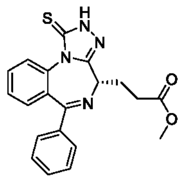

tautomer of compound 5

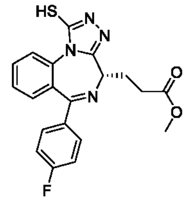

compound 6

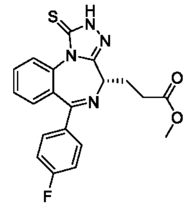

tautomer of compound 6

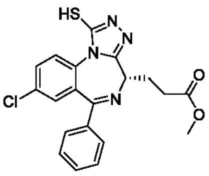

compound 7

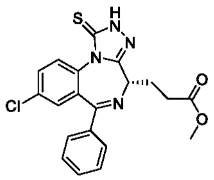

tautomer of compound 7

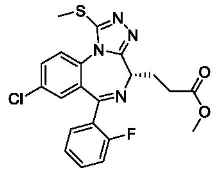

compound 8

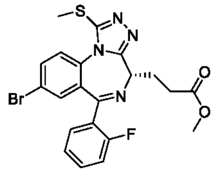

compound 9

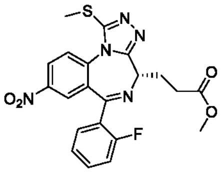

compound 10

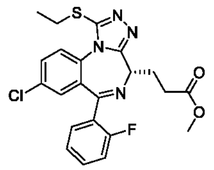

compound 11

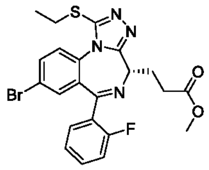

compound 12

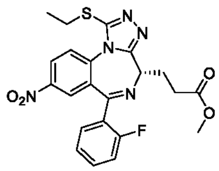

compound 13

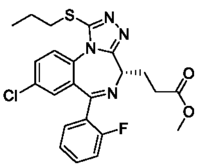

compound 14

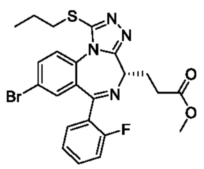

compound 15

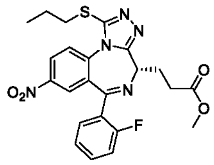

compound 16

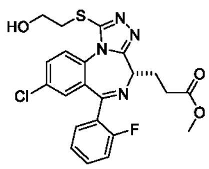

compound 17

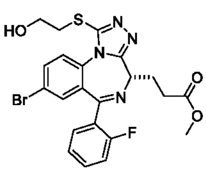

compound 18

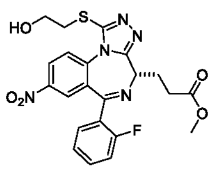

compound 19

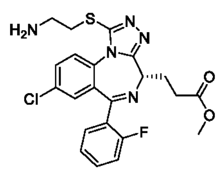

compound 20

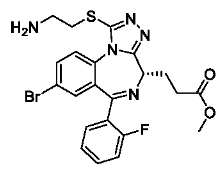

compound 21

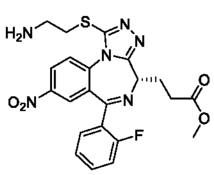

compound 22

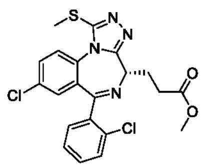

compound 23

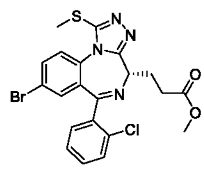

compound 24

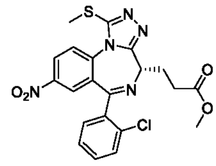

compound 25

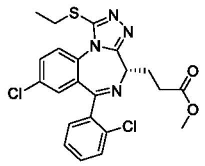
compound 26

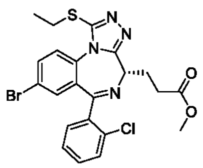
compound 27

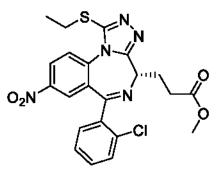
compound 28

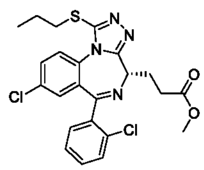
compound 29

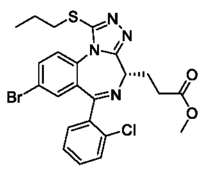
compound 30

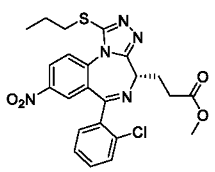
compound 31

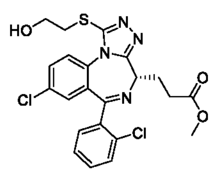
compound 32

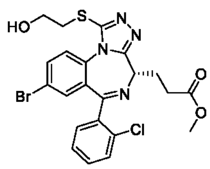
compound 33

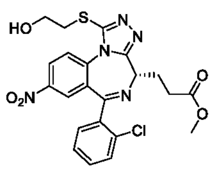
compound 34

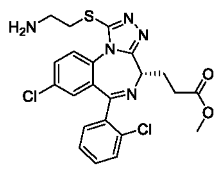
compound 35

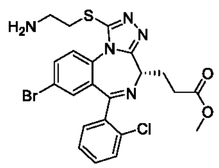
compound 36

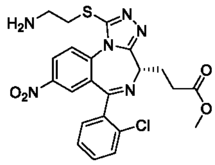
compound 37

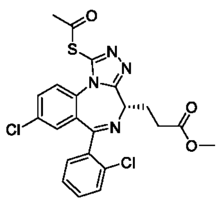
compound 38

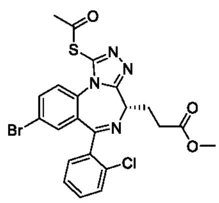
compound 39

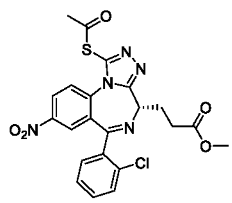
compound 40

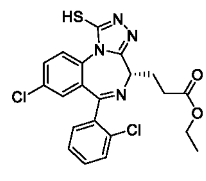
compound 41

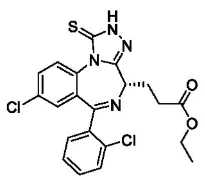
tautomer of compound 41

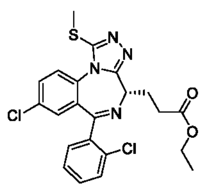
compound 42

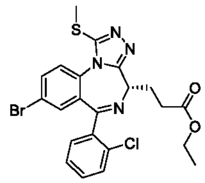
compound 43

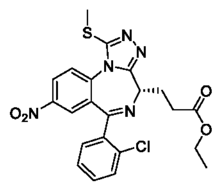
compound 44

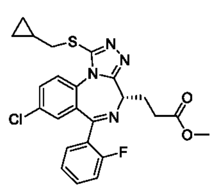
compound 45

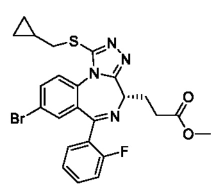
compound 46

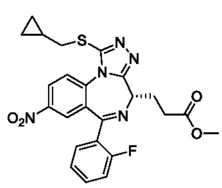
compound 47

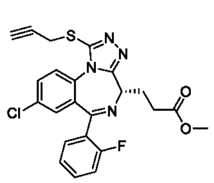
compound 48

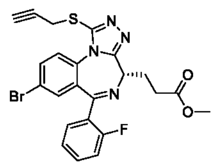
compound 49

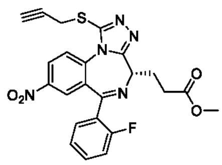
compound 50

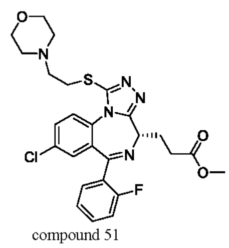
compound 51

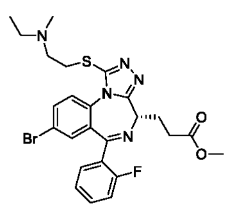
compound 52

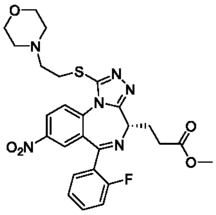
compound 53

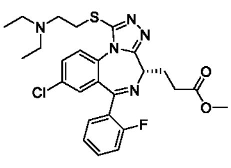
compound 54

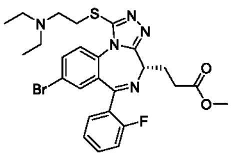
compound 55

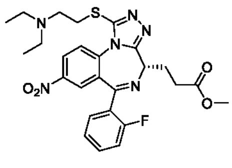
compound 56

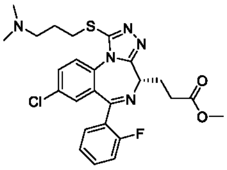
compound 57

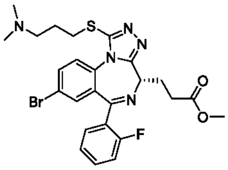
compound 58

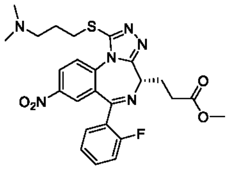
compound 59

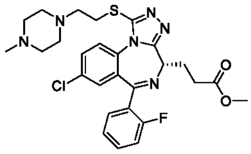
compound 60

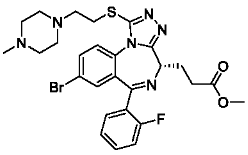
compound 61

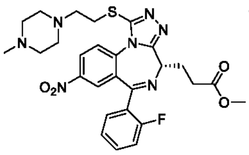
compound 62

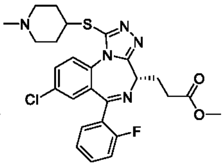
compound 63

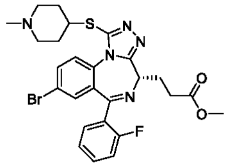
compound 64

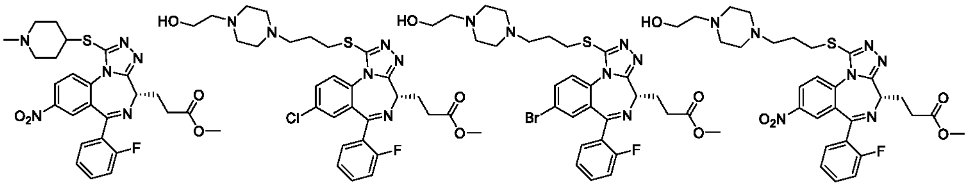
compound 65

compound 66

compound 67

compound 68

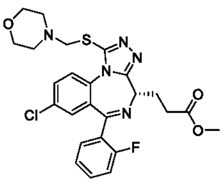
compound 69

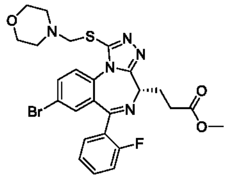
compound 70

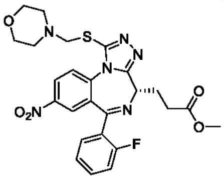
compound 71

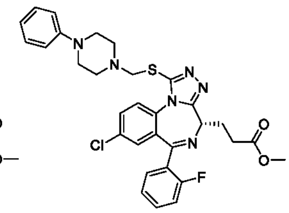
compound 72

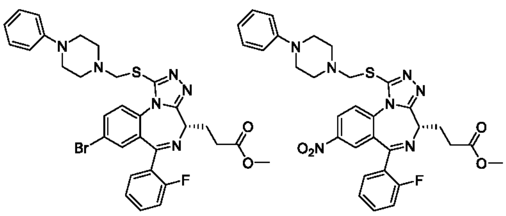
compound 73

compound 74

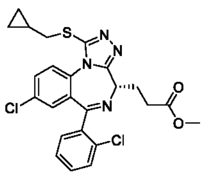
compound 75

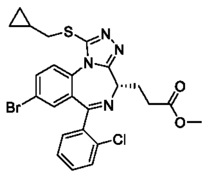
compound 76

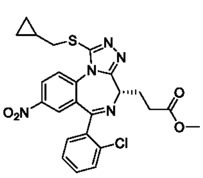
compound 77

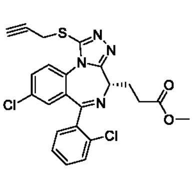
compound 78

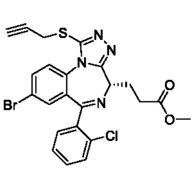
compound 79

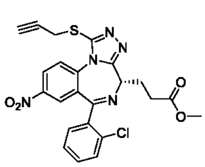
compound 80

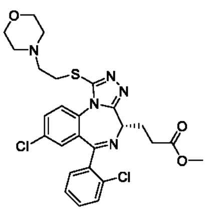
compound 81

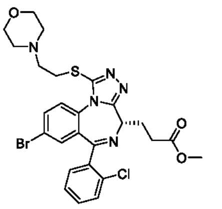
compound 82

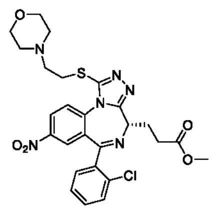
compound 83

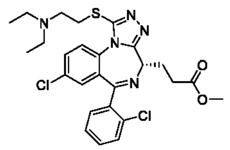
compound 84

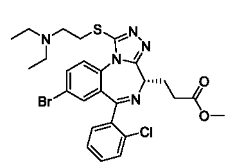
compound 85

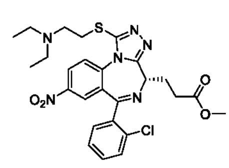
compound 86

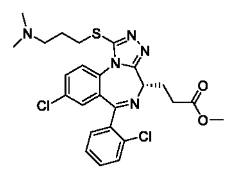
compound 87

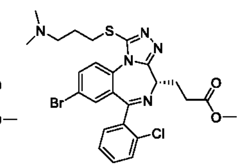
compound 88

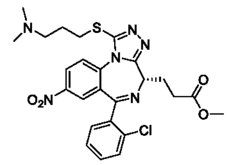
compound 89

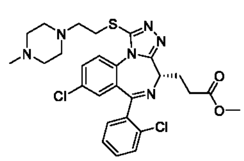
compound 90

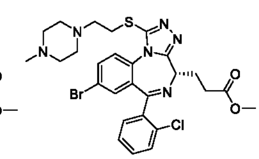
compound 91

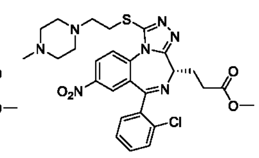
compound 92

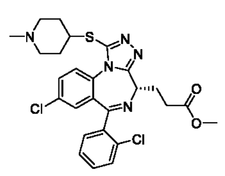
compound 93

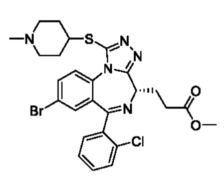
compound 94

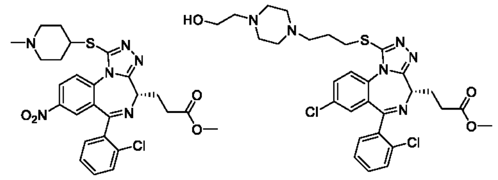
compound 95

compound 96

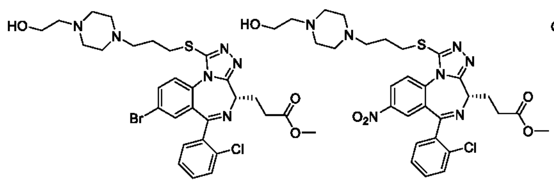
compound 97

compound 98

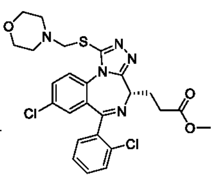
compound 99

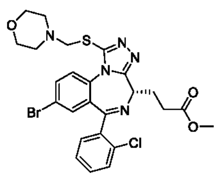
compound 100

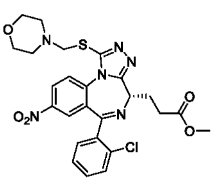
compound 101

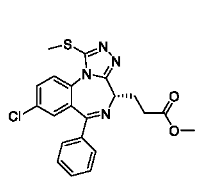
compound 102

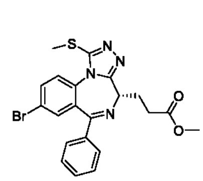
compound 103

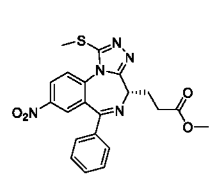
compound 104

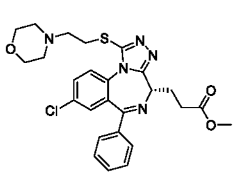
compound 105

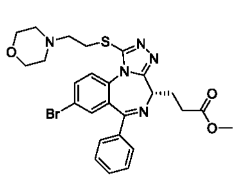
compound 106

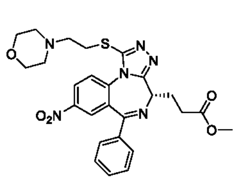
compound 107

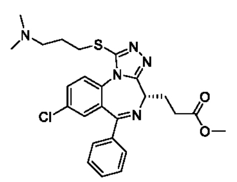
compound 108

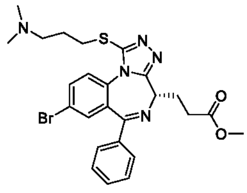
compound 109

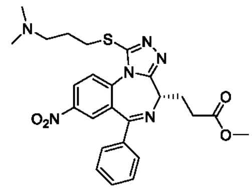
compound 110

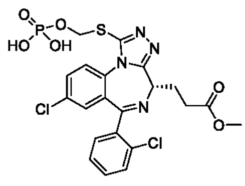
compound 111

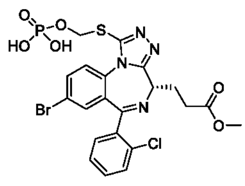
compound 112

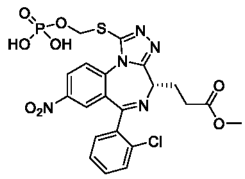
compound 113

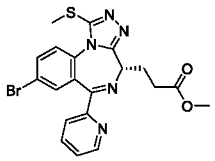
compound 114

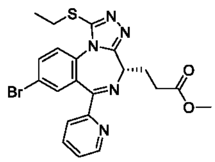
compound 115

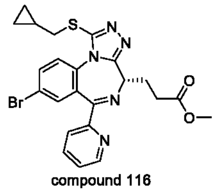
compound 116

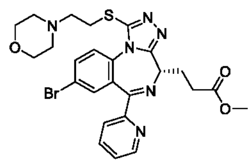
compound 117

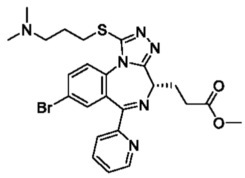
compound 118

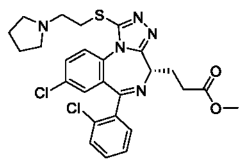
compound 119

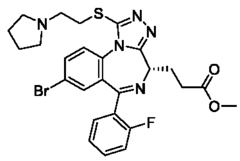
compound 120

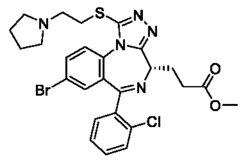
compound 121

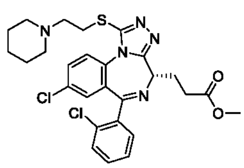
compound 122

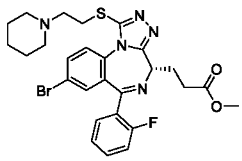
compound 123

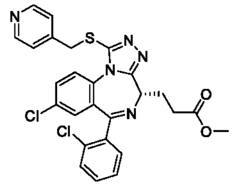
compound 124

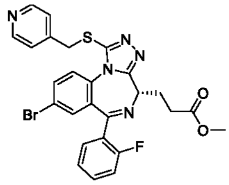
compound 125

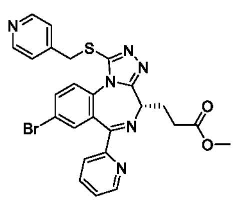
compound 126

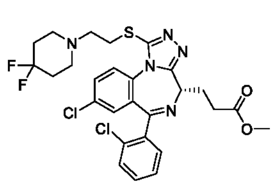
compound 127

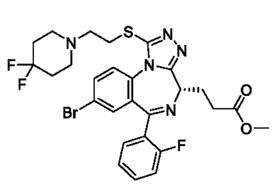
compound 128

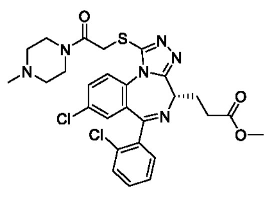
compound 129

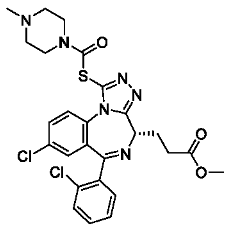

compound 130

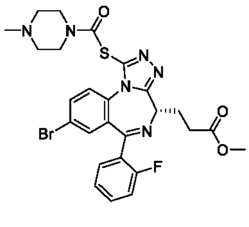

compound 131

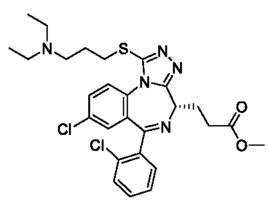

compound 132

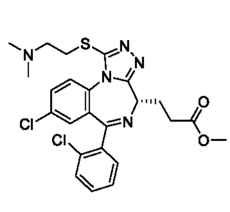

compound 133

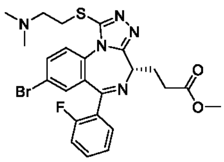

compound 134

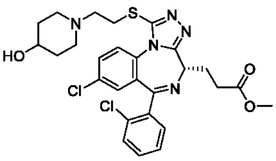

compound 135

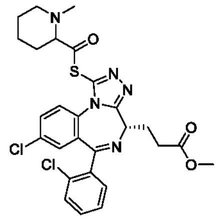

compound 136

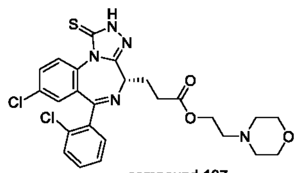

compound 137

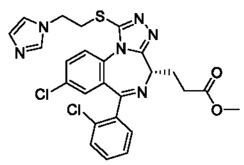

compound 138

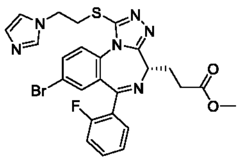

compound 139

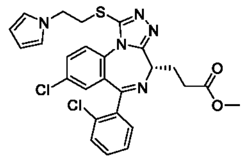

compound 140

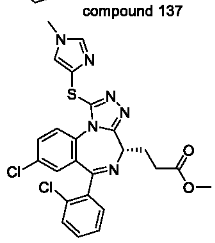

compound 141

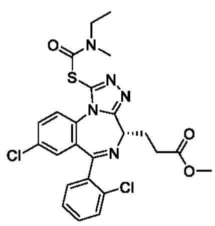

compound 142

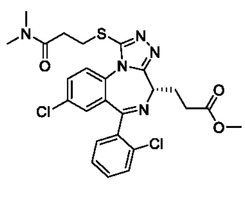

compound 143

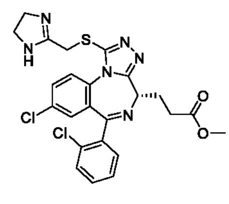

compound 144

Further, the compounds of the present disclosure may be present in the pharmaceutical composition in a content or an amount of about 10 mg to about 3000 mg, suitably 25-3000 mg, preferably 60-2700 mg, more preferably 60-1500 mg, particularly preferably 60-1000 mg, e.g., 60 mg, 80 mg, 100 mg, 150 mg, 200 mg, 300 mg, or 500 mg.

The pharmaceutically acceptable carrier is selected from water, oil, and other injectable solvents.

The pharmaceutical additive is selected from starch, glucose, lactose, brown sugar, gelatin, maltose, chalk, silica gel, sodium stearate, glyceryl monostearate, talc, sodium oxide, skimmed milk powder, glycerol, propylene glycol, ethanol, lecithin, glycine, mannitol, Tween 80, polysorbate, sodium carboxymethylcellulose, gelatin, pectin, albumin, trehalose, and dextran.

Further, the pharmaceutically acceptable salt thereof is selected from acetate, adipate, aspartate, benzoate, benzenesulfonate, bicarbonate/carbonate, bisulfate/sulfate, borate, camphorsulfonate, citrate, cyclamate, edisylate, ethanesulfonate, formate, fumarate, glucoheptonate, gluconate, glucuronate, hexafluorophosphate, hydrochloride/oxide, hydrobromide/bromide, hydroiodide/iodide, isethionate, lactate, malate, maleate, malonate, methanesulfonate, methylsulfate, naphthoate, 2-naphthalenesulfonate, nicotinate, nitrate, orotate, oxalate, palmitate, dihydronaphthoate, phosphate/hydrophosphate/dihydrophosphate, pyroglutamate, saccharate, stearate, succinate, tannate, tartrate, tosylate, trifluoroacetate and xinafoate, aluminum salt, arginine, benzathine, calcium salt, choline, diethylamine salt, diethanolamine salt, glycinate, lysinate, magnesium salt, meglumine salt, ethanolamine salt, sodium salt, potassium salt, ammonium salt, tromethamine salt, and zinc salt.

Further, provided is a preparation method for the compound according to a reaction route as follows:

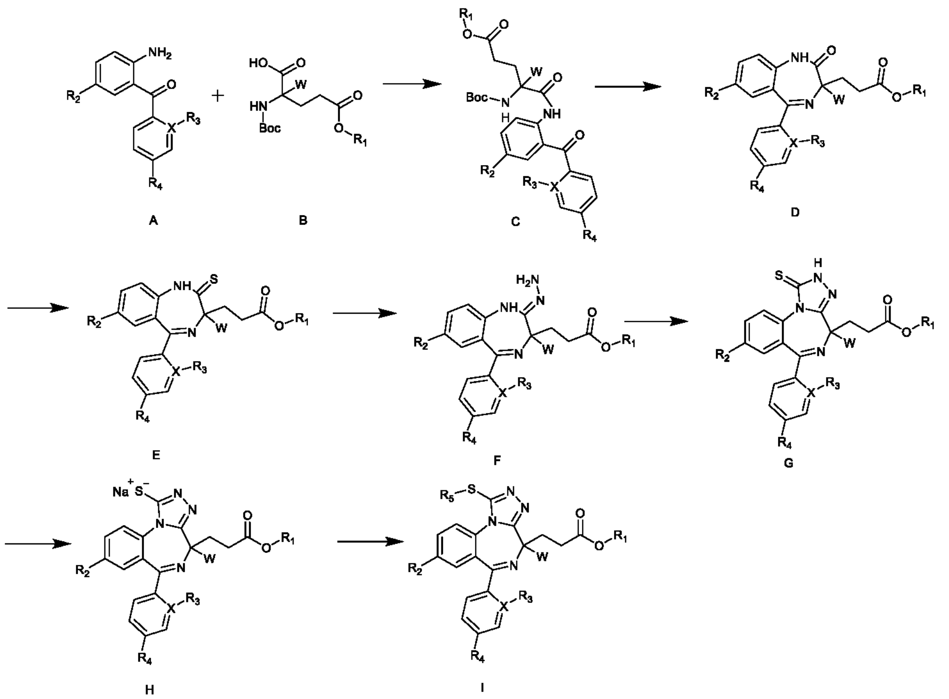


wherein $R_1$, $R_2$, $R_3$, $R_4$, $R_5$, and W are as defined in any one of claims 1 and 3, and the method comprises the following steps:

(1) subjecting the A and a chiral amino acid protected by $N_2$ or a derivative thereof to a condensation reaction to obtain an intermediate C, wherein
the reaction is preferably performed in the presence of a base and a condensing agent, wherein the base is for example, but not limited to, DIPEA, N-methylmorpholine, or TEA, and the condensing agent is for example, but not limited to, HATU, DCC, EDCI, PyBOP, BOP-Cl, or $T_3P$; the reaction is preferably performed in a suitable solvent, wherein the suitable solvent is for example, but not limited to, THF, DMF, or DCM; the reaction is preferably performed under an ice bath condition or at room temperature or under a solvent reflux condition; the chiral amino acid or the derivative thereof is for example, but not limited to, L-glutamic acid-5-methyl ester or L-glutamic acid-5-ethyl ester;
(2) removing the protective group of the intermediate C to obtain a benzodiazepine intermediate D, wherein
the reaction is preferably performed under an acidic condition, wherein the acidic condition is for example, but not limited to, trifluoroacetic acid or hydrochloric acid,
(3) subjecting the benzodiazepine intermediate D and Lawesson reagent or phosphorus pentasulfide to a reaction under heating and reflux conditions to obtain an active intermediate E, wherein
the reaction is preferably performed in a suitable solvent, wherein the suitable solvent is for example, but not limited to, toluene, or THF,
(4) subjecting the active intermediate E and hydrazine hydrate to a substitution reaction to obtain an intermediate F, wherein
the reaction is preferably performed in a suitable solvent, wherein the suitable solvent is for example, but not limited to, toluene or THF; the reaction is preferably performed under an ice bath condition or at room temperature or under a solvent reflux condition,
(5) subjecting the intermediate F and thiophosgene to a ring-closure reaction to obtain a target product G, wherein the reaction is preferably performed in a suitable solvent, wherein the suitable solvent is for example, but not limited to, DCM, DMF, or THF; the reaction is preferably performed under an ice bath condition or at room temperature or under a solvent reflux condition; the reaction is preferably performed under a basic condition, wherein the basic reaction is for example, but not limited to, triethylamine, DIPEA, or $K_2CO_3$,
(6) subjecting the intermediate G and a sodium salt to a reaction in an anhydrous solution to obtain a target product H, wherein
the reaction is preferably performed in a suitable solvent, wherein the suitable solvent is for example, but not limited to, DCM, $CH_3OH$, $CH_3CH_2OH$, $CH_3CH(OH)CH_3$, or THF; the reaction is preferably performed under an ice bath condition or at room temperature or under a solvent reflux condition; the reaction is preferably performed under a basic condition, wherein the basic condition is for example, but not limited to, sodium isopropoxide, sodium t-butoxide,

sodium methoxide, or sodium ethoxide, and

(7) subjecting the intermediate H to a substitution reaction to obtain a target product I, wherein

the reaction is preferably performed in a suitable solvent, wherein the suitable solvent is for example, but not limited to, DCM, DMF, or THF; the reaction is preferably performed under an ice bath condition or at room temperature or under a solvent reflux condition.

Further, provided is use of the compound or the composition for manufacturing a medicament for sedation and anesthesia, including use in conscious sedation during short-term diagnostic, surgical or endoscopic procedures, induction and maintenance of general anesthesia, and ICU sedation.

Further, provided is a method of sedation and anesthesia comprising administering an effective amount of the compound according to any one of claims 1 to 7 or the pharmaceutical composition according to claim 8 by an intravenous, intra-arterial, subcutaneous, intraperitoneal, intramuscular, or transdermal route.

Further, provided is a pharmaceutical formulation comprising a compound represented by general formula (I) or a pharmaceutically acceptable salt thereof as an active agent and a pharmaceutically acceptable carrier. The pharmaceutically acceptable carrier refers to one or more inert and non-toxic solid or liquid fillers, diluents, adjuvants, and the like, which do not adversely affect the active compound or the patient.

Further, the dosage form may be a suspension, an emulsion, an injection, a lyophilized powder injection, or other pharmaceutically common dosage form.

Further, the dosing regimen may be adjusted to provide the best desired response. For example, a single bolus may be given, several separate doses may be administered over time, or the dose may be proportionally reduced or increased as indicated by the exigency of the treatment situation. It is noted that dose values may vary with the type and severity of the condition to be alleviated, and may include single or multiple doses. It is further understood that for any particular individual, the specific dosage regimen will be adjusted over time according to the individual need and the professional judgment of the person administering the composition or supervising the administration of the composition.

It is an object of the present disclosure to provide a pharmaceutical composition comprising an effective amount of the compound of the present disclosure and one or more pharmaceutically acceptable carriers.

The pharmaceutically acceptable carriers that can be used in the pharmaceutical composition of the present disclosure include, but are not limited to, a sterile liquid, e.g., water, oil, and other injectable solvents, including those of petroleum, animal, vegetable, or synthetic source, e.g., peanut oil, soybean oil, mineral oil, sesame oil, and the like; water is an exemplary carrier when the pharmaceutical composition is administered intravenously; normal saline, glucose, aqueous glycerol solution, ethanol, propylene glycol, polyethylene glycol, or glycerol may also be used as a liquid carrier, particularly for an injectable liquid or an emulsion. Suitable pharmaceutical additives include starch, glucose, lactose, brown sugar, gelatin, maltose, chalk, silica gel, sodium stearate, glyceryl monostearate, talc, sodium oxide, skimmed milk powder, glycerol, propylene glycol, ethanol, lecithin, glycine, mannitol, Tween 80, polysorbate, sodium carboxymethylcellulose, gelatin, pectin, albumin, trehalose, dextran, and the like. The composition may further optionally contain a small amount of wetting agent, emulsifier, or pH buffer.

The pharmaceutical composition of the present disclosure may be administered by a suitable route. Preferably, the pharmaceutical composition of the present disclosure is administered by an intravenous, intra-arterial, subcutaneous, intraperitoneal, intramuscular, or transdermal route.

It is another object of the present disclosure to provide a kit comprising the compound or the pharmaceutical composition of the present disclosure.

It is a further object of the present disclosure to provide a method of sedation and anesthesia, comprising administering, preferably by an intravenous, intra-arterial, subcutaneous, intraperitoneal, intramuscular, or transdermal route, an effective amount of the compound or the pharmaceutical composition of the present disclosure, preferably for use in the following clinical treatment regimens: preoperative sedation during surgery; conscious sedation during short-term diagnosis, surgery, or an endoscopic procedure; induction and maintenance of general anesthesia prior to and/or concurrently with administration of other anesthetics and analgesics; ICU sedation; use for manufacturing a medicament for sedation and anesthesia and an anxiolytic medicament, including use in sedation, hypnosis, anxiolysis, and oblivion for early-wake insomnia due to dysfunction of excitation and inhibition of the brain.

It is a further object of the present disclosure to provide the compounds of the present disclosure for use as a medicament for sedation and anesthesia, preferably for intravenous administration in the following clinical treatment regimens: preoperative sedation during surgery; conscious sedation during short-term diagnosis, surgery, or an endoscopic procedure; induction and maintenance of general anesthesia prior to and/or concurrently with administration of other anesthetics and analgesics; ICU sedation; use for manufacturing a medicament for sedation and anesthesia and an anxiolytic medicament, including use in sedation, hypnosis, anxiolysis, and oblivion for early-wake insomnia due to dysfunction of excitation and inhibition of the brain.

It is a further object of the present disclosure to provide use of the compound or pharmaceutical composition

of the present disclosure for manufacturing a medicament for sedation and anesthesia, preferably for intravenous administration in the following clinical treatment regimens: preoperative sedation during surgery; conscious sedation during short-term diagnosis, surgery, or an endoscopic procedure; induction and maintenance of general anesthesia prior to and/or concurrently with administration of other anesthetics and analgesics; ICU sedation; use for manufacturing a medicament for sedation and anesthesia and an anxiolytic medicament, including use in sedation, hypnosis, anxiolysis, and oblivion for early-wake insomnia due to dysfunction of excitation and inhibition of the brain.

The amount of the compound of the present disclosure administered will depend on the individual being treated, the rate of administration, the disposition of the compound, and the judgment of the prescribing physician. Generally, an effective dose is about 0.0001 mg to about 50 mg/kg/day, e.g., about 0.01 to about 10 mg/kg/day (single or separate administration). For a person weighing 70 kg, this would total about 0.007 mg/day to about 3500 mg/day, for example, about 0.7 mg/day to about 700 mg/day. In some cases, dosage levels below the lower limit of the above range may be sufficient, while in other cases, a larger dosage may still be employed without causing any harmful side effects, provided that the larger dosage is first divided into several smaller dosages for administration throughout the day.

## Beneficial Effects

The beneficial effects of the present disclosure are as follows: the short-acting benzodiazepine derivatives of the present disclosure have the characteristics of fast onset of action, short acting time, strong depth, fast metabolism, and fast wake-up. In anesthesia experiments on mice and rats, the short-acting benzodiazepine derivatives of the present disclosure have comparable onset of action to remimazolam, but have significantly shortened acting time and wake-up time, and some of the compounds have significantly enhanced depth of action and have characteristics such as faster onset of action, shorter action time, greater action strength, faster metabolism, faster wake-up, and the like; in a long-term infusion anesthesia experiment on rats, compared to remimazolam, the short-acting benzodiazepine derivatives of the present disclosure have significantly shortened wake-up time and recovery time and have characteristics such as faster wake-up, faster recovery and the like; the benzodiazepine compounds of the present disclosure not only retain high affinity and selectivity for the $GABA_A$ receptor, but also have the following advantages by structural modification of benzodiazepine and modulation of the carboxylate group: predictable fast onset time for achieving sedation and anesthesia, short effective action time, strong depth of action, and short wake-up time, thereby reducing adverse inhibitory reactions on the cardiovascular system and the respiratory system and reducing the side effects on the nervous system of a patient, including problems such as sleepiness, dizziness, and the like.

## Definitions and Description of Terms

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should be construed as being within the scope of the present specification and/or the claims.

Unless otherwise stated, a numerical range set forth in the description and claims shall be construed as at least including each specific integer value within the range. For example, the numerical range "1-20" shall be construed as at least including each integer value in the numerical range "1-10", i.e., 1, 2, 3, 4, 5, 6, 7, 8, 9, and 10, and each integer value in the numerical range "11-20", i.e., 11, 12, 13, 14, 15, 16, 17, 18, 19 and 20.

The term "halogen" refers to fluorine, chlorine, bromine, and iodine.

The term "$C_{1-20}$ alkyl" should be understood to represent a linear or branched saturated monovalent hydrocarbyl group having 1-20 carbon atoms. For example, "$C_{1-10}$ alkyl" represents linear and branched chain alkyl groups having 1, 2, 3, 4, 5, 6, 7, 8, 9, or 10 carbon atoms, "$C_{1-8}$ alkyl" represents linear and branched chain alkyl groups having 1, 2, 3, 4, 5, 6, 7, or 8 carbon atoms, and "$C_{1-6}$ alkyl" represents linear and branched chain alkyl groups having 1, 2, 3, 4, 5, or 6 carbon atoms. The alkyl is, for example, methyl, ethyl, propyl, butyl, pentyl, hexyl, isopropyl, isobutyl, sec-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof.

The term "$C_{2-20}$ alkenyl" should be understood to represent a linear or branched monovalent hydrocarbyl group containing one or more double bonds and having 2-20 carbon atoms, preferably "$C_{2-10}$ alkenyl". "$C_{2-10}$ alkenyl" should be understood to preferably represent a linear or branched monovalent hydrocarbyl group containing one or more double bonds and having 2, 3, 4, 5, 6, 7, 8, 9, or 10 carbon atoms, more preferably "$C_{2-8}$ alkenyl". "$C_{2-10}$ alkenyl" should be understood to preferably represent a linear or branched monovalent hydrocarbyl group containing one or more double bonds and having 2, 3, 4, 5, 6, 7, or 8 carbon atoms, for example, having 2, 3, 4, 5, or 6 carbon atoms (i.e., $C_{2-6}$ alkenyl) or having 2 or 3 carbon atoms (i.e., $C_{2-3}$ alkenyl). It should be understood that in the case that the alkenyl comprises

more than one double bond, the double bonds can be separated from one another or conjugated. The alkenyl is, for example, vinyl, allyl, (*E*)-2-methylvinyl, (*Z*)-2-methylvinyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl,hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex1-enyl, (Z)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (Z)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl or 1-isopropylvinyl.

The term "$C_{2-20}$ alkynyl" should be understood to represent a linear or branched monovalent hydrocarbyl group containing one or more triple bonds and having 2-20 carbon atoms, preferably "$C_{2-10}$ alkynyl". The term "$C_{2-10}$ alkynyl" should be understood to preferably represent a linear or branched monovalent hydrocarbyl group containing one or more triple bonds and having 2, 3, 4, 5, 6, 7, 8, 9, or 10 carbon atoms, for example, having 2, 3, 4, 5, 6, 7, or 8 carbon atoms (i.e., "$C_{2-8}$ alkynyl"), having 2, 3, 4, 5, or 6 carbon atoms (i.e., "$C_{2-6}$ alkynyl"), or having 2 or 3 carbon atoms ("$C_{2-3}$ alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or 3,3-dimethylbut-1-ynyl. In particular, the alkynyl is ethynyl, prop-1-ynyl or prop-2-ynyl.

The term "$C_{3-10}$ cycloalkyl" should be understood to represent a saturated monovalent monocyclic or bicyclic (such as bridged or spiro) hydrocarbon ring or tricyclic alkane having 3, 4, 5, 6, 7, 8, 9 or 10 carbon atoms. The $C_{3-10}$ cycloalkyl may be monocyclic hydrocarbyl such as cyclopropyl, cyclobutyl, cyclopentyl, cyclohexyl, cycloheptyl, cyclooctyl, cyclononyl or cyclodecyl, or bicyclic hydrocarbyl such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonyl, 2,6-diazaspiro[3,4]octyl, or tricyclic hydrocarbyl such as adamantyl.

The term "3- to 10-membered heterocyclyl" refers to a saturated or unsaturated non-aromatic ring or ring system and contains at least one heteroatom selected from O, S and N. The heterocyclyl may be connected to the rest of the molecule through any one of the carbon atoms or the nitrogen atom (if present). The heterocyclyl may include fused or bridged rings as well as spiro rings. In particular, the heterocyclyl may include, but is not limited to: 4-membered rings such as azetidinyl and oxetanyl; 5-membered rings such as tetrahydrofuranyl, dioxolyl, pyrrolidinyl, imidazolidinyl, pyrazolidinyl and pyrrolinyl; 6-membered rings such as tetrahydropyranyl, piperidyl, morpholinyl, dithianyl, thiomorpholinyl, piperazinyl and trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[c]pyrrol-2(1*H*)-yl ring, or a 5,6-membered bicyclic ring such as a hexahydropyrrolo[1,2-*a*]pyrazin-2(1H)-yl ring. The heterocyclyl may be partially unsaturated, i.e., it may contain one or more double bonds, for example, but not limited to, dihydrofuranyl, dihydropyranyl, 2,5-dihydro-1*H*-pyrrolyl, 4*H*-[1,3,4]thiadiazinyl, 1,2,3,5-tetrahydrooxazyl, or 4*H*-[1,4]thiazinyl, or it may be benzo-fused, for example, but not limited to, dihydroisoquinolyl. When the 3- to 10-membered heterocyclyl is connected to another group to form the compound of the present disclosure, the group may be connected to the carbon atom on the 3- to 10-membered heterocyclyl, or may be connected to the heteroatom on the 3- to 10-membered heterocyclyl. For example, when the 3- to 10-membered heterocyclyl is selected from piperazinyl, the group may be connected to the nitrogen atom on the piperazinyl. Alternatively, when the 3- to 10-membered heterocyclyl is selected from piperidyl, the group may be connected to the nitrogen atom on the piperidyl ring or the carbon atom in the para position.

The term "$C_{6-14}$ aryl" should be understood to preferably represent an aromatic or partially aromatic monovalent monocyclic, bicyclic or tricyclic hydrocarbon ring having 6, 7, 8, 9, 10, 11, 12, 13 or 14 carbon atoms ("$C_{6-14}$ aryl"), in particular a ring having 6 carbon atoms ("$C_6$ aryl"), e.g., phenyl; biphenyl; a ring having 9 carbon atoms ("$C_9$ aryl"), e.g., indanyl or indenyl; a ring having 10 carbon atoms ("$C_{10}$ aryl"), e.g., tetrahydronaphthyl, dihydronaphthyl, or naphthyl; a ring having 13 carbon atoms ("$C_{13}$ aryl"), e.g., fluorenyl; or a ring having 14 carbon atoms ("$C_{14}$ aryl"), e.g., anthryl. When the $C_{6-14}$ aryl is substituted, it may be monosubstituted or polysubstituted. In addition, the substitution site is not limited, and may be, for example, ortho-substitution, para-substitution, or meta-substitution.

The term "5- to 14-membered heteroaryl" should be understood to represent a monovalent monocyclic, bicyclic (e.g., fused, bridged or spiro) or tricyclic aromatic ring system that has 5-14 ring atoms and contains one or more (e.g., 1-5) heteroatoms independently selected from N, O, and S, e.g., "5- to 14-membered heteroaryl". The term "5- to 14-membered heteroaryl" should be understood to represent a monovalent monocyclic, bicyclic or tricyclic aromatic ring system that has 5, 6, 7, 8, 9, 10, 11, 12, 13 or 14 ring atoms, in particular 5, 6, 9 or 10 carbon atoms, contains 1-5,

preferably 1-3 heteroatoms independently selected from N, O and S, and may be benzo-fused in each case. "Heteroaryl" also refers to a group in which a heteroaromatic ring is fused to one or more aryl, alicyclic or heterocyclyl rings, wherein the radical or site of attachment is on the heteroaromatic ring. When the 5- to 14-membered heteroaryl is connected to another group to form the compound of the present disclosure, the group may be connected to the carbon atom on the 5-to 14-membered heteroaryl ring, or may be connected to the heteroatom on the 5- to 14-membered heteroaryl ring. When the 5- to 14-membered heteroaryl is substituted, it may be monosubstituted or polysubstituted. In addition, the substitution site is not limited. For example, hydrogen connected to the carbon atom on the heteroaryl ring may be substituted, or hydrogen connected to the heteroatom on the heteroaryl ring may be substituted.

The term "spiro rings" refers to a ring system in which two rings share 1 ring-forming atom.

The term "fused rings" refers to a ring system in which two rings share 2 ring-forming atoms.

The term "bridged rings" refers to a ring system in which two rings share 3 or more ring-forming atoms.

The term "oxo" means that the carbon atom, nitrogen atom or sulfur atom in the substituent is substituted with an oxy group formed after oxidation (=O).

## DETAILED DESCRIPTION

In order to make the objects and technical solutions of the present disclosure clearer, the present disclosure is further illustrated below with reference to specific examples. It should be understood that these examples are merely intended to illustrate the present disclosure rather than limit the scope of the present disclosure. Moreover, specific experimental methods not mentioned in the following examples are performed according to the conventional experimental methods.

The abbreviations herein have the following meanings:

| Abbreviations | Meaning | Abbreviations | Meaning |
|---|---|---|---|
| DCM | Dichloromethane | BOP-Cl | Bis(2-oxo-3-oxazolidinyl) phosphinic chloride |
| DCC | Dicyclohexylcarbodiimide | $T_3P$ | Propylphosphonic anhydride |
| DIPEA | *N,N*-diisopropylethylamine | $Boc_2O$ | Di-*tert*-butyl dicarbonate |
| EDCI | 1-ethyl-(3-dimethylaminopropyl)carbodiimide hydrochloride | TEA | Triethylamine |
| MeOH | Methanol | TLC | Thin-layer chromatography |
| THF | Tetrahydrofuran | s | Singlet |
| EtOH | Ethanol | d | Doublet |
| DMF | *N,N*-dimethylfonnamide | t | Triplet |
| HATU | 2-(7 -azabenzotriazol)-*N,N,N',N'*-tetramethyluronium hexafluorophosphate | q | Quartet |
| LCMS | liquid chromatograph-mass spectrometer | dd | Double doublet |
| $P_2S_5$ | Phosphorus pentasulfide | m | Multiplet |
| PyBOP | Benzotriazol-1-yl-oxytripyrrolidino-phosphonium hexafluorophosphate | br | Broad |

The structure of the compound is confirmed by nuclear magnetic resonance spectroscopy ($^1H$ NMR, $^{13}C$ NMR) and mass spectrometry (MS); the reaction is monitored by thin-layer chromatography (TLC) or LCMS using the following developer systems: a dichloromethane and methanol system, a n-hexane and ethyl acetate system, and a petroleum ether and ethyl acetate system.

A 200-300 mesh silica gel is generally used as a stationary phase for the column chromatography; the system of the eluent includes: a dichloromethane and methanol system and a n-hexane and ethyl acetate system, the volume ratio of the solvent is adjusted according to the polarity of the compound.

In the following examples, the reaction temperature is room temperature (20 °C to 30 °C) unless otherwise specified.

Example 1: methyl (*S*)-3-(8-chloro-6-(2-fluorophenyl)-1-mercapto-4*H*-benzo[*f*]1,2,4]triazolo[4,3-*a*][1,4]diazepin-4-yl)propionate (compound 1) (tautomer of compound 1)

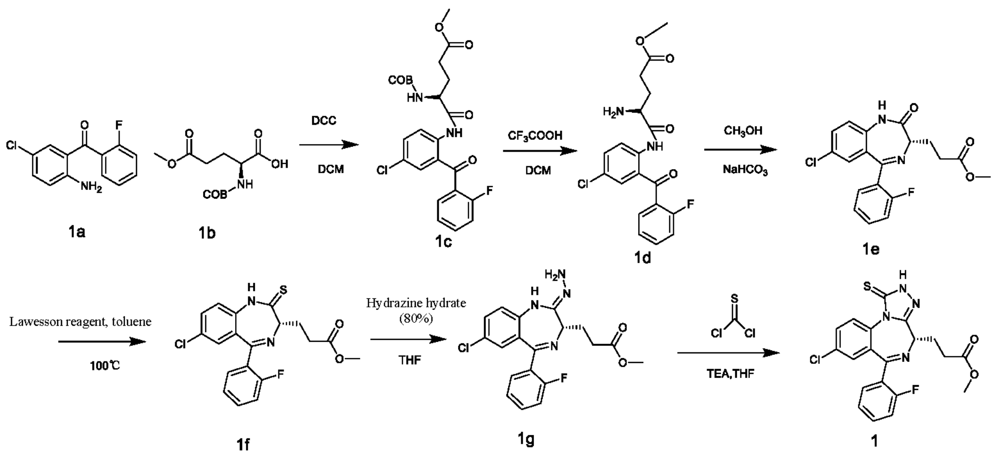

1a 1b 1c 1d 1e 1f 1g 1

Step 1: preparation of methyl (*S*)-5-((2-fluoro-benzoyl-4-chlorophenyl)amino)-4-((*tert*-butoxycarbonyl)amino)-5-oxopentanoate (compound 1c):
2-amino-5-chloro-2'-fluorobenzophenone (compound Ia, 11.48 g, 0.046 mol) and the compound *N-tert*-butoxycarbonyl-L-glutamic acid-5-methyl ester (compound Ib, 10 g, 0.038 mol) were dissolved in DCM (300 mL). The mixture was cooled to 0 °C, and DCC (9.49 g, 0.046 mmol) was added. The mixture was stirred for 24 h. After the reaction was completed as detected by LCMS, the reaction solution was filtered in vacuum. The filtrate was collected and concentrated under reduced pressure to evaporate the solvent. The residue was purified by column chromatography (petroleum ether/ethyl acetate, 4:1, v/v) to give methyl (*S*)-S-((2-fluoro-benzoyl-4-chlorophenyl)amino)-4-((*tert*-butoxycarbonyl)amino)-5-oxopentanoate as a white solid (compound 1c, 10.56 g, yield: 56.4%).

Step 2: preparation of methyl (S)-4-amino-5-((2-fluorobenzoyl-4-chlorophenyl)amino)-5-oxopentanate (compound Id): Methyl (*S*)-5-((2-fluoro-benzoyl-4-chlorophenyl)amino)-4-((*tert*-butoxycarbonyl)amino)-5 -oxopentanoate (compound 1c, 10.56 g) was dissolved in DCM (50 mL). TFA (50 mL) was added, and the mixture was stirred for 20 min until LCMS showed that the reaction was completed. The reaction solution was concentrated to give a residue, crude methyl (S)-4-amino-5-((2-fluorobenzoyl-4-chlorophenyl)amino)-5-oxopentanoate (compound Id, 8.4 g), which was used directly in the next step.

Step 3: preparation of methyl (*S*)-3-(7-chloro-2-oxo-5-(2-fluorophenyl)-2,3-dihydro-1*H*-benzo[*e*][1,4]diazepin-3-yl)propionate (compound Ie):
Methyl (*S*)-4-amino-5-((2-fluorobenzoyl-4-chlorophenyl)amino)-5-oxopentanoate (compound Id, 8.4 g) was dissolved in MeOH (100 mL) and adjusted to about pH 10 by the addition of $NaHCO_3$. The mixture was stirred for 24 h. After the reaction was completed as detected by LCMS, the reaction solution was filtered. The filtrate was poured into ice water and extracted with ethyl acetate. The organic phase was washed 3 times with water, dried, and concentrated, and the residue was purified by column chromatography (petroleum ether/ethyl acetate, 2:1, v/v) to obtain white methyl (S)-3-(7-chloro-2-oxo-5-(2-fluorophenyl)-2,3-dihydro-1H-benzo[e][1,4]diazepin-3-yl)propionate (compound Ie, 7.5 g).

Step 4: preparation of methyl (*S*)-3-(7-chloro-5-(2-fluorophenyl)-2-thio-2-3,3-dihydro-1*H*-benzo[*e*][1,4]diazepin-3-yl)propionate (compound If):
Methyl (*S*)-3-(7-chloro-2-oxo-5-(2-fluorophenyl)-2,3-dihydro-1*H*-benzo[*e*][1,4]diazepin-3-yl)propionate (compound 1e, 7.5 g) was dissolved in toluene (200 mL), and Lawesson reagent (4.86 g, 0.012 mol) was added. The compound was heated to 100 °C and stirred for 1.5 h until LCMS showed that the reaction was completed. A saturated sodium bicarbonate solution was added to remove excessive Lawesson reagent, and the mixture was extracted with ethyl acetate (20 mL x 3). The organic layers were combined, dried over anhydrous sodium sulfate, and concentrated under reduced pressure to evaporate the reaction solvent. The residue was purified by column chromatography (petroleum ether/ethyl acetate, 7:1, v/v) to obtain methyl (*S*)-3-(7-chloro-5-(2-fluorophenyl)-2-thio-2-3,3-dihydro-1*H*-benzo[*e*][1,4]diazepin-3-yl)propionate as a light yellow solid (compound 1f, 4.07 g, yield: 52.1%).

Step 5: preparation of methyl (*S*)-3-(7-chloro-5-(2-fluorophenyl)-2-hydrazino-2,3-dihydro-1*H*-benzo[*e*][1,4]diazepin-3-yl)propionate (compound 1g)
Methyl (*S*)-3-(7-chloro-5-(2-fluorophenyl)-2-thio-2-3,3-dihydro-1*H*-benzo[*e*][1,4]diazepin-3-yl)propionate (compound If, 4.07 g) was dissolved in 50 mL of THF, and the mixture was cooled to 0 °C. 80% hydrazine hydrate (1.56 g, 0.031 mol)

was added, and the mixture was stirred for 30 min until LCMS showed that the reaction was completed. Saturated NaCl was added to remove excess hydrazine hydrate, the mixture was extracted with DCM. The organic phases were combined, dried, and concentrated to obtain crude methyl (*S*)-3-(7-chloro-5-(2-fluorophenyl)-2-hydrazino-2,3-dihydro-1*H*-benzo[*e*][1,4]diazepin-3-yl)propionate (compound 1g, 3.1 g), which was used directly in the next step.

Step 6: preparation of methyl (*S*)-3-(8-bromo-6-(pyridin-2-yl)-1-thio-2-2,4-dihydro-1*H*-benzo[*f*][1,2,4]triazolo[4,3-α][1,4]diazepin-4-yl)propionate (tautomer of compound 1)
Methyl (*S*)-3-(7-chloro-5-(2-fluorophenyl)-2-hydrazino-2,3-dihydro-1*H*-benzo[*e*][1,4]diazepin-3-yl)propionate (compound 1g, 3.1 g) was dissolved in 80 mL of THF, followed by the addition of TEA (1.77 g, 0.018 mol), and the mixture was cooled to 0 °C. Thiophosgene (1.0 g, 0.009 mol) was added, and the mixture was stirred for 2 h until LCMS showed that the reaction was completed. The reaction solution was filtered in vacuum. The filtrate was collected and concentrated under reduced pressure to evaporate the reaction solvent. The residue was purified by column chromatography (petroleum ether/ethyl acetate, 4:1, v/v) to give methyl (*S*)-3-(8-bromo-6-(pyridin-2-yl)-1-thio-2-2,4-dihydro-1*H*-benzo[*f*][1,2,4]triazolo[4,3-*a*][1,4]diazepin-4-yl)propionate as a white solid (tautomer of compound 1, 1.46 g, yield: 42.5%).

$^1$H NMR (300 MHz, DMSO-$d_6$) δ: 14.17(s, 1H), 8.46 (d, *J* = 8.7Hz, 1H), 7.90-7.28 (m, 6H), 4.26 (t, *J*=6.1Hz, 1H), 3.62 (s, 3H), 2.70-2.45 (m, 4H); $^{13}$C NMR (75 MHz, DMSO-$d_6$) δ: 173.58, 166.94, 164.32, 161.78, 158.47, 153.86, 133.56, 132.55, 132.23, 131.91, 131.18, 129.03, 127.87, 127.02, 125.27, 116.78, 55.15, 51.85, 30.19, 26.26; LC-MS (ESI) m/z: 430.5 [M+H] $^+$.

Examples 2-7 were synthesized according to the methods as described in the above example, with the specific NMR, MS, and C NMR characterization data shown as follows:

Example 2: preparation of methyl (*S*)-3-(8-chloro-6-(2-chlorophenyl)-1-thio-2,4-dihydro-1*H*-benzo[*e*][1,2,4]triazolo[4,3-*a*][1,4]diazepin-4-yl)propionate (tautomer of compound 2)

$^1$H NMR (300 MHz, DMSO-$d_6$) δ: 14.15 (s, 1H), 8.46 (d, *J* = 8.8Hz, 1H), 7.87 (d, *J* = 7.4Hz, 1H), 7.66-7.50 (m, 4H), 7.16 (s, 1H), 4.34 (t, *J* = 5.8Hz, 1H), 3.62 (s, 3H), 2.70-2.45 (m, 4H); $^{13}$C NMR (75 MHz, DMSO-$d_6$) δ: 173.54, 167.16, 167.00, 153.68, 138.23, 132.42, 132.28, 132.12, 131.94, 131.73, 131.64, 131.07, 130.21, 128.52, 127.99, 127.96, 55.30, 51.85, 30.22, 26.16; LC-MS (ESI) m/z: 447.2 [M+H] $^+$.

Example 3: preparation of methyl (*S*)-3-(8-bromo-6-(pyridin-2-yl)-1-thio-2,4-dihydro-1*H*-benzo[*f*][1,2,4]triazolo[4,3-*a*][1,4]diazepin-4-yl)propionate (tautomer of compound 3)

$^1$H NMR (300 MHz, DMSO-$d_6$) δ: 14.17 (s, 1H), 8.59 (d, *J* = 4.3Hz, 1H), 8.37 (d, *J* = 8.8Hz, 1H), 8.13 (d, *J* = 7.9Hz, 1H), 8.03-7.98 (m, 2H), 7.60-7.54 (m, 2H), 4.34 (t, *J*=5.8Hz, 1H), 3.65 (s, 3H), 2.70-2.45 (m, 4H); $^{13}$C NMR (75 MHz, $CDCl_3$-$d_1$) δ: 173.70, 167.81, 166.51, 157.68, 155.34, 149.03, 136.91, 134.69, 134.01, 132.24, 130.18, 126.91, 125.28, 124.90, 123.99, 55.09, 51.71, 30.17, 26.18; LC-MS (ESI) m/z: 457.8 [M+H] $^+$.

Example 4: preparation of methyl (*S*)-3-(8-bromo-6-(2-fluorophenyl)-1-thio-2,4-dihydro-1*H*-benzo[*f*][1,2,4]triazolo[4,3-*a*][1,4]diazepin-4-yl) propionate (tautomer of compound 4)

$^1$H NMR (300 MHz, DMSO-$d_6$) δ: 11.14 (s, 1H), 8.38 (d, *J* = 9.1Hz, 1H), 7.97 (d, *J* = 8.9Hz, 1H), 7.66-7.54 (m, 2H), 7.43 (s, 1H), 7.36 (t, *J* = 7.5Hz, 1H), 7.24 (t, *J* = 9.8Hz, 1H), 4.27(t, *J* = 6.7Hz, 1H), 3.60 (s, 3H), 2.70-2.45 (m, 4H); $^{13}$C NMR (75 MHz, DMSO-$d_6$) δ: 173.60, 166.91, 164.26, 161.78, 158.48, 153.87, 134.09, 133.46, 132.45, 131.93, 131.60, 128.02, 127.05, 125.28, 120.85, 116.80, 55.14, 51.87, 30.20, 26.27; LC-MS (ESI) m/z: 475.1 [M+H] $^+$.

Example 5: preparation of methyl (*S*)-3-(6-phenyl-1-thio-2,4-dihydro-1*H*-benzo[*f*][1,2,4]triazolo[4,3-*a*][1,4]diazepin-4-yl)propionate (tautomer of compound 5)

$^1$H NMR (400 MHz, DMSO-$d_6$) δ: 14.06 (s, 1H), 8.43 (d, *J* = 8.0Hz, 1H), 7.80 (t, *J* = 7.3Hz, 1H), 7.60-7.40 (m, 7H), 4.17 (t, *J* = 5.7Hz, 1H), 3.62 (s, 3H), 2.70-2.40 (m, 4H); $^{13}$C NMR (75 MHz, $CDCl_3$-$d_1$) δ: 173.89, 169.62, 167.15, 154.17, 138.77, 133.17, 131.38, 131.10, 131.00, 129.80(2C), 129.57, 128.35(2C), 127.88, 125.67, 54.78, 51.87, 30.25, 26.21; LC-MS (ESI) m/z: 379.5[M+H]+.

Example 6: preparation of methyl (*S*)-3-(6-(4-fluorophenyl)-1-thio-2,4-dihydro-1*H*-benzo[*f*][1,2,4]triazolo[4,3-*a*][1,4]diazepin-4-yl)propionate (tautomer of compound 6)

$^1$H NMR (400 MHz, DMSO-$d_6$) δ: 14.06 (s, 1H), 8.43 (d, *J* = 8.3Hz, 1H), 7.81 (t, *J* = 8.8Hz, 1H), 7.60-7.55 (m, 3H), 7.43 (d, *J* = 9.0Hz, 1H), 7.28 (t, *J* = 7.7Hz, 2H), 4.17 (t, *J* = 7.6Hz, 1H), 3.62 (s, 3H), 2.70-2.40 (m, 4H); $^{13}$C NMR (75 MHz, $CDCl_3$-$d_1$) δ: 173.84, 168.39, 167.18, 166.24, 162.90, 154.14, 134.90, 133.16, 131.98, 131.18, 129.31, 127.98,

125.80, 115.58, 115.29, 54.77, 51.88, 30.22, 26.17; LC-MS (ESI) m/z: 397.3 $[M+H]^+$.

Example 7: preparation of methyl (*S*)-3-(8-chloro-6-phenyl)-1-thio-2,4-dihydro-1-benzo[*f*][1,2,4]triazolo[4,3-*a*][1,4]diazepin-4-yl)propionate (tautomer of compound 7)

$^1$H NMR (300 MHz, $CDCl_3$-*$d_1$*) δ: 11.34 (s, 1H), 8.57 (d, *J* = 8.8Hz, 1H),7.71-7.41 (m, 7H), 4.17 (t, *J* = 5.8Hz, 1H), 3.73 (s, 3H), 2.81-2.60 (m, 4H); $^{13}$C NMR (75 MHz, DMSO-*$d_6$*) δ: 173.62, 167.25, 166.82, 154.07, 138.40, 132.31, 132.24, 131.44, 131.20, 130.96, 130.45, 129.54, 128.91, 127.86, 55.36, 51.84, 30.23, 26.34; LC-MS (ESI) m/z: 412.7 $[M+H]^+$.

Example 8: preparation of methyl (*S*)-3-(8-chloro-6-(2-fluorophenyl)-1-(methylthio)-4*H*-benzo[*f*][1,2,4]triazolo[4,3-*a*][1,4]diazepin-4-yl)propionate (compound 8)

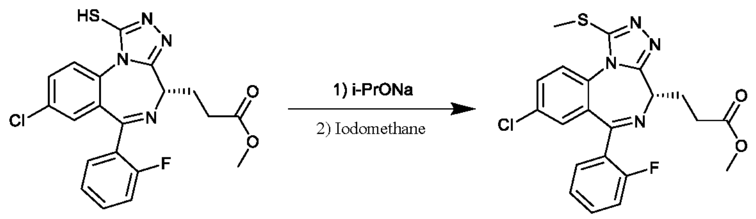


1 8

Methyl (*S*)-3-(8-chloro-6-(2-fluorophenyl)-1-mercapto-4*H*-benzo[*f*][1,2,4]triazolo[4,3-*a*][1,4]diazepin-4-yl)propionate (compound 1, 0.1 g) was dissolved in 8 mL of THF, and the mixture was cooled in an ice bath. A solution of 20% sodium isopropoxide in tetrahydrofuran was added dropwise, and the mixture was stirred at room temperature for 30 min after the dropwise addition was completed. The reaction solution was concentrated by rotary evaporation to remove the solvent to obtain a corresponding sodium salt. Iodomethane (0.049 g, 0.35 mmol) was added, and the mixture was heated and refluxed at 68 °C for 8 h. The reaction solution was concentrated under reduced pressure, and the residue was purified by column chromatography (PE:EA = 1:1) to obtain methyl (5)-3-(8-chloro-6-(2-fluorophenyl)-1-(methylthio)-4*H*-benzo[*f*][1,2,4]triazolo[4,3-*a*][1,4]diazepin-4-yl)propionate as a white solid (compound 8, 0.075 g, yield: 72.8%).

$^1$H NMR (300 MHz, $CDCl_3$-*$d_1$*) δ: 7.78-7.61 (m, 3H), 7.48 (q, *J* = 6.1Hz, 1H), 7.34-7.24 (m, 2H), 7.05 (t, *J* = 9.6Hz, 1H), 4.23 (t, *J* = 5.7Hz, 1H), 3.68 (s, 3H), 2.90-2.72 (m, 7H); $^{13}$C NMR (75 MHz, $CDCl_3$-*$d_1$*) δ 173.88, 163.67, 161.96, 158.65, 157.58, 151.07, 133.73, 132.71, 132.60, 131.62, 131.27, 130.35, 129.78, 127.05, 124.63, 116.46, 55.06, 51.63, 30.18, 26.55, 15.28; LC-MS (ESI) m/z: 445.3 $[M+H]^+$.

Examples 9-144 (compounds 9-144) were synthesized according to the methods as described in the above example, with the specific NMR, MS, and C NMR characterization data shown as follows:

Example 9: methyl (*S*)-3-(8-bromo-6-(2-fluorophenyl)-1-(methylthio)-4*H*-benzo[*f*][1,2,4]triazolo[4,3-*a*][1,4]diazepin-4-yl)propionate (compound 9)

$^1$H NMR (300 MHz, $CDCl_3$-*$d_1$*) δ 7.67 (dd, *J* = 7.3, 2.2 Hz, 1H), 7.59 (d, *J* = 2.0 Hz, 1H), 7.55-7.31 (m, 5H), 7.22 (dd, *J* = 7.2, 2.2 Hz, 1H), 5.22 (s, 1H), 3.68 (s, 3H), 2.53 (d, *J* = 28.9 Hz, 4H), 2.45-2.26 (m, 2H), 2.25 (d, *J* = 12.4 Hz, 1H); $^{13}$C NMR (75 MHz, $CDCl_3$-*$d_1$*) δ 173.78, 160.03, 150.29, 147.02, 140.17, 138.02, 131.75, 129.62, 129.44, 129.40, 125.51, 125.40, 122.44, 118.85, 117.15, 115.82, 54.38, 51.46, 32.25, 27.63, 14.05; LC-MS (ESI) m/z: 490.3 $[M+H]^+$.

Example 10: (*S*)-3-(8-nitro-6-(2-fluorophenyl)-1-(methylthio)-4*H*-benzo[*f*][1,2,4]triazolo[4,3-*a*][1,4]diazepin-4-yl)propionic acid (compound 10)

$^1$H NMR (300 MHz, $CDCl_3$-*$d_1$*) δ 8.30 (dd, *J* = 7.5, 2.0 Hz, 1H), 8.15 (d, *J* = 2.0 Hz, 1H), 7.75 (d, *J* = 7.5 Hz, 1H), 7.64 (dd, *J* = 7.2, 2.3 Hz, 1H), 7.50 - 7.31 (m, 2H), 7.27 (dd, *J* = 7.2, 2.4 Hz, 1H), 5.15 (s, 1H), 3.68 (s, 3H), 2.56 (s, 3H), 2.53 (d, *J* = 12.4 Hz, 1H), 2.40 (d, *J* = 12.4 Hz, 1H), 2.24 (d, *J* = 1.5 Hz, 2H); $^{13}$C NMR (75 MHz, $CDCl_3$-*$d_1$*) δ 173.68, 160.03, 150.29, 148.47, 146.96, 143.52, 141.28, 129.83, 129.75, 128.39, 124.87, 124.15, 123.29, 122.44, 120.52, 115.88, 57.87, 51.40, 32.25, 27.60, 14.05; LC-MS (ESI) m/z: 456.4 $[M+H]^+$.

Example 11: methyl (S)-3-(8-chloro-6-(2-fluorophenyl)-1-(ethylthio)-4*H*-benzo[*f*][1,2,4]triazolo[4,3-*a*][1,4]diazepin-4-yl)propionate (compound 11)

$^1$H NMR (300 MHz, $CDCl_3$-*d₁*) δ: 7.78-7.61 (m, 5H), 7.48 (q, *J* = 5.4 Hz, 1H), 7.34-7.24 (m, 2H), 7.05 (t, *J* = 9.4 Hz, 1H), 4.23 (t, *J* = 5.7 Hz, 1H), 3.68 (s, 3H), 3.25 (q, *J* = 7.2 Hz, 2H), 2.90-2.72 (m, 4H), 1.39 (t, *J* = 7.2Hz, 3H); $^{13}$C NMR (75 MHz, $CDCl_3$-$d_1$) δ: 173.88, 163.70, 162.02, 158.68, 157.41, 150.20, 133.69, 132.71, 131.51, 131.26, 130.40, 129.65, 127.06, 125.08, 124.56, 116.45, 55.10, 51.63, 30.20, 27.81, 26.54, 14.66; LC-MS (ESI) m/z: 458.9 $[M+H]^+$.

Example 12: methyl (*S*)-3-(8-bromo-6-(2-fluorophenyl)-1-(ethylthio)-4*H*-benzo[*f*][1,2,4]triazolo[4,3-*a*][1,4]diazepin-4-yl)propionate (compound 12)

$^1$H NMR (300 MHz, $CDCl_3$-$d_1$) δ 7.67 (dd, *J* = 7.1, 2.4 Hz, 1H), 7.59 (d, *J* = 2.0 Hz, 1H), 7.55-7.29 (m, 4H), 7.22 (dd, *J* = 7.0, 2.5 Hz, 1H), 5.22 (s, 1H), 3.68 (s, 3H), 3.18 (s, 2H), 2.50 (d, *J* = 12.4 Hz, 1H), 2.39 (d, *J* = 12.4 Hz, 1H), 2.37-2.19 (m, 2H), 1.39 (s, 3H); $^{13}$C NMR (75 MHz, $CDCl_3$-$d_1$) δ 173.78, 160.03, 147.02, 144.40, 140.17, 138.02, 131.75, 129.69, 129.44, 129.40, 127.05, 124.20, 122.44, 119.90, 116.44, 115.82, 54.38, 51.46, 32.25, 28.40, 27.60, 14.23; LC-MS (ESI) m/z: 504.4 $[M+H]^+$.

Example 13: methyl (*S*)-3-(8-nitro-6-(2-fluorophenyl)-1-(ethylthio)-4*H*-benzo[*f*][1,2,4]triazolo[4,3-*a*][1,4]diazepin-4-yl)propionate (compound 13)

$^1$H NMR (300 MHz, $CDCl_3$-$d_1$) δ 8.30 (dd, *J* = 7.5, 2.0 Hz, 1H), 8.17 (d, *J* = 2.0 Hz, 1H), 7.75 (d, *J* = 7.5 Hz, 1H), 7.66-7.56 (m, 1H), 7.47 - 7.31 (m, 2H), 7.32-7.22 (m, 1H), 5.15 (s, 1H), 3.68 (s, 3H), 3.18 (s, 2H), 2.53 (d, *J* = 12.4 Hz, 1H), 2.45-2.33 (m, 2H), 2.23 (d, *J* = 12.4 Hz, 1H), 1.40 (s, 3H); $^{13}$C NMR (75 MHz, $CDCl_3$-$d_1$) δ 173.90, 160.03, 148.47, 146.96, 144.00, 143.52, 141.28, 129.50, 129.44, 128.71, 124.87, 124.15, 122.86, 122.44, 120.52, 115.88, 54.38, 51.43, 32.25, 28.40, 27.60, 14.23; LC-MS (ESI) m/z: 470.5 $[M+H]^+$.

Example 14: methyl (*S*)-3-(8-chloro-6-(2-fluorophenyl)-1-(propylthio)-4*H*-benzo[*f*][1,2,4]triazolo[4,3-*a*][1,4]diazepin-4-yl)propionate (compound 14)

$^1$H NMR (300 MHz, $CDCl_3$-$d_1$) δ: 7.78-7.61 (m, 3H), 7.48 (q, *J* = 8.3 Hz, 1H), 7.34-7.24 (m, 2H), 7.05 (t, *J* = 9.0Hz, 1H), 4.23 (t, *J* = 6.2 Hz, 1H), 3.68 (s, 3H), 3.22 (t, *J* = 7.0 Hz, 2H), 2.90-2.72 (m, 4H), 1.77 (q, *J* = 7.1 Hz, 2H), 1.01 (t, *J* = 7.2 Hz, 3H); $^{13}$C NMR (75 MHz, $CDCl_3$-$d_1$) δ: 173.89, 163.69, 162.02, 158.68, 157.40, 150.42, 133.67, 132.70, 131.50, 131.27, 130.42, 129.65, 127.06, 125.11, 124.57, 116.45, 55.11, 51.63, 35.33, 30.21, 26.54, 22.68, 13.17; LC-MS (ESI) m/z: 473.1 $[M+H]^+$.

Example 15: methyl (*S*)-3-(8-bromo-6-(2-fluorophenyl)-1-(propylthio)-4*H*-benzo[*f*][1,2,4]triazolo[4,3-*a*][1,4]diazepin-4-yl)propionate (compound 15)

$^1$H NMR (300 MHz, $CDCl_3$-$d_1$) δ 7.67 (dd, *J* = 7.0, 2.4 Hz, 1H), 7.62-7.29 (m, 6H), 7.21 (dd, *J* = 6.9, 2.5 Hz, 1H), 5.17 (s, 1H), 3.68 (s, 3H), 3.24 (s, 2H), 2.50 (d, *J* = 12.4 Hz, 1H), 2.452.19 (m, 3H), 1.80 (d, *J* = 0.4 Hz, 3H), 1.01 (s, 3H); $^{13}$C NMR (75 MHz, $CDCl_3$-$d_1$) δ 173.78, 160.03, 147.02, 145.70, 140.80, 138.02, 131.75, 129.84, 129.69, 129.44, 127.05, 124.20, 122.44, 121.80, 116.44, 115.82, 54.38, 51.45, 32.86, 32.25, 27.67, 27.60, 14.10; LC-MS (ESI) m/z: 518.4 $[M+H]^+$.

Example 16: methyl (*S*)-3-(8-nitro-6-(2-fluorophenyl)-1-(propylthio)-4*H*-benzo[*f*][1,2,4]triazolo[4,3-a][1,4]diazepin-4-yl)propionate (compound 16)

$^1$H NMR (300 MHz, $CDCl_3$-$d_1$) δ 8.28 (dd, *J* = 7.5, 2.0 Hz, 1H), 8.17 (d, *J* = 1.9 Hz, 1H), 7.75 (d, *J* = 7.5 Hz, 1H), 7.64 (dd, *J* = 5.7, 3.8 Hz, 1H), 7.40 (dd, *J* = 5.6, 3.9 Hz, 2H), 7.28 (dd, *J* = 5.6, 3.9 Hz, 1H), 5.15 (s, 1H), 3.68 (s, 3H), 3.28 (s, 2H), 2.53 (d, *J* = 12.4 Hz, 1H), 2.40 (d, *J* = 12.4 Hz, 1H), 2.24 (d, *J* = 1.5 Hz, 2H), 1.79 (s, 2H), 1.03 (s, 3H); $^{13}$C NMR (75 MHz, $CDCl_3$-$d_1$) δ 173.90, 160.03, 148.47, 145.70, 144.82, 143.03, 142.03, 129.44, 128.90, 128.71, 124.87, 124.15, 122.86, 122.44, 120.52, 115.82, 54.33, 51.46, 33.02, 32.86, 27.67, 27.60, 14.09; LC-MS (ESI) m/z: 484.5 $[M+H]^+$.

Example 17: methyl (*S*)-3-(8-chloro-6-(2-fluorophenyl)-1-((2-hydroxyethyl)thio)-4*H*-benzo[*f*][1,2,4]triazolo[4,3-a][1,4]diazepin-4-yl)propionate (compound 17)

$^1$H NMR (300 MHz, $CDCl_3$-$d_1$) δ: 7.78-7.61 (m, 3H), 7.48 (q, *J* = 7.3 Hz, 1H), 7.34-7.24 (m, 2H), 7.05 (t, *J* = 10.0 Hz, 1H), 4.25-4.13 (m, 2H), 3.99 (q, *J*=5.6 Hz, 2H), 3.68 (s, 3H), 3.49-3.35 (m, 2H), 2.90-2.72 (m, 4H); $^{13}$C NMR (75 MHz, $CDCl_3$-$d_1$) δ: 173.81, 163.79, 162.01, 158.67, 157.51, 150.84, 133.95, 132.78, 131.69, 131.27, 130.17, 129.77, 126.92, 125.06, 124.58, 116.50, 61.90, 54.99, 51.66, 36.08, 30.13, 26.46; LC-MS (ESI) m/z: 475.1 $[M+H]^+$.

Example 18: methyl (*S*)-3-(8-bromo-6-(2-fluorophenyl)-1-((2-hydroxyethyl)thio)-4*H*-benzo[f][1,2,4]triazolo[4,3-a][1,4]diazepin-4-yl)propionate (compound 18)

$^1$H NMR (300 MHz, $CDCl_3$-$d_1$) δ 7.67 (dd, *J* = 7.0, 2.4 Hz, 1H), 7.62 - 7.46 (m, 2H), 7.47-7.29 (m, 2H), 7.21 (dd, *J* = 6.9, 2.5 Hz, 1H), 7.11 (d, *J* = 7.5 Hz, 1H), 5.15 (s, 1H), 4.58 (t, *J* = 5.5 Hz, 1H), 3.71-3.49 (m, 6H), 3.43 (d, *J* = 12.4 Hz, 1H), 2.50 (d, *J* = 12.4 Hz, 1H), 2.39 (d, *J* = 12.4 Hz, 1H), 2.24 (d, *J* = 1.6 Hz, 2H); $^{13}$C NMR (75 MHz, $CDCl_3$-$d_1$) δ 173.78, 160.03, 147.02, 145.12, 140.80, 138.02, 131.75, 129.84, 129.65, 129.44, 127.05, 124.20, 122.44, 121.80, 116.44, 115.82, 61.40, 54.38, 51.45, 34.93, 32.03, 27.60; LC-MS (ESI) m/z: 520.4 $[M+H]^+$.

Example 19: methyl (*S*)-3-(8-nitro-6-(2-fluorophenyl)-1-((2-hydroxyethyl)thio)-4*H*-benzo[*f*][1,2,4]triazolo[4,3-*a*][1,4]diazepin-4-yl)propionate (compound 19)

$^1$H NMR (300 MHz, $CDCl_3$-$d_1$) δ 8.28 (dd, *J* = 7.5, 2.0 Hz, 1H), 8.17 (d, *J* = 2.0 Hz, 1H), 7.75 (d, *J* = 7.5 Hz, 1H), 7.64 (dd, *J* = 5.7, 3.8 Hz, 1H), 7.40 (dd, *J* = 5.6, 3.9 Hz, 2H), 7.41-7.23 (m, 1H), 5.15 (s, 1H), 4.27 (t, *J* = 5.5 Hz, 1H), 3.71-3.54 (m, 6H), 3.51 (d, *J* = 12.4 Hz, 1H), 2.53 (d, *J* = 12.4 Hz, 1H), 2.40 (d, *J* = 12.4 Hz, 1H), 2.24 (d, *J* = 1.6 Hz, 2H); $^{13}$C NMR (75 MHz, $CDCl_3$-$d_1$) δ 173.90, 160.03, 148.47, 145.70, 144.82, 143.03, 142.03, 129.44, 128.90, 128.71, 124.87, 124.15, 122.86, 122.44, 121.02, 115.82, 61.63, 54.33, 51.46, 35.01, 33.02, 27.60; LC-MS (ESI) m/z: 486.5 $[M+H]^+$.

Example 20: methyl (*S*)-3-(8-chloro-6-(2-fluorophenyl)-1-((2-aminoethyl)thio)-4*H*-benzo[*f*][1,2,4]triazolo[4,3-a][1,4]diazepin-4-yl)propionate (compound 20)

$^1$H NMR (300 MHz, $CDCl_3$-$d_1$) δ: 7.78-7.61 (m, 3H), 7.48 (q, *J* = 5.4Hz, 1H), 7.34-7.24 (m, 2H), 7.05 (t, *J* = 9.0Hz, 1H), 4.38-4.21 (m, 3H), 3.68 (s, 3H), 3.46-3.22 (m, 4H), 2.90-2.72 (m, 4H); $^{13}$C NMR (75 MHz, $CDCl_3$-$d_1$) δ: 173.84, 163.79, 162.00, 158.65, 157.61, 150.05, 133.93, 132.74, 131.74, 131.24, 130.09, 129.69, 126.95, 125.11, 124.56, 116.48, 54.98, 51.65, 40.72, 31.58, 30.15, 26.45; LC-MS (ESI) m/z: 474.0 $[M+H]^+$.

Example 21: methyl (*S*)-3-(8-bromo-6-(2-fluorophenyl)-1-((2-aminoethyl)thio)-4*H*-benzo[f][1,2,4]triazolo[4,3-*a*][1,4]diazepin-4-yl)propionate (compound 21)

$^1$H NMR (300 MHz, $CDCl_3$-$d_1$) δ 7.67 (dd, *J* = 7.0, 2.5 Hz, 1H), 7.62-7.46 (m, 2H), 7.47-7.29 (m, 2H), 7.21 (dd, *J* = 6.9, 2.5 Hz, 1H), 7.04 (d, *J* = 7.5 Hz, 1H), 5.15 (s, 1H), 4.49 (d, *J* = 0.7 Hz, 1H), 4.37 (d, *J* = 0.7 Hz, 1H), 3.71-3.51 (m, 5H), 2.95 (s, 2H), 2.50 (d, *J* = 12.4 Hz, 1H), 2.39 (d, *J* = 12.4 Hz, 1H), 2.24 (d, *J* = 1.5 Hz, 2H); $^{13}$C NMR (75 MHz, $CDCl_3$-$d_1$) δ 173.78, 160.03, 147.02, 145.12, 140.80, 138.02, 131.75, 129.84, 129.69, 129.44, 127.05, 124.20, 122.44, 121.80, 116.44, 115.82, 54.38, 51.45, 39.99, 34.13, 32.03, 27.60; LC-MS (ESI) m/z: 519.4 $[M+H]^+$.

Example 22: methyl (*S*)-3-(8-nitro-6-(2-fluorophenyl)-1-((2-aminoethyl)thio)-4*H*-benzo[*f*][1,2,4]triazolo[4,3-*a*][1,4]diazepin-4-yl)propionate (compound 22)

$^1$H NMR (300 MHz, $CDCl_3$-$d_1$) δ 8.29 (dd, *J* = 7.5, 2.0 Hz, 1H), 8.17 (d, *J* = 2.0 Hz, 1H), 7.75 (d, *J* = 7.5 Hz, 1H), 7.64 (dd, *J* = 5.7, 3.8 Hz, 1H), 7.40 (dd, *J* = 5.6, 3.9 Hz, 2H), 7.41-7.23 (m, 1H), 5.15 (s, 1H), 4.49 (d, *J* = 0.7 Hz, 1H), 4.37 (d, *J* = 0.7 Hz, 1H), 3.65 (d, *J* = 17.9 Hz, 5H), 3.00-2.82 (m, 2H), 2.53 (d, *J* = 12.4 Hz, 1H), 2.40 (d, *J* = 12.4 Hz, 1H), 2.24 (d, *J* = 1.6 Hz, 2H); $^{13}$C NMR (75 MHz, $CDCl_3$-$d_1$) δ 173.90, 160.03, 148.47, 145.70, 144.82, 143.03, 142.03, 129.44, 128.90, 128.71, 124.87, 124.15, 122.86, 122.44, 120.52, 115.82, 54.33, 51.46, 39.99, 34.37, 33.02, 27.60; LC-MS (ESI) m/z: 485.5 $[M+H]^+$.

Example 23: methyl (*S*)-3-(8-chloro-6-(2-chlorophenyl)-1-(methylthio)-4*H*-benzo[*f*][1,2,4]triazolo[4,3-*a*][1,4]diazepin-4-yl)propionate (compound 23)

$^1$H NMR (300 MHz, $CDCl_3$-$d_1$) δ: 7.74 (d, *J* = 8.6 Hz, 1H), 7.64-7.54 (m, 2H), 7.43-7.33 (m, 3H), 7.19 (s, 1H), 4.28 (t, *J* = 6.4 Hz, 1H), 3.68 (s, 3H), 2.90-2.76 (m, 7H); $^{13}$C NMR (75 MHz, $CDCl_3$-$d_1$) δ: 173.83, 166.45, 157.49, 151.12, 138.02, 133.68, 132.84, 131.57, 131.35, 131.30, 131.00, 130.94, 130.25, 129.49, 127.19, 124.62, 55.10, 51.63, 30.18, 26.50, 15.25; LC-MS (ESI) m/z: 461.1 $[M+H]^+$.

Example 24: methyl (*S*)-3-(8-bromo-6-(2-chlorophenyl)-1-(methylthio)-4*H*-benzo[*f*][1,2,4]triazolo[4,3-*a*][1,4]diazepin-4-yl)propionate (compound 24)

$^1$H NMR (300 MHz, $CDCl_3$-$d_1$) δ 7.66 (dd, *J* = 7.4, 1.9 Hz, 1H), 7.62-7.44 (m, 3H), 7.51-7.27 (m, 3H), 7.34- 7.24 (m, 1H), 5.32 (s, 1H), 3.68 (s, 3H), 2.53 (d, *J* = 28.9 Hz, 4H), 2.45- 2.26 (m, 2H), 2.25 (d, *J* = 12.4 Hz, 1H); $^{13}$C NMR

(75 MHz, $CDCl_3$-*$d_1$*) δ 173.78, 150.29, 149.26, 147.02, 140.80, 133.13, 132.57, 131.75, 130.86, 129.62, 128.97, 128.62, 125.51, 124.07, 118.85, 118.19, 54.38, 51.46, 32.25, 27.63, 14.05; LC-MS (ESI) m/z: 506.8 $[M+H]^+$.

Example 25: methyl (*S*)-3-(8-nitro-6-(2-chlorophenyl)-1-(methylthio)-4*H*-benzo[*f*][1,2,4]triazolo[4,3-*a*][1,4]diazepin-4-yl)propionate (compound 25)

$^1H$ NMR (300 MHz, $CDCl_3$-*$d_1$*) δ 8.33 (dd, *J* = 7.5, 2.0 Hz, 1H), 8.17 (d, *J* = 2.1 Hz, 1H), 7.68-7.54 (m, 2H), 7.54-7.31 (m, 3H), 5.22 (s, 1H), 3.68 (s, 3H), 2.56 (s, 3H), 2.53 (d, *J* = 12.4 Hz, 1H), 2.40 (d, *J* = 12.4 Hz, 1H), 2.35-2.17 (m, 2H); $^{13}C$ NMR (75 MHz, $CDCl_3$-*$d_1$*) δ 173.78, 150.96, 150.29, 148.47, 143.37, 141.28, 134.20, 130.98, 130.35, 129.35, 128.71, 128.62, 124.17, 123.83, 122.86, 121.02, 54.38, 51.43, 32.25, 27.60, 14.05; LC-MS (ESI) m/z: 472.9 $[M+H]^+$.

Example 26: methyl (*S*)-3-(8-chloro-6-(2-chlorophenyl)-1-(ethylthio)-4*H*-benzo[*f*][1,2,4]triazolo[4,3-*a*][1,4]diazepin-4-yl)propionate (compound 26)

$^1H$ NMR (300 MHz, $CDCl_3$-*$d_1$*) δ: 7.74 (d, *J* = 8.5 Hz, 1H), 7.64-7.54 (m, 2H), 7.43-7.33 (m, 3H), 7.19 (s, 1H), 4.28 (t, *J* = 6.2 Hz, 1H), 3.68 (s, 3H), 3.34 (q, *J* = 7.6 Hz, 2H), 2.90-2.72 (m, 4H), 1.42 (t, *J* = 7.1 Hz, 3H); $^{13}C$ NMR (75 MHz, $CDCl_3$-*$d_1$*) δ: 173.83, 166.46, 157.23, 150.27, 138.05, 133.63, 132.82, 131.46, 131.32, 131.30, 130.98, 130.95, 130.21, 129.36, 127.19, 125.04, 55.14, 51.63, 30.20, 27.68, 26.49, 14.77; LC-MS (ESI) m/z: 475.1$[M+H]^+$.

Example 27: methyl (*S*)-3-(8-bromo-6-(2-chlorophenyl)-1-(ethylthio)-4*H*-benzo[*f*][1,2,4]triazolo[4,3-*a*][1,4]diazepin-4-yl)propionate (compound 27)

$^1H$ NMR (300 MHz, $CDCl_3$-*$d_1$*) δ 7.71-7.61 (m, 1H), 7.60 (d, *J* = 2.0 Hz, 1H), 7.57-7.25 (m, 6H), 5.32 (s, 1H), 3.68 (s, 3H), 3.18 (s, 2H), 2.50 (d, *J* = 12.4 Hz, 1H), 2.45-2.26 (m, 2H), 2.25 (d, *J* = 12.4 Hz, 1H), 1.39 (s, 3H); $^{13}C$ NMR (75 MHz, $CDCl_3$-*$d_1$*) δ 173.78, 149.26, 147.02, 144.40, 140.17, 133.47, 132.57, 131.75, 130.98, 129.65, 128.97, 128.62, 127.05, 124.07, 122.26, 116.44, 54.38, 51.48, 32.03, 29.80, 28.40, 14.23; LC-MS (ESI) m/z: 520.8$[M+H]^+$.

Example 28: methyl (*S*)-3-(8-nitro-6-(2-chlorophenyl)-1-(ethylthio)-4*H*-benzof[1,2,4]triazolo[4,3-*a*][1,4]diazepin-4-yl)propionate (compound 28)

$^1H$ NMR (300 MHz, $CDCl_3$-*$d_1$*) δ 8.30 (dd, *J* = 7.5, 2.0 Hz, 1H), 8.19 (d, *J* = 2.0 Hz, 1H), 7.75 (d, *J* = 7.5 Hz, 1H), 7.61 (dd, *J* = 7.4, 2.0 Hz, 1H), 7.54-7.31 (m, 3H), 5.22 (s, 1H), 3.68 (s, 3H), 3.18 (s, 2H), 2.53 (d, *J* = 12.4 Hz, 1H), 2.40 (d, *J* = 12.4 Hz, 1H), 2.24 (d, *J* = 1.5 Hz, 2H), 1.40 (s, 3H); $^{13}C$ NMR (75 MHz, $CDCl_3$-*$d_1$*) δ 173.90, 150.96, 147.02, 144.00, 143.37, 141.28, 133.92, 130.98, 130.35, 129.31, 128.71, 128.54, 124.07, 123.83, 122.86, 121.02, 54.38, 51.46, 32.25, 28.40, 27.60, 14.23; LC-MS (ESI) m/z: 486.9$[M+H]^+$.

Example 29: methyl (*S*)-3-(8-chloro-6-(2-chlorophenyl)-1-(propylthio)-4*H*-benzo[*f*][1,2,4]triazolo[4,3-*a*][1,4]diazepin-4-yl)propionate (compound 29)

$^1H$ NMR (300 MHz, $CDCl_3$-*$d_1$*) δ: 7.76-7.19 (m, 7H), 4.28 (t, *J*= 5.7 Hz, 1H), 3.68 (s, 3H), 3.30 (t, *J*=13.8Hz, 2H), 2.90-2.72 (m, 4H), 1.81 (q, *J* = 6.7 Hz, 2H), 1.04 (t, *J* = 6.9 Hz, 3H); $^{13}C$ NMR (75 MHz, $CDCl_3$-*$d_1$*) δ: 173.85, 166.46, 157.22, 150.50, 138.06, 133.61, 132.84, 131.47, 131.35, 131.29, 130.99, 130.95, 130.22, 129.35, 127.18, 125.09, 55.19, 51.64, 35.17, 30.23, 26.52, 22.73,13.23; LC-MS (ESI) m/z: 488.9 $[M+H]^+$.

Example 30: methyl (*S*)-3-(8-bromo-6-(2-chlorophenyl)-1-(propylthio)-4*H*-benzo[*f*][1,2,4]triazolo[4,3-*a*][1,4]diazepin-4-yl)propionate (compound 30)

$^1H$ NMR (300 MHz, $CDCl_3$-*$d_1$*) δ 7.99-7.88 (m, 2H), 7.79 (dd, *J* = 8.4, 2.5 Hz, 1H), 7.59-7.49 (m, 1H), 7.51-7.39 (m, 2H), 7.41-7.27 (m, 1H), 6.06-5.90 (m, 1H), 3.62 (s, 2H), 3.32 (dt, *J* = 14.5, 6.4 Hz, 1H), 3.17 (dt, *J* = 14.5, 6.4 Hz, 1H), 2.79-2.62 (m, 1H), 2.58-2.40 (m, 2H), 2.44-2.24 (m, 1H), 1.93-1.58 (m, 2H), 1.02 (t, *J* = 7.6 Hz, 3H); $^{13}C$ NMR (75 MHz, $CDCl_3$-*$d_1$*) δ 173.45, 162.53, 154.57, 153.88, 138.65, 136.35, 134.47, 133.31, 132.86, 130.41, 129.64, 129.59, 128.37, 126.63, 122.44, 119.72, 55.11, 51.90, 34.69, 29.81, 29.59, 22.63, 13.16; LC-MS (ESI) m/z: 532.03 $[M+H]^+$.

Example 31: methyl (*S*)-3-(8-nitro-6-(2-chlorophenyl)-1-(propylthio)-4*H*-benzo[*f*][1,2,4]triazolo[4,3-*a*][1,4]diazepin-4-yl)propionate (compound 31)

$^1H$ NMR (300 MHz, $CDCl_3$-*$d_1$*) δ 8.46 (dd, *J* = 8.5, 2.1 Hz, 1H), 8.36 (d, *J* = 2.1 Hz, 1H), 8.25 (d, *J* = 8.5 Hz,

1H), 7.57 (dd, *J* = 7.8, 1.5 Hz, 1H), 7.54-7.36 (m, 2H), 7.35 (td, *J* = 7.6, 1.5 Hz, 1H), 5.12-4.94 (m, 1H), 3.61 (s, 2H), 3.38-3.13 (m, 2H), 2.70-2.46 (m, 3H), 2.35-2.17 (m, 1H), 1.92-1.58 (m, 2H), 1.03 (t, *J*= 7.6 Hz, 3H); $^{13}$C NMR (75 MHz, $CDCl_3$-*$d_1$*) δ 173.50, 162.06, 154.57, 153.88, 146.01, 138.70, 137.82, 134.63, 130.41, 129.65, 128.42, 128.36, 128.20, 126.67, 125.98, 123.39, 55.11, 51.90, 34.69, 29.70, 29.62, 22.63, 13.16; LC-MS (ESI) m/z: 499.11 $[M+H]^+$.

Example 32: methyl (*S*)-3-(8-chloro-6-(2-chlorophenyl)-1-((2-hydroxyethyl)thio)-4*H*-benzo[*f*][1,2,4]triazolo[4,3-*a*][1,4]diazepin-4-yl)propionate (compound 32)

$^1$H NMR (300 MHz, $CDCl_3$-*$d_1$*) δ: 7.75 (d, *J* = 8.6 Hz, 1H), 7.64-7.55 (m, 2H), 7.45-7.33 (m, 3H), 7.19 (s, 1H), 4.28 (t, *J*=5.3 Hz, 1H), 4.11-3.96 (m, 3H), 3.68 (s, 3H), 3.51-3.42 (m, 2H), 2.90-2.72 (m, 4H); $^{13}$C NMR (75 MHz, $CDCl_3$-*$d_1$*) δ: 173.79, 166.57, 157.42, 150.92, 137.91, 133.98, 132.83, 131.65, 131.39, 131.29, 130.98, 130.71, 130.27, 129.48, 127.22, 125.07, 62.20, 55.08, 51.68, 36.05, 30.15, 26.42; LC-MS (ESI) m/z: 491.4 $[M+H]^+$.

Example 33: methyl (*S*)-3-(8-bromo-6-(2-chlorophenyl)-1-((2-hydroxyethyl)thio)-4*H*-benzo[*f*][1,2,4]triazolo[4,3-*a*][1,4]diazepin-4-yl)propionate (compound 33)

$^1$H NMR (300 MHz, $CDCl_3$-*$d_1$*) δ 7.99-7.88 (m, 2H), 7.81 (dd, *J* = 8.4, 2.5 Hz, 1H), 7.59-7.48 (m, 1H), 7.51-7.39 (m, 2H), 7.39-7.27 (m, 1H), 6.07-5.90 (m, 1H), 4.40 (t, *J* = 7.2 Hz, 1H), 3.75 (q, *J* = 7.0 Hz, 2H), 3.62 (s, 3H), 3.67 - 3.51 (m, 1H), 3.39 (dt, *J*= 15.0, 6.8 Hz, 1H), 2.78-2.60 (m, 1H), 2.60-2.38 (m, 2H), 2.41-2.22 (m, 1H); $^{13}$C NMR (75 MHz, $CDCl_3$-*$d_1$*) δ 173.42, 162.52, 154.54, 153.87, 138.65, 136.35, 134.89, 133.44, 132.85, 130.33, 129.69, 129.62, 128.28, 126.66, 122.43, 119.94, 60.25, 55.10, 51.89, 35.35, 29.70, 29.59; LC-MS (ESI) m/z: 534.01 $[M+H]^+$.

Example 34: methyl (S)-3-(8-nitro-6-(2-chlorophenyl)-1-((2-hydroxyethyl)thio)-4H-benzo[*f*][1,2,4]triazolo[4,3-*a*][1,4]diazepin-4-yl)propionate (compound 34)

$^1$H NMR (300 MHz, $CDCl_3$-*$d_1$*) δ 8.49-8.35 (m, 2H), 8.26 (d, *J* = 8.4 Hz, 1H), 7.57 (dd, *J* = 7.8, 1.4 Hz, 1H), 7.54-7.38 (m, 2H), 7.34 (td, *J* = 7.5, 1.5 Hz, 1H), 5.09-4.93 (m, 1H), 4.40 (t, *J*= 7.2 Hz, 1H), 3.82-3.63 (m, 2H), 3.61 (s, 3H), 3.68-3.40 (m, 2H), 2.77-2.60 (m, 1H), 2.61-2.42 (m, 2H), 2.35-2.16 (m, 1H); $^{13}$C NMR (75 MHz, $CDCl_3$-*$d_1$*) δ 173.40, 162.06, 154.59, 153.87, 146.02, 138.70, 137.80, 134.47, 130.33, 129.69, 128.29, 128.18, 127.85, 126.71, 125.90, 123.39, 60.27, 55.10, 51.90, 35.34, 29.65, 29.60; LC-MS (ESI) m/z: 501.09 $[M+H]^+$.

Example 35: methyl (*S*)-3-(8-chloro-6-(2-chlorophenyl)-1-((2-aminoethyl)thio)-4*H*-benzo[*f*][1,2,4]triazolo[4,3-*a*][1,4]diazepin-4-yl)propionate (compound 35)

$^1$H NMR (300 MHz, $CDCl_3$-*$d_1$*) δ: 7.79 (d, *J* = 8.7 Hz, 1H), 7.62-7.51 (m, 2H), 7.41-7.31 (m, 3H), 7.15 (s, 1H), 6.46 (s, 2H), 4.24 (t, *J* = 7.8 Hz, 1H), 3.77-3.52 (m, 7H), 2.82-2.73 (m, 4H); $^{13}$C NMR (75 MHz, $CDCl_3$-*$d_1$*) δ: 173.80, 166.72, 157.62, 150.17, 137.84, 134.11, 132.82, 131.95, 131.37, 131.23, 130.93, 130.39, 130.27, 129.39, 127.18, 125.15, 54.93, 51.71, 40.16, 31.60, 30.15, 26.31; LC-MS (ESI) m/z: 490.2 $[M+H]^+$.

Example 36: methyl (*S*)-3-(8-bromo-6-(2-chlorophenyl)-1-((2-aminoethyl)thio)-4*H*-benzo[*f*][1,2,4]triazolo[4,3-*a*][1,4]diazepin-4-yl)propionate (compound 36)

$^1$H NMR (300 MHz, $CDCl_3$-*$d_1$*) δ 7.98-7.88 (m, 2H), 7.81 (dd, *J* = 8.3, 2.5 Hz, 1H), 7.59-7.49 (m, 1H), 7.51-7.39 (m, 2H), 7.39-7.27 (m, 1H), 6.06-5.89 (m, 1H), 3.74 (t, *J* = 6.1 Hz, 2H), 3.62 (s, 2H), 3.48 (dt, *J* = 15.6, 5.8 Hz, 1H), 3.34 (dt, *J* = 15.6, 5.9 Hz, 1H), 3.18-2.88 (m, 2H), 2.79-2.61 (m, 1H), 2.59-2.37 (m, 2H), 2.41-2.22 (m, 1H); $^{13}$C NMR (75 MHz, $CDCl_3$-*$d_1$*) δ 173.42, 162.53, 154.45, 153.86, 138.65, 136.35, 134.80, 133.38, 132.85, 130.33, 129.69, 129.62, 128.28, 126.66, 122.43, 119.94, 55.10, 51.89, 40.95, 35.18, 29.70, 29.59; LC-MS (ESI) m/z: 533.03 $[M+H]^+$.

Example 37: methyl (*S*)-3-(8-nitro-6-(2-chlorophenyl)-1-((2-aminoethyl)thio)-4*H*-benzo[*f*][1,2,4]triazolo[4,3-*a*][1,4]diazepin-4-yl) propionate (compound 37)

$^1$H NMR (300 MHz, $CDCl_3$-*$d_1$*) δ 8.47 (dd, *J* = 8.5, 2.1 Hz, 1H), 8.37 (d, *J*= 2.1 Hz, 1H), 8.27 (d, *J*= 8.4 Hz, 1H), 7.56 (dd, *J* = 7.7, 1.5 Hz, 1H), 7.56-7.38 (m, 2H), 7.34 (td, *J* = 7.5, 1.5 Hz, 1H), 5.08-4.90 (m, 1H), 3.74 (t, *J* = 6.1 Hz, 2H), 3.64-3.33 (m, 4H), 3.14 (dp, *J* = 14.6, 6.0 Hz, 1H), 2.98 (dp, *J* = 14.6, 5.9 Hz, 1H), 2.74-2.57 (m, 1H), 2.62-2.42 (m, 2H), 2.26 (dtd, *J* = 11.0, 8.4, 7.7 Hz, 1H); $^{13}$C NMR (75 MHz, $CDCl_3$-*$d_1$*) δ 173.39, 162.06, 154.50, 153.88, 146.02, 138.70, 137.80, 134.48, 130.33, 129.69, 128.29, 128.17, 127.79, 126.71, 125.90, 123.39, 55.10, 51.90, 40.95, 35.19, 29.65, 29.60; LC-MS (ESI) m/z: 500.10 $[M+H]^+$.

Example 38: methyl (*S*)-3-(8-chloro-6-(2-chlorophenyl)-1-(acetylthio)-4*H*-benzo[*f*][1,2,4]triazolo[4,3-a1,4]diazepin-4-yl)propionate (compound 38)

$^1$H NMR (300 MHz, $CDCl_3$-$d_1$) δ: 8.49 (d, *J* = 8.8 Hz, 1H), 7.64 (d, *J* = 9.5 Hz, 1H), 7.51-7.39 (m, 4H), 7.15 (d, *J* = 3.2 Hz, 1H), 4.28 (t, *J* = 5.8 Hz, 1H), 3.68 (s, 3H), 2.80-2.62 (m, 4H), 2.30 (s, 3H); $^{13}$C NMR (75 MHz, $CDCl_3$-$d_1$) δ: 173.46, 168.18, 167.82, 166.20, 151.61, 141.72, 137.59, 134.00, 133.16, 132.08, 131.51, 131.07, 130.85, 130.49, 129.00, 127.37, 127.07, 54.77, 51.81, 29.81, 25.73, 20.63; LC-MS (ESI) m/z: 489.4 $[M+H]^+$.

Example 39: methyl (*S*)-3-(8-bromo-6-(2-chlorophenyl)-1-(acetylthio)-4*H*-benzo[*f*][1,2,4]triazolo[4,3-*a*][1,4]diazepin-4-yl)propionate (compound 39)

$^1$H NMR (300 MHz, $CDCl_3$-$d_1$) δ 8.10 (d, *J*= 8.3 Hz, 1H), 7.96 (d, *J* = 2.5 Hz, 1H), 7.79 (dd, *J* = 8.4, 2.5 Hz, 1H), 7.57 - 7.27 (m, 4H), 6.06-5.93 (m, 1H), 3.62 (s, 3H), 2.71-2.44 (m, 3H), 2.45-2.22 (m, 4H); $^{13}$C NMR (75 MHz, $CDCl_3$-$d_1$) δ 185.25, 173.42, 162.53, 154.37, 148.67, 138.65, 136.35, 134.79, 133.71, 132.84, 130.33, 129.63, 129.62, 128.28, 126.66, 122.42, 119.94, 55.10, 51.89, 30.14, 29.65, 29.59; LC-MS (ESI) m/z: 532.00 $[M+H]^+$.

Example 40: methyl (*S*)3-(8-nitro-6-(2-chlorophenyl)-1-(acetylthio)-4*H*-benzo*f*[1,2,4]triazolo[4,3-*a*][1,4]diazepin-4-yl)propionate (compound 40)

$^1$H NMR (300 MHz, $CDCl_3$-$d_1$) δ 8.54-8.36 (m, 2H), 8.34 (d, *J*= 2.0 Hz, 1H), 7.55 (ddd, *J* = 7.7, 6.0, 1.8 Hz, 2H), 7.53-7.32 (m, 2H), 5.99 (ddd, *J* = 8.2, 7.4, 0.8 Hz, 1H), 3.61 (s, 2H), 2.71-2.40 (m, 3H), 2.42 (s, 3H), 2.44-2.25 (m, 1H); $^{13}$C NMR (75 MHz, $CDCl_3$-$d_1$) δ 185.25, 173.39, 162.06, 154.37, 148.19, 146.02, 138.70, 138.07, 134.47, 130.33, 129.69, 128.29, 128.21, 128.17, 126.71, 125.90, 123.40, 55.10, 51.90, 30.14, 29.65, 29.60; LC-MS (ESI) m/z: 499.07 $[M+H]^+$.

Example 41: ethyl (*S*)-3-(8-chloro-6-(2-chlorophenyl)-1-mercapto-4*H*-benzo[*f*][1,2,4]triazolo[4,3-*a*][1,4]diazepin-4-yl)propionate (compound 41)

$^1$H NMR (300 MHz, $CDCl_3$-$d_1$) δ: 12.39 (s, 1H), 8.52 (d, *J* = 8.7 Hz, 1H), 7.62 (d, *J* = 8.6 Hz, 1H), 7.64-7.38 (m, 4H), 7.14 (s, 1H), 4.28 (t, *J* = 6.3 Hz, 1H), 4.14 (q, *J* = 7.0 Hz, 2H), 2.76-2.57 (m, 4H), 1.23 (t, *J* = 7.1 Hz, 3H); $^{13}$C NMR (75 MHz, $CDCl_3$-$d_1$) δ: 173.32, 168.00, 167.13, 153.25, 137.67, 133.88, 133.07, 131.81, 131.39, 131.17, 130.91, 130.82, 130.40, 129.14, 127.01, 60.70, 54.92, 30.18, 25.84, 14.19; LC-MS (ESI) m/z: 461.0 $[M+H]^+$.

Example 42: ethyl (*S*)-3-(8-chloro-6-(2-chlorophenyl)-1-(methylthio)-4*H*-benzo[*f*][1,2,4]triazolo[4,3-a][1,4]diazepin-4-yl)propionate (compound 42)

$^1$H NMR (300 MHz, $CDCl_3$-$d_1$) δ: 7.75 (d, *J*= 8.5 Hz, 1H), 7.64-7.56 (m, 2H), 7.43-7.31 (m, 3H),7.19 (s, 1H), 4.28 (t, *J* = 5.6 Hz, 1H), 4.14 (q, *J* = 7.0 Hz, 2H), 2.90-2.76 (m, 7H) 1.24 (t, *J* = 6.6 Hz, 3H); $^{13}$C NMR (75 MHz, $CDCl_3$-$d_1$) δ: 173.44, 166.45, 157.55, 151.14, 138.05, 133.70, 132.88, 131.60, 131.38, 131.02, 130.28, 129.51, 127.22, 124.63, 60.43, 55.14, 30.46, 26.54, 15.27, 14.24; LC-MS (ESI) m/z: 475.1 $[M+H]^+$.

Example 43: ethyl (*S*)-3-(8-bromo-6-(2-chlorophenyl)-1-(methylthio)-4*H*-benzo[*f*][1,2,4]triazolo[4,3-*a*][1,4]diazepin-4-yl)propionate (compound 43)

$^1$H NMR (300 MHz, $CDCl_3$-$d_1$) δ 7.99-7.87 (m, 2H), 7.80 (dd, *J*= 8.4, 2.5 Hz, 1H), 7.53 (dd, *J*= 8.0, 1.6 Hz, 1H), 7.51-7.39 (m, 2H), 7.39-7.27 (m, 1H), 5.02 (ddd, *J*= 8.3, 7.6, 0.7 Hz, 1H), 4.21-3.97 (m, 2H), 2.64 (s, 3H), 2.73 - 2.26 (m, 4H), 1.18 (t, *J* = 6.9 Hz, 3H); $^{13}$C NMR (75 MHz, $CDCl_3$-$d_1$) δ 172.72, 162.53, 154.47, 153.45, 138.65, 136.35, 134.56, 133.15, 132.85, 130.33, 129.69, 129.62, 128.28, 126.67, 122.44, 119.73, 60.67, 55.10, 30.27, 29.68, 16.53, 14.18; LC-MS (ESI) m/z: 518.02 $[M+H]^+$.

Example 44: ethyl (*S*)-3-(8-nitro-6-(2-chlorophenyl)-1-(methylthio)-4*H*-benzo[*f*][1,2,4]triazolo[4,3-*a*][1,4]diazepin-4-yl)propionate (compound 44)

$^1$H NMR (300 MHz, $CDCl_3$-$d_1$) δ 8.52-8.35 (m, 2H), 8.25 (d, *J*= 8.4 Hz, 1H), 7.56 (dd, *J*= 7.7, 1.5 Hz, 1H), 7.53-7.39 (m, 2H), 7.33 (td, *J* = 7.5, 1.5 Hz, 1H), 5.02-4.87 (m, 1H), 4.21-3.97 (m, 2H), 2.75-2.58 (m, 1H), 2.64 (s, 3H), 2.61-2.39 (m, 2H), 2.33-2.15 (m, 1H), 1.18 (t, *J*= 6.9 Hz, 3H); $^{13}$C NMR (75 MHz, $CDCl_3$-$d_1$) δ 172.77, 161.98, 154.47, 153.47, 146.09, 138.70, 137.56, 134.51, 130.33, 129.67, 128.29, 128.17, 127.79, 126.66, 125.92, 123.39, 60.68, 55.10, 30.29, 29.68, 16.53, 14.17; LC-MS (ESI) m/z: 485.09 $[M+H]^+$.

Example 45: methyl (*S*)-3-(8-chloro-1-((cyclopropylmethyl)thio)-6-(2-fluorophenyl)-4*H*-benzo[*f*][1,2,4]triazolo[4,3-α][1,4]diazepin-4-yl)propionate (compound 45)

$^1$H NMR (300 MHz, $CDCl_3$-*d₁*) δ: 7.90 (s, 2H), 7.62 (dt, *J* = 25.3, 7.0 Hz, 2H), 7.37 (d, *J* = 14.2 Hz, 2H), 7.22 (t, *J* = 9.6 Hz, 1H), 4.28 (t, *J*= 6.4 Hz, 1H), 3.62 (s, 3H), 3.07 (d, *J*= 7.2 Hz, 2H), 2.77-2.57 (m, 4H), 1.05 (s, 1H), 0.45 (d, *J*= 7.9 Hz, 2H), 0.24-0.12 (m, 2H); $^{13}$C NMR (75 MHz, $CDCl_3$-$d_1$) δ: 173.57, 163.45, 161.94, 161.87, 161.44, 156.52, 153.90, 134.08, 133.03, 132.44, 132.38, 131.36, 130.59, 130.53, 129.90, 127.85, 127.79, 127.77, 127.69, 124.73, 124.71, 122.90, 116.37, 116.21, 55.18, 51.94, 38.04, 29.44, 29.28, 11.77, 6.27; LC-MS (ESI) m/z: 486.1 $[M+H]^+$.

Example 46: methyl (*S*)-3-(8-bromo-1-((cyclopropylmethyl)thio)-6-(2-fluorophenyl)-4*H*-benzo[*f*][1,2,4]triazolo[4,3-*a*][1,4]diazepin-4-yl)propionate (compound 46)

$^1$H NMR (300 MHz, $CDCl_3$-$d_1$) δ 7.97-7.83 (m, 2H), 7.80 (dd, *J* = 8.4, 2.5 Hz, 1H), 7.53 (dddd, *J* = 15.6, 7.6, 5.0, 1.6 Hz, 2H), 7.28 (dtd, *J* = 16.5, 7.7, 1.4 Hz, 2H), 4.70-4.53 (m, 1H), 3.61 (s, 2H), 3.36-3.21 (m, 1H), 3.09-2.96 (m, 1H), 2.71-2.53 (m, 1H), 2.58-2.42 (m, 2H), 2.36 (dtd, *J* = 10.9, 8.4, 7.6 Hz, 1H), 1.40-1.20 (m, 5H); $^{13}$C NMR (75 MHz, $CDCl_3$-$d_1$) δ 173.43, 163.16, 162.00, 161.89, 159.80, 154.35, 153.86, 136.35, 133.54, 132.91, 131.57, 131.46, 130.46, 130.36, 128.86, 128.82, 126.92, 126.66, 124.26, 124.22, 122.43, 119.98, 115.98, 115.71, 55.26, 51.89, 37.41, 29.65, 29.60, 13.37, 6.76; LC-MS (ESI) m/z: 528.06 $[M+H]^+$.

Example 47: methyl (*S*)-3-(8-nitro-1-((cyclopropylmethyl)thio)-6-(2-fluorophenyl)-4*H*-benzo[*f*][1,2,4]triazolo[4,3-α][1,4]diazepin-4-yl)propionate (compound 47)

$^1$H NMR (300 MHz, $CDCl_3$-$d_1$) δ 8.46 (dd, *J* = 8.5, 2.1 Hz, 1H), 8.35 - 8.19 (m, 2H), 7.63-7.44 (m, 2H), 7.30 (dtd, *J* = 13.2, 7.7, 1.4 Hz, 2H), 4.93 (t, *J* = 8.1 Hz, 1H), 3.61 (s, 2H), 3.42-3.29 (m, 1H), 3.05-2.91 (m, 1H), 2.71-2.50 (m, 2H), 2.55-2.27 (m, 2H), 1.39-1.20 (m, 5H);$^{13}$C NMR (75 MHz, $CDCl_3$-$d_1$) δ 173.39, 163.12, 161.47, 161.36, 159.76, 154.35, 153.88, 146.14, 137.98, 131.60, 131.49, 130.48, 130.37, 128.16, 127.31, 127.25, 127.07, 126.81, 126.00, 124.32, 124.28, 123.39, 115.95, 115.68, 55.26, 51.90, 37.41, 29.65, 29.61, 13.37, 6.76; LC-MS (ESI) m/z: 495.14 $[M+H]^+$.

Example 48: methyl (*S*)-3-(8-chloro-6-(2-fluorophenyl)-1-(prop-2-yn-1-ylthio)-4*H*-benzo[*f*][1,2,4]triazolo[4,3-a][1,4]diazepin-4-yl)propionate (compound 48)

$^1$H NMR (400 MHz, $CDCl_3$-$d_1$) δ: 7.95-7.85 (m, 2H), 7.68-7.53 (m, 2H), 7.42-7.30 (m, 2H), 7.28-7.18 (m, 1H), 4.30 (dd, *J* = 8.0, 5.5 Hz, 1H), 3.98 (qd, *J* = 16.4, 2.6 Hz, 2H), 3.62 (s, 3H), 3.17 (t, *J* = 2.6 Hz, 1H), 2.80-2.54 (m, 4H); $^{13}$C NMR (75 MHz, $CDCl_3$-$d_1$) δ: 173.35, 163.77, 161.57, 161.23, 161.14, 156.64, 153.26, 134.21, 133.16, 132.95, 132.63, 131.36, 130.71, 130.55, 129.34, 127.76, 127.65, 127.58, 127.39, 124.83, 124.53, 123.25, 116.64, 116.33, 79.82, 72.44, 55.37, 51.62, 29.43, 29.30, 20.68; LC-MS (ESI) m/z: 469.2 $[M+H]^+$.

Example 49: methyl (*S*)-3-(8-bromo-6-(2-fluorophenyl)-1-(prop-2-yn-1-ylthio)-4*H*-benzo[*f*][1,2,4]triazolo[4,3-α][1,4]diazepin-4-yl)propionate (compound 49)

$^1$H NMR (300 MHz, $CDCl_3$-$d_1$) δ 7.91 (d, *J* = 8.4 Hz, 1H), 7.88-7.74 (m, 2H), 7.63-7.44 (m, 2H), 7.37-7.19 (m, 2H), 4.90 (t, *J* = 8.0 Hz, 1H), 4.07 (dd, *J*= 12.4, 3.0 Hz, 1H), 3.85 (dd, *J*= 12.4, 3.0 Hz, 1H), 3.62 (s, 2H), 2.71-2.27 (m, 5H); $^{13}$C NMR (75 MHz, $CDCl_3$-$d_1$) δ 173.39, 163.17, 162.00, 161.89, 159.80, 154.01, 153.86, 136.35, 133.51, 132.85, 131.57, 131.46, 130.43, 130.33, 128.86, 128.82, 126.93, 126.66, 124.29, 124.25, 122.43, 119.81, 115.98, 115.71, 79.08, 72.66, 55.26, 51.89, 29.65, 29.59, 20.39; LC-MS (ESI) m/z:512.03 $[M+H]^+$.

Example 50: methyl (*S*)-3-(8-nitro-6-(2-fluorophenyl)-1-(prop-2-yn-1-ylthio)-4*H*-benzo[*f*][1,2,4]triazolo[4,3-*a*][1,4]diazepin-4-yl)propionate (compound 50)

$^1$H NMR (300 MHz, $CDCl_3$-$d_1$) δ 8.48 (dd, *J* = 8.5, 2.1 Hz, 1H), 8.33 (d, *J* = 2.1 Hz, 1H), 8.22 (d, *J* = 8.5 Hz, 1H), 7.63-7.45 (m, 2H), 7.29 (dtd, *J* = 16.4, 7.7, 1.4 Hz, 2H), 4.90 (t, *J* = 8.1 Hz, 1H), 4.08 (dd, *J* = 12.3, 3.0 Hz, 1H), 3.87 (dd, *J* = 12.3, 3.0 Hz, 1H), 3.61 (s, 2H), 2.73-2.26 (m, 5H); $^{13}$C NMR (75 MHz, $CDCl_3$-$d_1$) δ 173.41, 163.13, 161.47, 161.36, 159.77, 154.02, 153.88, 146.14, 137.89, 131.58, 131.46, 130.43, 130.33, 128.17, 127.36, 127.32, 127.07, 126.81, 126.05, 124.33, 124.29, 123.39, 115.98, 115.71, 79.07, 72.67, 55.26, 51.90, 29.65, 29.61, 20.39; LC-MS (ESI) m/z: 479.11 $[M+H]^+$.

Example 51: methyl (*S*)-3-(8-chloro-6-(2-fluorophenyl)-1-((2-morpholinoethyl)thio)-4*H*-benzo*f*[1,2,4]triazolo[4,3-$\alpha$][1,4]diazepin-4-yl)propionate (compound 51)

$^1$H NMR (300 MHz, $CDCl_3$-*d₁*) $\delta$: 7.93 (s, 2H), 7.75-7.52 (m, 2H), 7.45-7.31 (m, 2H), 7.30-7.18 (m, 1H), 4.31 (d, *J*= 5.9 Hz, 1H), 3.63 (s, 3H), 3.55-3.45 (m, 4H), 3.35 (d, *J*= 6.7 Hz, 2H), 2.86-2.55 (m, 6H), 2.36 (s, 4H); $^{13}$C NMR (75 MHz, $CDCl_3$-$d_1$) $\delta$: 173.54, 163.45, 161.75, 161.42, 161.17, 156.34, 153.67, 134.62, 133.22, 132.15, 132.07, 131.46, 130.36, 130.22, 129.77, 127.83, 127.72, 127.68, 127.61, 124.84, 124.71, 122.83, 116.41, 116.33, 66.57, 55.24, 53.94, 53.56, 51.78, 30.26, 29.56, 29.35; LC-MS (ESI) m/z: 547.1 $[M+H]^+$.

Example 52: methyl (*S*)-3-(8-bromo-6-(2-fluorophenyl)-1-((2-morpholinoethyl)thio)-4*H*-benzo[*f*][1,2,4]triazolo[4,3-*a*][1,4]diazepin-4-yl)propionate (compound 52)

$^1$H NMR (300 MHz, $CDCl_3$-$d_1$) $\delta$: 8.06 (dd, *J* = 8.7, 2.2 Hz, 1H), 7.85 (d, *J* = 8.7 Hz, 1H), 7.74-7.47 (m, 3H), 7.37 (t, *J* = 7.5 Hz, 1H), 7.25 (dd, *J* = 10.6, 8.6 Hz, 1H), 4.30 (t, *J* = 6.6 Hz, 1H), 3.64 (s, 3H), 3.53-3.46 (m, 4H), 3.35 (d, *J* = 6.8 Hz, 2H), 2.89-2.56 (m, 6H), 2.36 (s, 4H); $^{13}$C NMR (75 MHz, $CDCl_3$-$d_1$) $\delta$ 173.40, 163.21, 162.00, 161.89, 159.85, 154.56, 153.86, 136.19, 133.51, 132.95, 131.61, 131.50, 130.38, 130.28, 128.98, 128.94, 126.92, 126.66, 124.32, 124.28, 122.44, 119.83, 115.98, 115.71, 65.42, 55.26, 53.50, 52.67, 51.93, 30.41, 29.65, 29.61; LC-MS (ESI) m/z: 591.90 $[M+H]^+$.

Example 53: methyl (*S*)-3-(8-nitro-6-(2-fluorophenyl)-1-((2-morpholinoethyl)thio)-4*H*-benzo[*f*][1,2,4]triazolo[4,3-*a*][1,4]diazepin-4-yl)propionate (compound 53)

$^1$H NMR (300 MHz, $CDCl_3$-$d_1$) $\delta$ 8.52 (dd, *J* = 8.5, 2.1 Hz, 1H), 8.38 (d, *J* = 2.1 Hz, 1H), 8.22 (d, *J* = 8.4 Hz, 1H), 7.64-7.46 (m, 2H), 7.37-7.20 (m, 2H), 6.05-5.91 (m, 1H), 3.66-3.50 (m, 7H), 3.37 (dt, *J* = 14.3, 5.2 Hz, 1H), 2.81 (dt, *J* = 12.0, 5.2 Hz, 1H), 2.71 - 2.40 (m, 6H), 2.42-2.22 (m, 3H). 13C NMR (75 MHz, $CDCl_3$-$d_1$) $\delta$ 173.37, 163.30, 161.48, 161.38, 159.94, 154.57, 153.88, 146.17, 137.95, 131.60, 131.49, 130.54, 130.43, 128.17, 127.60, 127.56, 127.08, 126.81, 125.94, 124.26, 124.22, 123.35, 115.98, 115.71, 65.46, 55.26, 53.55, 52.62, 51.89, 30.41, 29.67, 29.61; LC-MS (ESI) m/z: 555.60 $[M+H]^+$.

Example 54: methyl (*S*)-3-(8-chloro-1-((2-(diethylamino)ethyl)thio)-6-(2-fluorophenyl)-4*H*-benzo[*f*][1,2,4]triazolo[4,3-*a*][1,4]diazepin-4-yl)propionate (compound 54)

$^1$H NMR (300 MHz, $CDCl_3$-$d_1$) $\delta$: 7.90 (d, *J* = 1.8 Hz, 2H), 7.69-7.53 (m, 2H), 7.41-7.31 (m, 2H), 7.29-7.17 (m, 1H), 4.27 (dd, *J* = 7.7, 5.5 Hz, 1H), 3.61 (s, 3H), 3.30-3.22 (m, 2H), 2.80-2.68 (m, 3H), 2.68-2.52 (m, 4H), 2.45 (q, *J* = 7.1 Hz, 6H), 0.90 (t, *J* = 7.1 Hz, 6H); $^{13}$C NMR (75 MHz, $CDCl_3$-$d_1$) $\delta$: 173.49, 163.48, 161.84, 161.77, 161.52, 156.36, 153.37, 134.73, 133.25, 132.58, 132.39, 131.41, 130.67, 130.46, 129.73, 127.74, 127.62, 127.58, 127.39, 124.68, 124.51, 122.76, 116.45, 116.26, 55.22, 51.83, 51.53, 47.82, 30.37, 29.51, 29.33, 11.51; LC-MS (ESI) m/z: 531.6 $[M+H]^+$.

Example 55: methyl (*S*)-3-(8-bromo-1-((2-(diethylamino)ethyl)thio)-6-(2-fluorophenyl)-4*H*-benzo[*f*][1,2,4]triazolo[4,3-*a*][1,4]diazepin-4-yl)propionate (compound 55)

$^1$H NMR (300 MHz, $CDCl_3$-$d_1$) $\delta$ 7.96-7.84 (m, 2H), 7.79 (dd, *J* = 8.4, 2.5 Hz, 1H), 7.53 (dddd, *J*= 16.9, 7.6, 5.0, 1.6 Hz, 2H), 7.28 (dtd, *J* = 16.4, 7.7, 1.4 Hz, 2H), 6.05-5.89 (m, 1H), 3.62 (s, 2H), 3.51 (dt, *J* = 14.1, 5.2 Hz, 1H), 3.35 (dt, *J* = 14.1, 5.2 Hz, 1H), 2.84 (dt, *J* = 12.2, 5.2 Hz, 1H), 2.77-2.60 (m, 2H), 2.66-2.47 (m, 4H), 2.53-2.40 (m, 2H), 2.43-2.24 (m, 1H), 1.01 (t, *J* = 7.2 Hz, 6H); $^{13}$C NMR (75 MHz, $CDCl_3$-$d_1$) $\delta$ 173.41, 163.21, 162.00, 161.89, 159.85, 154.56, 153.86, 136.19, 133.43, 132.95, 131.62, 131.52, 130.53, 130.42, 128.86, 128.82, 126.92, 126.66, 124.26, 124.22, 122.42, 119.83, 115.98, 115.71, 55.26, 51.93, 50.88, 47.11, 30.42, 29.65, 29.61, 11.17; LC-MS (ESI) m/z: 575.51 $[M+H]^+$.

Example 56: methyl (*S*)-3-(8-nitro-1-((2-(diethylamino)ethyl)thio)-6-(2-fluorophenyl)-4*H*-benzo[*f*][1,2,4]triazolo[4,3-*a*][1,4]diazepin-4-yl)propionate (compound 56)

$^1$H NMR (300 MHz, $CDCl_3$-$d_1$) $\delta$ 8.49 (dd, *J* = 8.5, 2.1 Hz, 1H), 8.32 (d, *J* = 2.1 Hz, 1H), 8.21 (d, *J* = 8.5 Hz, 1H), 7.54 (dddd, *J* = 20.4, 7.6, 5.0, 1.7 Hz, 2H), 7.37-7.20 (m, 2H), 4.89 (ddd, *J* = 8.2, 7.5, 0.6 Hz, 1H), 3.62 (s, 2H), 3.47 (dt, *J* = 14.1, 5.2 Hz, 1H), 3.33 (dt, *J* = 14.1, 5.2 Hz, 1H), 2.87 (dt, *J* = 12.1, 5.2 Hz, 1H), 2.79-2.26 (m, 9H), 1.01 (t, *J* = 7.2 Hz, 6H); $^{13}$C NMR (75 MHz, DMSO-$d_6$) $\delta$ 173.42, 163.09, 161.48, 161.38, 159.73, 154.51, 153.88, 146.17, 137.95, 131.60, 131.49, 130.49, 130.38, 128.17, 127.60, 127.56, 127.08, 126.81, 126.00, 124.26, 124.22, 123.37, 115.98, 115.71, 55.26, 51.91, 50.88, 47.11, 30.42, 29.67, 29.61, 11.17; LC-MS (ESI) m/z: 541.61 $[M+H]^+$.

Example 57: methyl (*S*)-3-(8-chloro-1-((3-(dimethylamino)propyl)thio)-6-(2-fluorophenyl)-4*H*-benzo[*f*][1,2,4]triazolo[4,3-*a*][1,4]diazepin-4-yl)propionate (compound 57)

$^1$H NMR (300 MHz, $CDCl_3$-$d_1$) δ: 7.92-7.84 (m, 2H), 7.65 (td, *J*= 7.6, 1.8 Hz, 1H), 7.58 (tdd, *J* = 7.5, 5.2, 1.8 Hz, 1H), 7.42-7.31 (m, 2H), 7.22 (dd, *J* = 10.9, 8.3 Hz, 1H), 4.28 (dd, *J* = 7.7, 5.5 Hz, 1H), 3.61 (s, 3H), 3.24-3.06 (m, 2H), 2.78-2.52 (m, 4H), 2.39 (tt, *J* = 6.9, 4.3 Hz, 2H), 2.18 (s, 6H), 1.79 (q, *J* = 7.1 Hz, 2H); $^{13}$C NMR (75 MHz, $CDCl_3$-$d_1$) δ: 173.72, 163.68, 161.90, 161.82, 161.54, 156.38, 153.73, 134.21, 133.12, 132.51, 132.36, 131.73, 130.66, 130.48, 129.41, 127.88, 127.60, 127.47, 127.39, 124.63, 124.49, 122.88, 116.47, 116.28, 58.73, 55.48, 51.63, 45.28, 31.91, 29.53, 29.33, 26.74; LC-MS (ESI) m/z: 517.14 [M+H]$^+$.

Example 58: methyl (*S*)-3-(8-bromo-1-((3-(dimethylamino)propyl)thio)-6-(2-fluorophenyl)-4*H*-benzo[*f*][1,2,4]triazolo[4,3-*a*][1,4]diazepin-4-yl)propionate (compound 58)

$^1$H NMR (300 MHz, $CDCl_3$-$d_1$) δ 7.97-7.83 (m, 2H), 7.79 (dd, *J* = 8.4, 2.5 Hz, 1H), 7.53 (dddd, *J* = 16.9, 7.6, 4.9, 1.6 Hz, 2H), 7.28 (dtd, *J*= 13.1, 7.7, 1.4 Hz, 2H), 6.06-5.91 (m, 1H), 3.62 (s, 3H), 3.37-3.12 (m, 2H), 2.70-2.53 (m, 2H), 2.58-2.40 (m, 2H), 2.45-2.24 (m, 2H), 2.21 (s, 5H), 1.94 (ddq, *J* = 21.9, 13.0, 6.5 Hz, 2H), $^{13}$C NMR (75 MHz, $CDCl_3$-$d_1$) δ 173.41, 163.21, 162.00, 161.89, 159.85, 154.56, 153.86, 136.19, 133.43, 132.95, 131.57, 131.46, 130.53, 130.42, 128.86, 128.82, 126.92, 126.66, 124.26, 124.22, 122.42, 119.83, 115.98, 115.72, 57.69, 55.26, 51.89, 44.74, 30.52, 29.65, 29.61, 27.44; LC-MS (ESI) m/z: 560.49 [M+H]$^+$.

Example 59: methyl (*S*)-3-(8-nitro-1-((3-(dimethylamino)propyl)thio)-6-(2-fluorophenyl)-4*H*-benzo[*f*][1,2,4]triazolo[4,3-*a*][1,4]diazepin-4-yl)propionate (compound 59)

$^1$H NMR (300 MHz, $CDCl_3$-$d_1$) δ 8.48 (dd, *J* = 8.5, 2.1 Hz, 1H), 8.31 (d, *J* = 2.1 Hz, 1H), 8.22 (d, *J* = 8.5 Hz, 1H), 7.63-7.45 (m, 2H), 7.37-7.20 (m, 2H), 4.95 (t, *J*= 8.1 Hz, 1H), 3.62 (s, 3H), 3.30 (dt, *J*= 14.4, 6.4 Hz, 1H), 3.15 (dt, *J*= 14.4, 6.4 Hz, 1H), 2.71-2.26 (m, 6H), 2.21 (s, 5H), 1.91 (ddq, *J* = 20.6, 13.0, 6.5 Hz, 2H); $^{13}$C NMR (75 MHz, $CDCl_3$-$d_1$) δ 173.43, 163.12, 161.48, 161.38, 159.76, 154.59, 153.88, 146.17, 137.96, 131.60, 131.49, 130.51, 130.41, 128.17, 127.29, 127.25, 127.08, 126.81, 126.00, 124.26, 124.22, 123.37, 115.95, 115.68, 57.69, 55.26, 51.91, 44.74, 30.52, 29.65, 29.61, 27.43; LC-MS (ESI) m/z: 527.59 [M+H]$^+$.

Example 60: methyl (*S*)-3-(8-chloro-6-(2-fluorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 60)

$^1$H NMR (300 MHz, $CDCl_3$-$d_1$) δ: 7.96-7.83 (m, 3H), 7.71-7.55 (m, 3H), 7.43-7.32 (m, 3H), 7.23 (dd, *J* = 11.0, 8.3 Hz, 2H), 4.28 (dd, *J* = 7.7, 5.5 Hz, 2H), 3.62 (s, 3H), 2.78-2.56 (m, 11H); $^{13}$C NMR (75 MHz, $CDCl_3$-$d_1$) δ: 173.69, 163.57, 161.84, 161.73, 161.56, 156.48, 153.38, 134.67, 133.55, 132.47, 132.34, 131.73, 130.35, 130.13, 129.57, 127.65, 127.58, 127.37, 127.29, 124.67, 124.61, 122.48, 116.37, 116.24, 55.32, 53.46, 52.78, 52.55, 51.78, 45.84, 30.26, 29.74, 29.36; LC-MS (ESI) m/z: 557.18 [M+H]$^+$.

Example 61: methyl (*S*)-3-(8-bromo-6-(2-fluorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 61)

$^1$H NMR (300 MHz, $CDCl_3$-$d_1$) δ 7.96-7.84 (m, 2H), 7.80 (dd, *J* = 8.3, 2.4 Hz, 1H), 7.54 (dddd, *J* = 15.2, 7.6, 5.0, 1.6 Hz, 2H), 7.28 (dtd, *J* = 17.6, 7.7, 1.4 Hz, 2H), 6.06-5.90 (m, 1H), 3.66 - 3.50 (m, 4H), 3.31 (dt, *J* = 14.1, 5.2 Hz, 1H), 2.93 (dt, *J*= 12.1, 5.2 Hz, 1H), 2.72-2.60 (m, 2H), 2.65-2.49 (m, 4H), 2.55-2.40 (m, 2H), 2.43-2.21 (m, 7H). $^{13}$C NMR (75 MHz, $CDCl_3$-$d_1$) δ 173.36, 163.19, 162.00, 161.89, 159.83, 154.56, 153.86, 136.38, 133.51, 132.95, 131.61, 131.50, 130.38, 130.28, 128.98, 128.94, 126.92, 126.66, 124.32, 124.28, 122.44, 119.83, 115.98, 115.71, 55.26, 53.62, 52.88, 52.57, 51.93, 45.16, 30.41, 29.67, 29.59; LC-MS (ESI) m/z: 602.54 [M+H]$^+$.

Example 62: methyl (*S*)-3-(8-nitro-6-(2-fluorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 62)

$^1$H NMR (300 MHz, $CDCl_3$-$d_1$) δ 8.52 (dd, *J* = 8.4, 2.1 Hz, 1H), 8.37 (d, *J* = 2.1 Hz, 1H), 8.20 (d, *J* = 8.5 Hz, 1H), 7.64-7.46 (m, 2H), 7.28 (dtd, *J* = 18.3, 7.7, 1.4 Hz, 2H), 5.98 (ddd, *J* = 8.2, 7.5, 0.6 Hz, 1H), 3.66-3.49 (m, 4H), 3.29 (dt, *J* = 14.1, 5.2 Hz, 1H), 2.84 (dt, *J* = 12.1, 5.2 Hz, 1H), 2.80-2.45 (m, 8H), 2.52-2.29 (m, 5H), 2.25 (s, 3H); $^{13}$C NMR (75 MHz, $CDCl_3$-$d_1$) δ 173.36, 163.30, 161.48, 161.38, 159.94, 154.51, 153.88, 146.17, 138.11, 131.60, 131.49, 130.54, 130.43, 128.17, 127.60, 127.56, 127.08, 126.81, 125.94, 124.26, 124.22, 123.35, 115.98, 115.71, 55.26, 53.62, 52.88, 52.57, 51.93, 45.16, 30.41, 29.67, 29.61; LC-MS (ESI) m/z: 568.64 [M+H]$^+$.

Example 63: methyl (*S*)-3-(8-chloro-6-(2-fluorophenyl)-1-((1-methylpiperidin-4-yl)thio)-4*H*-benzo[*f*][1,2,4]triazolo[4,3-α][1,4]diazepin-4-yl)propionate (compound 63)

$^1$H NMR (300 MHz, $CDCl_3$-$d_1$) δ: 7.94-7.71 (m, 3H), 7.62 (tdd, *J* = 7.6, 5.3, 1.9 Hz, 1H), 7.50-7.25 (m, 3H), 2.63 (dt, *J* = 17.0, 8.0 Hz, 2H), 2.15-2.09 (m, 3H), 1.94 (d, *J* = 15.2 Hz, 6H), 1.86-1.74 (m, 1H), 1.61-1.42 (m, 2H), 1.26 (p, *J* = 4.6, 3.6 Hz, 2H); $^{13}$C NMR (75 MHz, $CDCl_3$-$d_1$) δ: 173.62, 163.43, 161.72, 161.63, 161.53, 154.68, 153.31, 133.68, 133.10, 132.42, 132.23, 131.41, 130.63, 130.48, 129.88, 127.82, 127.79, 127.73, 127.45, 124.68, 124.56, 123.36, 116.38, 116.23, 55.16, 53.81, 51.92, 45.91, 39.48, 31.55, 29.39, 29.25; LC-MS (ESI) m/z: 528.63 $[M+H]^+$.

Example 64: methyl (*S*)-3-(8-bromo-6-(2-fluorophenyl)-1-((1-methylpiperidin-4-yl)thio)-4H-benzo[*f*][1,2,4]triazolo[4,3-*a*][1,4]diazepin-4-yl)propionate (compound 64)

$^1$H NMR (300 MHz, $CDCl_3$-$d_1$) δ 7.95-7.85 (m, 2H), 7.80 (dd, *J* = 8.4, 1.9 Hz, 1H), 7.64-7.45 (m, 2H), 7.28 (dtd, *J* = 16.4, 7.7, 1.3 Hz, 2H), 6.05-5.91 (m, 1H), 3.62 (s, 2H), 3.27 (p, *J* = 5.8 Hz, 1H), 2.74 (ddd, *J* = 12.2, 7.8, 5.9 Hz, 2H), 2.68-2.51 (m, 1H), 2.58-2.42 (m, 3H), 2.48-2.32 (m, 2H), 2.37-2.29 (m, 0H), 2.29 (s, 3H), 2.04-1.79 (m, 4H). $^{13}$C NMR (75 MHz, $CDCl_3$-$d_1$) δ 173.41, 163.21, 162.00, 161.89, 159.85, 153.44, 153.01, 136.19, 133.07, 132.95, 131.60, 131.49, 130.53, 130.42, 128.86, 128.82, 126.92, 126.66, 124.26, 124.22, 122.42, 119.83, 115.98, 115.71, 55.26, 54.28, 51.93, 45.68, 34.94, 31.76, 29.65, 29.61; LC-MS (ESI) m/z: 573.50 $[M+H]^+$.

Example 65: methyl (*S*)-3-(8-nitro-6-(2-fluorophenyl)-1-((1-methylpiperidin-4-yl)thio)-4*H*-benzo[*f*][1,2,4]triazolo[4,3-*a*][1,4]diazepin-4-yl)propionate (compound 65)

$^1$H NMR (300 MHz, $CDCl_3$-$d_1$) δ 8.51 (dd, *J*= 8.4, 1.9 Hz, 1H), 8.40 (d, *J*= 1.9 Hz, 1H), 8.22 (d, *J*= 8.5 Hz, 1H), 7.55 (dddd, *J* = 17.0, 7.6, 5.0, 1.7 Hz, 2H), 7.29 (dtd, *J* = 14.3, 7.7, 1.4 Hz, 2H), 6.06-5.90 (m, 1H), 3.62 (s, 2H), 3.28 (p, *J* = 5.8 Hz, 1H), 2.77 (ddd, *J* = 12.2, 7.8, 5.8 Hz, 2H), 2.71-2.24 (m, 6H), 2.29 (s, 3H), 2.06-1.80 (m, 4H). $^{13}$C NMR (75 MHz, $CDCl_3$-$d_1$) δ 173.37, 163.09, 161.48, 161.38, 159.73, 153.42, 153.03, 146.17, 137.52, 131.60, 131.49, 130.51, 130.41, 128.17, 127.60, 127.56, 127.08, 126.81, 126.05, 124.26, 124.22, 123.35, 115.98, 115.71, 55.26, 54.28, 51.91, 45.68, 34.92, 31.67, 29.67, 29.61; LC-MS (ESI) m/z: 539.60 $[M+H]^+$.

Example 66: methyl (*S*)-3-(8-chloro-6-(2-fluorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 66)

$^1$H NMR (300 MHz, $CDCl_3$-$d_1$) δ: 7.89 (s, 2H), 7.70-7.53 (m, 2H), 7.42-7.31 (m, 2H), 7.22 (dd, *J* = 10.9, 8.3 Hz, 1H), 4.35 (s, 1H), 4.27 (dd, *J* = 7.8, 5.5 Hz, 1H), 3.45 (q, *J* = 6.0 Hz, 2H), 3.33 (s, 4H), 3.21-3.03 (m, 2H), 2.80-2.57 (m, 4H), 2.41-2.21 (m, 10H), 1.74 (q, *J* = 6.9 Hz, 2H); $^{13}$C NMR (75 MHz, $CDCl_3$-$d_1$) δ: 173.51, 163.39, 161.74, 161.67, 161.24, 156.51, 153.86, 134.09, 133.33, 132.18, 132.28, 131.50, 130.54, 130.47, 129.88, 127.75, 127.58, 127.72, 127.61, 124.72, 124.66, 122.55, 116.36, 116.02, 59.79, 57.51, 55.23, 55.12, 52.68, 52.08, 51.89, 31.15, 29.43, 29.18, 27.65; LC-MS (ESI) m/z: 602.3 $[M+H]^+$.

Example 67: methyl (*S*)-3-(8-bromo-6-(2-fluorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 67)

$^1$H NMR (300 MHz, $CDCl_3$-$d_1$) δ 7.94-7.77 (m, 3H), 7.54 (dddd, *J* = 8.5, 7.5, 5.0, 1.4 Hz, 2H), 7.37-7.18 (m, 2H), 6.07-5.90 (m, 1H), 4.26 (t, *J* = 7.3 Hz, 1H), 3.66 - 3.45 (m, 4H), 3.36-3.12 (m, 2H), 2.87 (t, *J* = 5.3 Hz, 3H), 2.72-2.53 (m, 7H), 2.54 (dt, *J* = 3.6, 1.8 Hz, 1H), 2.56-2.42 (m, 2H), 2.49-2.31 (m, 1H), 2.36-2.22 (m, 1H), 1.88 (pd, *J* = 6.5, 4.1 Hz, 2H). $^{13}$C NMR (75 MHz, $CDCl_3$-$d_1$) δ 173.40, 163.37, 162.00, 161.89, 160.00, 154.56, 153.86, 136.38, 133.59, 132.95, 131.61, 131.50, 130.39, 130.28, 128.98, 128.94, 126.93, 126.66, 124.21, 124.17, 122.44, 119.76, 115.91, 115.64, 59.24, 58.71, 55.29, 55.11, 52.38, 52.36, 51.93, 30.47, 29.67, 29.59, 27.31; LC-MS (ESI) m/z: 645.59 $[M+H]^+$.

Example 68: methyl (*S*)-3-(8-nitro-6-(2-fluorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 68)

$^1$H NMR (300 MHz, $CDCl_3$-$d_1$) δ 8.52 (dd, *J*= 8.4, 1.9 Hz, 1H), 8.41 (d, *J*= 1.8 Hz, 1H), 8.22 (d, *J*= 8.3 Hz, 1H), 7.63-7.48 (m, 2H), 7.28 (dtd, *J* = 21.6, 7.7, 1.4 Hz, 2H), 6.04-5.89 (m, 1H), 4.27 (t, *J* = 7.3 Hz, 1H), 3.66-3.49 (m, 4H), 3.35 (dt, J= 14.4, 6.4 Hz, 1H), 3.19 (dt, *J* = 14.4, 6.4 Hz, 1H), 2.87 (t, *J*= 5.3 Hz, 3H), 2.72-2.58 (m, 4H), 2.64-2.44 (m, 6H), 2.47-2.23 (m, 2H), 1.85 (ddq, *J* = 29.5, 13.0, 6.5 Hz, 2H). $^{13}$C NMR (75 MHz, $CDCl_3$-$d_1$) δ 173.36, 163.30, 161.56, 161.45, 159.94, 154.60, 153.88, 146.16, 138.12, 131.61, 131.50, 130.46, 130.35, 128.16, 127.78, 127.74, 127.08, 126.81, 125.94, 124.35, 124.31, 123.35, 115.91, 115.64, 59.24, 58.71, 55.26, 55.11, 52.44, 52.41, 51.94, 30.45, 29.67,

29.59, 27.31; LC-MS (ESI) m/z: 612.69 [M+H]$^+$.

Example 69: methyl (*S*)-3-(8-chloro-6-(2-fluorophenyl)-1-((morpholinomethyl)thio)-4*H*-benzo[*f*][1,2,4]triazolo[4,3-*a*][1,4]diazepin-4-yl)propionate (compound 69)

$^1$H NMR (300 MHz, $CDCl_3$-*d$_1$*) δ: 8.47 (d, *J* = 8.9 Hz, 1H), 7.87 (dd, *J* = 8.9, 2.5 Hz, 1H), 7.69-7.42 (m, 3H), 7.41-7.31 (m, 2H), 7.25 (dd, *J* = 10.8, 8.3 Hz, 1H), 5.19 (d, *J* = 13.4 Hz, 1H), 5.03 (d, *J* = 13.4 Hz, 1H), 4.33 (dd, *J* = 8.7, 5.1 Hz, 1H), 3.32 (s, 1H), 2.88-2.54 (m, 7H), 2.49-2.39 (m, 1H), 1.24 (s, 1H); $^{13}$C NMR (75 MHz, $CDCl_3$-*d$_1$*) δ: 173.44, 163.43, 161.90, 161.83, 161.34, 153.79, 153.74, 134.22, 133.01, 132.43, 132.37, 131.35, 130.53, 130.46, 129.92, 127.75, 127.65, 127.51, 127.49, 124.71, 124.61, 123.02, 116.42, 116.01, 66.71, 58.33, 55.58, 51.74, 43.81, 29.41, 29.17; LC-MS (ESI) m/z: 531.2 [M+H]$^+$.

Example 70: methyl (*S*)-3-(8-bromo-6-(2-fluorophenyl)-1-((morpholinomethyl)thio)-4*H*-benzo[*f*][1,2,4]triazolo[4,3-*a*][1,4]diazepin-4-yl)propionate (compound 70)

$^1$H NMR (300 MHz, $CDCl_3$-*d$_1$*) δ: 7.97-7.83 (m, 2H), 7.81 (dd, *J* = 8.4, 2.5 Hz, 1H), 7.53 (dddd, *J* = 16.9, 7.6, 5.0, 1.6 Hz, 2H), 7.36-7.19 (m, 2H), 6.04-5.90 (m, 1H), 4.24 (d, *J*= 13.4 Hz, 1H), 3.99 (d, *J* = 13.4 Hz, 1H), 3.75-3.56 (m, 7H), 2.93 (ddd, *J* = 12.7, 6.5, 5.5 Hz, 2H), 2.80-2.40 (m, 5H), 2.41-2.23 (m, 1H). $^{13}$C NMR (75 MHz, $CDCl_3$-*d$_1$*) δ 173.41, 163.21, 162.00, 161.89, 159.85, 153.86, 149.17, 136.19, 133.62, 132.95, 131.62, 131.52, 130.53, 130.42, 128.86, 128.82, 126.92, 126.66, 124.26, 124.22, 122.42, 119.83, 115.98, 115.71, 66.31, 58.28, 55.26, 52.38, 51.93, 29.65, 29.61; LC-MS (ESI) m/z: 575.47 [M+H]$^+$.

Example 71: methyl (*S*)-3-(8-nitro-6-(2-fluorophenyl)-1-((morpholinomethyl)thio)-4*H*-benzo[*f*][1,2,4]triazolo[4,3-*a*][1,4]diazepin-4-yl)propionate (compound 71)

$^1$H NMR (300 MHz, $CDCl_3$-*d$_1$*) δ 8.50 (dd, *J* = 8.4, 2.1 Hz, 1H), 8.33 (d, *J* = 2.1 Hz, 1H), 8.23 (d, *J* = 8.5 Hz, 1H), 7.65-7.46 (m, 2H), 7.29 (dtd, *J* = 14.4, 7.7, 1.4 Hz, 2H), 4.75 (t, *J* = 8.2 Hz, 1H), 4.20 (d, *J* = 13.4 Hz, 1H), 4.06 (d, *J* = 13.4 Hz, 1H), 3.74-3.54 (m, 7H), 2.93 (ddd, *J* = 12.7, 6.8, 5.3 Hz, 2H), 2.73 (ddd, *J* = 12.7, 6.9, 5.2 Hz, 2H), 2.72 - 2.48 (m, 2H), 2.53-2.27 (m, 2H). $^{13}$C NMR (75 MHz, $CDCl_3$-*d$_1$*) δ 173.37, 163.09, 161.48, 161.38, 159.73, 153.86, 149.17, 146.17, 137.95, 131.60, 131.49, 130.51, 130.41, 128.17, 127.65, 127.61, 127.08, 126.81, 126.05, 124.26, 124.22, 123.35, 115.98, 115.71, 66.32, 58.28, 55.26, 52.43, 51.91, 29.67, 29.61; LC-MS (ESI) m/z: 541.57 [M+H]$^+$.

Example 72: methyl (*S*)-3-(8-chloro-6-(2-fluorophenyl)-1-(((4-phenylpiperazin-1-yl)methyl)thio)-4*H*-benzo[*f*][1,2,4]triazolo[4,3-*a*][1,4]diazepin-4-yl)propionate (compound 72)

$^1$H NMR (300 MHz, $CDCl_3$-*d$_1$*) δ: 8.47 (d, *J*= 8.9 Hz, 1H), 7.92-7.83 (m, 1H), 7.68-7.51 (m, 2H), 7.40-7.30 (m, 2H), 7.29-7.14 (m, 3H), 6.93 (t, J = 9.3 Hz, 2H), 6.76 (t, *J*= 7.2 Hz, 1H), 5.28 (d, *J* = 13.4 Hz, 1H), 5.10 (d, *J* = 13.4 Hz, 1H), 4.37- 4.23 (m, 1H), 3.33 (s, 2H), 3.13 (t, *J* = 4.9 Hz, 3H), 3.02-2.85 (m, 4H), 2.82-2.56 (m, 4H), 2.45 (dd, *J* = 10.1, 6.7 Hz, 1H); $^{13}$C NMR (75 MHz, $CDCl_3$-*d$_1$*) δ: 173.46, 163.40, 161.84, 161.81, 161.33, 153.79, 153.34, 150.64, 134.18, 133.22, 132.16, 132.12, 131.39, 130.49, 130.33, 129.94, 129.11, 127.75, 127.63, 127.53, 127.49, 124.63, 124.45, 123.18, 118.61, 116.59, 116.44, 116.31, 58.36, 55.28, 51.91, 51.72, 48.41, 29.39, 29.19; LC-MS (ESI) m/z: 605.22 [M+H]$^+$.

Example 73: methyl (*S*)-3-(8-bromo-6-(2-fluorophenyl)-1-(((4-phenylpiperazin-1-yl)methyl)thio)-4*H*-benzo[*f*][1,2,4]triazolo[4,3-*a*][1,4]diazepin-4-yl)propionate (compound 73)

$^1$H NMR (300 MHz, $CDCl_3$-*d$_1$*) δ: 7.87 (d, *J* = 2.4 Hz, 1H), 7.80-7.62 (m, 2H), 7.57 (dddd, *J* = 7.6, 6.7, 3.9, 1.4 Hz, 2H), 7.29 (td, *J* = 7.5, 1.4 Hz, 1H), 7.23-7.11 (m, 3H), 6.90-6.81 (m, 3H), 6.02-5.87 (m, 1H), 4.34-4.09 (m, 2H), 3.76 (s, 2H), 3.34 (ddd, *J* = 11.8, 6.4, 4.2 Hz, 2H), 3.22 (ddd, *J*= 11.8, 6.5, 4.2 Hz, 2H), 2.76 (ddd, *J*= 11.7, 6.4, 4.2 Hz, 2H), 2.61-2.52 (m, 1H), 2.60-2.32 (m, 4H), 2.41-2.15 (m, 1H); $^{13}$C NMR (75 MHz, $CDCl_3$-*d$_1$*) δ: 173.34, 163.22, 162.45, 161.76, 160.45, 153.56, 151.31, 149.21, 136.45, 133.70, 132.32, 131.37, 131.67, 130.78, 130.43, 129.23, 128.68, 128.45, 126.57, 126.34, 124.23, 124.12, 122.45, 119.56, 118.50, 116.12, 115.91, 115.64, 58.79, 55.33, 51.53, 51.74, 48.44, 29.32, 29.52; LC-MS (ESI) m/z: 649.58 [M+H]$^+$.

Example 74: methyl (*S*)-3-(8-nitro-6-(2-fluorophenyl)-1-(((4-phenylpiperazin-1-yl)methyl)thio)-4*H*-benzo[*f*][1,2,4]triazolo[4,3-*a*][1,4]diazepin-4-yl)propionate (compound 74)

$^1$H NMR (300 MHz, $CDCl_3$-*d$_1$*) δ: 8.48-8.37 (m, 2H), 8.00 (d, *J* = 8.2 Hz, 1H), 7.56-7.38 (m, 2H), 7.23 (td, *J* =

7.6, 1.3 Hz, 1H), 7.14-7.01 (m, 3H), 6.88-6.73 (m, 3H), 4.56 (ddd, *J* = 8.2, 7.2, 0.7 Hz, 1H), 4.13 (d, *J* = 13.4 Hz, 1H), 4.01 (d, *J* = 13.3 Hz, 1H), 3.43 (s, 2H), 3.21 (ddd, *J* = 11.8, 6.3, 4.2 Hz, 2H), 3.01 (ddd, *J* = 11.8, 6.5, 4.2 Hz, 2H), 2.74 (ddd, *J* = 11.8, 6.4, 4.2 Hz, 2H), 2.65-2.54 (m, 1H), 2.60-2.47 (m, 2H), 2.53-2.42 (m, 1H), 2.48-2.33 (m, 1H), 2.34-2.16 (m, 1H); $^{13}$C NMR (75 MHz, $CDCl_3$-$d_1$) δ: 173.34, 163.54, 161.56, 161.36, 160.78, 153.98, 151.45, 149.54, 146.16, 137.22, 131.51, 131.65, 130.54, 130.76, 129.17, 128.20, 127.55, 127.12, 127.01, 126.71, 126.02, 124.43, 124.23, 123.45, 118.08, 116.11, 115.87, 115.45, 58.56, 55.68, 51.45, 51.67, 48.44, 29.78, 29.68; LC-MS (ESI) m/z: 615.68 $[M+H]^+$.

Example 75: methyl (*S*)-3-(8-chloro-6-(2-chlorophenyl)-1-((cyclopropylmethyl)thio)-4*H*-benzo[*f*][1,2,4]triazolo[4,3-*a*][1,4]diazepin-4-yl)propionate (compound 75)

$^1$H NMR (300 MHz, $CDCl_3$-$d_1$) δ: 7.93-7.81 (m, 2H), 7.66 (dd, *J* = 6.0, 3.3 Hz, 1H), 7.53 (dq, *J* = 6.1, 4.0, 3.4 Hz, 2H), 7.51-7.42 (m, 1H), 7.19 (d, *J* = 2.3 Hz, 1H), 4.31 (dd, *J* = 7.9, 5.5 Hz, 1H), 3.26-3.07 (m, 2H), 2.79-2.57 (m, 3H), 1.11 (ddt, *J* = 10.7, 7.5, 3.7 Hz, 1H), 0.58-0.45 (m, 2H), 0.34-0.21 (m, 2H); $^{13}$C NMR (75 MHz, $CDCl_3$-$d_1$) δ: 173.43, 162.78, 156.44, 153.88, 137.85, 134.75, 133.44, 133.26, 131.45, 131.39, 129.69, 129.45, 128.57, 128.38, 127.21, 122.56, 54.73, 51.82, 38.13, 29.58, 29.14, 11.63, 6.31; LC-MS (ESI) m/z: 503.0$[M+H]^+$.

Example 76: methyl (*S*)-3-(8-bromo-6-(2-chlorophenyl)-1-((cyclopropylmethyl)thio)-4*H*-benzo[*f*][1,2,4]triazolo[4,3-*a*][1,4]diazepin-4-yl)propionate (compound 76)

$^1$H NMR (300 MHz, $CDCl_3$-$d_1$) δ: 7.81 (d, *J* = 2.4 Hz, 1H), 7.74 (d, *J* = 8.4 Hz, 1H), 7.70-7.55 (m, 2H), 7.53-7.41 (m, 2H), 7.32 (ddd, *J* = 7.8, 5.1, 3.8 Hz, 1H), 4.79-4.50 (m, 1H), 3.70 (s, 2H), 3.43 (dd, *J* = 13.5, 4.8 Hz, 1H), 3.13 (dd, *J* = 13.6, 4.8 Hz, 1H), 2.71-2.43 (m, 3H), 2.53-2.28 (m, 1H), 1.50-1.33 (m, 1H), 1.42-1.14 (m, 4H); $^{13}$C NMR (75 MHz, $CDCl_3$-$d_1$) δ: 173.45, 162.31, 154.35, 153.57, 138.87, 136.17, 134.12, 133.45, 132.28, 130.98, 129.34, 129.47, 128.38, 126.68, 122.44, 119.76, 55.43, 51.67, 37.43, 29.81, 29.62, 13.54, 6.60; LC-MS (ESI) m/z: 545.88 $[M+H]^+$.

Example 77: methyl (*S*)-3-(8-nitro-6-(2-chlorophenyl)-1-((cyclopropylmethyl)thio)-4*H*-benzo[*f*][1,2,4]triazolo[4,3-*a*][1,4]diazepin-4-yl)propionate (compound 77)

$^1$H NMR (300 MHz, $CDCl_3$-$d_1$) δ: 8.49 (d, *J*= 2.1 Hz, 1H), 8.37 (dd, *J*= 8.5, 2.1 Hz, 1H), 8.02 (d, *J*= 8.5 Hz, 1H), 7.67-7.48 (m, 3H), 7.30 (ddd, *J* = 8.0, 6.9, 1.7 Hz, 1H), 4.67-4.43 (m, 1H), 3.34 (s, 2H), 3.10-3.01 (m, 1H), 2.90-2.85 (m, 1H), 2.59-2.33 (m, 2H), 2.39-2.23 (m, 1H), 2.20-2.05 (m, 1H), 1.46-1.21 (m, 5H); $^{13}$C NMR (75 MHz, $CDCl_3$-$d_1$) δ: 173.50, 162.65, 154.76, 153.55, 146.81, 138.09, 137.32, 134.46, 130.71, 129.98, 128.12, 128.23, 128.46, 126.87, 125.67, 123.75, 55.43, 51.56, 37.56, 29.65, 29.23, 13.45, 6.76; LC-MS (ESI) m/z: 511.98 $[M+H]^+$.

Example 78: methyl (*S*)-3-(8-chloro-6-(2-chlorophenyl)-1-(prop-2-yn-1-ylthio)-4*H*-benzo[*f*][1,2,4]triazolo[4,3-*a*][1,4]diazepin-4-yl)propionate (compound 78)

$^1$H NMR (300 MHz, $CDCl_3$-$d_1$) δ: 7.94-7.80 (m, 2H), 7.69-7.60 (m, 1H), 7.58-7.48 (m, 2H), 7.45 (qd, *J* = 5.5, 2.5 Hz, 1H), 7.20 (d, *J* = 2.4 Hz, 1H), 4.33 (dd, *J* = 7.9, 5.4 Hz, 1H), 4.15-3.96 (m, 2H), 3.60 (s, 3H), 3.21 (t, *J* = 2.5 Hz, 1H), 2.78-2.54 (m, 4H); $^{13}$C NMR (75 MHz, $CDCl_3$-$d_1$) δ: 173.25, 162.95, 156.03, 153.88, 137.76, 134.79, 133.90, 133.06, 131.54, 131.29, 129.88, 129.65, 128.82, 128.67, 127.31, 123.03, 79.13, 72.19, 54.94 51.94, 29.57, 29.24, 20.22; LC-MS (ESI) m/z: 485.1 $[M+H]^+$.

Example 79: methyl (*S*)-3-(8-bromo-6-(2-chlorophenyl)-1-(prop-2-yn-1-ylthio)-4*H*-benzo[*f*][1,2,4]triazolo[4,3-*a*][1,4]diazepin-4-yl)propionate (compound 79)

$^1$H NMR (300 MHz, $CDCl_3$-$d_1$) δ: 7.97 (d, *J*= 2.5 Hz, 1H), 7.76 (d, *J*= 8.4 Hz, 1H), 7.55 (dd, *J*= 8.5, 2.4 Hz, 1H), 7.48-7.31 (m, 3H), 7.18 (ddd, *J*= 7.8, 5.7, 3.2 Hz, 1H), 4.67-4.52 (m, 1H), 4.01 (dd, *J* = 12.3, 3.0 Hz, 1H), 3.89 (dd, *J* = 12.4, 3.0 Hz, 1H), 3.71 (s, 2H), 2.65-2.32 (m, 3H), 2.29-2.05 (m, 2H); $^{13}$C NMR (75 MHz, $CDCl_3$-$d_1$) δ: 173.12, 162.65, 154.30, 153.09, 138.23, 136.76, 134.09, 133.19, 132.65, 130.65, 129.45, 129.76, 128.78, 126.13, 122.56, 119.73, 79.01, 72.66, 55.11, 51.70, 29.57, 29.76, 20.41; LC-MS (ESI) m/z: 529.87 $[M+H]^+$.

Example 80: methyl (*S*)-3-(8-nitro-6-(2-chlorophenyl)-1-(prop-2-yn-1-ylthio)-4*H*-benzo[*f*][1,2,4]triazolo[4,3-*a*][1,4]diazepin-4-yl)propionate (compound 80)

$^1$H NMR (300 MHz, $CDCl_3$-$d_1$) δ: 8.49-8.34 (m, 2H), 8.09 (d, *J*= 8.4 Hz, 1H), 7.55-7.40 (m, 3H), 7.30 (ddd, *J* = 7.7, 7.2, 1.7 Hz, 1H), 4.89 (t, *J* = 8.0 Hz, 1H), 4.12 (dd, *J* = 12.4, 3.0 Hz, 1H), 3.89 (dd, *J* = 12.4, 3.0 Hz, 1H), 3.60 (s,

2H), 2.87-2.63 (m, 2H), 2.49-2.33 (m, 1H), 2.37-2.29 (m, 1H), 2.21 (t, *J* = 3.0 Hz, 1H); $^{13}$C NMR (75 MHz, $CDCl_3$-*$d_1$*) δ: 173.50, 162.06, 154.02, 153.88, 146.01, 138.70, 137.82, 134.63, 130.41, 129.65, 128.39, 128.36, 128.20, 126.67, 125.98, 123.39, 79.06, 72.67, 55.11, 51.90, 29.70, 29.62, 20.40; LC-MS (ESI) m/z: 495.97 $[M+H]^+$.

Example 81: methyl (*S*)-3-(8-chloro-6-(2-chlorophenyl)-1-((2-morpholinoethyl)thio)-4*H*-benzo[*f*][1,2,4]triazolo[4,3-*a*][1,4]diazepin-4-yl)propionate (compound 81)

$^1$H NMR (300 MHz, $CDCl_3$-*$d_1$*) δ: 7.92 (d, *J*= 1.8 Hz, 2H), 7.72-7.64 (m, 1H), 7.58-7.46 (m, 3H), 7.27-7.16 (m, 1H), 4.34 (t, *J* = 6.6 Hz, 1H), 3.62 (s, 3H), 3.55 (t, *J* = 4.5 Hz, 4H), 3.43 (s, 2H), 2.81-2.67 (m, 2H), 2.63 (t, *J* = 6.6 Hz, 4H), 2.40 (s, 4H); $^{13}$C NMR (75 MHz, $CDCl_3$-*$d_1$*) δ: 173.34, 162.85, 156.47, 153.39, 137.34, 134.56, 133.33, 133.10, 131.49, 131.36, 129.24, 129.11, 128.87, 128.59, 127.39, 122.55, 66.23, 54.88, 53.99, 53.54, 51.78, 30.35, 29.88, 29.68; LC-MS (ESI) m/z: 564.1 $[M+H]^+$.

Example 82: methyl (*S*)-3-(8-bromo-6-(2-chlorophenyl)-1-((2-morpholinoethyl)thio)-4*H*-benzo[*f*][1,2,4]triazolo[4,3-*a*][1,4]diazepin-4-yl)propionate (compound 82)

$^1$H NMR (300 MHz, $CDCl_3$-*$d_1$*) δ: 8.04 (dd, *J*= 8.7, 2.3 Hz, 1H), 7.83 (d, *J* = 8.7 Hz, 1H), 7.68 (dd, *J*= 6.0, 3.3 Hz, 1H), 7.60-7.45 (m, 3H), 7.33 (d, *J* = 2.2 Hz, 1H), 4.33 (t, *J* = 6.6 Hz, 1H), 3.62 (s, 3H), 3.56-3.51 (m, 4H), 3.39 (d, *J* = 6.2 Hz, 2H), 2.82-2.66 (m, 2H), 2.62 (t, *J* = 6.7 Hz, 4H), 2.45-2.32 (m, 4H); $^{13}$C NMR (75 MHz, $CDCl_3$-*$d_1$*) δ: 173.54, 162.56, 154.86, 153.45, 138.96, 136.11, 134.57, 133.69, 132.98, 130.01, 129.24, 129.30, 128.45, 126.35, 122.57, 119.97, 65.12, 55.45, 53.55, 52.57, 51.98, 30.65, 29.80, 29.89; LC-MS (ESI) m/z: 606.7 $[M+H]^+$.

Example 83: methyl (*S*)-3-(8-nitro-6-(2-chlorophenyl)-1-((2-morpholinoethyl)thio)-4*H*-benzo[*f*][1,2,4]triazolo[4,3-*a*][1,4]diazepin-4-yl)propionate (compound 83)

$^1$H NMR (300 MHz, $CDCl_3$-*$d_1$*) δ: 8.56-8.43 (m, 2H), 8.13 (d, *J* = 8.3 Hz, 1H), 7.59-7.47 (m, 1H), 7.52-7.38 (m, 2H), 7.37-7.25 (m, 1H), 5.11-4.96 (m, 1H), 3.54-3.33 (m, 8H), 3.19 (dt, *J* = 14.1, 5.2 Hz, 1H), 2.89-2.65 (m, 3H), 2.57-2.32 (m, 6H), 2.20-2.11 (m, 1H); $^{13}$C NMR (75 MHz, $CDCl_3$-*$d_1$*) δ: 173.11, 162.45, 154.44, 153.90, 146.22, 138.67, 137.48, 135.92, 130.33, 129.68, 128.45, 128.85, 128.68, 126.15, 125.68, 123.42, 65.82, 55.15, 53.47, 52.98, 51.75, 30.09, 29.61, 29.01; LC-MS (ESI) m/z: 571.05 $[M+H]^+$.

Example 84: methyl (*S*)-3-(8-chloro-6-(2-chlorophenyl)-1-((2-(diethylamino)ethyl)thio)-4*H*-benzo[*f*][1,2,4]triazolo[4,3-*a*][1,4]diazepin-4-yl)propionate (compound 84)

$^1$H NMR (300 MHz, $CDCl_3$-*$d_1$*) δ: 8.06-7.74 (m, 2H), 7.66 (s, 1H), 7.49 (d, *J*= 27.1 Hz, 3H), 7.19 (s, 1H), 3.60 (s, 3H), 3.32 (s, 2H), 2.69 (s, 5H), 0.93 (s, 6H); $^{13}$C NMR (75 MHz, $CDCl_3$-*$d_1$*) δ: 173.45, 162.23, 156.68, 153.44, 137.88, 134.21, 133.45, 133.88, 131.73, 131.46, 129.96, 129.78, 128.75, 128.53, 127.38, 122.45, 54.59, 51.83, 51.67, 47.65, 30.57, 29.99, 29.43, 11.87; LC-MS (ESI) m/z: 548.1 $[M+H]^+$.

Example 85: methyl (*S*)-3-(8-bromo-6-(2-chlorophenyl)-1-((2-(diethylamino)ethyl)thio)-4*H*-benzo[*f*][1,2,4]triazolo[4,3-*a*][1,4]diazepin-4-yl)propionate (compound 85)

$^1$H NMR (300 MHz, $CDCl_3$-*$d_1$*) δ: 7.95 (d, *J* = 2.4 Hz, 1H), 7.79-7.61 (m, 2H), 7.55-7.40 (m, 3H), 7.28 (ddd, *J* = 7.7, 6.4, 2.4 Hz, 1H), 4.76-4.62 (m, 1H), 3.68-3.51 (m, 3H), 3.43 (dt, *J* = 14.2, 5.2 Hz, 1H), 2.87 (dt, *J* = 12.1, 5.2 Hz, 1H), 2.79-2.56 (m, 3H), 2.63-2.46 (m, 4H), 2.52-2.39 (m, 1H), 2.43-2.24 (m, 1H), 1.03 (t, *J* = 7.2 Hz, 6H); $^{13}$C NMR (75 MHz, $CDCl_3$-*$d_1$*) δ: 173.47, 162.55, 154.56, 153.88, 138.65, 136.19, 134.57, 133.30, 132.51, 130.41, 129.80, 129.31, 128.32, 126.70, 122.48, 119.74, 55.12, 51.92, 50.90, 47.11, 30.42, 29.81, 29.62, 11.17; LC-MS (ESI) m/z: 590.97 $[M+H]^+$.

Example 86: methyl (*S*)-3-(8-nitro-6-(2-chlorophenyl)-1-((2-(diethylamino)ethyl)thio)-4*H*-benzo[*f*][1,2,4]triazolo[4,3-*a*][1,4]diazepin-4-yl)propionate (compound 86)

$^1$H NMR (300 MHz, $CDCl_3$-*$d_1$*) δ: 8.54 (dd, *J* = 8.5, 2.1 Hz, 1H), 8.41 (d, *J* = 2.1 Hz, 1H), 8.29 (d, *J* = 8.3 Hz, 1H), 7.60 (dd, *J* = 7.7, 1.5 Hz, 1H), 7.56-7.39 (m, 2H), 7.31 (td, *J* = 7.5, 1.4 Hz, 1H), 5.98 (ddd, *J*= 8.2, 7.3, 0.7 Hz, 1H), 3.76-3.56 (m, 4H), 3.33 (dt, *J* = 14.3, 5.2 Hz, 1H), 2.81 (dt, *J* = 12.1, 5.2 Hz, 1H), 2.63-2.49 (m, 8H), 2.32-2.14 (m, 1H), 1.01 (t, *J* = 7.3 Hz, 6H); $^{13}$C NMR (75 MHz, $CDCl_3$-*$d_1$*) δ: 173.23, 162.11, 154.63, 153.23, 146.87, 138.86, 137.98, 134.58, 130.23, 129.96, 128.42, 128.18, 128.49, 126.26, 125.86, 123.14, 55.75, 51.87, 50.97, 47.31, 30.65, 29.43, 29.61, 11.32; LC-MS (ESI) m/z: 557.07 $[M+H]^+$.

Example 87: methyl (*S*)-3-(8-chloro-6-(2-chlorophenyl)-1-((3-(dimethylamino)propyl)thio)-4*H*-benzo[*f*][1,2,4]triazolo[4,3-*a*][1,4]diazepin-4-yl)propionate (compound 87)

$^1H$ NMR (300 MHz, $CDCl_3$-$d_1$) δ: 7.93-7.80 (m, 2H), 7.66 (dd, *J*= 5.9, 3.4 Hz, 1H), 7.53 (dq, *J*= 6.1, 3.9, 3.5 Hz, 2H), 7.50-7.42 (m, 1H), 7.19 (d, *J* = 2.3 Hz, 1H), 4.31 (dd, *J* = 7.9, 5.5 Hz, 1H), 3.60 (s, 3H), 3.31-3.23 (m, 1H), 3.16 (dt, *J* = 13.4, 7.2 Hz, 1H), 2.78-2.57 (m, 4H), 2.36 (s, 2H), 2.17 (s, 6H), 1.86-1.73 (m, 2H); $^{13}C$ NMR (75 MHz, $CDCl_3$-$d_1$) δ: 173.98, 162.36, 156.78, 153.43, 137.67, 134.84, 133.56, 133.42, 131.86, 131.46, 129.86, 129.08, 128.68, 128.49, 127.17, 122.98, 58.03, 54.10, 51.27, 45.55, 31.36, 29.71, 29.59, 26.59; LC-MS (ESI) m/z: 534.07 $[M+H]^+$.

Example 88: methyl (*S*)-3-(8-bromo-6-(2-chlorophenyl)-1-((3-(dimethylamino)propyl)thio)-4*H*-benzo[*f*][1,2,4]triazolo[4,3-*a*][1,4]diazepin-4-yl)propionate (compound 88)

$^1H$ NMR (300 MHz, $CDCl_3$-$d_1$) δ: 7.99 (d, *J*= 8.4 Hz, 1H), 7.87-7.70 (m, 2H), 7.67 (dd, *J*= 7.6, 1.8 Hz, 1H), 7.56 (dd, *J* = 7.6, 1.6 Hz, 1H), 7.39 (dtd, *J* = 23.1, 7.4, 1.7 Hz, 2H), 6.01-5.91 (m, 1H), 3.65 (s, 3H), 3.38 (dt, *J* = 14.4, 6.4 Hz, 1H), 3.10 (dt, *J* = 14.4, 6.5 Hz, 1H), 2.67-2.34 (m, 6H), 2.22 (s, 5H), 2.01 (dp, *J* = 12.9, 6.4 Hz, 1H), 1.88 (dp, *J* = 12.8, 6.4 Hz, 1H); $^{13}C$ NMR (75 MHz, $CDCl_3$-$d_1$) δ: 173.14, 162.23, 154.50, 153.43, 138.22, 136.18, 134.79, 133.26, 132.96, 130.16, 129.09, 129.34, 128.98, 126.12, 122.43, 119.21, 57.64, 55.86, 51.43, 44.53, 30.15, 29.70, 29.34, 27.78; LC-MS (ESI) m/z: 576.92 $[M+H]^+$.

Example 89: methyl (*S*)-3-(8-nitro-6-(2-chlorophenyl)-1-((3-(dimethylamino)propyl)thio)-4*H*-benzo[*f*][1,2,4]triazolo[4,3-*a*][1,4]diazepin-4-yl)propionate (compound 89)

$^1H$ NMR (300 MHz, $CDCl_3$-$d_1$) δ: 8.58 (dd, *J*= 8.5, 2.1 Hz, 1H), 8.43 (d, *J*= 2.1 Hz, 1H), 8.34 (d, *J*= 8.5 Hz, 1H), 7.55 (dd, *J*= 7.7, 1.4 Hz, 1H), 7.50 - 7.38 (m, 2H), 7.32 (td, *J* = 7.5, 1.5 Hz, 1H), 4.99 (t, *J* = 8.1 Hz, 1H), 3.61 (s, 3H), 3.26 - 3.04 (m, 2H), 2.83 - 2.37 (m, 6H), 2.31 (s, 5H), 1.95 (ddt, *J* = 17.5, 12.8, 6.3 Hz, 2H); $^{13}C$ NMR (75 MHz, $CDCl_3$-$d_1$) δ: 173.14, 162.40, 154.60, 153.77, 146.41, 138.33, 137.69, 134.87, 130.44, 129.82, 128.58, 128.69, 127.77, 126.86, 125.93, 123.39, 57.73, 55.26, 51.98, 44.99, 30.58, 29.88, 29.09, 27.54; LC-MS (ESI) m/z: 543.09 $[M+H]^+$.

Example 90: methyl (*S*)-3-(8-chloro-6-(2-chlorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 90)

$^1H$ NMR (300 MHz, $CDCl_3$-$d_1$) δ: 7.91 (dd, *J* = 8.7, 2.4 Hz, 1H), 7.84 (d, *J* = 8.7 Hz, 1H), 7.71-7.63 (m, 1H), 7.59-7.50 (m, 2H), 7.50-7.41 (m, 1H), 7.21 (d, *J* = 2.4 Hz, 1H), 4.32 (dd, *J* = 7.9, 5.5 Hz, 1H), 3.60 (s, 3H), 2.92-2.56 (m, 10H); $^{13}C$ NMR (75 MHz, $CDCl_3$-$d_1$) δ: 173.76, 162.91, 156.09, 153.49, 137.91, 134.24, 133.76, 133.42, 131.90, 131.11, 129.46, 129.36, 128.87, 128.71, 127.90, 122.23, 54.29, 53.70, 52.40, 52.22, 51.53, 45.66, 30.98, 29.56, 29.29;LC-MS (ESI) m/z: 573.19 $[M+H]^+$.

Example 91: methyl (*S*)-3-(8-bromo-6-(2-chlorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 91)

$^1H$ NMR (300 MHz, $CDCl_3$-$d_1$) δ: 8.03-7.87 (m, 2H), 7.86 (dd, *J*= 8.3, 2.4 Hz, 1H), 7.60 (ddd, *J* = 7.7, 5.3, 1.6 Hz, 2H), 7.42 (td, *J* = 7.5, 1.7 Hz, 1H), 7.32 (td, *J* = 7.6, 1.5 Hz, 1H), 6.10-5.98 (m, 1H), 3.69-3.56 (m, 4H), 3.33 (dt, *J* = 14.2, 5.2 Hz, 1H), 2.90 (dt, *J* = 12.1, 5.2 Hz, 1H), 2.78-2.33 (m, 13H), 2.36-2.26 (s, 3H); $^{13}C$ NMR (75 MHz, $CDCl_3$-$d_1$) δ: 173.23, 162.76, 154.34, 153.67, 138.98, 136.34, 134.59, 133.16, 132.90, 130.46, 129.51, 129.33, 128.40, 126.10, 122.21, 119.70, 55.37, 53.48, 52.69, 52.16, 51.95, 45.17, 30.34, 29.11, 29.60; LC-MS (ESI) m/z: 617.99 $[M+H]^+$.

Example 92: methyl (*S*)-3-(8-nitro-6-(2-chlorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 92)

$^1H$ NMR (300 MHz, $CDCl_3$-$d_1$) δ: 8.56 (dd, *J*= 7.2, 1.5 Hz, 1H), 8.33 (d, *J*= 1.5 Hz, 1H), 7.87 (d, *J*= 7.7 Hz, 1H), 7.69-7.59 (m, 1H), 7.56-7.33 (m, 3H), 5.95-5.83 (m, 1H), 3.56 (s, 3H), 3.50-3.28 (m, 2H), 2.88-2.60 (m, 2H), 2.67-2.49 (m, 7H), 2.55-2.45 (m, 4H), 2.50-2.42 (m, 1H), 2.42-2.29 (m, 0H), 2.30 (s, 3H); $^{13}C$ NMR (75 MHz, $CDCl_3$-$d_1$) δ: 173.36, 162.07, 154.51, 153.88, 146.14, 138.72, 137.95, 134.98, 130.33, 129.65, 128.27, 128.25, 128.17, 126.71, 125.83, 123.35, 55.10, 53.62, 52.88, 52.57, 51.93, 45.16, 30.41, 29.67, 29.61; LC-MS (ESI) m/z: 584.19 $[M+H]^+$.

Example 93: methyl (*S*)-3-(8-chloro-6-(2-chlorophenyl)-1-((1-methylpiperidin-4-yl)thio)-4*H*-benzo[*f*][1,2,4]triazolo[4,3-α][1,4]diazepin-4-yl)propionate (compound 93)

$^1$H NMR (300 MHz, $CDCl_3$-*d*$_1$) δ: 7.92-7.78 (m, 2H), 7.70-7.60 (m, 1H), 7.58-7.41 (m, 3H), 7.18 (d, *J* = 2.4 Hz, 1H), 4.31 (dd, *J* = 7.8, 5.5 Hz, 1H), 3.36 (d, *J* = 18.0 Hz, 4H), 2.79-2.54 (m, 5H), 2.13 (s, 3H), 2.10-1.92 (m, 3H), 1.90-1.79 (m, 1H), 1.66 (dtd, *J* = 13.2, 10.2, 9.7, 3.5 Hz, 1H), 1.51 (dtd, *J* = 13.8, 10.3, 3.8 Hz, 1H), 1.24 (s, 1H), 1.19-0.97 (m, 1H); $^{13}$C NMR (75 MHz, $CDCl_3$-*d*$_1$) δ: 173.56, 162.78, 154.34, 153.98, 137.09, 134.34, 133.39, 133.14, 131.98, 131.01, 129.84, 129.37, 128.81, 128.58, 127.23, 123.67, 54.45, 53.76, 51.43, 45.98, 39.09, 31.34, 29.71, 29.01; LC-MS (ESI) m/z: 544.17 $[M+H]^+$.

Example 94: methyl (*S*)-3-(8-bromo-6-(2-chlorophenyl)-1-((1-methylpiperidin-4-yl)thio)-4*H*-benzo[*f*][1,2,4]triazolo[4,3-*a*][1,4]diazepin-4-yl)propionate (compound 94)

$^1$H NMR (300 MHz, $CDCl_3$-*d*$_1$) δ 8.02-7.93 (m, 1H), 7.87-7.74 (m, 2H), 7.68-7.57 (m, 1H), 7.53-7.37 (m, 3H), 5.75 (t, *J* = 8.3 Hz, 1H), 3.74 (p, *J* = 4.3 Hz, 1H), 2.95 (ddd, *J* = 12.6, 7.7, 5.0 Hz, 2H), 2.81-2.69 (m, 2H), 2.72-2.64 (m, 1H), 2.67-2.52 (m, 1H), 2.38-2.22 (m, 2H), 2.25 (s, 2H), 2.11-1.94 (m, 2H), 1.76-1.60 (m, 2H); $^{13}$C NMR (75 MHz, $CDCl_3$-*d*$_1$) δ 173.44, 162.92, 154.83, 153.07, 136.68, 136.32, 134.54, 132.69, 130.92, 129.67, 129.29, 128.62, 126.95, 122.39, 118.51, 55.15, 52.59, 51.85, 45.28, 39.32, 30.13, 29.31, 29.03;LC-MS (ESI) m/z: 589.07 $[M+H]^+$.

Example 95: methyl (*S*)-3-(8-nitro-6-(2-chlorophenyl)-1-((1-methylpiperidin-4-yl)thio)-4*H*-benzo[*f*][1,2,4]triazolo[4,3-*a*][1,4]diazepin-4-yl)propionate (compound 95)

$^1$H NMR (300 MHz, $CDCl_3$-*d*$_1$) δ 8.65 (dd, *J* = 8.9, 2.0 Hz, 1H), 8.44 (d, *J* = 2.0 Hz, 1H), 8.20 (d, *J* = 8.9 Hz, 1H), 7.68-7.52 (m, 1H), 7.53-7.35 (m, 3H), 5.75 (t, *J* = 8.3 Hz, 1H), 3.76 (p, *J* = 4.3 Hz, 1H), 2.92 (ddd, *J* = 12.6, 7.7, 5.0 Hz, 2H), 2.82-2.63 (m, 2H), 2.73-2.64 (m, 1H), 2.67-2.52 (m, 1H), 2.41-2.32 (m, 2H), 2.24 (s, 2H), 2.11-1.95 (m, 2H), 1.76-1.60 (m, 2H). $^{13}$C NMR (75 MHz, $CDCl_3$-*d*$_1$) δ 173.44, 162.54, 154.39, 153.21, 145.72, 137.91, 137.16, 134.54, 130.92, 129.44, 129.27, 128.61, 128.47, 126.86, 125.17, 123.04, 55.05, 52.59, 51.85, 45.22, 39.42, 30.26, 29.38, 29.04; LC-MS (ESI) m/z: 556.15 $[M+H]^+$.

Example 96: methyl (*S*)-3-(8-chloro-6-(2-chlorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 96)

$^1$H NMR (300 MHz, $CDCl_3$-*d*$_1$) δ: 7.93-7.84 (m, 2H), 7.74-7.65 (m, 1H), 7.58-7.50 (m, 2H), 7.50-7.47 (m, 1H), 7.15 (d, *J* = 2.3 Hz, 1H), 4.33-4.26 (m, 2H), 3.47 (q, *J* = 5.9 Hz, 2H), 3.43-3.35 (m, 0H), 3.31 (s, 2H), 3.26 (dt, *J* = 13.8, 7.0 Hz, 1H), 3.12 (dt, *J* = 13.4, 7.2 Hz, 1H), 2.78-2.57 (m, 3H), 2.32 (s, 10H), 1.90-1.71 (m, 2H); $^{13}$C NMR (75 MHz, $CDCl_3$-*d*$_1$) δ: 173.23, 162.34, 156.65, 153.22, 137.74, 134.46, 133.56, 133.06, 131.88, 131.72, 129.74, 129.36, 128.80, 128.55, 127.38, 122.21, 59.43, 57.76, 55.63, 54.52, 52.37, 52.27, 51.28, 31.15, 29.36, 29.27, 27.46; LC-MS (ESI) m/z: 617.23 $[M+H]^+$.

Example 97: methyl (*S*)-3-(8-bromo-6-(2-chlorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 97)

$^1$H NMR (300 MHz, $CDCl_3$-*d*$_1$) δ 8.02-7.92 (m, 1H), 7.87-7.73 (m, 2H), 7.68-7.57 (m, 1H), 7.53-7.37 (m, 3H), 5.75 (t, *J* = 8.3 Hz, 1H), 4.11 (t, *J*= 6.3 Hz, 1H), 3.62 (s, 2H), 3.60-3.45 (m, 2H), 3.24 (td, *J*= 6.9, 1.9 Hz, 2H), 2.78-2.54 (m, 2H), 2.60-2.52 (m, 1H), 2.56-2.43 (m, 2H), 2.52-2.45 (m, 1H), 2.29-2.35 (m, 6H), 2.40-2.21 (m, 2H), 1.85 (tt, *J* = 6.9, 5.8 Hz, 2H); $^{13}$C NMR (75 MHz, $CDCl_3$-*d*$_1$) δ 173.41, 162.97, 156.25, 153.66, 136.68, 136.32, 134.34, 134.42, 132.59, 130.92, 129.67, 129.26, 128.51, 126.45, 122.37, 118.53, 59.43, 57.28, 55.15, 54.51, 52.52, 51.92, 51.75, 30.28, 29.37, 29.07, 27.65; LC-MS (ESI) m/z: 662.13 $[M+H]^+$.

Example 98: methyl (*S*)-3-(8-nitro-6-(2-chlorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 98)

$^1$H NMR (300 MHz, $CDCl_3$-*d*$_1$) δ 8.65 (dd, *J* = 8.9, 2.0 Hz, 1H), 8.46 (d, *J* = 2.0 Hz, 1H), 8.20 (d, *J* = 8.9 Hz, 1H), 7.67-7.66 (m, 1H), 7.53-7.34 (m, 3H), 5.75 (t, *J* = 8.3 Hz, 1H), 4.11 (t, *J* = 6.3 Hz, 1H), 3.65 (s, 2H), 3.63 - 3.55 (m, 2H), 3.24 (td, J= 6.9, 1.9 Hz, 2H), 2.78-2.56 (m, 2H), 2.60-2.51 (m, 1H), 2.56-2.42 (m, 3H), 2.49-2.34 (m, 7H), 2.44-2.35 (m, 1H), 2.39-2.18 (m, 2H), 1.81 (tt, *J* = 6.9, 5.8 Hz, 2H); $^{13}$C NMR (75 MHz, $CDCl_3$-*d*$_1$) δ 173.44, 162.54, 156.32, 153.82, 145.72, 138.32, 137.06, 134.54, 130.92, 129.41, 129.27, 128.61, 128.42, 126.96, 125.17, 123.04, 59.43, 57.27, 55.05, 54.51, 52.52, 51.92, 51.85, 30.28, 29.37, 29.07, 27.55; LC-MS (ESI) m/z: 629.20 $[M+H]^+$.

Example 99: methyl (*S*)-3-(8-chloro-6-(2-chlorophenyl)-1-((morpholinomethyl)thio)-4*H*-benzo[*f*][1,2,4]triazolo[4,3-*a*][1,4]diazepin-4-yl)propionate (compound 99)

$^1$H NMR (300 MHz, $CDCl_3$-*d₁*) δ: 8.42 (d, *J*= 8.8 Hz, 1H), 7.86 (dd, *J*= 8.8, 2.5 Hz, 1H), 7.70-7.60 (m, 1H), 7.58-7.51 (m, 2H), 7.51-7.42 (m, 1H), 7.16 (d, *J*= 2.4 Hz, 1H), 5.21-5.08 (m, 2H), 4.38 (dd, *J*= 8.6, 5.2 Hz, 1H), 3.60 (s, 3H), 3.32 (s, 1H), 2.88-2.54 (m, 7H), 2.48-2.38 (m, 1H); $^{13}$C NMR (75 MHz, $CDCl_3$-$d_1$) δ: 173.36, 162.28, 153.47, 153.28, 137.19, 134.03, 133.47, 133.19, 131.63, 131.57, 129.72, 129.56, 128.86, 128.55, 127.05, 123.53, 66.32, 58.17, 54.59, 51.73, 43.82, 29.47, 29.18; LC-MS (ESI) m/z: 547.34 $[M+H]^+$.

Example 100: methyl (*S*)-3-(8-bromo-6-(2-chlorophenyl)-1-((morpholinomethyl)thio)-4*H*-benzo[*f*][1,2,4]triazolo[4,3-*a*][1,4]diazepin-4-yl)propionate (compound 100)

$^1$H NMR (300 MHz, $CDCl_3$-$d_1$) δ 7.93 (dd, *J* = 1.9, 0.7 Hz, 1H), 7.87-7.74 (m, 2H), 7.64-7.51 (m, 1H), 7.53-7.37 (m, 3H), 5.75 (t, *J* = 8.3 Hz, 1H), 4.08 (d, *J* = 3.6 Hz, 2H), 3.53 (s, 3H), 3.69-3.52 (m, 4H), 2.68-2.52 (m, 2H), 2.48-2.46 (m, 4H), 2.38-2.29 (m, 1H), 2.33-2.21 (m, 1H). $^{13}$C NMR (75 MHz, $CDCl_3$-$d_1$) δ 173.44, 162.92, 153.77, 153.55, 137.68, 136.38, 134.61, 134.54, 132.69, 130.92, 129.73, 129.21, 128.61, 126.95, 122.30, 118.53, 66.63, 56.98, 55.05, 51.65, 46.16, 29.48, 29.27;LC-MS (ESI) m/z: 591.01 $[M+H]^+$.

Example 101: methyl (*S*)-3-(8-nitro-6-(2-chlorophenyl)-1-((morpholinomethyl)thio)-4*H*-benzo[*f*][1,2,4]triazolo[4,3-*a*][1,4]diazepin-4-yl)propionate (compound 101)

$^1$H NMR (300 MHz, $CDCl_3$-$d_1$) δ 8.65 (dd, *J* = 9.0, 2.1 Hz, 1H), 8.46 (d, *J* = 2.2 Hz, 1H), 8.20 (d, *J* = 8.9 Hz, 1H), 7.67-7.56 (m, 1H), 7.53-7.37 (m, 3H), 5.72 (t, *J*= 8.3 Hz, 1H), 4.08 (d, *J*= 3.6 Hz, 2H), 3.63 (s, 3H), 3.69-3.54 (m, 4H), 2.72-2.57 (m, 2H), 2.58-2.42 (m, 4H), 2.38-2.25 (m, 1H), 2.33-2.18 (m, 1H); $^{13}$C NMR (75 MHz, $CDCl_3$-$d_1$) δ 173.44, 162.54, 153.83, 153.78, 145.72, 138.23, 137.06, 134.57, 130.82, 129.44, 129.37, 128.64, 128.47, 126.96, 125.17, 123.04, 66.63, 56.91, 55.05, 51.82, 46.16, 29.38, 29.06;LC-MS (ESI) m/z: 558.13 $[M+H]^+$.

Example 102: methyl (*S*)-3-(8-chloro-1-(methylthio)-6-phenyl-4*H*-benzo[*f*][1,2,4]triazolo[4,3-*a*][1,4]diazepin-4-yl)propionate (compound 102)

$^1$H NMR (300 MHz, $CDCl_3$-$d_1$) δ: 7.97-7.83 (m, 2H), 7.57-7.40 (m, 6H), 4.23 (dd, *J* = 7.6, 5.5 Hz, 1H), 3.63 (s, 3H), 2.82-2.53 (m, 7H); $^{13}$C NMR (75 MHz, $CDCl_3$-$d_1$) δ: 173.47, 166.48, 153.48, 153.37, 139.58, 133.75, 133.42, 132.62, 130.46, 130.27, 128.69, 128.43, 128.12, 122.65, 55.63, 51.73, 29.83, 29.07, 16.74; LC-MS (ESI) m/z: 427.06 $[M+H]^+$.
Example 103: methyl (*S*)-3-(8-bromo-1-(methylthio)-6-phenyl-4*H*-benzo[*f*][1,2,4]triazolo[4,3-*a*][1,4]diazepin-4-yl)propionate (compound 103)
$^1$H NMR (300 MHz, $CDCl_3$-$d_1$) δ 7.82-7.70 (m, 2H), 7.66-7.54 (m, 2H), 7.53 (d, *J* = 8.1 Hz, 1H), 7.52 - 7.43 (m, 2H), 7.48 - 7.39 (m, 1H), 5.64 (t, *J*= 8.3 Hz, 1H), 2.72 (s, 3H), 2.74-2.51(m, 2H), 2.38-2.24 (m, 1H), 2.33-2.18 (m, 1H); $^{13}$C NMR (75 MHz, $CDCl_3$-$d_1$) δ: 173.44, 166.20, 153.33, 152.80, 138.56, 136.32, 133.69, 133.29, 129.80, 129.08, 128.49, 128.41, 122.38, 118.45, 55.26, 51.82, 29.38, 29.06, 16.60; LC-MS (ESI) m/z: 472.04 $[M+H]^+$.

Example 104: methyl (*S*)-3-(8-nitro-1-(methylthio)-6-phenyl-4*H*-benzo1,2,4]triazolo[4,3-*a*][1,4]diazepin-4-yl)propionate (compound 104)

$^1$H NMR (300 MHz, $CDCl_3$-$d_1$) δ 8.61 (dd, *J* = 8.9, 2.1 Hz, 1H), 8.59 (d, *J* = 2.2 Hz, 1H), 8.12 (d, J = 9.0 Hz, 1H), 7.69-7.66 (m, 2H), 7.55-7.43 (m, 2H), 7.48-7.34 (m, 1H), 5.64 (t, *J* = 8.3 Hz, 1H), 2.72 (s, 3H), 2.78-2.52 (m, 2H), 2.38-2.23 (m, 1H), 2.33-2.18 (m, 1H); $^{13}$C NMR (75 MHz, $CDCl_3$-$d_1$) δ: 173.44, 166.07, 153.35, 153.07, 145.66, 138.78, 137.34, 129.81, 128.93, 128.42, 128.42, 128.42, 125.72, 123.07, 55.32, 51.81, 29.34, 29.16, 16.62; LC-MS (ESI) m/z: 438.12 $[M+H]^+$.

Example 105: methyl (*S*)-3-(8-chloro-1-((2-morpholinoethyl)thio)-6-phenyl-4*H*-benzo[*f*][1,2,4]triazolo[4,3-*a*][1,4]diazepin-4-yl)propionate (compound 105)

$^1$H NMR (300 MHz, $CDCl_3$-$d_1$) δ: 7.92 (d, *J* = 2.5 Hz, 2H), 7.52-7.42 (m, 6H), 4.23 (t, *J* = 6.4 Hz, 1H), 3.63 (s, 3H), 3.43 (t, *J* = 4.6 Hz, 4H), 2.75-2.53 (m, 6H), 2.33-2.27 (m, 3H), 2.09 (s, 1H); $^{13}$C NMR (75 MHz, $CDCl_3$-$d_1$) δ: 172.36, 166.71, 156.82, 153.23, 139.62, 133.81, 133.53, 132.55, 130.71, 130.52, 128.64, 128.37, 128.10, 122.51, 66.73, 55.87, 53.44, 53.31, 51.34, 30.36, 29.52, 29.37; LC-MS (ESI) m/z: 526.19 $[M+H]^+$.

Example 106: methyl (*S*)-3-(8-bromo-1-((2-morpholinoethyl)thio)-6-phenyl-4*H*-benzo[*f*][1,2,4]triazolo[4,3-*a*][1,4]diazepin-4-yl)propionate (compound 106)

$^1$H NMR (300 MHz, $CDCl_3$-$d_1$) δ: 8.09 (d, *J*= 10.9 Hz, 1H), 7.87 (d, *J* = 8.7 Hz, 1H), 7.61-7.48 (m, 6H), 4.25 (t, *J* = 6.5 Hz, 1H), 3.65 (s, 3H), 3.56-3.39 (m, 6H), 2.81-2.58 (m, 6H), 2.34 (s, 4H); $^{13}$C NMR (75 MHz, $CDCl_3$-$d_1$) δ 173.34, 165.99, 155.26, 153.74, 138.36, 136.51, 134.19, 133.09, 129.84, 129.04, 128.59, 128.40, 122.38, 118.45, 66.51, 55.23, 54.17, 52.82, 51.81, 30.47, 29.32, 29.08; LC-MS (ESI) m/z: 572.00 $[M+H]^+$.

Example 107: methyl (*S*)-3-(8-nitro-1-((2-morpholinoethyl)thio)-6-phenyl-4*H*-benzo[*f*][1,2,4]triazolo[4,3-*a*][1,4]diazepin-4-yl)propionate (compound 107)

$^1$H NMR (300 MHz, $CDCl_3$-$d_1$) δ 8.61 (dd, *J* = 8.9, 2.1 Hz, 1H), 8.49 (d, *J* = 2.2 Hz, 1H), 8.12 (d, *J*= 9.0 Hz, 1H), 7.69-7.56 (m, 2H), 7.55-7.43 (m, 2H), 7.48-7.39 (m, 1H), 5.61 (t, *J* = 8.3 Hz, 1H), 3.64-3.43 (m, 7H), 3.35 (td, *J* = 6.3, 0.8 Hz, 2H), 2.73-2.76 (m, 3H), 2.61-2.41 (m, 4H), 2.41-2.14 (m, 2H); $^{13}$C NMR (75 MHz, $CDCl_3$-$d_1$) δ: 173.44, 166.04, 155.51, 153.77, 145.66, 138.78, 137.67, 129.60, 128.98, 128.49, 128.46, 128.42, 125.71, 123.07, 66.23, 55.42, 54.12, 52.79, 51.85, 30.47, 29.35, 29.07; LC-MS (ESI) m/z: 537.19 $[M+H]^+$.

Example 108: methyl (*S*)-3-(8-chloro-1-((3-(dimethylamino)propyl)thio)-6-phenyl-4*H*-benzo[*f*][1,2,4]triazolo[4,3-*a*][1,4]diazepin-4-yl)propionate (compound 108)

$^1$H NMR (300 MHz, $CDCl_3$-$d_1$) δ: 7.97-7.85 (m, 2H), 7.57-7.40 (m, 6H), 4.23 (dd, *J* = 7.6, 5.5 Hz, 1H), 3.61 (s, 2H), 3.11 (td, *J* = 7.2, 2.7 Hz, 2H), 2.82-2.54 (m, 4H), 2.24-2.16 (m, 2H), 2.02 (s, 6H), 1.69 (dq, *J* = 14.5, 7.1 Hz, 2H), 1.27-1.11 (m, 1H); $^{13}$C NMR (75 MHz, $CDCl_3$-$d_1$) δ: 173.57, 166.82, 156.35, 153.86, 139.08, 133.50, 133.02, 132.63, 130.71, 130.21, 128.66, 128.68, 128.42, 122.91, 58.46, 55.26, 51.91, 45.36, 31.13, 29.46, 29.28, 26.47; LC-MS (ESI) m/z: 499.56 $[M+H]^+$.

Example 109: methyl (*S*)-3-(8-bromo-1-((3-(dimethylamino)propyl)thio)-6-phenyl-4*H*-benzo[*f*][1,2,4]triazolo[4,3-*a*][1,4]diazepin-4-yl)propionate (compound 109)

$^1$H NMR (300 MHz, $CDCl_3$-$d_1$) δ 7.82-7.70 (m, 2H), 7.66-7.39 (m, 7H), 5.64 (t, *J* = 8.3 Hz, 1H), 3.62 (s, 3H), 3.23 (d, *J* = 12.4 Hz, 1H), 2.78 - 2.61 (m, 1H), 2.67-2.57 (m, 1H), 2.57 (t, *J* = 5.7 Hz, 2H), 2.38-2.29 (m, 1H), 2.33-2.14 (m, 1H), 1.92-1.74 (m, 2H); $^{13}$C NMR (75 MHz, $CDCl_3$-$d_1$) δ 173.44, 165.99, 156.25, 153.75, 138.56, 136.31, 134.08, 133.09, 129.80, 129.05, 128.49, 128.40, 122.32, 118.45, 58.21, 55.26, 51.85, 44.82, 30.04, 29.35, 29.08, 26.61; LC-MS (ESI) m/z: 543.11 $[M+H]^+$.

Example 110: methyl (*S*)-3-(8-nitro-1-((3-(dimethylamino)propyl)thio)-6-phenyl-4*H*-benzo[*f*][1,2,4]triazolo[4,3-*a*][1,4]diazepin-4-yl)propionate (compound 110)

$^1$H NMR (300 MHz, $CDCl_3$-$d_1$) δ 8.63 (dd, *J*= 8.9, 2.1 Hz, 1H), 8.49 (d, *J* = 2.2 Hz, 1H), 8.11 (d, *J* = 9.0 Hz, 1H), 7.69-7.56 (m, 2H), 7.55-7.39 (m, 3H), 5.64 (t, *J* = 8.3 Hz, 1H), 3.63 (s, 3H), 3.23 (d, *J* = 12.4 Hz, 1H), 2.78-2.64 (m, 1H), 2.67-2.55 (m, 1H), 2.57 (t, *J* = 5.7 Hz, 2H), 2.38-2.23 (m, 1H), 2.33-2.28 (m, 1H), 1.92-1.74 (m, 2H); $^{13}$C NMR (75 MHz, $CDCl_3$-$d_1$) δ 173.44, 166.04, 156.33, 153.75, 145.66, 138.78, 137.81, 129.81, 128.98, 128.49, 128.43, 128.42, 125.61, 123.07, 58.21, 55.42, 51.85, 44.72, 30.08, 29.33, 29.07, 26.61; LC-MS (ESI) m/z: 509.19 $[M+H]^+$.

Example 111: methyl (*S*)-3-(8-chloro-6-(2-chlorophenyl)-1-(((phosphonooxy)methyl)thio)-4*H*-benzo [*f*][1,2,4]triazolo[4,3-*a*][1,4]diazepin-4-yl)propionate (compound 111)

$^1$H NMR (300 MHz, $CDCl_3$-$d_1$) δ: 7.82 (d, *J* = 8.2 Hz, 1H), 7.87 (s, 2H), 7.67-7.60 (m, 2H), 7.51-7.40 (m, 4H), 5.83 (d, *J* = 8.5 Hz, 2H), 5.75 (t, *J*= 8.3 Hz, 1H), 2.74-2.56 (m, 2H), 2.39-2.23 (m, 2H); $^{13}$C NMR (75 MHz, $CDCl_3$-$d_1$) δ: 173.42, 161.14, 157.56, 157.13, 154.10, 137.57, 134.72, 134.19, 132.66, 131.29, 131.26, 129.70, 129.62, 128.68, 128.46, 127.31, 123.05, 66.32, 66.28, 58.25, 51.94, 29.93, 29.01; LC-MS (ESI) m/z: 557.34 $[M+H]^+$.

Example 112: methyl (*S*)-3-(8-bromo-6-(2-chlorophenyl)-1-(((phosphonooxy)methyl)thio)-4*H*-benzo[*f*][1,2,4]triazolo[4,3-*a*][1,4]diazepin-4-yl)propionate (compound 112)

$^1$H NMR (300 MHz, $CDCl_3$-$d_1$) δ 7.99 (dd, *J* = 1.9, 0.7 Hz, 1H), 7.87 (s, 2H), 7.84-7.64 (m, 2H), 7.67-7.53 (m, 1H), 7.53-7.35 (m, 3H), 5.83 (d, *J* = 8.4 Hz, 2H), 5.75 (t, *J* = 8.3 Hz, 1H), 2.78- 2.51 (m, 2H), 2.38-2.29 (m, 1H), 2.33-2.21 (m, 1H); $^{13}$C NMR (75 MHz, $CDCl_3$-$d_1$) δ 173.30, 161.23, 157.01, 156.93, 153.98, 136.52, 136.24, 135.16, 133.90,

132.76, 130.92, 129.32, 128.64, 128.61, 126.95, 122.23, 118.29, 66.01, 65.93, 58.26, 51.55, 29.53, 29.22;LC-MS (ESI) m/z: 601.96 $[M+H]^+$.

Example 113: methyl (*S*)-3-(8-nitro-6-(2-chlorophenyl)-1-(((phosphonooxy)methyl)thio)-4*H*-benzo[*f*][1,2,4]triazolo[4,3-*a*][1,4]diazepin-4-yl)propionate (compound 113)

$^1$H NMR (300 MHz, $CDCl_3$-*d$_1$*) δ 8.65 (dd, *J* = 9.0, 2.1 Hz, 1H), 8.41 (d, *J* = 2.2 Hz, 1H), 8.29 (d, *J* = 8.9 Hz, 1H), 7.87 (s, 2H), 7.67-7.57 (m, 1H), 7.53 - 7.37 (m, 3H), 5.81 (d, *J* = 8.4 Hz, 2H), 5.75 (t, *J* = 8.3 Hz, 1H), 2.77-2.52 (m, 2H), 2.38-2.18 (m, 2H). $^{13}$C NMR (75 MHz, $CDCl_3$-*d$_1$*) δ: 173.30, 160.78, 157.01, 157.04, 154.03, 145.72, 137.80, 136.97, 133.90, 130.72, 129.30, 128.61, 128.46, 128.44, 126.75, 124.61, 123.23, 66.11, 65.82, 58.24, 51.72, 29.53, 29.52; LC-MS (ESI) m/z: 567.04 $[M+H]^+$.

Example 114: methyl (*S*)-3-(8-bromo-6-(pyridin-2-yl)-1-methylthio-4*H*-benzo[*f*][1,2,4]triazolo[4,3-*a*][1,4]diazepin-4-yl)propionate (compound 114)

$^1$H NMR (300 MHz, $CDCl_3$-*d$_1$*) δ: 8.79 (dd, *J*= 3.5, 1.2 Hz, 1H), 7.86 - 7.75 (m, 3H), 7.70 - 7.65 (m, 1H), 7.54 (dd, *J* = 7.8, 1.5 Hz, 1H), 7.33-7.30 (m, 1H), 6.13 (t, *J* = 5.3 Hz, 1H), 3.66 (s, 2H), 2.72 (s, 3H), 2.66 (td, *J* = 7.8, 1.2 Hz, 3H), 2.62 - 2.51 (m, 2H). $^{13}$C NMR (75 MHz, $CDCl_3$-*d$_1$*) δ 173.38, 162.32, 155.83, 153.99, 150.80, 147.41, 137.47, 136.64, 136.60, 134.48, 127.86, 125.25, 122.80, 122.65, 120.45, 57.05, 52.01, 29.61, 29.27, 16.69. LC-MS (ESI) m/z:472.3$[M+H]^+$.

Example 115: methyl (*S*)-3-(8-bromo-6-(pyridin-2-yl)-1-ethylthio-4*H*-benzo[*f*][1,2,4]triazolo[4,3-*a*][1,4]diazepin-4-yl)propionate (compound 115)

$^1$H NMR (300 MHz, $CDCl_3$-*d$_1$*) δ: 8.78 (dd, *J* = 3.5, 1.3 Hz, 1H), 7.85 - 7.74 (m, 3H), 7.67 (t, *J* = 1.3 Hz, 1H), 7.54 (dd, *J* = 7.9, 1.3 Hz, 1H), 7.33-7.30 (m, 1H), 6.13 (t, *J* = 5.2 Hz, 1H), 3.65 (s, 3H), 3.29 - 3.18 (m, 2H), 2.70 - 2.52 (m, 4H), 1.40 (t, *J* = 7.2 Hz, 3H). $^{13}$C NMR (75 MHz, $CDCl_3$-*d$_1$*) δ 173.72, 160.73, 157.51, 153.83, 151.63, 147.12, 137.61, 137.47, 136.40, 134.75, 125.25, 124.56, 123.44, 122.65, 120.39, 66.22, 57.42, 52.01, 29.61, 29.23, 14.70. LC-MS (ESI) m/z:486.1 $[M+H]^+$.

Example 116: methyl (*S*)-3-(8-bromo-6-(pyridin-2-yl)-1-((cyclopropylmethyl)thio)-4*H*-benzo[*f*][1,2,4]triazolo[4,3-*a*][1,4]diazepin-4-yl)propionate (compound 116)

$^1$H NMR (300 MHz, $CDCl_3$-*d$_1$*) δ: 8.79 (dd, J= 3.6, 1.3 Hz, 1H), 7.82 (td, *J*= 7.6, 1.2 Hz, 1H), 7.80 - 7.71 (m, 2H), 7.67 (d, *J*= 2.2 Hz, 1H), 7.56 (dd, *J* = 7.9, 1.4 Hz, 1H), 7.33-7.31 (m, 1H), 6.13 (t, *J* = 5.2 Hz, 1H), 3.66 (s, 3H), 3.02 (d, *J* = 4.8 Hz, 2H), 2.70 - 2.51 (m, 4H), 1.40 - 1.24 (m, 5H). $^{13}$C NMR (75 MHz, $CDCl_3$-*d$_1$*) δ 173.58, 162.37, 156.38, 154.59, 149.57, 147.13, 137.57, 137.28, 136.60, 134.78, 127.93, 125.30, 122.76, 122.64, 120.55, 59.05, 51.80, 39.41, 29.45, 29.28, 10.89, 6.19. LC-MS (ESI) m/z: 512.4$[M+H]^+$.

Example 117: methyl (*S*)-3-(8-bromo-6-(pyridin-2-yl)-1-((2-morpholinoethyl)thio)-4*H*-benzo[*f*][1,2,4]triazolo[4,3-*a*][1,4]diazepin-4-yl)propionate (compound 117)

$^1$H NMR (300 MHz, $CDCl_3$-*d$_1$*) δ: 8.58 (d, *J* = 4.2 Hz, 1 H), 8.18 (d, *J* = 7.9 Hz, 1 H), 7.85 (dd, *J* = 7.2, 1.3 Hz, 1 H), 7.79 (dd, *J*= 6.5, 4.2 Hz, 1 H), 7.66 (d, *J*= 8.8 Hz, 2 H), 7.41-7.37 (m, 1 H), 4.27 (t, *J*= 5.1 Hz, 1 H), 3.69 (s, 3H), 3.64 (t, *J* = 4.8 Hz, 4 H), 3.52-3.41 (m, 2 H), 2.91-2.82 (m, 4 H), 2.74 (t, *J* = 6.7 Hz, 2H),2.46 (t, *J* = 4.5 Hz, 4 H). $^{13}$C NMR (75 MHz, $CDCl_3$-*d$_1$*) δ 173.62, 162.11, 156.69, 154.59, 148.12, 147.31, 137.47, 137.09, 136.60, 135.16, 128.24, 125.26, 122.73, 122.59, 120.96, 65.69, 58.05, 54.45, 53.31, 51.93, 32.18, 29.54, 29.28. LC-MS (ESI) m/z: 571.1 $[M+H]^+$.

Example 118: methyl (*S*)-3-(8-bromo-6-(pyridin-2-yl)-1-(3-((dimethylamino)propyl)thio)-4*H*-benzo[*f*][1,2,4]triazolo[4,3-*a*][1,4]diazepin-4-yl)propionate (compound 118)

$^1$H NMR (300 MHz, $CDCl_3$-*d$_1$*) δ: 8.79 (dd, *J*= 3.6, 1.2 Hz, 1H), 7.81 (td, *J* = 7.6, 1.2 Hz, 1H), 7.78 - 7.72 (m, 2H), 7.67 (dd, *J*= 1.7, 0.9 Hz, 1H), 7.56 (dd, *J* = 7.9, 1.4 Hz, 1H), 7.33-7.30 (m, 1H), 6.13 (t, *J*= 5.4 Hz, 1H), 3.65 (s, 3H), 3.27 (t, *J* = 6.4 Hz, 2H), 2.73 - 2.62 (m, 2H), 2.60 - 2.48 (m, 4H), 2.25 (s, 6H), 1.93 (pd, *J* = 6.5, 1.1 Hz, 2H). $^{13}$C NMR (75 MHz, $CDCl_3$-*d$_1$*) δ 173.33, 162.11, 156.72, 154.49, 147.89, 146.31, 137.30, 137.09, 136.67, 135.32, 128.24, 125.26, 122.74, 122.32, 120.94, 58.49, 58.05, 51.96, 44.90, 32.55, 29.54, 29.28, 26.44. LC-MS (ESI) m/z:543.2 $[M+H]^+$.

Example 119: methyl (*S*)-3-(8-chloro-6-(2-chlorophenyl)-1-(2-((pyrrolidin-1-yl)ethyl)thio)-4*H*-benzo[*f*][1,2,4]triazolo[4,3-*a*][1,4]diazepin-4-yl)propionate (compound 119)

$^1$H NMR (300 MHz, $CDCl_3$-$d_1$) δ: 7.91 (d, *J* = 2.0 Hz, 1H), 7.61 (dd, *J* = 7.1, 2.2 Hz, 1H), 7.51 - 7.37 (m, 4H), 7.29 (d, *J* = 7.5 Hz, 1H), 5.22 (s, 1H), 3.68 (s, 3H), 3.48 (d, *J* = 0.9 Hz, 2H), 2.68 - 2.58 (m, 4H), 2.49 (s, 2H), 2.44 - 2.31 (m, 2H), 2.30 - 2.19 (m, 2H), 1.80 (d, *J* = 12.5 Hz, 4H). $^{13}$C NMR (75 MHz, $CDCl_3$-$d_1$) δ 173.30, 160.77, 154.13, 148.12, 138.12, 135.83, 134.71, 132.61, 130.82, 130.51, 130.43, 129.29, 129.04, 128.19, 126.36, 122.81, 57.76, 55.23, 54.02, 51.98, 32.51, 29.56, 29.39, 23.39. LC-MS (ESI) m/z:544.1 [M+H]$^+$.

Example 120: methyl (*S*)-3-(8-bromo-6-(2-fluorophenyl)-1-(2-((pyrrolidin-1-yl)ethyl)thio)-4*H*-benzo[*f*][1,2,4]triazolo[4,3-*a*][1,4]diazepin-4-yl)propionate (compound 120)

$^1$H NMR (300 MHz, $CDCl_3$-$d_1$) δ: 7.72 (dd, *J* = 8.4, 2.6 Hz, 1H), 7.61 - 7.56 (m, 2H), 7.55 - 7.43 (m, 2H), 7.28 (td, *J* = 7.5, 1.5 Hz, 1H), 7.21 (dd, *J* = 7.8, 1.5 Hz, 1H), 6.10 (t, *J* = 5.2 Hz, 1H), 3.64 (s, 3H), 3.45 (t, *J* = 5.1 Hz, 2H), 2.74 (t, *J* = 4.9 Hz, 4H), 2.65 - 2.51 (m, 6H), 1.89-182 (m, 4H). $^{13}$C NMR (75 MHz, $CDCl_3$-$d_1$) δ 173.65, 163.32, 159.17, 154.22, 147.98, 136.64, 136.54, 132.59, 131.54, 130.23, 128.57, 127.70, 124.00, 121.79, 120.47, 115.76, 58.29, 55.23, 54.02, 51.93, 32.18, 29.54, 29.30, 23.40. LC-MS (ESI) m/z:572.1 [M+H]$^+$.

Example 121: methyl (*S*)-3-(8-bromo-6-(2-chlorophenyl)-1-((2-(pyrrolidin-1-yl)ethyl)thio)-4*H*-benzo[*f*][1,2,4]triazolo[4,3-*a*][1,4]diazepin-4-yl)propionate (compound 121)

$^1$H NMR (300 MHz, $CDCl_3$-$d_1$) δ: 7.75 (dd, *J* = 8.4, 2.6 Hz, 1H), 7.68 - 7.58 (m, 2H), 7.55 (dd, *J* = 7.7, 1.6 Hz, 1H), 7.49 (dd, *J* = 7.8, 1.6 Hz, 1H), 7.40 (td, *J* = 7.6, 1.6 Hz, 1H), 7.33 (td, *J* = 7.5, 1.5 Hz, 1H), 6.11 (t, *J* = 5.2 Hz, 1H), 3.64 (s, 3H), 3.45 (t, *J* = 5.1 Hz, 2H), 2.74 (t, *J* = 4.9 Hz, 4H), 2.65 - 2.51 (m, 6H), 1.86 (td, *J* = 4.9, 2.7 Hz, 4H). $^{13}$C NMR (75 MHz, $CDCl_3$-$d_1$) δ 173.57, 160.33, 154.78, 149.12, 137.49, 136.40, 136.44, 134.71, 133.11, 131.17, 130.63, 129.36, 128.19, 126.09, 121.84, 120.47, 57.87, 55.33, 54.02, 51.93, 32.14, 29.54, 29.39, 23.33. LC-MS (ESI) m/z:588.0 [M+H]$^+$. Example 122: methyl (*S*)-3-(8-chloro-6-(2-chlorophenyl)-1-(2-((piperidin-1-yl)ethyl)thio)-4*H*-benzo[*f*][1,2,4]triazolo[4,3-*a*][1,4]diazepin-4-yl)propionate (compound 122)

$^1$H NMR (300 MHz, $CDCl_3$-$d_1$) δ: 7.77 (d, *J* = 8.7 Hz, 1 H), 7.61 (dd, *J* = 2.4 Hz, 8.7 Hz, 1 H), 7.57 - 7.53 (m, 1 H), 7.41 (dd, *J* = 5.6, 3.0 Hz, 2 H), 7.37 (d, *J* = 3.6 Hz, 1 H), 7.17 (d, *J* = 2.4 Hz, 1 H), 4.26 (t, *J* = 6.9 Hz, 1 H), 3.67 (s, 3 H), 3.50 (t, *J* = 7.1 Hz, 2 H), 2.83 (t, *J* = 3.7 Hz, 4 H), 2.74 (t, *J* = 7.1 Hz, 2 H), 2.47 (s, 4 H), 1.62 - 1.56 (m, 4 H), 1.45 (s, 2 H). $^{13}$C NMR (75 MHz, $CDCl_3$-$d_1$) δ 173.63, 160.77, 154.20, 148.15, 138.23, 135.93, 134.66, 132.61, 130.83, 130.54, 130.43, 129.22, 129.10, 128.13, 126.35, 122.71, 57.76, 54.95, 54.06, 51.97, 32.18, 29.54, 29.39, 26.64, 23.96. LC-MS (ESI) m/z: 558.2[M+H]$^+$.

Example 123: methyl (*S*)-3-(8-bromo-6-(2-fluorophenyl)-1-(2-((piperidin-1-yl)ethyl)thio)-4*H*-benzo[*f*][1,2,4]triazolo[4,3-*a*][1,4]diazepin-4-yl)propionate (compound 123)

$^1$H NMR (300 MHz, $CDCl_3$-$d_1$) δ: 7.73 (dd, *J* = 8.4, 2.6 Hz, 1H), 7.62 - 7.56 (m, 2H), 7.53 - 7.43 (m, 2H), 7.29 (td, *J* = 7.5, 1.5 Hz, 1H), 7.17 (dd, *J* = 7.9, 1.5 Hz, 1H), 6.10 (t, *J* = 5.2 Hz, 1H), 3.64 (s, 3H), 3.45 (t, *J* = 5.1 Hz, 2H), 2.68 - 2.51 (m, 10H), 1.58-1.54 (m, 4H), 1.48 - 1.37 (m, 2H). $^{13}$C NMR (75 MHz, $CDCl_3$-$d_1$) δ 173.60, 163.21, 159.19, 154.15, 148.12, 136.35, 136.54, 132.44, 131.52, 130.23, 128.53, 127.70, 124.00, 121.85, 120.25, 115.78, 58.29, 54.90, 54.06, 51.93, 32.18, 29.54, 29.30, 26.83, 24.03. LC-MS (ESI) m/z: 586.1[M+H] $^+$.

Example 124: methyl (*S*)-3-(8-chloro-6-(2-chlorophenyl)-1-(2-((pyridin-4-yl)methyl)thio)-4*H*-benzo[*f*][1,2,4]triazolo[4,3-*a*][1,4]diazepin-4-yl)propionate (compound 124)

$^1$H NMR (300 MHz, $CDCl_3$-$d_1$) δ: 8.47 (d, *J* = 5.2 Hz, 2H), 7.93 - 7.83 (m, 1H), 7.78 (d, *J* = 8.7 Hz, 1H), 7.67 - 7.56 (m, 1H), 7.56 - 7.47 (m, 2H), 7.46 - 7.34 (m, 3H), 7.23 - 7.09 (m, 1H), 4.64 - 4.43 (m, 2H), 4.30 (t, *J* = 6.2 Hz, 1H), 3.59 (s, 3H), 2.67 - 2.57 (m, 4H). $^{13}$C NMR (75 MHz, $CDCl_3$-$d_1$) δ 173.65, 161.70, 154.16, 149.79, 147.48, 146.26, 138.07, 135.66, 134.71, 132.61, 130.82, 130.51, 130.43, 129.36, 129.04, 128.19, 126.36, 124.08, 122.76, 57.76, 51.96, 39.84, 29.54, 29.39. LC-MS (ESI) m/z: 538.1[M+H]$^+$.

Example 125: methyl (*S*)-3-(8-bromo-6-(2-fluorophenyl)-1-(2-((pyridin-4-yl)methyl)thio)-4*H*-benzo[*f*][1,2,4]triazolo[4,3-*a*][1,4]diazepin-4-yl)propionate (compound 125)

$^1$H NMR (300 MHz, $CDCl_3$-$d_1$) δ: 8.62 - 8.57 (m, 2H), 7.73 (dd, *J* = 8.4, 2.6 Hz, 1H), 7.59 (d, *J* = 2.5 Hz, 1H), 7.58 - 7.51 (m, 2H), 7.47 (td, *J* = 7.7, 1.6 Hz, 1H), 7.33 - 7.26 (m, 3H), 7.26 - 7.20 (m, 1H), 6.10 (t, *J* = 5.2 Hz, 1H), 4.53

(s, 2H), 3.64 (s, 3H), 2.67 - 2.57 (m, 2H), 2.57 - 2.51 (m, 2H). $^{13}$C NMR (75 MHz, $CDCl_3$-*d*$_1$) δ 173.62, 163.35, 159.17, 154.16, 149.79, 147.48, 146.26, 136.60, 136.54, 132.67, 131.51, 130.23, 128.53, 127.70, 124.09, 124.00, 121.79, 120.47, 115.76, 58.29, 51.92, 39.84, 29.54, 29.30. LC-MS (ESI) m/z:566.4 $[M+H]^+$.

Example 126: methyl (*S*)-3-(8-bromo-6-(pyridin-2-yl)-1-(((pyridin-4-yl)methyl)thio)-4*H*-benzo[*f*][1,2,4]triazolo[4,3-*a*][1,4]diazepin-4-yl)propionate (compound 126)

$^1$H NMR (300 MHz, $CDCl_3$-*d*$_1$) δ: 8.62 - 8.58 (m, 1H), 8.53 - 8.46 (m, 2H), 8.18 (d, *J* = 7.9 Hz, 1H), 7.84 (td, *J* = 7.8, 1.7 Hz, 1H), 7.75 (dd, *J* = 8.7, 2.2 Hz, 1H), 7.67 (d, *J* = 2.2 Hz, 1H), 7.54 (d, *J* = 8.7 Hz, 1H), 7.42-7.38 (m, 1H), 7.36 - 7.32 (m, 2H), 4.55 - 4.37 (m, 2H), 4.30 - 4.21 (m, 1H), 3.69 (s, 3H), 2.93 - 2.77 (m, 4H). $^{13}$C NMR (75 MHz, $CDCl_3$-*d*$_1$) δ 173.41, 161.93, 156.42, 154.52, 149.59, 147.48, 147.33, 146.06, 137.37, 137.19, 136.60, 135.48, 128.31, 125.25, 124.08, 122.76, 122.61, 120.94, 58.05, 51.96, 39.84, 29.54, 29.34. LC-MS (ESI) m/z: 549.4$[M+H]^+$.

Example 127: methyl (*S*)-3-(8-chloro-6-(2-chlorophenyl)-1-(2-((4,4-difluoropiperidin-1-yl)ethyl)thio)-4*H*-benzo[*f*][1,2,4]triazolo[4,3-*a*][1,4]diazepin-4-yl)propionate (compound 127)

$^1$H NMR (300 MHz, $CDCl_3$-*d*$_1$) δ: 7.68 - 7.59 (m, 2H), 7.62 - 7.52 (m, 2H), 7.53 - 7.45 (m, 1H), 7.41 - 7.32 (m, 2H), 6.11 (t, *J* = 5.2 Hz, 1H), 3.66 (s, 3H), 3.46 (t, *J* = 5.2 Hz, 2H), 2.74 - 2.62 (m, 6H), 2.65 - 2.51 (m, 4H), 2.25 - 2.12 (m, 4H). $^{13}$C NMR (75 MHz, $CDCl_3$-*d*$_1$) δ 172.98, 160.92, 154.16, 148.12, 137.98, 135.93, 134.65, 132.65, 131.03, 130.83, 130.56, 129.18, 129.10, 128.07, 126.30, 122.69, 118.58, 57.87, 54.60, 51.78, 48.83, 32.57, 31.95, 9.54, 29.49. LC-MS (ESI) m/z: 594.4$[M+H]^+$.

Example 128: methyl (*S*)-3-(8-bromo-6-(2-fluorophenyl)-1-(2-((4,4-difhioropiperidin-1-yl)ethyl)thio))-4*H*-benzo[*f*][1,2,4]triazolo[4,3-*a*][1,4]diazepin-4-yl)propionate (compound 128)

$^1$H NMR (300 MHz, $CDCl_3$-d$_1$) δ: 7.74 (dd, *J* = 8.4, 2.4 Hz, 1H), 7.62 - 7.54 (m, 2H), 7.54 - 7.44 (m, 2H), 7.33 - 7.21 (m, 2H), 4.32 (t, *J* = 5.3, 6.8 Hz, 1H), 3.66 (s, 3H), 3.46 (t, *J* = 5.2, 7.4 Hz, 2H), 2.74 - 2.68 (m, 3H), 2.68 - 2.62 (m, 4H), 2.61 (dd, *J* = 1.9, 1.2 Hz, 1H), 2.61 - 2.51 (m, 2H), 2.25 - 2.12 (m, 4H). $^{13}$C NMR (75 MHz, $CDCl_3$-d$_1$) δ 173.33, 163.02, 159.33, 154.16, 148.12, 136.55, 136.54, 132.67, 131.53, 130.23, 128.58, 127.70, 123.91, 121.85, 120.45, 118.58, 115.77, 58.29, 54.60, 51.94, 48.71, 32.61, 32.23, 29.34, 29.63. LC-MS (ESI) m/z: 622.5$[M+H]^+$.

Example 129: methyl (*S*)-3-(8-chloro-6-(2-chlorophenyl)-1-((2-(4-methylpiperazin-1-y1)2-oxyethy1)thio)-4*H*-benzo[*f*][1,2,4]triazolo[4,3-*a*][1,4]diazepin-4-yl)propionate (compound 129)

$^1$H NMR (300 MHz, $CDCl_3$-*d*$_1$) δ: 7.72 (d, *J* = 8.4 Hz, 1H), 7.64 - 7.57 (m, 2H), 7.55 (d, *J* = 2.4 Hz, 1H), 7.53 - 7.45 (m, 1H), 7.41 - 7.32 (m, 2H), 6.11 (t, *J* = 5.2 Hz, 1H), 4.08 (d, *J* = 2.7 Hz, 2H), 3.66 (s, 3H), 3.49 (t, *J* = 5.3 Hz, 4H), 2.65 - 2.50 (m, 8H), 2.23 (s, 3H). $^{13}$C NMR (75 MHz, $CDCl_3$-*d*$_1$) δ 173.37, 171.54, 160.92, 154.17, 147.98, 137.98, 136.05, 134.72, 132.65, 131.16, 130.83, 130.56, 129.18, 129.10, 128.33, 126.44, 122.74, 56.97, 54.43, 51.80, 46.07, 45.25, 35.71, 29.47, 29.78. LC-MS (ESI) m/z: 587.5$[M+H]^+$.

Example 130: methyl (*S*)-3-(8-chloro-6-(2-chlorophenyl)-1-(2-(4-methylpiperazine-1-carbony1)thio)-4*H*-benzo[*f*][1,2,4]triazolo[4,3-*a*][1,4]diazepin-4-yl)propionate (compound 130)

$^1$H NMR (300 MHz, $CDCl_3$-*d*$_1$) δ: 7.69 (d, *J* = 8.3 Hz, 1H), 7.61 - 7.54 (m, 2H), 7.54 - 7.45 (m, 2H), 7.41 - 7.30 (m, 2H), 6.12 (t, *J* = 5.4 Hz, 1H), 3.77 - 3.62 (m, 7H), 2.74 - 2.63 (m, 2H), 2.60 - 2.45 (m, 6H), 2.29 (s, 3H). $^{13}$C NMR (75 MHz, $CDCl_3$-*d*$_1$) δ 173.22, 164.87, 160.77, 154.72, 143.84, 137.98, 136.55, 134.71, 132.60, 130.83, 130.54, 130.43, 129.20, 129.10, 128.16, 126.36, 122.85, 57.81, 54.27, 52.08, 47.39, 45.36, 29.54, 29.57. LC-MS (ESI) m/z: 573.1$[M+H]^+$.

Example 131: methyl (*S*)-3-(8-bromo-6-(2-fluorophenyl)-1-(2-(4-methylpiperazine-1-carbonyl)thio))-4*H*-benzo [f][1,2,4]triazolo[4,3-*a*][1,4]diazepin-4-yl)propionate (compound 131)

$^1$H NMR (300 MHz, $CDCl_3$-*d*$_1$) δ: 7.75 (dd, *J* = 8.4, 2.6 Hz, 1H), 7.59 (d, *J* = 2.5 Hz, 1H), 7.56 - 7.44 (m, 3H), 7.28 (td, *J* = 7.6, 1.4 Hz, 1H), 7.22 (dd, *J* = 7.7, 1.3 Hz, 1H), 6.12 (t, *J* = 5.4 Hz, 1H), 3.77 - 3.68 (m, 2H), 3.68 - 3.63 (m, 5H), 2.74 - 2.63 (m, 2H), 2.57 (ddd, *J* = 7.9, 5.5, 1.0 Hz, 2H), 2.55 - 2.45 (m, 4H), 2.29 (s, 3H). $^{13}$C NMR (75 MHz, $CDCl_3$-*d*$_1$) δ: 173.61, 167.58, 160.70, 148.74, 143.80, 143.14, 132.49, 131.38, 131.10, 129.83, 129.44, 129.40, 124.54, 124.20, 121.80, 115.88, 115.73, 54.33, 54.18, 51.71, 51.50, 44.98, 33.02, 31.91. LC-MS (ESI) m/z: 601.10 $[M+H]^+$.

Example 132: methyl (*S*)-3-(8-chloro-6-(2-chlorophenyl)-1-(3-((diethylamino)propyl)thio)-4*H*-benzo[*f*][1,2,4]triazolo[4,3-*a*][1,4]diazepin-4-yl)propionate (compound 132)

$^1$H NMR (300 MHz, $CDCl_3$-$d_1$) δ: 7.91 (d, *J* = 1.6 Hz, 1H), 7.62 (dd, *J* = 7.2, 2.2 Hz, 1H), 7.51 - 7.37 (m, 5H), 5.32 (s, 1H), 3.68 (s, 3H), 3.25 (d, *J* = 2.5 Hz, 2H), 2.65 - 2.49 (m, 6H), 2.44 - 2.28 (m, 4H), 1.99 (d, *J* = 2.2 Hz, 2H), 0.99 (s, 6H). $^{13}$C NMR (75 MHz, $CDCl_3$-$d_1$) δ: 173.62, 145.70, 144.01, 141.27, 138.92, 132.79, 130.73, 130.17, 129.99, 129.43, 129.37, 128.94, 128.72, 128.62, 124.77, 120.33, 54.20, 53.56, 51.40, 47.08, 33.51, 33.02, 31.91, 30.11, 11.83. LC-MS (ESI) m/z: 560.16 $[M+H]^+$.

Example 133: methyl (*S*)-3-(8-chloro-6-(2-chlorophenyl)-1-(2-((dimethylamino)ethyl)thio)-4*H*-benzo[*f*][1,2,4]triazolo[4,3-*a*][1,4]diazepin-4-yl)propionate (compound 133)

$^1$H NMR (300 MHz, $CDCl_3$-$d_1$) δ: 7.75 (d, *J*= 8.7 Hz, 1H), 7.59 (dd, *J* = 8.7, 2.4 Hz, 1H), 7.55 (dd, *J* = 5.8, 3.4 Hz, 1H), 7.45 - 7.39 (m, 2H), 7.37 - 7.32 (m, 1H), 7.17 (d, *J* = 2.3 Hz, 1H), 4.26 (t, *J* = 6.0Hz, 1 H), 3.67 (s, 3 H), 3.62 - 3.40 (m, 2 H), 2.91 - 2.78 (m, 4H), 2.72 (h, *J* = 6.1 Hz, 2H), 2.30 (s, 6H). $^{13}$C NMR (75 MHz, $CDCl_3$-$d_1$) δ: 173.88, 145.68, 144.42, 143.87, 138.88, 133.17, 130.68, 130.54, 129.82, 129.72, 129.46, 129.41, 128.74, 124.66, 122.11, 54.20, 52.83, 51.40, 42.92, 37.46, 33.02, 31.93. LC-MS (ESI) m/z: 518.11 $[M+H]^+$.

Example 134: methyl (*S*)-3-(8-bromo-6-(2-fluorophenyl)-1-(2-((dimethylamino)ethyl)thio)-4*H*-benzo[*f*][1,2,4]triazolo[4,3-*a*][1,4]diazepin-4-yl)propionate (compound 134)

$^1$H NMR (300 MHz, $CDCl_3$-$d_1$) δ: 7.73 (dd, *J* = 8.5, 2.5 Hz, 1H), 7.61 - 7.55 (m, 2H), 7.54 - 7.43 (m, 2H), 7.29 (td, *J* = 7.7, 1.5 Hz, 1H), 7.23 (dd, *J* = 7.9, 1.5 Hz, 1H), 4.30 (t, *J* = 5.2 Hz, 1H), 3.64 (s, 3H), 3.46 (t, *J* = 5.2 Hz, 2H), 2.71 (dt, *J* = 12.4, 5.2 Hz, 1H), 2.69 - 2.57 (m, 3H), 2.60 - 2.51 (m, 2H), 2.29 (s, 6H). $^{13}$C NMR (75 MHz, $CDCl_3$-$d_1$) δ: 173.78, 160.38, 145.61, 144.39, 143.49, 134.16, 131.97, 130.87, 130.41, 130.05, 129.44, 124.54, 124.20, 122.40, 115.82, 115.55, 54.33, 52.69, 51.40, 42.92, 37.46, 33.02, 30.26. LC-MS (ESI) m/z: 546.09 $[M+H]^+$.

Example 135: methyl (*S*)-3-(8-chloro-6-(2-chlorophenyl)-1-(2-((4-hydroxypiperidin-1-yl)ethyl)thio)-4*H*-benzo[*f*][1,2,4]triazolo[4,3-*a*][1,4]diazepin-4-yl)propionate (compound 135)

$^1$H NMR (300 MHz, $CDCl_3$-$d_1$) δ: 7.91 (d, *J* = 2.0 Hz, 1H), 7.66 (dd, *J* = 7.0, 2.4 Hz, 1H), 7.50 - 7.35 (m, 4H), 7.29 (d, *J* = 7.5 Hz, 1H), 5.25 (s, 1H), 3.68 (s, 3H), 3.63 - 3.51 (m, 2H), 3.48 (d, *J* = 0.9 Hz, 2H), 2.62 (d, *J* = 2.4 Hz, 4H), 2.55 (s, 2H), 2.44 - 2.32 (m, 2H), 2.33 (d, *J* = 7.0 Hz, 1H), 2.25 (d, *J* = 12.5 Hz, 1H), 1.79 (d, *J* = 7.5 Hz, 4H). $^{13}$C NMR (75 MHz, $CDCl_3$-$d_1$) δ:173.61, 145.63, 145.00, 143.04, 138.86, 132.79, 130.72, 130.15, 129.99, 129.37, 128.72, 128.60, 128.19, 124.67, 119.77, 67.85, 54.33, 51.50, 50.74, 50.54, 33.99, 33.02, 32.94, 31.92. LC-MS (ESI) m/z: 574.14 $[M+H]^+$.

Example 136: methyl (*S*)-3-(8-chloro-6-(2-chlorophenyl)-1-(2-((4-methylpiperidine-2-carbonyl)ethyl)thio)-4*H*-benzo[*f*][1,2,4]triazolo[4,3-*a*][1,4]diazepin-4-yl)propionate (compound 136)

$^1$H NMR (300 MHz, $CDCl_3$-$d_1$) δ: 7.98 (d, *J* = 2.0 Hz, 1H), 7.66 - 7.59 (m, 1H), 7.50 - 7.44 (m, 1H), 7.46 - 7.33 (m, 3H), 7.29 (d, *J* = 7.5 Hz, 1H), 5.25 (s, 1H), 4.42 (s, 1H), 3.68 (s, 3H), 2.62 (d, *J* = 10.6 Hz, 2H), 2.49 (d, *J* = 12.3 Hz, 1H), 2.41 - 2.30 (m, 5H), 2.25 (d, *J* = 12.5 Hz, 1H), 2.02 (d, *J* = 21.1 Hz, 2H), 1.65 (s, 1H), 1.59 (d, *J* = 4.6 Hz, 2H), 1.53 (s, 1H). $^{13}$C NMR (75 MHz, $CDCl_3$-$d_1$) δ: 175.67, 173.61, 148.74, 145.69, 143.49, 138.91, 132.79, 130.71, 130.16, 129.99, 129.84, 129.37, 128.72, 128.60, 128.19, 124.68, 122.11, 62.34, 54.53, 54.33, 51.45, 41.64, 33.02, 31.91, 28.57, 26.60, 22.95. LC-MS (ESI) m/z:571.12$[M+H]^+$.

Example 137: (*S*)-3-(8-chloro-6-(2-chlorophenyl)-1-thio-2,4-dihydro-1*H*-benzo[*f*][1,2,4]triazolo[4,3-*a*][1,4]diazepin-4-yl)propionic acid-2-morpholinoethyl ester (compound 137)

$^1$H NMR (300 MHz, $CDCl_3$-$d_1$) δ: 13.8(s, 1H), 7.80 (d, *J* = 8.5 Hz, 1H), 7.58 - 7.50 (m, 2H), 7.50 - 7.45 (m, 1H), 7.45 - 7.36 (m, 2H), 7.34 (d, *J* = 2.4 Hz, 1H), 4.56 (t, *J* = 6.0 Hz, 1H), 4.22 (t, *J* = 6.5 Hz, 2H), 3.60 - 3.48 (m, 4H), 2.85 (t, *J* = 6.4, 8.2 Hz, 2H), 2.72 - 2.57 (m, 4H), 2.53 - 2.45 (m, 2H), 2.43 - 2.28 (m, 2H). $^{13}$C NMR (75 MHz, $CDCl_3$-$d_1$) δ: 173.82, 172.99, 145.29, 143.07, 143.02, 132.79, 130.64, 130.52, 129.38, 129.37, 129.30, 128.94, 128.72, 128.62, 124.74, 122.85, 66.04, 65.90, 55.35, 54.26, 53.77, 30.86, 30.56. LC-MS (ESI) m/z: 546.11 $[M+H]^+$.

Example 138: methyl (*S*)-3-(8-chloro-6-(2-chlorophenyl)-1-(2-((1*H*-imidazol-1-yl)ethyl)thio)-4*H*-benzo[*f*][1,2,4]triazolo[4,3-*a*][1,4]diazepin-4-yl)propionate (compound 138)

$^1$H NMR (300 MHz, $CDCl_3$-$d_1$) δ: 7.85 (s, 1H), 7.63 (d, *J* = 8.3 Hz, 1H), 7.59 - 7.51 (m, 3H), 7.47 (dd, *J* = 7.7, 1.7 Hz, 1H), 7.38 (td, *J* = 23.6, 7.4, 1.6 Hz, 2H), 7.03 (s, 2H), 6.10 (t, *J* = 5.2 Hz, 1H), 4.27 (td, *J* = 5.0, 1.6 Hz, 2H), 3.66 - 3.60 (m, 5H), 2.66 - 2.51 (m, 4H). $^{13}$C NMR (75 MHz, $CDCl_3$-$d_1$) δ:173.87, 145.66, 145.05, 143.04, 139.39, 138.93, 133.92, 132.79, 130.68, 130.55, 129.71, 129.46, 129.44, 129.37, 129.31, 128.94, 128.62, 124.73, 120.33, 54.20, 51.40, 49.21, 36.04, 33.07, 31.90. LC-MS (ESI) m/z: 541.09 $[M+H]^+$.

Example 139: methyl (*S*)-3-(8-bromo-6-(2-fluorophenyl)-1-(2-((1*H*-imidazol-1-yl)ethyl)thio)-4*H*-benzo[*f*][1,2,4]triazolo[4,3-*a*][1,4]diazepin-4-yl)propionate (compound 139)

$^1$H NMR (300 MHz, $CDCl_3$-$d_1$) δ: 7.85 (s, 1H), 7.72 (dd, *J* = 8.4, 2.6 Hz, 1H), 7.61 - 7.54 (m, 2H), 7.52 (dd, *J* = 7.9, 1.5 Hz, 1H), 7.47 (td, *J* = 7.7, 1.6 Hz, 1H), 7.28 (td, *J* = 7.5, 1.5 Hz, 1H), 7.21 (dd, *J* = 7.8, 1.5 Hz, 1H), 7.03 (s, 2H), 6.10 (t, *J* = 5.2 Hz, 1H), 4.27 (td, *J* = 5.0, 1.7 Hz, 2H), 3.66 - 3.60 (m, 5H), 2.67 - 2.51 (m, 4H). $^{13}$C NMR (75 MHz, $CDCl_3$-$d_1$) δ 172.39, 160.89, 154.16, 148.42, 137.37, 136.33, 136.43, 134.20, 132.08, 131.13, 130.83, 129.13, 128.19, 126.39, 121.84, 120.47, 57.37, 55.24, 54.02, 51.93, 32.18, 28.94, 29.34, 23.27. LC-MS (ESI) m/z:569.1$[M+H]^+$.

Example 140: methyl (*S*)-3-(8-chloro-6-(2-chlorophenyl)-1-(2-((1*H*-pyrrol-1-yl)ethyl)thio)-4*H*-benzo[*f*][1,2,4]triazolo[4,3-*a*][1,4]diazepin-4-yl)propionate (compound 140)

$^1$H NMR (300 MHz, $CDCl_3$-$d_1$) δ: 7.87 (dd, *J* = 8.7, 2.4 Hz, 1 H), 7.78 (d, *J* = 8.7 Hz, 1 H), 7.65 (dd, *J* = 5.9, 3.3 Hz, 1 H), 7.55 - 7.49 (m, 2 H), 7.49 - 7.42 (m, 1 H), 7.18 (d, *J* = 2.3 Hz, 1 H), 6.76 (t, *J* = 2.1 Hz, 2 H), 5.99 (t, *J* = 2.1 Hz, 2 H), 4.32 (t, *J* = 6.4 Hz, 1 H), 4.24 (t, *J* = 6.5 Hz, 2 H), 3.60 (s, 3 H), 2.68 (t, *J* = 6.4 Hz, 2 H), 2.52-2.50 (m, 4 H). $^{13}$C NMR (75 MHz, $CDCl_3$-$d_1$) δ:173.90, 145.62, 145.01, 143.04, 138.89, 132.79, 130.67, 130.54, 129.72, 129.46, 129.44, 129.37, 128.94, 128.62, 125.93, 124.70, 120.33, 119.18, 54.20, 53.61, 51.39, 36.04, 33.00, 31.89. LC-MS (ESI) m/z: 540.09 $[M+H]^+$.

Example 141: methyl (*S*)-3-(8-chloro-6-(2-chlorophenyl)-1-((1-methyl-1*H*-imidazol-4-yl)thio)-4*H*-benzo[*f*][1,2,4]triazolo[4,3-*a*][1,4]diazepin-4-yl)propionate (compound 141)

$^1$H NMR (300 MHz, $CDCl_3$-$d_1$) δ: 8.03 (s, 1H), 7.97 - 7.90 (m, 2H), 7.61 (dd, *J* = 7.5, 2.0 Hz, 1H), 7.53 - 7.34 (m, 5H), 5.17 (s, 1H), 3.68 (s, 6H), 2.56 - 2.45 (m, 2H), 2.30 - 2.19 (m, 2H). $^{13}$C NMR (75 MHz, $CDCl_3$-$d_1$) δ:173.94, 156.53, 145.64, 145.36, 144.40, 138.85, 137.66, 133.15, 132.20, 130.65, 130.54, 129.82, 129.70, 129.46, 129.44, 128.94, 128.70, 124.72, 122.11, 54.20, 51.40, 33.80, 33.05, 30.26; LC-MS (ESI) m/z: 527.07 $[M+H]^+$.

Example 142: methyl (*S*)-3-(8-chloro-6-(2-chlorophenyl)-1-((ethyl(methyl)carbamoyl)thio)-4*H*-benzo[*f*][1,2,4]triazolo[4,3-*a*][1,4]diazepin-4-yl)propionate (compound 142)

1HNMR (300MHz, $CDCl_3$-$d_1$) δ 7.75 (d, J = 8.4 Hz, 1H), 7.60 - 7.51 (m, 3H), 7.50 (dd, J = 7.8, 1.4 Hz, 1H), 7.41 (td, J = 7.7, 1.8 Hz, 1H), 7.34 (td, J = 7.5, 1.4 Hz, 1H), 4.30 (t, J = 8.2 Hz, 1H), 3.64 (s, 3H), 3.45 (q, J = 7.2 Hz, 2H), 3.02 (s, 3H), 2.75 - 2.59 (m, 2H), 2.59-2.53 (m, 2H), 1.22 (t, J = 7.2 Hz, 3H). $^{13}$C NMR (75 MHz, $CDCl_3$-$d_1$) δ:173.90, 162.05, 148.74, 146.39, 143.80, 138.87, 133.16, 130.61, 130.53, 129.82, 129.75, 129.46, 129.45, 129.25, 128.74, 124.69, 124.25, 54.19, 51.41, 43.61, 34.27, 32.98, 30.26, 12.47. LC-MS (ESI) m/z: 532.09 $[M+H]^+$.

Example 143: methyl (*S*)-3-(8-chloro-6-(2-chlorophenyl)-1-(3-((dimethylamino)-3-oxopropyl)thio)-4*H*-benzo[*f*][1,2,4]triazolo[4,3-*a*][1,4]diazepin-4-yl)propionate (compound 143)

$^1$H NMR (300 MHz, $CDCl_3$-$d_1$) δ: 7.89 (d, *J* = 1.9 Hz, 1H), 7.61 (dd, *J* = 7.1, 2.2 Hz, 1H), 7.54 - 7.37 (m, 6H), 5.32 (s, 1H), 3.68 (s, 3H), 3.39 (s, 2H), 2.86 (s, 5H), 2.80 - 2.69 (m, 2H), 2.44 - 2.28 (m, 3H), 2.25 (d, *J* = 12.5 Hz, 1H). $^{13}$C NMR (75 MHz, $CDCl_3$-$d_1$) δ: 173.78, 172.42, 145.67, 144.02, 143.04, 138.84, 132.79, 130.60, 130.55, 129.74, 129.46, 129.37, 129.02, 128.93, 128.62, 124.74, 120.33, 54.22, 51.42, 36.42, 35.74, 33.03, 31.88, 31.67. LC-MS (ESI) m/z:546.1$[M+H]^+$.

Example 144: methyl (*S*)-3-(8-chloro-6-(2-chlorophenyl)-1-(((4,5-dihydro-1*H*-imidazol-2-yl)methyl)thio)-4*H*-benzo[*f*][1,2,4]triazolo[4,3-*a*][1,4]diazepin-4-yl)propionate (compound 144)

$^1$H NMR (300 MHz, $CDCl_3$-$d_1$) δ: 7.91 (d, *J* = 2.0 Hz, 1H), 7.61 (dd, *J* = 7.2, 2.3 Hz, 1H), 7.54 - 7.37 (m, 4H),

7.31 (d, $J$ = 7.5 Hz, 1H), 6.83 (s, 1H), 5.17 (s, 1H), 4.32 (s, 2H), 3.68 (s, 3H), 3.59 - 3.50 (m, 4H), 2.49 (d, $J$ = 12.5 Hz, 1H), 2.40 (d, $J$ = 12.5 Hz, 1H), 2.30 - 2.19 (m, 2H). $^{13}C$ NMR (75 MHz, $CDCl_3$-$d_1$) δ: 173.87, 159.44, 145.60, 143.90, 141.65, 138.93, 133.18, 130.69, 130.56, 129.82, 129.73, 129.46, 129.44, 128.91, 128.70, 124.73, 122.11, 54.18, 51.38, 44.30,44.27,41.81,33.04,31.95. LC-MS (ESI) m/z: 529.1 $[M+H]^+$.

Pharmacological Activity Assay:

In anesthetic drug experiments, latency generally refers to the time from the start of administration to the loss of consciousness of the subject. The shorter latency is preferred, indicating a rapid onset of action after administration. The duration of anesthesia generally refers to the duration of time from the loss of consciousness to the restoration of consciousness of the subject. The duration of the drug will vary from animal model to animal species. Too long anesthesia may produce adverse cardiovascular and respiratory responses, such as adverse neurological effects to the subject, including sleepiness and dizziness; meanwhile, too short duration of anesthesia may affect the anesthesia effect, causing problems such as increase of anesthesia dosage during the operation.

Example 145. Test for Drug-Induced Disappearance of Righting Reflex in Mice

KM mice (female, 18-25 g) were randomly grouped and subjected to a single administration by rapid bolus via the tail vein. The latency and duration of disappearance of righting reflex in the mice were recorded. The experimental results are shown in Table 1 below.

Table 1. Test for drug-induced disappearance of righting reflex in mice

| Compound (dose) | Latency (min) | Duration (min) | Compound (dose) | Latency (min) | Duration (min) |
|---|---|---|---|---|---|
| Compound 1 (30 mg/kg) | 0.26 | 3.51 | Compound 69 (30 mg/kg) | 0.90 | 21.61 |
| Tautomer of compound 1 (30 mg/kg) | 0.21 | 3.47 | Compound 70 (30 mg/kg) | 1.01 | 18.61 |
| Compound 2 (30 mg/kg) | 0.58 | 5.27 | Compound 71 (60 mg/kg) | 0.56 | 12.10 |
| Tautomer of compound 2 (30 mg/kg) | 1.10 | 10.35 | Compound 72 (30 mg/kg) | 0.42 | 13.12 |
| Compound 3 (30 mg/kg) | 0.48 | 7.37 | Compound 72 (60 mg/kg) | 1.52 | 10.67 |
| Tautomer of compound 3 (30 mg/kg) | 0.65 | 8.27 | Compound 73 (30 mg/kg) | 0.86 | 12.12 |
| Compound 4 (30 mg/kg) | 0.88 | 9.21 | Compound 74 (30 mg/kg) | 0.47 | 6.16 |
| Tautomer of compound 4 (30 mg/kg) | 0.38 | 2.95 | Compound 75 (30 mg/kg) | 0.51 | 6.53 |
| Compound 5 (30 mg/kg) | 1.11 | 13.27 | Compound 76 (30 mg/kg) | 0.76 | 5.65 |
| Tautomer of compound 5 (30 mg/kg) | 0.73 | 7.82 | Compound 77 (30 mg/kg) | 0.38 | 5.69 |
| Compound 6 (30 mg/kg) | 0.59 | 9.93 | Compound 78 (30 mg/kg) | 1.05 | 12.67 |
| Tautomer of compound 6 (30 mg/kg) | 0.73 | 8.84 | Compound 79 (30 mg/kg) | 0.57 | 6.93 |
| Compound 7 (30 mg/kg) | 1.14 | 12.26 | Compound 80 (60 mg/kg) | 0.86 | 13.57 |
| Tautomer of compound 7 (30 mg/kg) | 0.95 | 9.22 | Compound 81 (60 mg/kg) | 0.21 | 6.41 |
| Compound 8 (30 mg/kg) | 0.36 | 8.35 | Compound 81 (100 mg/kg) | 0.22 | 12.38 |
| Compound 9 (30 mg/kg) | 0.84 | 12.12 | Compound 82 (30 mg/kg) | 0.31 | 8.37 |

(continued)

| Compound (dose) | Latency (min) | Duration (min) | Compound (dose) | Latency (min) | Duration (min) |
|---|---|---|---|---|---|
| Compound 10 (30 mg/kg) | 0.76 | 3.15 | Compound 83 (30 mg/kg) | 0.28 | 7.89 |
| Compound 11 (30 mg/kg) | 0.77 | 6.37 | Compound 83 (60 mg/kg) | 0.19 | 14.58 |
| Compound 11 (60 mg/kg) | 0.83 | 7.98 | Compound 84 (30 mg/kg) | 0.31 | 21.9 |
| Compound 12 (30 mg/kg) | 0.74 | 17.21 | Compound 85 (60 mg/kg) | 0.67 | 13.12 |
| Compound 13 (30 mg/kg) | 0.23 | 2.87 | Compound 86 (30 mg/kg) | 0.62 | 18.62 |
| Compound 14 (30 mg/kg) | 0.78 | 9.12 | Compound 87 (60 mg/kg) | 0.73 | 21.17 |
| Compound 15 (30 mg/kg) | 1.03 | 8.47 | Compound 88 (30 mg/kg) | 0.53 | 15.82 |
| Compound 16 (30 mg/kg) | 0.79 | 6.25 | Compound 89 (60 mg/kg) | 0.83 | 9.88 |
| Compound 16 (60 mg/kg) | 0.28 | 6.6 | Compound 90 (30 mg/kg) | 0.67 | 15.26 |
| Compound 17 (30 mg/kg) | 1.09 | 15.35 | Compound 91 (30 mg/kg) | 1.80 | 17.33 |
| Compound 18 (30 mg/kg) | 0.31 | 4.74 | Compound 92 (60 mg/kg) | 2.00 | 10.12 |
| Compound 18 (60 mg/kg) | 0.33 | 9.16 | Compound 93 (30 mg/kg) | 0.58 | 12.41 |
| Compound 19 (30 mg/kg) | 0.89 | 16.22 | Compound 94 (60 mg/kg) | 0.61 | 13.85 |
| Compound 20 (30 mg/kg) | 0.27 | 9.82 | Compound 95 (30 mg/kg) | 0.82 | 12.41 |
| Compound 21 (30 mg/kg) | 0.35 | 2.83 | Compound 96 (30 mg/kg) | 0.49 | 7.98 |
| Compound 21 (60 mg/kg) | 0.2 | 6.4 | Compound 97 (60 mg/kg) | 0.93 | 17.07 |
| Compound 22 (30 mg/kg) | 0.58 | 6.53 | Compound 98 (30 mg/kg) | 1.31 | 14.71 |
| Compound 23 (60 mg/kg) | 0.25 | 8.46 | Compound 99 (60 mg/kg) | 2.63 | 19.37 |
| Compound 23 (100 mg/kg) | 0.16 | 14.6 | Compound 100 (30 mg/kg) | 0.64 | 9.75 |
| Compound 24 (60 mg/kg) | 0.24 | 6.83 | Compound 101 (30 mg/kg) | 0.85 | 12.05 |
| Compound 25 (60 mg/kg) | 0.31 | 5.89 | Compound 102 (30 mg/kg) | 1.13 | 17.24 |
| Compound 26 (30 mg/kg) | 1.5 | 3.57 | Compound 102 (60 mg/kg) | 0.39 | 7.47 |
| Compound 26 (60 mg/kg) | 0.18 | 8.06 | Compound 103 (30 mg/kg) | 0.74 | 14.42 |
| Compound 27 (30 mg/kg) | 0.35 | 5.93 | Compound 104 (30 mg/kg) | 0.77 | 9.78 |
| Compound 28 (60 mg/kg) | 0.46 | 4.24 | Compound 105 (30 mg/kg) | 0.63 | 13.94 |

(continued)

| Compound (dose) | Latency (min) | Duration (min) | Compound (dose) | Latency (min) | Duration (min) |
|---|---|---|---|---|---|
| Compound 29 (30 mg/kg) | 0.57 | 9.25 | Compound 105 (60 mg/kg) | 0.49 | 13.17 |
| Compound 30 (30 mg/kg) | 0.42 | 7.44 | Compound 106 (30 mg/kg) | 0.33 | 12.37 |
| Compound 31 (30 mg/kg) | 2.85 | 8.98 | Compound 106 (60 mg/kg) | 0.09 | 3.26 |
| Compound 32 (30 mg/kg) | 0.73 | 20.73 | Compound 107 (30 mg/kg) | 0.45 | 13.64 |
| Compound 33 (60 mg/kg) | 0.21 | 6.41 | Compound 108 (60 mg/kg) | 0.59 | 11.73 |
| Compound 33 (100 mg/kg) | 0.22 | 12.38 | Compound 109 (60 mg/kg) | 0.97 | 13.27 |
| Compound 34 (30 mg/kg) | 0.3 | 21.9 | Compound 110 (30 mg/kg) | 0.22 | 10.16 |
| Compound 35 (60 mg/kg) | 0.18 | 40.26 | Compound 110 (60 mg/kg) | 0.21 | 15.27 |
| Compound 36 (60 mg/kg) | 0.34 | 8.82 | Compound 111 (30 mg/kg) | 0.67 | 18.36 |
| Compound 37 (30 mg/kg) | 0.49 | 7.63 | Compound 112 (30 mg/kg) | 0.78 | 19.21 |
| Compound 37 (60 mg/kg) | 0.21 | 7.68 | Compound 112 (60 mg/kg) | 0.46 | 12.62 |
| Compound 38 (30 mg/kg) | 0.67 | 12.94 | Compound 113 (60 mg/kg) | 0.58 | 9.49 |
| Compound 39 (60 mg/kg) | 0.19 | 9.67 | Compound 114 (30 mg/kg) | 0.41 | 20.32 |
| Compound 40 (30 mg/kg) | 0.37 | 8.94 | Compound 115 (15 mg/kg) | 0.18 | 1.26 |
| Compound 41 (60 mg/kg) | 0.40 | 8.59 | Compound 115 (30 mg/kg) | 0.13 | 5.02 |
| Compound 42 (30 mg/kg) | 0.65 | 4.68 | Compound 116 (30 mg/kg) | 0.11 | 10.89 |
| Compound 43 (30 mg/kg) | 0.38 | 12.38 | Compound 117 (30 mg/kg) | 0.14 | 2.03 |
| Compound 43 (60 mg/kg) | 0.84 | 7.25 | Compound 118 (30 mg/kg) | 0.32 | 15.44 |
| Compound 44 (30 mg/kg) | 1.28 | 16.27 | Compound 119 (30 mg/kg) | 0.46 | 30.86 |
| Compound 45 (30 mg/kg) | 0.65 | 11.37 | Compound 120 (30 mg/kg) | 0.29 | 19.47 |
| Compound 46 (30 mg/kg) | 0.69 | 10.83 | Compound 121 (60 mg/kg) | 0.18 | 16.67 |
| Compound 47 (60 mg/kg) | 0.82 | 10.52 | Compound 122 (30 mg/kg) | 0.30 | 28.06 |
| Compound 48 (60 mg/kg) | 1.25 | 8.54 | Compound 123 (15 mg/kg) | 0.30 | 1.38 |
| Compound 49 (30 mg/kg) | 0.73 | 9.65 | Compound 123 (30 mg/kg) | 0.23 | 53.03 |
| Compound 50 (30 mg/kg) | 0.93 | 15.27 | Compound 124 (15 mg/kg) | 0.18 | 7.67 |

(continued)

| Compound (dose) | Latency (min) | Duration (min) | Compound (dose) | Latency (min) | Duration (min) |
|---|---|---|---|---|---|
| Compound 51 (60 mg/kg) | 0.25 | 8.46 | Compound 124 (30 mg/kg) | 0.23 | 13.31 |
| Compound 51 (100 mg/kg) | 0.16 | 14.6 | Compound 125 (30 mg/kg) | 0.19 | 7.81 |
| Compound 52 (30 mg/kg) | 1.3 | 5.38 | Compound 126 (30 mg/kg) | 0.16 | 5.05 |
| Compound 52 (60 mg/kg) | 0.94 | 7.22 | Compound 127 (30 mg/kg) | 0.43 | 18.11 |
| Compound 53 (60 mg/kg) | 0.42 | 9.98 | Compound 128 (30 mg/kg) | 0.42 | 7.98 |
| Compound 54 (30 mg/kg) | 0.24 | 6.83 | Compound 129 (30 mg/kg) | 0.56 | 13.27 |
| Compound 55 (30 mg/kg) | 0.74 | 13.84 | Compound 130 (30 mg/kg) | 0.52 | 18.27 |
| Compound 56 (30 mg/kg) | 0.92 | 10.22 | Compound 131 (30 mg/kg) | 0.56 | 6.84 |
| Compound 57 (60 mg/kg) | 0.31 | 5.89 | Compound 132 (30 mg/kg) | 0.87 | 18.05 |
| Compound 58 (30 mg/kg) | 0.25 | 7.76 | Compound 133 (30 mg/kg) | 0.26 | 2.24 |
| Compound 58 (60 mg/kg) | 0.33 | 11.17 | Compound 134 (30 mg/kg) | 0.27 | 6.54 |
| Compound 59 (60 mg/kg) | 0.37 | 9.62 | Compound 135 (30 mg/kg) | 0.22 | 10.64 |
| Compound 60 (30 mg/kg) | 0.49 | 12.27 | Compound 136 (30 mg/kg) | 0.36 | 17.5 |
| Compound 61 (30 mg/kg) | 0.82 | 12.28 | Compound 137 (30 mg/kg) | 0.67 | 21.45 |
| Compound 62 (60 mg/kg) | 0.86 | 10.05 | Compound 138 (30 mg/kg) | 0.27 | 6.94 |
| Compound 63 (30 mg/kg) | 1.23 | 15.25 | Compound 139 (30 mg/kg) | 0.38 | 19.99 |
| Compound 64 (60 mg/kg) | 0.99 | 14.27 | Compound 140 (30 mg/kg) | 0.45 | 21.65 |
| Compound 65 (30 mg/kg) | 1.74 | 17.25 | Compound 141 (30 mg/kg) | 0.74 | 8.76 |
| Compound 66 (60 mg/kg) | 0.89 | 4.27 | Compound 142 (30 mg/kg) | 0.21 | 10.35 |
| Compound 67 (30 mg/kg) | 0.84 | 11.37 | Compound 143 (30 mg/kg) | 0.64 | 9.62 |
| Compound 68 (60 mg/kg) | 0.68 | 15.02 | Compound 144 (30 mg/kg) | 0.45 | 5.32 |

As shown in Table 1, the compounds of the present disclosure had the anesthetic effect of fast onset of action and faster wake-up in a mouse experiment.

Example 146: Test for Drug-Induced Disappearance of Righting Reflex in Rats

SD rats (male, 200-280 g) were randomly grouped and subjected to a single administration by rapid bolus via the tail vein. The latency and duration of righting reflex in the rats were recorded.

Table 2. Test for drug-induced disappearance righting reflex in rats

| Compound (dose) | Latency (min) | Duration (min) |
|---|---|---|
| Compound 1 | 0.25 | 13.1 |
| tautomer of compound 1 | 0.68 | 11.03 |
| Compound 4 | 0.35 | 5.97 |
| tautomer of compound 4 | 0.48 | 15.4 |
| Compound 10 | 0.32 | 6.02 |
| Compound 13 | 0.58 | 8.03 |
| Compound 23 | 0.36 | 6.86 |
| Compound 24 | 0.28 | 16.4 |
| Compound 25 | 0.45 | 18.6 |
| Compound 26 | 0.36 | 9.76 |
| Compound 29 | 0.86 | 6.75 |
| Compound 33 | 0.52 | 9.68 |
| Compound 34 | 0.22 | 15.6 |
| Compound 35 | 0.25 | 35.3 |
| Compound 36 | 0.28 | 38.6 |
| Compound 37 | 0.22 | 25.1 |
| Compound 39 | 0.38 | 9.28 |
| Compound 51 | 0.27 | 10.5 |
| Compound 52 | 0.33 | 22.7 |
| Compound 53 | 0.18 | 16.3 |
| Compound 59 | 0.22 | 19.5 |
| Compound 82 | 0.41 | 23.8 |
| Compound 83 | 0.16 | 31.7 |
| Compound 97 | 0.18 | 28.6 |
| Compound 102 | 0.37 | 11.5 |
| Compound 119 | 0.43 | 13.27 |
| Compound 126 | 0.26 | 27.83 |
| Compound 129 | 0.37 | 17.28 |
| Compound 135 | 0.14 | 16.64 |
| Compound 141 | 0.21 | 24.19 |

As shown in Table 2 above, the compounds of the present disclosure had the anesthetic effect of fast onset of action and fast wake-up in a rat experiment.

Example 147. Experiment on Induction of Anesthesia in Rats by Continuous Infusion of Drug for 20 min

SD rats (male, 200-280 g) were randomly grouped and subjected to administration of an initial dose by rapid bolus via the tail vein, followed by a continuous infusion with a maintenance dose for 20 min. After the infusion was completed, the duration of the righting reflex and the time from wake-up to recovery in rats were recorded.

Table. 3. Experiment on induction of anesthesia in rats by continuous infusion of drug for 20 min

| Compound | Initial dose | Maintenance dose | Duration of anesthesia | Time from wake-up to recovery |
|---|---|---|---|---|
| Compound 1 | 20 mg/kg | 0.2 (mg/kg)/min | 2.82 min | 11.2min |
| tautomer of compound 1 | 20 mg/kg | 0.2 (mg/kg)/min | 3.29 min | 9.6 min |
| Compound 4 | 20 mg/kg | 0.2 (mg/kg)/min | 5.21min | 15.6min |
| tautomer of compound 4 | 20 mg/kg | 0.2 (mg/kg)/min | 4.84min | 10.3min |
| Compound 10 | 20 mg/kg | 0.2 (mg/kg)/min | 5.35min | 13.6min |
| Compound 13 | 20 mg/kg | 0.2 (mg/kg)/min | 5.22min | 11.4min |
| Compound 23 | 20 mg/kg | 0.2 (mg/kg)/min | 7.22min | 13.2min |

(continued)

| Compound | Initial dose | Maintenance dose | Duration of anesthesia | Time from wake-up to recovery |
|---|---|---|---|---|
| Compound 24 | 20 mg/kg | 0.2 (mg/kg)/min | 15.6min | 22.3min |
| Compound 25 | 20 mg/kg | 0.2 (mg/kg)/min | 7.35min | 10.2min |
| Compound 26 | 20 mg/kg | 0.2 (mg/kg)/min | 8.22min | 9.46min |
| Compound 29 | 20 mg/kg | 0.2 (mg/kg)/min | 9.18min | 13.6min |
| Compound 33 | 20 mg/kg | 0.2 (mg/kg)/min | 17.3min | 20.2min |
| Compound 34 | 20 mg/kg | 0.2 (mg/kg)/min | 48.2min | 35.6min |
| Compound 35 | 20 mg/kg | 0.2 (mg/kg)/min | 51.3min | 33.8min |
| Compound 36 | 20 mg/kg | 0.2 (mg/kg)/min | 9.37min | 17.5min |
| Compound 37 | 20 mg/kg | 0.2 (mg/kg)/min | 6.68min | 10.5min |
| Compound 39 | 20 mg/kg | 0.2 (mg/kg)/min | 7.86min | 11.2min |
| Compound 51 | 20 mg/kg | 0.2 (mg/kg)/min | 8.36min | 22.6min |
| Compound 52 | 20 mg/kg | 0.2 (mg/kg)/min | 6.25min | 16.2min |
| Compound 53 | 20 mg/kg | 0.2 (mg/kg)/min | 4.17min | 13.5min |
| Compound 59 | 20 mg/kg | 0.2 (mg/kg)/min | 4.89min | 31.3min |
| Compound 82 | 20 mg/kg | 0.2 (mg/kg)/min | 10.27min | 20.8min |
| Compound 83 | 20 mg/kg | 0.2 (mg/kg)/min | 28.66min | 16.3min |
| Compound 97 | 20 mg/kg | 0.2 (mg/kg)/min | 6.43min | 17.4min |
| Compound 102 | 20 mg/kg | 0.2 (mg/kg)/min | 4.27min | 25.5min |
| Compound 119 | 20 mg/kg | 0.2 (mg/kg)/min | 12.83min | 32.7min |
| Compound 126 | 20 mg/kg | 0.2 (mg/kg)/min | 9.52min | 20.5min |
| Compound 129 | 20 mg/kg | 0.2 (mg/kg)/min | 10.22min | 17.7min |
| Compound 135 | 20 mg/kg | 0.2 (mg/kg)/min | 7.24min | 13.6min |
| Compound 141 | 20 mg/kg | 0.2 (mg/kg)/min | 13.21min | 22.19min |

As shown in Table 3 above, the compounds of the present disclosure could maintain anesthesia in animals under continuous infusion, and faster wake-up and faster recovery were observed after completion of the continuous infusion.

Example 148. Assay on Effect of Drugs on GABA-Activated Current in Cells by Cell Patch Clamp

Test compounds were dissolved at various concentrations in an extracellular buffer (140 mM NaCl, 4.7mM KCl, 10 mM HEPES, 2 mM $CaCl_2$, 10 mM glucose, 1 mM $MgCl_2$, pH 7.4). HEK 293T cells were seeded on glass coverslips and cultured in DMEM media at 37 °C with 5% $CO_2$ for 24 h. GABA $Cl^-$ current was subjected to whole cell recording using an HEKA EPC 10USB patch clamp amplifier. 2 μM GABA was used for exciting $Cl^-$ current, with the membrane potential clamped at -60 mV. The cells were treated with the test compound and 2 μM GABA simultaneously, and the induction effect on and $E_{max}$ of $Cl^-$ current in the same cell were recorded.

Table 4. Effect on GABA-activated current in cells

| Compound | Enhancement percentage $E_{max}$ for 2 μM GABA current (within 3-30 μM) |
|---|---|
| Compound 1 | 216% (20μM) |
| tautomer of compound 1 | 234% (20μM) |
| Compound 4 | 252% (20μM) |
| tautomer of compound 4 | 238% (20μM) |
| Compound 10 | 202% (20μM) |
| Compound 13 | 246% (20μM) |
| Compound 23 | 278% (20μM) |
| Compound 24 | 282% (20μM) |
| Compound 25 | 242% (20μM) |
| Compound 26 | 249% (20μM) |
| Compound 29 | 251% (20μM) |

(continued)

| Compound | Enhancement percentage $E_{max}$ for 2 μM GABA current (within 3-30 μM) |
|---|---|
| Compound 33 | 279% (20μM) |
| Compound 34 | 302% (20μM) |
| Compound 35 | 318% (20μM) |
| Compound 36 | 227% (20μM) |
| Compound 37 | 267% (20μM) |
| Compound 39 | 272% (20μM) |
| Compound 51 | 304% (20μM) |
| Compound 52 | 293% (20μM) |
| Compound 53 | 285% (20μM) |
| Compound 59 | 252% (20μM) |
| Compound 82 | 311% (20μM) |
| Compound 83 | 287% (20μM) |
| Compound 97 | 238% (20μM) |
| Compound 102 | 291% (20μM) |
| Compound 119 | 278% (20μM) |
| Compound 126 | 306% (20μM) |
| Compound 129 | 257% (20μM) |
| Compound 135 | 263% (20μM) |
| Compound 141 | 295% (20μM) |

Maximum enhancement percentage $E_{max}$ for 2 μM GABA current and corresponding concentration (μM): 100% for normal humans. The greater the percentage, the lower the corresponding concentration, and the greater the depth of anesthesia.

Table 5. Effect on GABA-activated current in cells

| Compound | Positive enhancement $EC_{50}$ (μM) for 2 μM GABA-induced current |
|---|---|
| Compound 1 | 0.85 |
| tautomer of compound 1 | 0.72 |
| Compound 4 | 0.69 |
| tautomer of compound 4 | 0.65 |
| Compound 10 | 0.67 |
| Compound 13 | 0.58 |
| Compound 23 | 0.52 |
| Compound 24 | 0.48 |
| Compound 25 | 0.64 |
| Compound 26 | 0.71 |
| Compound 29 | 0.74 |
| Compound 33 | 0.44 |
| Compound 34 | 0.38 |
| Compound 35 | 0.36 |
| Compound 36 | 0.58 |
| Compound 37 | 0.53 |
| Compound 39 | 0.88 |
| Compound 51 | 0.41 |
| Compound 52 | 0.47 |
| Compound 53 | 0.53 |
| Compound 59 | 0.79 |
| Compound 82 | 0.39 |
| Compound 83 | 0.57 |

(continued)

| Compound | Positive enhancement $EC_{50}$ ($\mu$M) for 2 $\mu$M GABA-induced current |
|---|---|
| Compound 97 | 0.84 |
| Compound 102 | 0.48 |
| Compound 119 | 0.43 |
| Compound 126 | 0.45 |
| Compound 129 | 0.42 |
| Compound 135 | 0.75 |
| Compound 141 | 0.61 |
| Note: positive enhancement $EC_{50}$ ($\mu$M) for 2 $\mu$M GABA-induced current: the concentration of drug required to achieve a certain depth of anesthesia is as low as possible. | |

As shown in Tables 4 and 5 above, the compounds of the present disclosure had a suitable depth of anesthesia, indicating that the compounds of the present disclosure have an excellent anesthetic effect.

Example 149. Rate of Drug Passing Through *In Vitro* Blood-Brain Barrier

A Transwell device (pore size: 5.0 $\mu$m) was placed in a 24-well cell culture well; then hCMEC/D3 cells at $1\times10^5$ cells/mL were transferred to the upper microwells of Transwell at 150 $\mu$L/well, and cell culture medium was added to the lower microwells. After the bottom was covered by a single layer of the cells, the medium in the wells was replaced with EBM-2 complete medium containing 1% FBS, and the cells were cultured in a $CO_2$ incubator for 10 days until a fully dense single layer of the cells was formed. When a fully dense single layer of the cells was formed, the compound at 0.1 mg/mL was added to the lower medium, and after 5 min, the concentration of the compound in the upper medium was measured to calculate the permeability:

$$P_{compound}\ \% = C_{upper\ compound} / C_{lower\ compound} \times 100\%$$

Table 6. Test of rate of drug penetrating through blood-brain barrier

| Compound | *In vitro* blood-brain barrier, compound permeability at 5 min |
|---|---|
| Compound 1 | 72.2% |
| tautomer of compound 1 | 76.8% |
| Compound 4 | 68.6% |
| tautomer of compound 4 | 75.8% |
| Compound 10 | 58.7% |
| Compound 13 | 81.6% |
| Compound 23 | 65.3% |
| Compound 24 | 73.4% |
| Compound 25 | 81.5% |
| Compound 26 | 52.8% |
| Compound 29 | 77.6% |
| Compound 33 | 83.4% |
| Compound 34 | 85.6% |
| Compound 35 | 84.6% |
| Compound 36 | 71.6% |
| Compound 37 | 84.6% |
| Compound 39 | 82.6% |
| Compound 51 | 78.6% |
| Compound 52 | 80.1% |
| Compound 53 | 69.9% |
| Compound 59 | 75.7% |

(continued)

| Compound | *In vitro* blood-brain barrier, compound permeability at 5 min |
|---|---|
| Compound 82 | 80.1% |
| Compound 83 | 77.3% |
| Compound 97 | 84.6% |
| Compound 102 | 87.1% |
| Compound 119 | 83.3% |
| Compound 126 | 85.6% |
| Compound 129 | 75.9% |
| Compound 135 | 80.2% |
| Compound 141 | 76.6% |

As shown in Table 6 above, the compounds had the function of rapidly passing through the blood-brain barrier.

Example 150. Test of *In Vitro* Plasma Half-Life of Drug

Preparation of plasma: after the SD rats were fasted and kept with water for 12 h, blood was collected through orbital venous plexus. The freshly collected blood was added into a centrifuge tube containing heparin sodium and centrifuged at 5000 rpm for 10 min at 4 °C. The supernatant was slowly pipetted and subpackaged. Finally, the collected SD rat plasma was sealed, marked, and stored at -20 °C for later use.

Preparation of target compound sample: the target compound was precisely weighed by an analytical balance and prepared into a stock solution at a concentration of 2 mg/mL (solvent: $V_{acetone}$:$V_{Tween\ 80}$:$V_{normal\ saline}$ = 1:1:3) for later use.

Determination of *in vitro* half-life of target compound sample: 20 μL of test compound stock solution and 80 μL of rat plasma were mixed uniformly. Nine parallel ep tubes were taken, marked as corresponding test tubes of 0 min, 1 min, 2 min, 3 min, 5 min, 10 min, 15 min, 20 min, and 30 min, respectively, and placed in a water bath at 37 °C for incubation for 0 min, 1 min, 2 min, 3 min, 5 min, 10 min, 15 min, 20 min, and 30 min, respectively. 50 μL of mixed solution were taken from the corresponding ep tube at each time point, and 100 mL of acetonitrile was added for precipitating protein. The mixture was vortexed for 10 min using a mixer and centrifuged at 12000 rpm for 15 min. Then, the supernatant was centrifuged at 12000 rpm for 10 min. The optimal detection method for the target compound in the MRM mode was determined using Intellistat software in UPLC-MS/MS, the peak area of the corresponding mass spectrum was integrated, and the plasma half-life of the compound was calculated.

Table 7. Test results for *in vitro* plasma half-life of drugs

| Compound | *In vitro* plasma half-life (min) |
|---|---|
| Tautomer of compound 1 | 4.65 |
| Compound 4 | 1.73 |
| Compound 30 | 0.45 |
| Compound 32 | 0.27 |
| Compound 33 | 0.89 |
| Compound 35 | 0.21 |
| Compound 36 | 0.57 |
| Compound 39 | 0.43 |
| Compound 52 | 0.36 |
| Compound 53 | 0.33 |
| Compound 59 | 0.25 |
| Compound 82 | 0.37 |
| Compound 83 | 0.28 |
| Compound 97 | 0.61 |
| Compound 102 | 0.27 |
| Compound 126 | 0.41 |
| Compound 135 | 0.33 |

As shown in Table 7 above, the compounds had the function of rapidly passing through the blood-brain barrier. Example 151. Determination of Whether Drugs Have Potential Inhibitory Effect on Voltage-Gated Potassium Channel hERG by Fluorescence Polarization Technique

In this experiment, $IC_{50}$ was calculated by detecting the effect of compounds at 8 concentrations on the current of the hERG channel. The procedures were performed exactly as for Predictor™ hERG fluorescence polarization assay kit. Before measuring fluorescence polarization, the assay plate was covered to protect the reagents from light and evaporation, and after incubation at room temperature for 2-4 h, the sample plate was centrifuged to read the fluorescence polarization value, with the excitation light at 540 nm and the emission light at 573 nm. The data showed that the cardiotoxicity of the compounds of the present disclosure was significantly lower than that of the marketed drug midazolam.

Table 8. Results for drug effects on current of hERG channel

| Compound | $IC_{50}/\mu M$ |
|---|---|
| 126 | 72.53 |
| 135 | 31.86 |
| Midazolam | 7.09 |

The above pharmacological data showed that the compounds of general formula (I) had the characteristics of fast onset of action, strong action depth, and fast wake-up, and the preferred compound had almost no accumulation.

Lyophilized powder for injection:

Example 152. Lyophilized Powder for Injection Containing Compound 32 as Active Agent:

Compound 32 (10 g) and glycine (100 g) were added to a flask, water for injection was added to 1 L, and the pH of the solution was adjusted to 3.5. The solution was subpackaged into 1000 vials, and prepared into lyophilized powder for injection by a conventional lyophilization method.

It should be understood that various modifications or changes may be made by those skilled in the art after reading the above teachings of the present disclosure, and these equivalent forms also fall within the scope defined by the claims appended hereto.

## Claims

1. A novel benzodiazepine compound, comprising a compound represented by general formula (I), and a pharmaceutically acceptable salt, a stereoisomer, a tautomer, a polymorph, a solvate, a metabolite or a prodrug thereof:

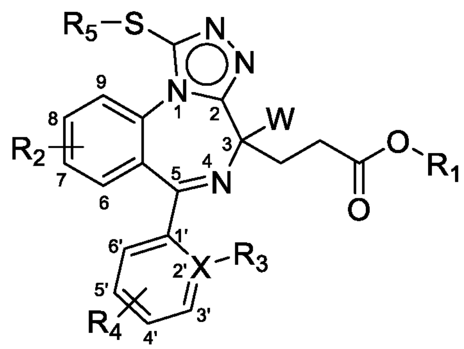

(I)

wherein:

$R_1$ is selected from hydrogen, $C_{1-20}$ alkyl, $C_{2-20}$ alkenyl, $C_{2-20}$ alkynyl, 3- to 10-membered cycloalkyl or $C_{1-10}$ alkylene-3-to 10-membered cycloalkyl, 3- to 10-membered heterocyclyl or $C_{1-10}$ alkylene-3- to 10-membered

heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3-to 10-membered heterocyclyl or $C_{1-10}$ alkylene-3- to 10-membered heterocyclyl, 6-to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more substituents independently selected from halogen, hydroxy, cyano, amino, nitro, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

$R_2$ is located at any position of the benzene ring and is monosubstituted or polysubstituted;

$R_2$ is selected from hydrogen, halogen, hydroxy, cyano, $R_6R_7N-(CH_2)_n-$, $(R_6)(R_7)N-(CH_2)_n-$, trifluoromethyl, nitro, amino, $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3-to 10-membered heterocyclyl or $C_{1-10}$ alkylene-3- to 10-membered heterocyclyl, 6-to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more substituents independently selected from halogen, hydroxy, cyano, amino, nitro, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

X is selected from C and N, and when X is C, $R_3$ is selected from hydrogen, halogen, hydroxy, cyano, $R_6R_7N-(CH_2)_n-$, $(R_6)(R_7)N-(CH_2)_n-$, trifluoromethyl, nitro, amino, $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3-to 10-membered heterocyclyl or $C_{1-10}$ alkylene-3- to 10-membered heterocyclyl, 6-to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more substituents independently selected from halogen, hydroxy, cyano, amino, nitro, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl; when X is N, the $R_3$ substituent is absent;

$R_4$ is located at any position of the benzene ring or the pyridine ring and is monosubstituted or polysubstituted;

$R_4$ is selected from hydrogen, halogen, hydroxy, cyano, $R_6R_7N-(CH_2)_n-$, $(R_6)(R_7)N-(CH_2)_n-$, trifluoromethyl, nitro, amino, $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3-to 10-membered heterocyclyl or $C_{1-10}$ alkylene-3- to 10-membered heterocyclyl, 6-to 14-membered aryl or $C_{1-10}$ alkylene-6- to 14-membered aryl, or 5- to 14-membered heteroaryl or $C_{1-10}$ alkylene-5- to 14-membered heteroaryl is substituted with substituents independently selected from halogen, hydroxy, cyano, amino, nitro, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

$R_5$ is selected from hydrogen, $C_{1-20}$ alkyl, $C_{1-20}$ acyl, $C_{2-20}$ alkenyl, $C_{2-20}$ alkynyl, $C_{1-10}$ alkylene-phosphate group, $P(O)(OH)_2-C_{1-10}$ alkylene, 3- to 10-membered cycloalkyl or $C_{1-10}$ alkylene-3- to 10-membered cycloalkyl, $R_6R_7N-(CH_2)_n-$, $(R_6)(R_7)N-(CH_2)_n-$, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{1-20}$ acyl, $C_{2-20}$ alkenyl, $C_{2-20}$ alkynyl, 3- to 10-membered cycloalkyl or $C_{1-10}$ alkylene-3- to 10-

membered cycloalkyl, $R_6R_7N-(CH_2)_n-$, $(R_6)(R_7)N-(CH_2)_n-$, 3- to 10-membered heterocyclyl or $C_{1-10}$ alkylene-3- to 10-membered heterocyclyl, 6- to 14-membered aryl or $C_{1-10}$ alkylene-6- to 14-membered aryl, 5- to 14-membered heteroaryl or $C_{1-10}$ alkylene-5- to 14-membered heteroaryl is substituted with substituents selected from halogen, oxo (= O), $C_{1-10}$ alkylene-halogen, hydroxy, $C_{1-10}$ alkylene-hydroxy, $R_6R_7N-(CH_2)_n-$, $(R_6)(R_7)N-(CH_2)_n-$, cyano, $C_{1-10}$ alkylene-cyano, nitro, $C_{1-10}$ alkylene-nitro, amino, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

$R_6$ or $R_7$ are selected from hydrogen, $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, or 5- to 14-membered heteroaryl or $C_{1-10}$ alkylene-5- to 14-membered heteroaryl is each optionally substituted with one or more substituents independently selected from halogen, $C_{1-10}$ alkylene-halogen, hydroxy, $C_{1-10}$ alkylene-hydroxy, $(R_6)(R_7)N-(CH_2)_n-$, cyano, $C_{1-10}$ alkylene-cyano, nitro, $C_{1-10}$ alkylene-nitro, amino, $C_{1-20}$ alkyl, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

n is selected from 1 to 20;

W is selected from hydrogen, halogen, hydroxy, cyano, $R_6R_7N-(CH_2)_n-$, $(R_6)(R_7)N-(CH_2)_n-$, trifluoromethyl, nitro, amino, $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl;

the $C_{1-20}$ alkyl, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, or 5- to 14-membered heteroaryl or $C_{1-10}$ alkylene-5- to 14-membered heteroaryl is selected from halogen, $C_{1-10}$ alkylene-halogen, hydroxy, $C_{1-10}$ alkylene-hydroxy, $R_6R_7N-(CH2)_n-$, $(R_6)(R_7)N-(CH_2)_n-$, cyano, $C_{1-10}$ alkylene-cyano, nitro, $C_{1-10}$ alkylene-nitro, amino, $C_{1-20}$ alkyl, C, $C_{1-20}$ alkoxy, $C_{2-20}$ alkenyl, $C_{2-20}$ alkynyl, 3- to 10-membered cycloalkyl or $C_{1-10}$ alkylene-3- to 10-membered cycloalkyl, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and

5- to 14-membered heteroaryl or $C_{1-10}$ alkylene-5- to 14-membered heteroaryl;

wherein the hydrogen described above and the hydrogen contained in the groups described above are selected from protium, deuterium, and tritium.

**2.** The novel benzodiazepine compound according to claim 1, wherein:

$R_1$ is selected from hydrogen, $C_{1-6}$ alkyl, and $C_{1-10}$ alkylene-5- to 14-membered heteroaryl;

$R_2$ is substituted at positions selected from positions 6, 7, 8, and 9;

$R_2$ is selected from hydrogen, halogen, hydroxy, $R_6R_7N-(CH_2)_{1-10}-$, trifluoromethyl, nitro, cyano, amino, $C_{1-6}$ alkyl, $C_{1-6}$ alkoxy, $C_{2-6}$ alkenyl, and $C_{2-6}$ alkynyl;

$R_3$ selected from hydrogen, halogen, hydroxy, $R_6R_7N-(CH_2)_{1-10}-$, trifluoromethyl, nitro, cyano, amino, $C_{1-6}$ alkyl, $C_{1-6}$ alkoxy, $C_{2-6}$ alkenyl, and $C_{2-6}$ alkynyl;

$R_4$ is substituted at positions selected from positions 3’, 4’, and 5’;

$R_4$ selected from hydrogen, halogen, hydroxy, $R_6R_7N-(CH_2)_{1-10}-$, trifluoromethyl, nitro, cyano, amino, $C_{1-6}$ alkyl, $C_{1-6}$ alkoxy, $C_{2-6}$ alkenyl, and $C_{2-6}$ alkynyl;

$R_5$ is selected from hydrogen, $C_{1-10}$ alkyl, $C_{1-10}$ alkylene-phosphate group, 3- to 10-membered cycloalkyl or $C_{1-10}$ alkylene-3- to 10-membered cycloalkyl, $R_6R_7N-(CH_2)_n-$, and 3- to 10-membered heterocyclyl or $C_{1-10}$ alkylene-3- to 10-membered heterocyclyl;

$R_6$ or $R_7$ is selected from $C_{1-6}$ alkyl;

n is selected from 1 to 10;

W is selected from hydrogen, halogen, hydroxy, $R_6R_7N-(CH_2)_{1-10}-$, trifluoromethyl, nitro, cyano, $C_{1-6}$ alkyl, $C_{1-6}$ alkoxy,

$C_{2-6}$ alkenyl, and $C_{2-6}$ alkynyl.

**3.** The novel benzodiazepine compound according to claim 1, wherein:

$R_1$ is selected from hydrogen, methyl, ethyl, propyl, isopropyl, *n*-butyl, isobutyl, *tert*-butyl, *n*-pentyl, isopentyl, neopentyl, and $C_{1-6}$ alkylene-morpholinyl;
$R_2$ is substituted at a position selected from position 7;
$R_2$ is selected from hydrogen, chlorine, bromine, fluorine, nitro, cyano, amino, $R_6P_7N$-$(CH_2)_{1-6}$-, and trifluoromethyl;
$R_3$ is selected from hydrogen, chlorine, bromine, fluorine, nitro, cyano, amino, $R_6R_7N$-$(CH_2)_{1-6}$-, and trifluoromethyl;
$R_4$ is substituted at a position selected from position 4';
$R_4$ is selected from hydrogen, chlorine, bromine, fluorine, nitro, cyano, amino, $P_6R_7N$-$(CH_2)_{1-6}$-, and trifluoromethyl;
$R_5$ is selected from hydrogen, methyl, ethyl, methylene-phosphate group, $C_{1-10}$ alkylene-morpholinyl, $C_{1-10}$ alkylene-pyrrolyl, $C_{1-10}$ alkylene-imidazolyl, $C_{1-10}$ alkylene-piperidinyl, 2-(4-hydroxypiperidin-1-yl)ethyl, $C_{1-10}$ alkylene-piperidinyl, $C_{1-10}$ alkylene-piperazinyl, 2-(4-methylpiperazin-1-yl)ethyl, 3-(4-(2-hydroxyethyl)piperazin-1-yl)propyl, 2-(4-methylpiperazin-1-yl)-2-oxyethyl, 2-(4-methylpiperazin)-1-carbonyl, (4-phenylpiperazin-1-yl)methyl, 2-hydroxyethyl, 2-aminoethyl, acetyl, prop-2-yn-1-yl, dimethylaminomethyl, dimethylaminoethyl, dimethylamino-*n*-propyl, dimethylamino-isopropyl, dimethylamino-*n*-butyl, dimethylamino-isobutyl, dimethylamino-*tert*-butyl, dimethylamino-*n*-pentyl, dimethylamino-isopentyl, dimethylamino-neopentyl, cyclopropylmethyl, cyclopropylethyl, cyclopropyl-*n*-propyl, cyclopropylisopropyl, 1-methylpiperidin-4-yl, 1-ethyl-carbamoyl, 1-methyl-carbamoyl, (dimethylamino)-3-oxopropyl, and (4,5-dihydro-1*H*-imidazol-2-yl)methyl;
$R_6$ or $R_7$ is selected from methyl, ethyl, propyl, isopropyl, *n*-butyl, isobutyl, *tert*-butyl, *n*-pentyl, isopentyl, and neopentyl;
n is selected from 1-6;
W is selected from hydrogen, hydroxy, chlorine, bromine, fluorine, nitro, cyano, $R_6R_7N$-$(CH_2)_{1-6}$-, and trifluoromethyl.

**4.** The novel benzodiazepine compound according to claim 1, wherein:

X is C, $R_2$ and $R_3$ are halogen, and $R_5$ is selected from hydrogen, $C_{1-20}$ alkyl, $C_{1-20}$ acyl, $C_{2-20}$ alkenyl, $C_{2-20}$ alkynyl, $C_{1-10}$ alkylene-phosphate group, 3- to 10-membered cycloalkyl or $C_{1-10}$ alkylene-3- to 10-membered cycloalkyl, $R_6R_7N$-$(CH_2)_n$-, 3- to 10-membered heterocyclyl or $C_{1-10}$ alkylene-3- to 10-membered heterocyclyl, 6- to 14-membered aryl or
$C_{1-10}$ alkylene-6- to 14-membered aryl, and 5- to 14-membered heteroaryl or $C_{1-10}$ alkylene-5- to 14-membered heteroaryl;
X is N, $R_5$ is selected from hydrogen, $C_{1-20}$ alkyl, $C_{1-20}$ acyl, $C_{2-20}$ alkenyl, $C_{2-20}$ alkynyl, $C_{1-10}$ alkylene-phosphate group, 3- to 10-membered cycloalkyl or $C_{1-10}$ alkylene-3- to 10-membered cycloalkyl, $R_6R_7N$-$(CH_2)_n$-, 3- to 10-membered heterocyclyl or $C_{1-10}$ alkylene-3- to 10-membered heterocyclyl, 6- to 14-membered aryl or $C_{1-10}$ alkylene-6- to 14-membered aryl, and 5- to 14-membered heteroaryl or $C_{1-10}$ alkylene-5- to 14-membered heteroaryl.

**5.** The novel benzodiazepine compound according to claim 1, wherein:

X is C, $R_2$ and $R_3$ are halogen, $R_5$ is selected from hydrogen, $C_{1-10}$ alkyl, $C_{1-10}$ alkylene-phosphate group, 3- to 10-membered cycloalkyl or $C_{1-10}$ alkylene-3- to 10-membered cycloalkyl, $R_6R_7N$-$(CH_2)_n$-, and 3- to 10-membered heterocyclyl or $C_{1-10}$ alkylene-3- to 10-membered heterocyclyl;
X is N, and $R_5$ is selected from hydrogen, $C_{1-10}$ alkyl, $C_{1-10}$ alkylene-phosphate group, 3- to 10-membered cycloalkyl or
$C_{1-10}$ alkylene-3- to 10-membered cycloalkyl, $R_6R_7N$-$(CH_2)_n$-, and 3- to 10-membered heterocyclyl or $C_{1-10}$ alkylene-3-to 10-membered heterocyclyl.

**6.** The novel benzodiazepine compound according to claim 1, wherein:

X is C, $R_2$ and $R_3$ are halogen, and $R_5$ is selected from hydrogen, methyl, ethyl, methylene-phosphate group, $C_{1-10}$ alkylene-morpholinyl, $C_{1-10}$ alkylene-pyrrolyl, $C_{1-10}$ alkylene-imidazolyl, 2-(4-hydroxypiperidin-1-yl)ethyl, $C_{1-10}$ alkylene-piperidinyl, $C_{1-10}$ alkylene-piperazinyl, 2-(4-methylpiperazin-1-yl)ethyl, 3-(4-(2-hydroxyethyl)piperazin-1-yl)propyl, 2-(4-methylpiperazin-1-yl)-2-oxyethyl, 2-(4-methylpiperazin)-1-carbonyl, (4-phenylpiperazin-1-yl)methyl, 2-hydroxyethyl, 2-aminoethyl, acetyl, prop-2-yn-1-yl, dimethylaminomethyl, dimethylaminoethyl, dimethylamino-*n*-propyl, dimethylamino-isopropyl, dimethylamino-*n*-butyl, dimethylamino-isobutyl, dimethylamino-*tert*-butyl, dimethylamino-*n*-pentyl, dimethylamino-isopentyl, dimethylamino-neopentyl, cyclopropylmethyl, cyclopropylethyl, cyclopropyl-*n*-propyl, cyclopropylisopropyl, 1-methylpiperidin-4-yl, 1-ethyl-carbamoyl, 1-methyl-carbamoyl, (dimethylamino)-3-oxopropyl, and (4,5-dihydro-1*H*-imidazol-2-yl)methyl;

X is N, and $R_5$ is selected from hydrogen, methyl, ethyl, methylene-phosphate group, $C_{1-10}$ alkylene-morpholinyl, $C_{1-10}$ alkylene-pyrrolyl, $C_{1-10}$ alkylene-imidazolyl, 2-(4-hydroxypiperidin-1-yl)ethyl, $C_{1-10}$ alkylene-piperidinyl, $C_{1-10}$ alkylene-piperazinyl, 2-(4-methylpiperazin-1-yl)ethyl, 3-(4-(2-hydroxyethyl)piperazin-1-yl)propyl, 2-(4-methylpiperazin-1-yl)-2-oxyethyl, 2-(4-methylpiperazin)-1-carbonyl, (4-phenylpiperazin-1-yl)methyl, 2-hydroxyethyl, 2-aminoethyl, acetyl, prop-2-yn-1-yl, dimethylaminomethyl, dimethylaminoethyl, dimethylamino-*n*-propyl, dimethylamino-isopropyl, dimethylamino-*n*-butyl, dimethylamino-isobutyl, dimethylatnino-*tert*-butyl, dimethylatnino-*n*-pentyl, dimethylamino-isopentyl, dimethylamino-neopentyl, cyclopropylmethyl, cyclopropylethyl, cyclopropyl-*n*-propyl, cyclopropylisopropyl, 1-methylpiperidin-4-yl, 1-ethyl-carbamoyl, 1-methyl-carbamoyl, (dimethylamino)-3-oxopropyl, and (4,5-dihydro-1*H*-imidazol-2-yl)methyl.

**7.** The novel benzodiazepine compound according to claim 1, wherein the compound is selected from:

methyl (*S*)-3-(8-chloro-6-(2-fluorophenyl)-1-mercapto-4*H*-benzo[*f*][1,2,4]triazolo[4,3-*a*][1,4]dlazepin-4-yl)propionate (compound 1),
methyl (*S*)-3-(8-chloro-6-(2-fluorophenyl)-1-thio-2,4-dihydro-1*H*-benzo[*f*][1.2,4]triazolo[4.3-*a*][1.4]diazepin-4-yl)propionate (tautomer of compound 1),
methyl (*S*)-3-(8-chloro-6-(2-chlorophenyl)-1-mercapto-4*H*-benzo[*f*][1,2,4]triazolo[4,3-*a*][1,4]diazepm-4-yl)propionate (compound 2),
methyl (*S*)-3-(8-chloro-6-(2-chlorophenyl)-1-thio-2,4-dihydro-1*H*-benzo[*f*][1,2,4]triazolo[4,3-*a*][1,4]diazepin-4-yl)propionate (tautomer of compound 2),
methyl (*S*)-3-(8-bromo-6-(pyridin-2-yl)-1-mercapto-4*H*-benzo[*f*][1,2,4]triazolo[4,3-*a*][1,4]diazepin-4-yl)propionate (compound 3),
methyl (*S*)-3-(8-bromo-6-(pyridin-2-yl)-1-thio-2,4-dihydro-1*H*-benzo[*f*][1,2,4]triazolo[4,3-*a*][1,4]diazepm-4-yl)propionate (tautomer of compound 3),
methyl (*S*)-3-(8-bromo-6-(2-fluorophenyl)-1-mercapto-4*H*-benzo[*f*][1,2,4]triazolo[4,3-*a*][1,4]diazepin-4-yl)propionate (compound 4),
methyl (*S*)-3-(8-bromo-6-(2-fluorophenyl)-1-thio-2,4-dihydro-1*H*-benzo[*f*][1,2,4]triazolo[4,3-*a*][1,4]diazepin-4-yl) propionate (tautomer of compound 4),
methyl (*S*)-3-(6-phenyl-1-mercapto-4*H*-benzo[*f*][1,2,4]triazolo[4,3-*a*][1,4]diazepin-4-yl)propionate (compound 5),
methyl (*S*)-3-(6-phenyl-1-thio-2,4-dihydro-1*H*-benzo[*f*][1,2,4]triazolo[4,3-*a*][1,4]diazepin-4-yl)propionate (tautomer of compound 5),
methyl (*S*)-3-(6-(4-fluorophenyl)-1-mercapto-4*H*-benzo[*f*][1.2.4]triazolo[4.3-*a*][1,4]diazepin-4-yl)propionate (compound 6),
methyl (*S*)-3-(6-(4-fluorophenyl)-1-thio-2,4-dihydro-1*H*-benzo[*f*][1,2,4]triazolo[4,3-*a*][1,4]diazepm-4-yl)propionate (tautomer of compound 6),
methyl (*S*)-3-(8-chloro-6-phenyl-l-mercapto-4*H*-benzo[*f*][1,2,4]triazolo[4,3-*a*][1,4]diazepm-4-yl)propionate (compound 7),
methyl (*S*)-3-(8-chloro-6-phenyl-1-thio-2,4-dihydro-1*H*-benzo[*f*][1,2,4]triazolo[4,3-*a*][1,4]diazepin-4-yl)propionate (tautomer of compound 7),
methyl (*S*)-3-(8-chloro-6-(2-fluorophenyl)-1-(methylthio)-4*H*-benzo[*f*][1,2,4]triazolo[4,3-*a*][1,4]diazepin-4-yl)propionate (compound 8),
methyl (*S*)-3-(8-bromo-6-(2-fluorophenyl)-1-(methylthio)-4*H*-benzo[*f*][1,2,4]triazolo[4,3-*a*][1,4]diazepin-4-yl)propionate (compound 9),
methyl (*S*)-3-(8-bromo-6-(2-fluorophenyl)-1-(methylthio)-4*H*-benzo[*f*][1,2,4]triazolo[4,3-*a*][1,4]diazepin-4-yl)propionate (compound 10),
methyl (*S*)-3-(8-chloro-6-(2-fluorophenyl)-1-(ethylthio)-4*H*-benzo[*f*][1,2,4]triazolo[4,3-*a*][1,4]diazepin-4-yl)propionate (compound 11),
methyl (*S*)-3-(8-bromo-6-(2-fluorophenyl)-1-(ethylthio)-4*H*-benzo[*f*][1,2,4]triazolo[4,3-*a*][1,4]diazepin-4-yl)propionate (compound 12),
methyl (*S*)-3-(8-nitro-6-(2-fluorophenyl)-1-(ethylthio)-4*H*-benzo[*f*][1,2,4]triazolo[4,3-*a*][1,4]diazepin-4-yl)propionate (compound 13),

methyl (*S*)-3-(8-chloro-6-(2-fluorophenyl)-1-(propylthio)-4*H*-benzo[*f*][1,2,4]triazolo[4,3-*a*][1,4]diazepin-4-yl)propionate (compound 14),
methyl (*S*)-3-(8-bromo-6-(2-fluorophenyl)-1-(propylthio)-4*H*-benzo[*f*][1,2,4]triazolo[4,3-*a*][1,4]diazepin-4-yl)propionate (compound 15),
methyl (*S*)-3-(8-nitro-6-(2-fluorophenyl)-1-(propylthio)-4*H*-benzo[*f*][1,2,4]triazolo[4,3-*a*][1,4]diazepin-4-yl)propionate (compound 16),
methyl (*S*)-3-(8-chloro-6-(2-fluorophenyl)-1-((2-hydroxyethyl)thio)-4*H*-benzo[*f*][1,2,4]triazolo[4,3-*a*][1,4]diazepin-4-yl)propionate (compound 17),
methyl (*S*)-3-(8-bromo-6-(2-fluorophenyl)-1-((2-hydroxyethyl)thio)-4*H*-benzo[*f*][1,2,4]triazolo[4,3-*a*][1,4]diazepin-4-yl)propionate (compound 18),
methyl (*S*)-3-(8-nitro-6-(2-fluorophenyl)-1-((2-hydroxyethyl)thio)-4*H*-benzo[*f*][1,2,4]triazolo[4,3-*a*][1,4]diazepin-4-yl)propionate (compound 19),
methyl (*S*)-3-(8-chloro-6-(2-fluorophenyl)-1-((2-aminoethyl)thio)-4*H*-benzo[*f*][1,2,4]triazolo[4,3-*a*][1,4]diazepin-4-yl)propionate (compound 20),
methyl (*S*)-3-(8-bromo-6-(2-fluorophenyl)-1-((2-aminoethyl)thio)-4*H*-benzo[*f*][1,2,4]triazolo[4,3-*a*][1,4]diazepin-4-yl)propionate (compound 21),
methyl (*S*)-3-(8-nitro-6-(2-fluorophenyl)-1-((2-aminoethyl)thio)-4*H*-benzo[*f*][1,2,4]triazolo[4,3-*a*][1,4]diazepin-4-yl)propionate (compound 22),
methyl (*S*)-3-(8-chloro-6-(2-chlorophenyl)-1-(methylthio)-4*H*-benzo[*f*][1,2,4]triazolo[4,3-*a*][1,4]diazepin-4-yl)propionate (compound 23),
methyl (*S*)-3-(8-bromo-6-(2-chlorophenyl)-1-(methylthio)-4*H*-benzo[*f*][1,2,4]triazolo[4,3-*a*][1,4]diazepin-4-yl)propionate (compound 24),
methyl (S)-3 -(8-nitro-6-(2-chlorophenyl)-1-(methylthio)-4H-benzo[*f*][1,2,4]triazolo[4,3-*a*][1,4]diazepin-4-yl)propionate (compound 25),
methyl (*S*)-3-(8-chloro-6-(2-chlorophenyl)-1-(ethylthio)-4*H*-benzo[*f*][1,2,4]triazolo[4,3-*a*][1,4]diazepin-4-yl)propionate (compound 26),
methyl (*S*)-3-(8-bromo-6-(2-chlorophenyl)-1-(ethylthio)-4*H*-benzo[*f*][1,2,4]triazolo[4,3-*a*][1,4]diazepin-4-yl)propionate (compound 27),
methyl (*S*)-3-(8-nitro-6-(2-chlorophenyl)-1-(ethylthio)-4*H*-benzo[*f*][1,2,4]triazolo[4,3-*a*][1,4]diazepin-4-yl)propionate (compound 28),
methyl (*S*)-3-(8-chloro-6-(2-chlorophenyl)-1-(propylthio)-4*H*-benzo[*f*][1,2,4]triazolo[4,3-*a*][1,4]diazepin-4-yl)propionate (compound 29),
methyl (*S*)-3-(8-bromo-6-(2-chlorophenyl)-1-(propylthio)-4*H*-benzo[*f*][1,2,4]triazolo[4,3-*a*][1,4]diazepin-4-yl)propionate (compound 30),
methyl (*S*)-3-(8-nitro-6-(2-chlorophenyl)-1-(propylthio)-4*H*-benzo[*f*][1,2,4]triazolo[4,3-*a*][1,4]diazepin-4-yl)propionate (compound 31),
methyl (*S*)-3-(8-chloro-6-(2-chlorophenyl)-1-((2-hydroxyethyl)thio)-4*H*-benzo[*f*][1,2,4]triazolo[4,3-*a*][1,4]diazepin-4-yl)propionate (compound 32),
methyl (*S*)-3-(8-bromo-6-(2-chlorophenyl)-1-((2-hydroxyethyl)thio)-4*H*-benzo[*f*][1,2,4]tnazolo[4,3-*a*][1,4]diazepin-4-yl)propionate (compound 33),
methyl (*S*)-3-(8-nitro-6-(2-chlorophenyl)-1-((2-hydroxyethyl)thio)-4*H*-benzo[*f*][1,2,4]triazolo[4,3-*a*][1,4]diazepin-4-yl)propionate (compound 34),
methyl (*S*)-3-(8-chloro-6-(2-chlorophenyl)-1-((2-ammoethyl)thio)-4*H*-benzo[*f*][1,2,4]triazolo[4,3-*a*][1,4]diazepin-4-yl)propionate (compound 35),
methyl (*S*)-3-(8-bromo-6-(2-chlorophenyl)-1-((2-atninoethyl)thio)-4*H*-benzo[*f*][1,2,4]triazolo[4,3-*a*][1,4]diazepin-4-yl)propionate (compound 36),
methyl (*S*)-3-(8-nitro-6-(2-chlorophenyl)-1-((2-aminoethyl)thio)-4*H*-benzo[*f*][1,2,4]triazolo[4,3-*a*][1,4]diazepin-4-yl)propionate (compound 37),
methyl (*S*)-3-(8-chloro-6-(2-chlorophenyl)-1-(acetylthio)-4*H*-benzo[*f*][1,2,4]triazolo[4,3-*a*][1,4] diazepin-4-yl)propionate (compound 38),
methyl (*S*)-3-(8-bromo-6-(2-chlorophenyl)-1-(acetylthio)-4*H*-benzo[*f*][1,2,4]triazolo[4,3-*a*][1,4]diazepin-4-yl)propionate (compound 39),
methyl (*S*)-3-(8-nitro-6-(2-chlorophenyl)-1-(acetylthio)-4*H*-benzo[*f*][1,2,4]triazolo[4,3-*a*][1,4]diazepin-4-yl)propionate (compound 40),
ethyl (*S*)-3-(8-chloro-6-(2-chlorophenyl)-1-mercapto-4*H*-benzo[*f*][1,2,4]triazolo[4,3-*a*][1,4]diazepin-4-yl)propionate (compound 41),
ethyl (*S*)-3-(8-chloro-6-(2-chlorophenyl)-1-thio-2,4-dihydro-1*H*-benzo[*f*][1,2,4]triazolo[4,3-*a*[4,3]diazepin-4-yl)propionate (tautomer of compound 41),

ethyl (*S*)-3-(8-chloro-6-(2-chlorophenyl)-1-(methylthio)-4H-benzo[*f*][1,2,4]triazolo[4,3-*a*][1,4]diazepin-4-yl)propionate (compound 42),
ethyl (*S*)-3-(8-chloro-6-(2-chlorophenyl)-1-(methylthio)-4*H*-benzo[*f*][1,2,4]triazolo[4,3-*a*][1,4]diazepin-4-yl)propionate (compound 43),
ethyl (*S*)-3-(8-nitro-6-(2-chlorophenyl)-1-(methylthio)-4*H*-benzo[*f*][1,2,4]triazolo[4,3-*a*][1,4]diazepin-4-yl)propionate (compound 44),
methyl (*S*)-3-(8-chloro-6-(2-fhiorophenyl)-1-((cyclopropyhnethyl)thio)-4*H*-benzo[*f*][1,2,4]triazolo[4,3-*a*][1,4]diazepm-4-yl)propionate (compound 45),
methyl (*S*)-3-(8-bromo-6-(2-fluorophenyl)-1-((cyclopropyhnethyl)thio)-4*H*-benzo[*f*][1,2,4]triazolo[4,3-*a*][1,4]diazepin-4-yl)propionate (compound 46),
methyl (*S*)-3-(8-nitro-6-(2-fluorophenyl)-1-((cyclopropylmethyl)thio)-4*H*-benzo[*f*][1,2,4]triazolo[4,3-*a*][1,4]diazepin-4-yl)propionate (compound 47),
methyl (*S*)-3-(8-chloro-6-(2-fluorophenyl)-1-(propargylthio)-4*H*-benzo[*f*][1,2,4]triazolo[4,3-*a*][1,4]diazepin-4-yl)propionate (compound 48),
methyl (*S*)-3-(8-bromo-6-(2-fluorophenyl)-1-(propargylthio)-4*H*-benzo[*f*][1,2,4]triazolo[4,3-*a*][1,4]diazepin-4-yl)propionate (compound 49),
methyl (*S*)-3-(8-nitro-6-(2-fhiorophenyl)-1-(propargylthio)-4*H*-benzo[*f*][1,2,4]triazolo[4,3-*a*][1,4]diazepin-4-yl)propionate (compound 50),
methyl (*S*)-3-(8-chloro-6-(2-fluorophenyl)-1-((2-morpholinoethyl)thio)-4*H*-benzo[*f*][1,2,4]triazolo[4,3-*a*][1,4]diazepin-4-yl)propionate (compound 51),
methyl (*S*)-3-(8-bromo-6-(2-fluorophenyl)-1-((2-morpholinoethyl)thio)-4*H*-benzo[*f*][1,2,4]triazolo[4,3-*a*][1,4]diazepin-4-yl)propionate (compound 52),
methyl (*S*)-3-(8-nitro-6-(2-fluorophenyl)-1-((2-morpholinocthyl)thio)-4*H*-benzo[*f*][1,2,4]triazolo[4,3-*a*][1,4]diazepin-4-yl)propionate (compound 53),
methyl (*S*)-3-(8-chloro-6-(2-fluorophenyl)-1-((2-(diethylatnino)ethyl)thio)-4*H*-benzo[*f*][1,2,4]triazolo[4,3-*a*][1,4]diazepin-4-yl)propionate (compound 54),
methyl (*S*)-3-(8-bromo-6-(2-fluorophenyl)-1-((2-(diethylatnino)ethyl)thio)-4*H*-benzo[*f*][1,2,4]triazolo[4,3-*a*][1,4]diazepin-4-yl)propionate (compound 55),
methyl (*S*)-3-(8-nitro-6-(2-fluorophenyl)-1-((2-(diethylamino)ethyl)thio)-4*H*-benzo[*f*][1,2,4]triazolo[4,3-*a*][1,4]diazepin-4-yl)propionate (compound 56),
methyl (*S*)-3-(8-chloro-6-(2-fluorophenyl)-1-((3-(dimethylamino)propyl)thio)-4*H*-benzo[*f*][1,2,4]triazolo[4,3-*a*][1,4]diazepin-4-yl)propionate (compound 57),
methyl (*S*)-3-(8-bromo-6-(2-fluorophenyl)-1-((3-(dimethylamino)propyl)thio)-4*H*-benzo[*f*][1,2,4]triazolo[4,3-*a*][1,4]diazepin-4-yl)propionate (compound 58),
methyl (*S*)-3-(8-nitro-6-(2-fluorophenyl)-1-((3-(dimethylamino)propyl)thio)-4*H*-benzo[*f*][1,2,4]triazolo[4,3-*a*][1,4]diazepin-4-yl)propionate (compound 59),
methyl (*S*)-3-(8-chloro-6-(2-fluorophenyl)-1-((2-(4-methylpiperazin-1-yl)ethyl)thio)-4*H*-benzo[f][1,2,4]triazolo[4,3-*a*][1,4]diazepin-4-yl)propionate (compound 60),
methyl (*S*)-3-(8-bromo-6-(2-fluorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 61),
methyl (*S*)-3-(8-nitro-6-(2-fluorophenyl)-1-((2-(4-methylpiperazin-1-yl)ethyl)thio)-4H-benzo[*f*][1,2,4]triazolo[4,3-*a*][1,4]diazepin-4-yl)propionate (compound 62),
methyl (*S*)-3-(8-chloro-6-(2-fluorophenyl)-1-((1-methylpiperidin-4-yl)thio)-4*H*-benzo[*f*][1,2,4]triazolo[4,3-*a*][1,4]diazepin-4-yl)propionate (compound 63),
methyl (*S*)-3-(8-bromo-6-(2-fluorophenyl)-1-((1-metlhylpiperidin-4-yl)thio)-4*H*-benzo[*f*][1,2,4]triazolo[4,3-*a*][1,4]diazepin-4-yl)propionate (compound 64),
methyl (*S*)-3-(8-nitro-6-(2-fluorophenyl)-1-((1-methylpiperidin-4-yl)thio)-4*H*-benzo[*f*][1,2,4]triazolo[4,3-*a*][1,4]diazepin-4-yl)propionate (compound 65),
methyl (*S*)-3 -(8-chloro-6-(2-fluorophenyl)-1-((3-(4-(2-hydroxyethyl)piperazin-1-yl)propyl)thio)-4*H*-benzo[*f*][1,2,4]triazolo [4,3-*a*][1,4]diazepin-4-yl)propionate (compound 66),
methyl (*S*)-3-(8-bromo-6-(2-fluorophenyl)-1-((3-(4- (2-hydroxyethyl)piperazin-1-yl)propyl)thio)-4*H*-benzo[*f*][1,2,4]triazolo[4,3-*a*][1,4]diazepin-4-yl)propionate (compound 67),
methyl (*S*)-3-(8-nitro-6-(2-fluorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 68),
methyl (*S*)-3-(8-chloro-6-(2-fluorophenyl)-1-((morpholinomethyl)thio)-4*H*-benzo[*f*][1,2,4]triazolo[4,3-*a*][1,4]diazepin-4-yl)propionate (compound 69),
methyl (*S*)-3-(8-bromo-6-(2-fluorophenyl)-1-((morpholinomethyl)thio)-4*H*-benzo[*f*][1,2,4]triazolo[4,3-*a*][1,4]diazepin-4-yl)propionate (compound 70),

methyl (*S*)-3-(8-nitro-6-(2-fluorophenyl)-1-((morpholinomethyl)thio)-4*H*-benzo[*f*][1,2,4]triazolo[4,3-*a*][1,4]diazepin-4-yl)propionate (compound 71),
methyl (*S*)-3-(8-chloro-6-(2-fluorophenyl)-1-(((4-phenylpiperazin-1-yl)methyl)thio)-4*H*-benzo[*f*][1,2,4]triazolo[4,3 - *a*][1,4]diazepin-4-yl)propionate (compound 72),
methyl (*S*)-3-(8-bromo-6-(2-fluorophenyl)-1-(((4-phenylpiperazin-1-yl)methyl)thio)-4*H*-benzo[*f*][1,2,4]triazolo[4,3 - *a*][1,4]diazepin-4-yl)propionate (compound 73),
methyl (*S*)-3-(8-nitro-6-(2-fluorophenyl)-1-(((4-phenylpiperazin-1-yl)methyl)thio)-4*H*-benzo[*f*][1,2,4]triazolo[4,3 - *a*][1,4]diazepin-4-yl)propionate (compound 74),
methyl (*S*)-3-(8-chloro-6-(2-chlorophenyl)-1-((cyclopropylmethyl)thio)-4*H*-benzo[*f*][1,2,4]triazolo[4,3-*a*][1,4]diazepin-4-yl)propionate (compound 75),
methyl (*S*)-3-(8-bromo-6-(2-chlorophenyl)-1-((cyclopropylmethyl)thio)-4*H*-benzo[*f*][1,2,4]triazolo[4,3-*a*][1,4]diazepin-4-yl)propionate (compound 76),
methyl (*S*)-3-(8-nitro-6-(2-chlorophenyl)-1-((cyclopropylmethyl)thio)-4*H*-benzo[*f*][1,2,4]triazolo[4,3-*a*][1,4]diazepin-4-yl)propionate (compound 77),
methyl (*S*)-3-(8-chloro-6-(2-chlorophenyl)-1-(propargylthio)-4*H*-benzo[*f*][1,2,4]triazolo[4,3-*a*][1,4]diazepin-4-yl)propionate (compound 78),
methyl (*S*)-3-(8-bromo-6-(2-chlorophenyl)-1-(propargylthio)-4*H*-benzo[*f*][1,2,4]triazolo[4,3-*a*][1,4]diazepin-4-yl)propionate (compound 79),
methyl (*S*)-3-(8-nitro-6-(2-chlorophenyl)-1-(propargylthio)-4*H*-benzo[*f*][1,2,4]triazolo[4,3-*a*][1,4]diazepin-4-yl)propionate (compound 80),
methyl (*S*)-3-(8-chloro-6-(2-chlorophenyl)-1-((2-morpholinoethyl)thio)-4*H*-benzo[*f*][1,2,4]triazolo[4,3-*a*][1,4]diazepin-4-yl)propionate (compound 81),
methyl (*S*)-3-(8-bromo-6-(2-chlorophenyl)-1-((2-morpholinoethyl)thio)-4*H*-benzo[*f*][1,2,4]triazolo[4,3-*a*][1,4]diazepin-4-yl)propionate (compound 82),
methyl (*S*)-3-(8-nitro-6-(2-chlorophenyl)-1-((2-morpholinoethyl)thio)-4*H*-benzo[*f*][1,2,4]triazolo[4,3-*a*][1,4]diazepin-4-yl)propionate (compound 83),
methyl (*S*)-3-(8-chloro-6-(2-chlorophenyl)-1-((2-(diethylatnino)ethyl)thio)-4*H*-benzo[*f*][1,2,4]triazolo[4,3-*a*][1,4]diazepin-4-yl)propionate (compound 84),
methyl (*S*)-3-(8-bromo-6-(2-chlorophenyl)-1-((2-(diethylamino)ethyl)thio)-4*H*-benzo[*f*][1,2,4]triazolo[4,3-*a*][1,4]diazepin-4-yl)propionate (compound 85),
methyl (*S*)-3-(8-nitro-6-(2-chlorophenyl)-1-((2-(diethylatnino)ethyl)thio)-4*H*-benzo[*f*][1,2,4]triazolo[4,3-*a*][1,4]diazepin-4-yl)propionate (compound 86),
methyl (*S*)-3-(8-chloro-6-(2-chlorophenyl)-1-((3-(dimethylamino)propyl)thio)-4*H*-benzo[*f*][1,2,4]triazolo[4,3-*a*][1,4]diazepin-4-yl)propionate (compound 87),
methyl (*S*)-3-(8-bromo-6-(2-chlorophenyl)-1-((3-(dimethylamino)propyl)thio)-4*H*-benzo[*f*][1,2,4]triazolo[4,3-*a*][1,4]diazepin-4-yl)propionate (compound 88),
methyl (*S*)-3-(8-nitro-6-(2-chlorophenyl)-1-((3-(dimethylamino)propy)propyl)thio)-4*H*-benzo[*f*][1,2,4]triazolo[4,3 - *a*][1,4]diazepin-4-yl)propionate (compound 89),
methyl (*S*)-3-(8-chloro-6-(2-chlorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 90),
methyl (*S*)-3-(8-bromo-6-(2-chlorophenyl)-1-((2-(4-methylpiperazin-1-yl)ethyl)thio)-4*H*-benzo[*f*][1,2,4]triazolo[4,3-*a*][1,4]diazepin-4-yl)propionate (compound 91),
methyl (*S*)-3-(8-nitro-6-(2-chlorophenyl)-1-((2-(4-methylpiperazin-1-yl)ethyl)thio)-4*H*-benzo[f*]*[1,2,4]triazolo[4,3-*a*][1,4]diazepin-4-yl)propionate (compound 92),
methyl (*S*)-3-(8-chloro-6-(2-chlorophenyl)-1-((1-methylpipen-din-4-yl)thio)-4*H*-benzo[*f*][1,2,4]triazolo[4,3-*a*][1,4]diazepin-4-yl)propionate (compound 93),
methyl (*S*)-3-(8-bromo-6-(2-chlorophenyl)-1-((1-methylpipen-din-4-yl)thio)-4*H*-benzo[*f*][1,2,4]triazolo[4,3-*a*][1,4]diazepin-4-yl)propionate (compound 94),
methyl (*S*)-3-(8-nitro-6-(2-chlorophenyl)-1-((1-methylpiperidin-4-yl)thio)-4*H*-benzo[*f*][1,2,4]triazolo[4,3-*a*][1,4]diazepin-4-yl)propionate (compound 95),
methyl (*S*)-3-(8-chloro-6-(2-chlorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 96),
methyl (*S*)-3-(8-bromo-6-(2-chlorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 97),
methyl (*S*)-3-(8-nitro-6-(2-chlorophenyl)-1-((3-(4-(2-hydroxyethyl)piperazin-1-yl)propyl)thio)-4*H*-benzo[*f*][1,2,4]triazolo[4,3-*a*][1,4]diazepin-4-yl)propionate (compound 98),
methyl (*S*)-3-(8-chloro-6-(2-chlorophenyl)-1-((morpholinomethyl)thio)-4*H*-benzo[*f*][1,2,4]triazolo[4,3-*a*][1,4]diazepin-4-yl)propionate (compound 99),

methyl (*S*)-3-(8-bromo-6-(2-chlorophenyl)-1-((morpholinomethyl)thio)-4*H*-benzo[*f*][1,2,4]triazolo[4,3-*a*][1,4]diazepin-4-yl)propionate (compound 100),
methyl (*S*)-3-(8-nitro-6-(2-chlorophenyl)-1-((morpholinomethyl)thio)-4*H*-benzo[*f*][1,2,4]triazolo[4,3-*a*][1,4]diazepin-4-yl)propionate (compound 101),
methyl (*S*)-3-(8-chloro-1-(methylthio)-6-phenyl-4*H*-benzo[*f*][1,2,4]triazolo[4,3-*a*][1,4]diazepin-4-yl)propionate (compound 102),
methyl (*S*)-3-(8-bromo-1-(methylthio)-6-phenyl-4*H*-benzo[*f*][1,2,4]triazolo[4,3-*a*][1,4]diazepin-4-yl)propionate (compound 103),
methyl (*S*)-3-(8-nitro-1-(methylthio)-6-phenyl-4*H*-benzo[*f*][1,2,4]triazolo[4,3-*a*][1,4]diazepin-4-yl)propionate (compound 104),
methyl (*S*)-3-(8-chloro-1-((2-morpholinoethyl)thio)-6-phenyl-4*H*-benzo[*f*][1,2,4]triazolo[4,3-*a*][1,4]diazepin-4-yl)propionate (compound 105),
methyl (*S*)-3-(8-bromo-1-((2-morpholinoethyl)thio)-6-phenyl-4*H*-benzo[*f*][1,2,4]triazolo[4,3-*a*][1,4]diazepin-4-yl)propionate (compound 106),
methyl (*S*)-3-(8-nitro-1-((2-morpholinoethyl)thio)-6-phenyl-4*H*-benzo[*f*][1,2,4]triazolo[4,3-*a*][1,4]diazepin-4-yl)propionate (compound 107),
methyl (*S*)-3-(8-chloro-1-((3-(dimethylamino)propyl)thio)-6-phenyl-4*H*-benzo[*f*][1,2,4]triazolo[4,3-*a*][1,4]diazepin-4-yl)propionate (compound 108),
methyl (*S*)-3-(8-bromo-1-((3-(dimethylamino)propyl)thio)-6-phenyl-4*H*-benzo[*f*][1,2,4]triazolo[4,3-*a*][1,4]diazepm-4-yl)propionate (compound 109),
methyl (*S*)-3-(8-nitro-1-((3-(dimethylamino)propyl)thio)-6-phenyl-4H-benzo[*f*][1,2,4]triazolo[4,3-*a*][1,4]diazepin-4-yl)propionate (compound 110),
methyl (*S*)-3-(8-chloro-6-(2-chlorophenyl)-1-(((phosphonooxy)methyl)thio[*f*][1,2,4]triazolo[4,3-*a*][1,4]diazepin-4-yl)propionate (compound 111),
methyl (*S*)-3-(8-bromo-6-(2-chlorophenyl)-1-(((phosphonooxy)methyl)thio)-4*H*-benzo[*f*][1,2,4]triazolo[4,3-*a*][1,4]diazepin-4-yl)propionate (compound 112),
methyl (*S*)-3-(8-nitro-6-(2-chlorophenyl)-1-(((phosphonooxy)methyl)thio[*f*][1,2,4]triazolo[4,3-*a*][1,4]diazepin-4-yl)propionate (compound 113),
methyl (*S*)-3-(8-bromo-6-(pyridin-2-yl)-1-methylthio-4*H*-benzo[*f*][1,2,4]triazolo[4,3-*a*][1,4]diazepin-4-yl)propionate (compound 114),
methyl (*S*)-3-(8-bromo-6-(pyridin-2-yl)-1-ethylthio-4*H*-benzo[*f*][1,2,4]triazolo[4,3-*a*][1,4]diazepin-4-yl)propionate (compound 115),
methyl (*S*)-3-(8-bromo-6-(pyridin-2-yl)-1-((cyclopropylmethyl)thio)-4*H*-benzo[*f*][1,2,4]triazolo[4,3-*a*][1,4]diazepm-4-yl)propionate (compound 116),
methyl (*S*)-3-(8-bromo-6-(pyridin-2-yl)-1-((2-morpholinoethyl)thio)-4*H*-benzo[*f*][1,2,4]triazolo[4,3-*a*][1,4]diazepm-4-yl)propionate (compound 117),
methyl (*S*)-3-(8-bromo-6-(pyridin-2-yl)-1-(3-((dimethylamino)propyl)thio)-4*H*-benzo[*f*][1,2,4]triazolo[4,3-*a*][1,4]diazepin-4-yl)propionate (compound 118),
methyl (*S*)-3-(8-chloro-6-(2-chlorophenyl)-1-(2-((pyrrolidin-1-yl)ethyl)thio)-4*H*-benzo[*f*][1,2,4]triazolo[4,3-*a*][1,4]diazepin-4-yl)propionate (compound 119),
methyl (*S*)-3-(8-bromo-6-(2-fluorophenyl)-1-(2-((pyrrolidin-1-yl)ethyl)thio)-4*H*-benzo[*f*][1,2,4]triazolo[4,3-*a*][1,4]diazepin-4-yl)propionate (compound 120),
methyl (*S*)-3-(8-bromo-6-(2-chlorophenyl)-1-((2-(pyrrolidin-1-yl)ethyl)thio)-4*H*-benzo[*f*][1,2,4]triazolo[4,3-*a*][1,4]diazepin-4-yl)propionate (compound 121),
methyl (*S*)-3-(8-chloro-6-(2-chlorophenyl)-1-(2-((pipendin-1-yl)ethyl)thio)-4*H*-benzo[*f*][1,2,4]triazolo[4,3-*a*][1,4]diazepin-4-yl)propionate (compound 122),
methyl (*S*)-3-(8-bromo-6-(2-fluorophenyl)-1-(2-((pipendin-1-yl)ethyl)thio)-4*H*-benzo[*f*][1,2,4]triazolo[4,3-*a*][1,4]diazepin-4-yl)propionate (compound 123),
methyl (*S*)-3-(8-chloro-6-(2-chlorophenyl)-1-(2-((pyndin-4-yl)methyl)thio)-4*H*-benzo[*f*][1,2,4]triazolo[4,3-*a*][1,4]diazepin-4-yl)propionate (compound 124),
methyl (*S*)-3-(8-bromo-6-(2-fluorophenyl)-1-(2-((pyndin-4-yl)methyl)thio)-4*H*-benzo[*f*][1,2,4]triazolo[4,3-*a*][1,4]diazepin-4-yl)propionate (compound 125),
methyl (*S*)-3-(8-bromo-6-(pyridin-2-yl)-1-(((pyridin-4-yl)methyl)thio)-4*H*-benzo[*f*][1,2,4]triazolo[4,3-*a*][1,4]diazepin-4-yl)propionate (compound 126),
methyl (*S*)-3-(8-chloro-6-(2-chlorophenyl)-1-(2-((4,4-difluoropipendin-1-yl)ethyl)thio)-4*H*-benzo[*f*][1,2,4]triazolo[4,3-*a*][1,4]diazepin-4-yl)propionate (compound 127),
methyl (*S*)-3-(8-bromo-6-(2-fluorophenyl)-1-(2-((4,4-difluoropipendin-1-yl)ethyl)thio))-4*H*-benzo[*f*][1,2,4]triazolo[4,3-*a*][1,4]diazepin-4-yl)propionate (compound 128),

methyl (*S*)-3-(8-chloro-6-(2-chlorophenyl)-1-((2-(4-methylpiperazin-1-yl)-2-oxyethyl)thio)-4*H*-benzo[*f*][1,2,4]triazolo[4,3-*a*][1,4]diazepin-4-yl)propionate (compound 129),
methyl (*S*)-3-(8-chloro-6-(2-chlorophenyl)-1-(2-(4-methylpiperazine-1-carbonyl)thio)-4*H*-benzo[*f*][1,2,4]triazolo[4,3-*a*][1,4]diazepin-4-yl)propionate (compound 130),
methyl (*S*)-3-(8-bromo-6-(2-fluorophenyl)-1-(2-(4-methylpiperazine-l-carbonyl)thio))-4*H*-benzo[*f*][1,2,4]triazolo[4,3-*a*][1,4]diazepin-4-yl)propionate (compound 131),
methyl (*S*)-3-(8-chloro-6-(2-chlorophenyl)-1-(3-((diethylamino)propyl)thio)-4*H*-benzo[*f*][1,2,4]triazolo[4,3-*a*][1,4]diazepin-4-yl)propionate (compound 132),
methyl (*S*)-3-(8-chloro-6-(2-chlorophenyl)-1-(2-((dimethylamino)ethyl)thio)-4*H*-benzo[*f*][1,2,4]triazolo[4,3-*a*][1,4]diazepin-4-yl)propionate (compound 133),
methyl (*S*)-3-(8-bromo-6-(2-fluorophenyl)-1-(2-((dimethylatnino)ethyl)thio)-4*H*-benzo[*f*][1,2,4]triazolo[4,3-*a*][1,4]diazepin-4-yl)propionate (compound 134),
methyl (*S*)-3-(8-chloro-6-(2-chlorophenyl)-1-(2-((4-hydroxypiperidin-1-yl)ethyl)thio)-4*H*-benzo[*f*][1,2,4]triazolo[4,3- *a*][1,4]diazepin-4-yl)propionate (compound 135),
methyl (*S*)-3-(8-chloro-6-(2-chlorophenyl)-1-(2-((4-methylpiperidin-2-carbonyl)ethyl)thio)-4*H*-benzo[*f*][1,2,4]triazolo[4,3-*a*][1,4]diazepin-4-yl)propionate (compound136),
(*S*)-3-(8-chloro-6-(2-chlorophenyl)-1-thio-2,4-dihydro-1*H*-benzo[*f*][1,2,4]triazolo[4,3-*a*][1,4]diazepin-4-yl)propionic acid-2-morpholinoethyl ester (compound 137),
methyl (*S*)-3-(8-chloro-6-(2-chlorophenyl)-1-(2-((1*H*-imidazol-1-yl)ethyl)thio)-4*H*-benzo[*f*][1,2,4]triazolo[4,3-*a*][1,4]diazepin-4-yl)propionate (compound 138),
methyl (*S*)-3-(8-bromo-6-(2-fluorophenyl)-1-(2-((1*H*-imidazol-1-yl)ethyl)thio)-4*H*-benzo[*f*][1,2,4]triazolo[4,3-*a*][1,4]diazepin-4-yl)propionate (compound 139),
methyl (*S*)-3-(8-chloro-6-(2-chlorophenyl)-1-(2-((1*H*-pyrrol-1-yl)ethyl)thio)-4*H*-benzo[*f*][1,2,4]triazolo[4,3-*a*][1,4]diazepin-4-yl)propionate (compound 140),
methyl (*S*)-3-(8-chloro-6-(2-chlorophenyl)-1-((1-methyl-1*H*-imidazol-4-yl)thio)-4*H*-benzo[*f*][1,2,4]triazolo[4,3-*a*][1,4]diazepin-4-yl)propionate (compound 141),
methyl (*S*)-3-(8-chloro-6-(2-chlorophenyl)-1-((ethyl(methyl)carbamoyl)thio)-4*H*-benzo[*f*][1,2,4]triazolo[4,3-*a*][1,4]diazepin-4-yl)propionate (compound 142),
methyl (*S*)-3-(8-chloro-6-(2-chlorophenyl)-1-(3-((dimethylamino)-3-oxopropyl)thio)-4*H*-benzo[*f*][1,2,4]triazolo[4,3-*a*][1,4]diazepin-4-yl)propionate (compound 143), and
methyl (*S*)-3 -(8-chloro-6-(2-chlorophenyl)-1-(((4,5-dihydro-1*H*-imidazol-2-yl)methyl)thio)-4*H*-benzo[*f*][1,2,4]triazolo[4,3-*a*][1,4]diazepin-4-yl)propionate (compound 144);
some of the compounds have the following structures:

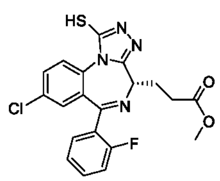
compound 1

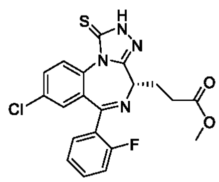
tautomer of compound 1

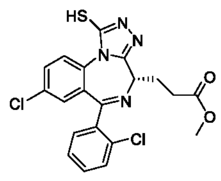
compound 2

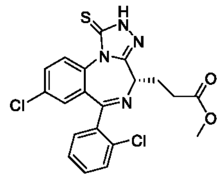
tautomer of compound 2

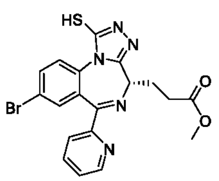
compound 3

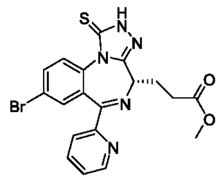
tautomer of compound 3

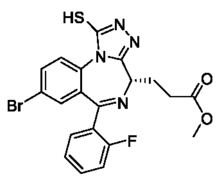
compound 4

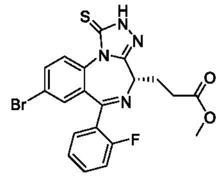
tautomer of compound 4

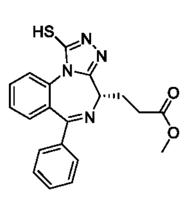
compound 5

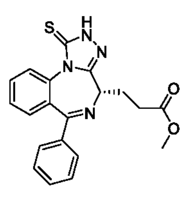
tautomer of compound 5

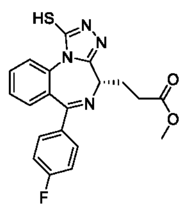
compound 6

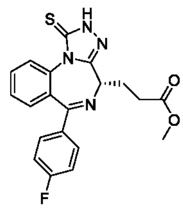
tautomer of compound 6

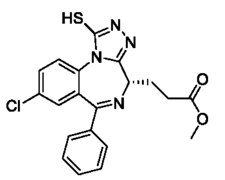
compound 7

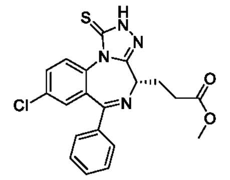
tautomer of compound 7

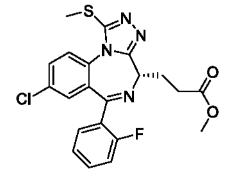
compound 8

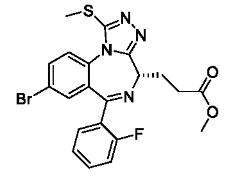
compound 9

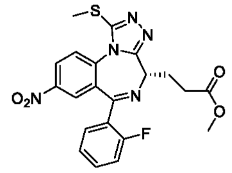
compound 10

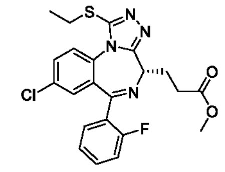
compound 11

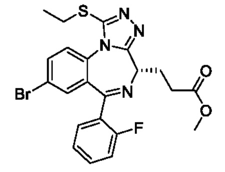
compound 12

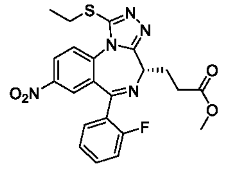
compound 13

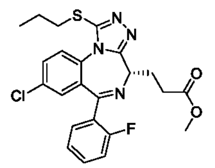
compound 14

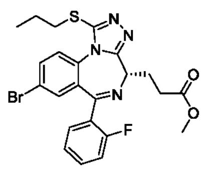
compound 15

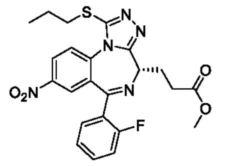
compound 16

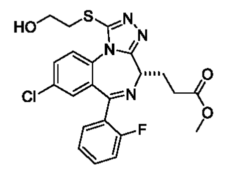
compound 17

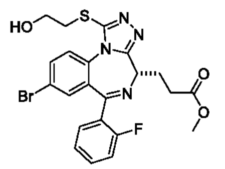
compound 18

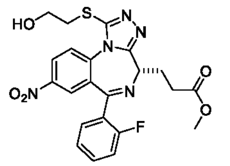
compound 19

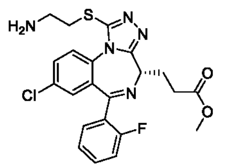
compound 20

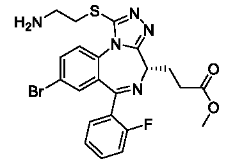
compound 21

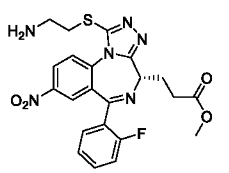
compound 22

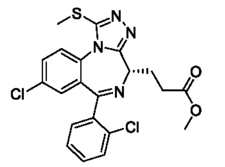
compound 23

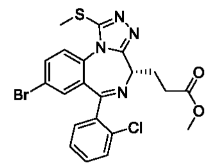
compound 24

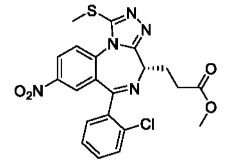
compound 25

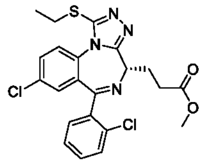
compound 26

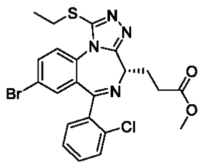
compound 27

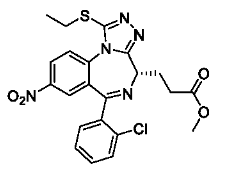
compound 28

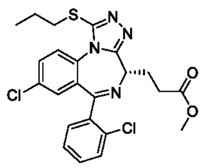
compound 29

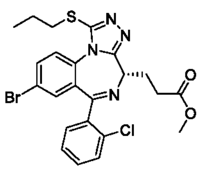
compound 30

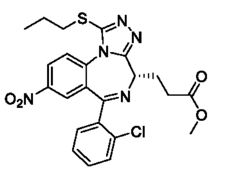
compound 31

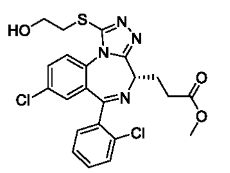
compound 32

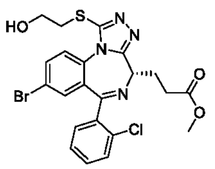
compound 33

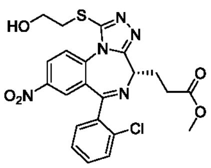
compound 34

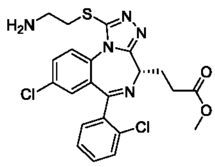
compound 35

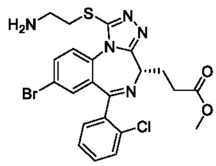
compound 36

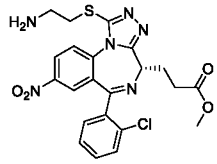
compound 37

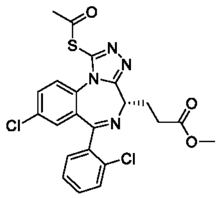
compound 38

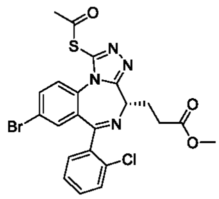
compound 39

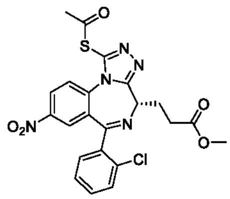
compound 40

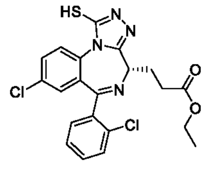
compound 41

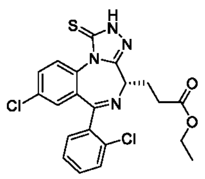
tautomer of compound 41

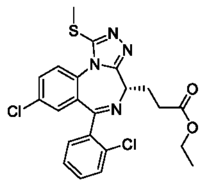
compound 42

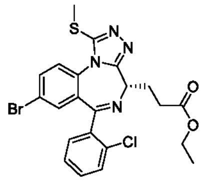
compound 43

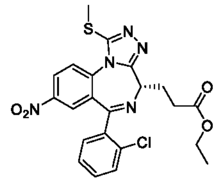
compound 44

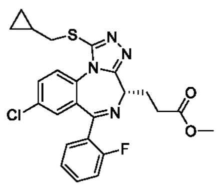
**compound 45**

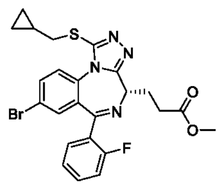
**compound 46**

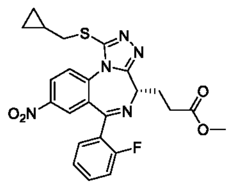
**compound 47**

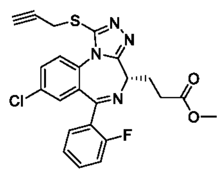
**compound 48**

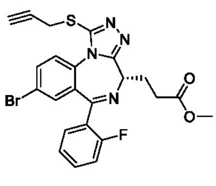
**compound 49**

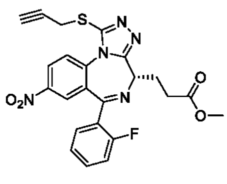
**compound 50**

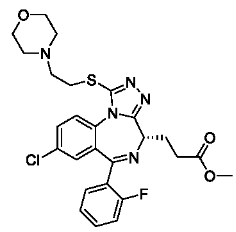
**compound 51**

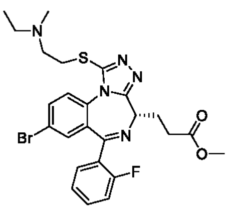
**compound 52**

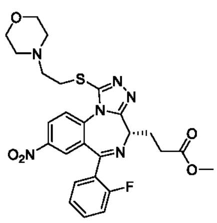
compound 53

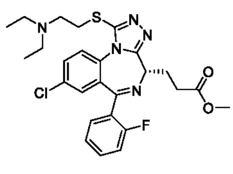
compound 54

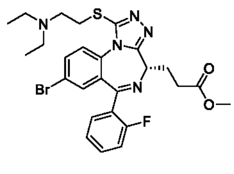
compound 55

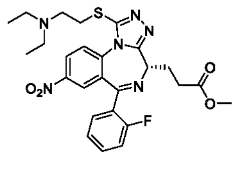
compound 56

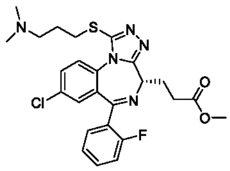
compound 57

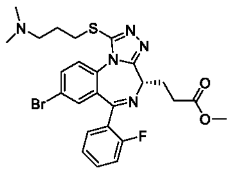
compound 58

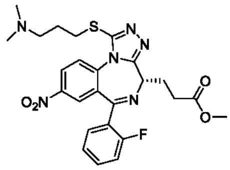
compound 59

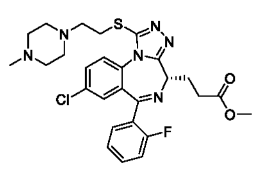
compound 60

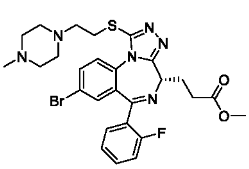
compound 61

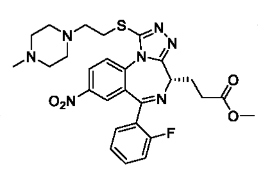
compound 62

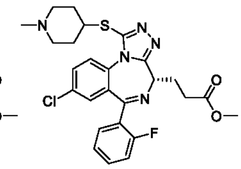
compound 63

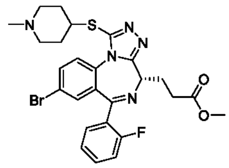
compound 64

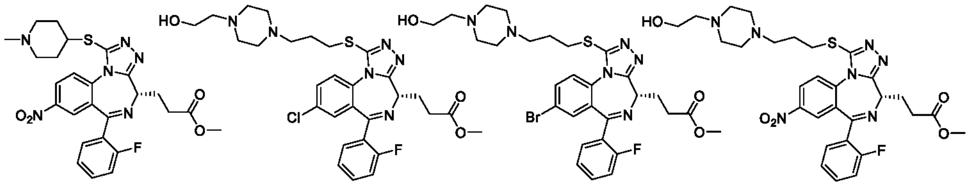
compound 65

compound 66

compound 67

compound 68

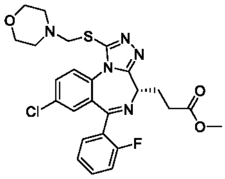
compound 69

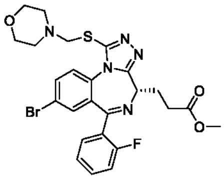
compound 70

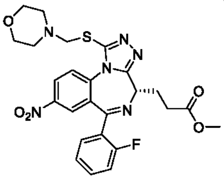
compound 71

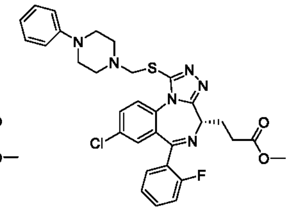
compound 72

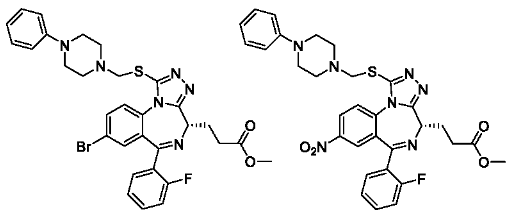
compound 73

compound 74

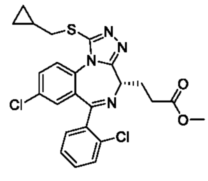
compound 75

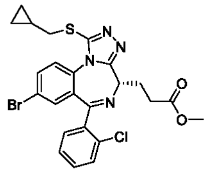
compound 76

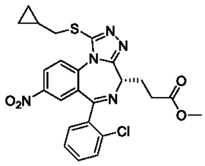
compound 77

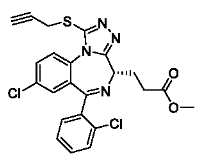
compound 78

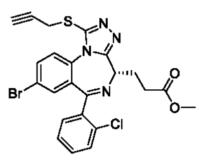
compound 79

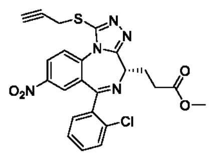
compound 80

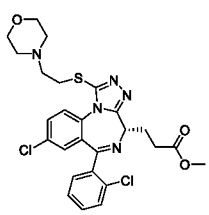
compound 81

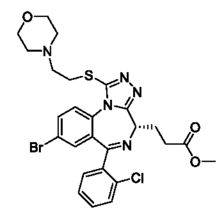
compound 82

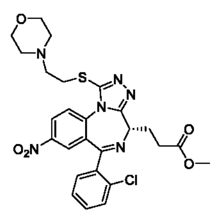
compound 83

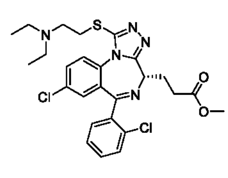
compound 84

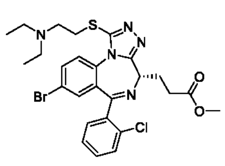
compound 85

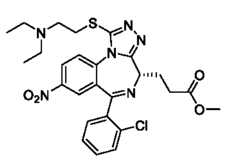
compound 86

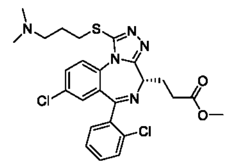
compound 87

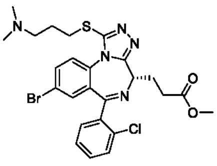
compound 88

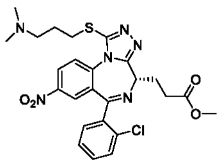
compound 89

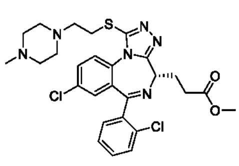
compound 90

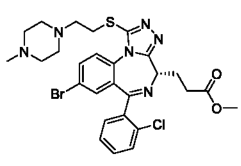
compound 91

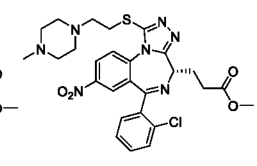
compound 92

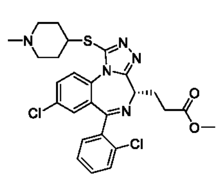
compound 93

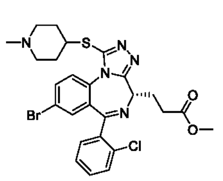
compound 94

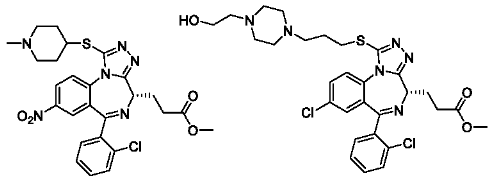
compound 95

compound 96

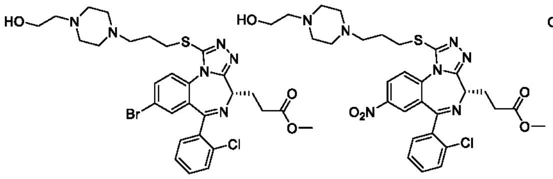
compound 97

compound 98

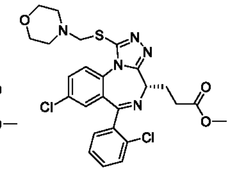
compound 99

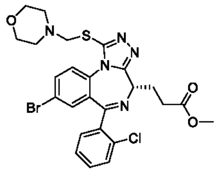
compound 100

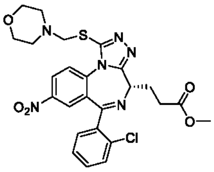
compound 101

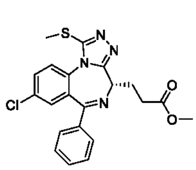
compound 102

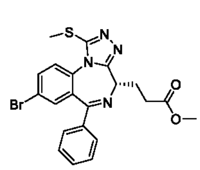
compound 103

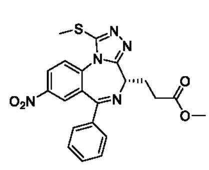
compound 104

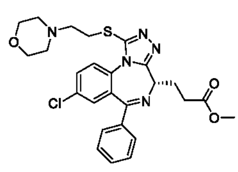
compound 105

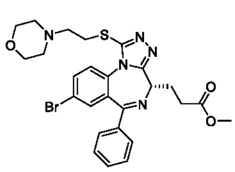
compound 106

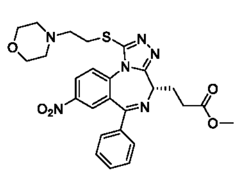
compound 107

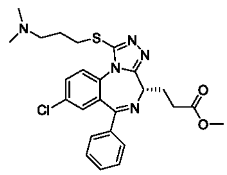
compound 108

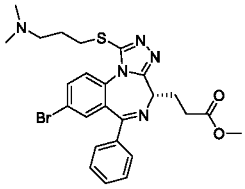
compound 109

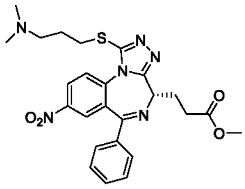
compound 110

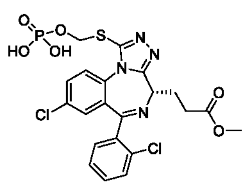
compound 111

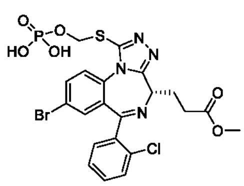
compound 112

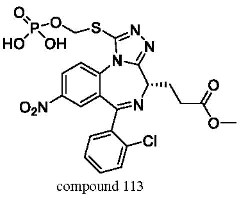

compound 113

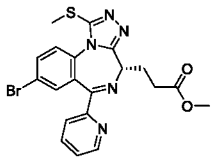

compound 114

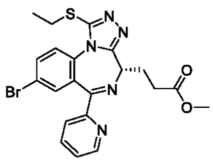

compound 115

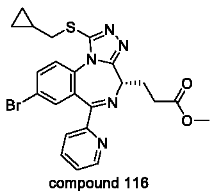

compound 116

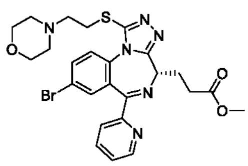

compound 117

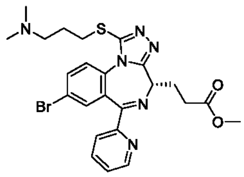

compound 118

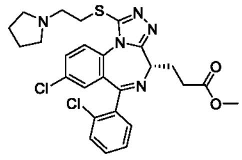

compound 119

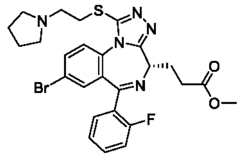

compound 120

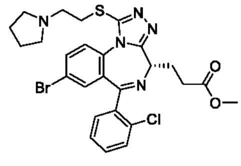

compound 121

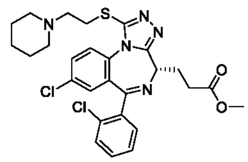

compound 122

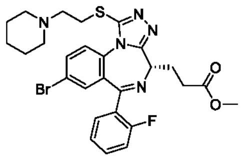

compound 123

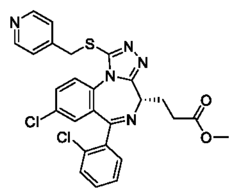

compound 124

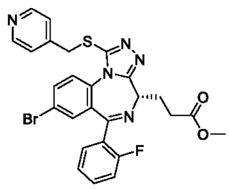

compound 125

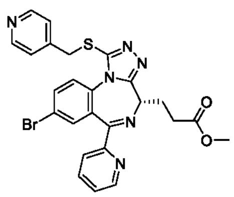

compound 126

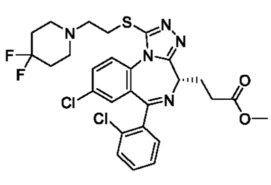

compound 127

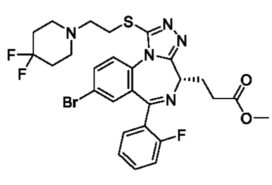

compound 128

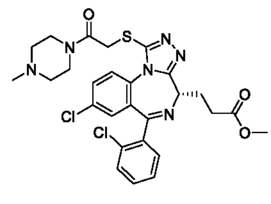

compound 129

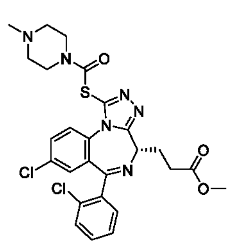

compound 130

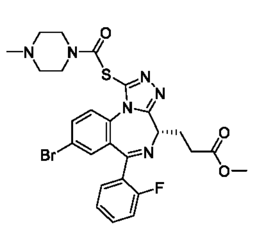

compound 131

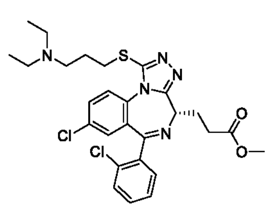

compound 132

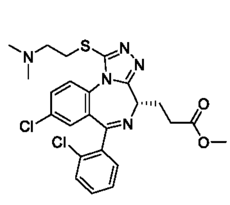

compound 133

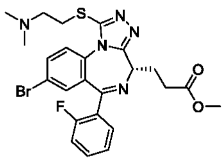
compound 134

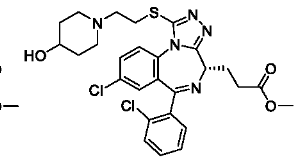
compound 135

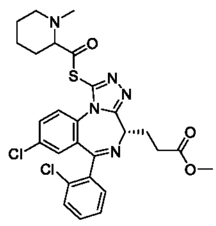
compound 136

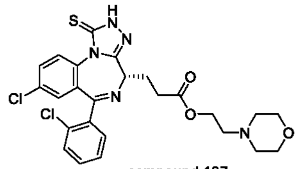
compound 137

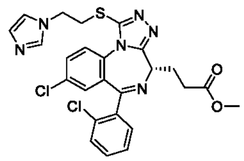
compound 138

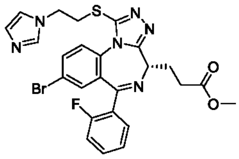
compound 139

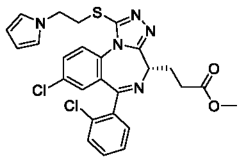
compound 140

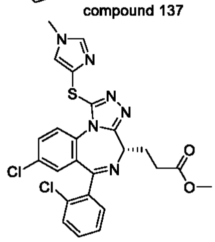
compound 141

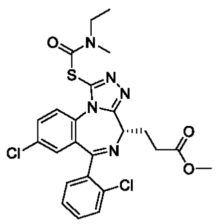
compound 142

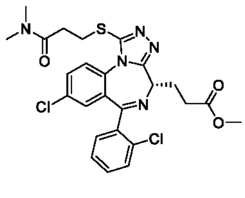
compound 143

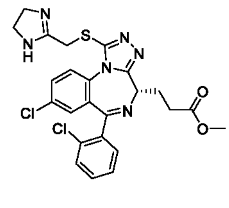
compound 144

**8.** A pharmaceutical composition comprising an effective amount of the compound according to any one of claims 1 to 7 and one or more pharmaceutically acceptable carriers, wherein the effective amount is 10 mg to 3000 mg.

**9.** The compound according to any one of claims 1 to 7 or the pharmaceutical composition according to claim 5, wherein the pharmaceutically acceptable salt thereof is selected from acetate, adipate, aspartate, benzoate, benzenesulfonate, bicarbonate/carbonate, bisulfate/sulfate, borate, camphorsulfonate, citrate, cyclamate, edisylate, ethanesulfonate, formate, fumarate, glucoheptonate, gluconate, glucuronate, hexafluorophosphate, hydrochloride/oxide, hydrobromide/bromide, hydroiodide/iodide, isethionate, lactate, malate, maleate, malonate, methanesulfonate, methylsulfate, naphthoate, 2-naphthalenesulfonate, nicotinate, nitrate, orotate, oxalate, palmitate, dihydronaphthoate, phosphate/hydrophosphate/dihydrophosphate, pyroglutamate, saccharate, stearate, succinate, tannate, tartrate, tosylate, trifluoroacetate and xinafoate, aluminum salt, arginine, benzathine, calcium salt, choline, diethylamine salt, diethanolamine salt, glycinate, lysinate, magnesium salt, meglumine salt, ethanolamine salt, sodium salt, potassium salt, ammonium salt, tromethamine salt, and zinc salt.

**10.** A preparation method for the compound according to any one of claims 1 to 7, wherein the method is according to a reaction route as follows:

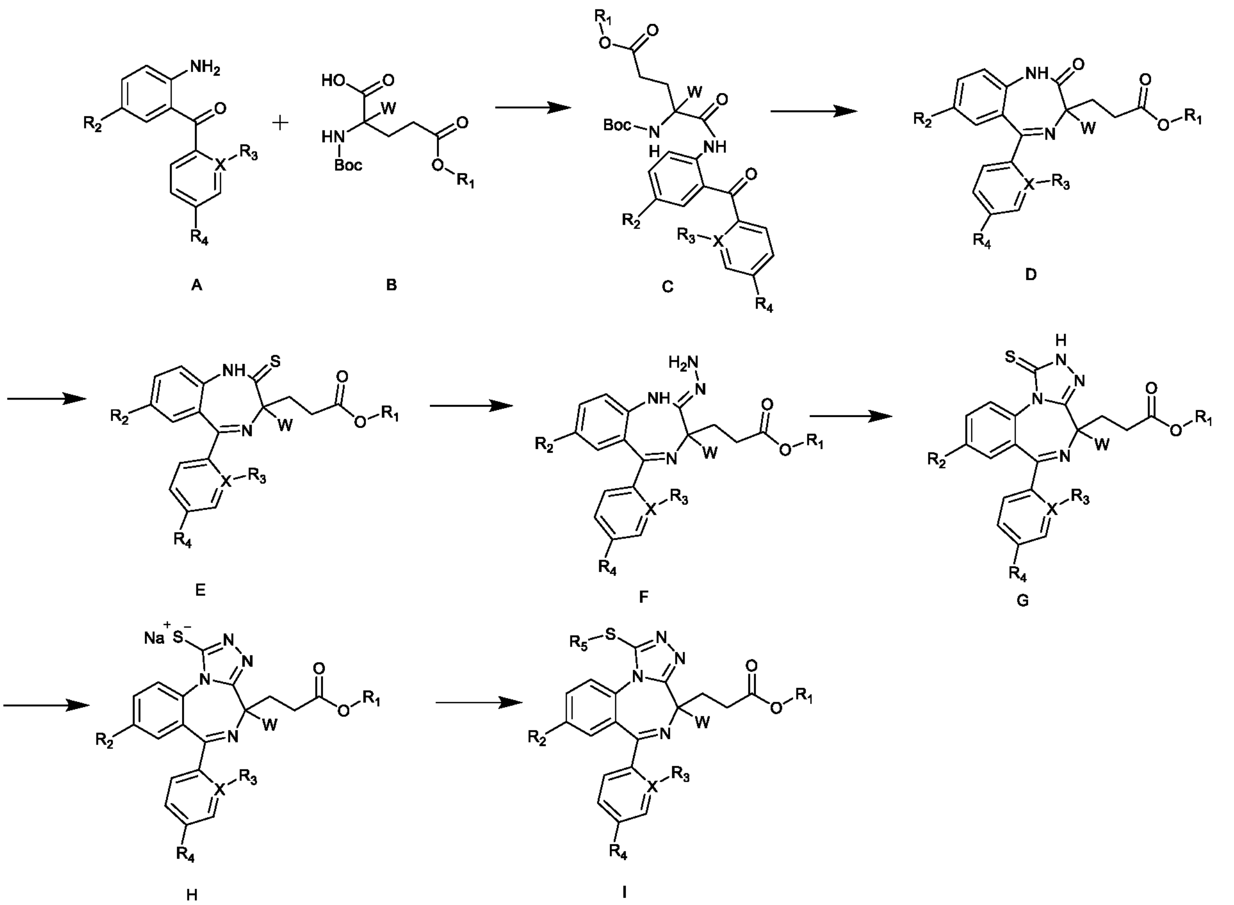

A B C D E F G H I

wherein $R_1$, $R_2$, $R_3$, $R_4$, $R_5$, and W are as defined in any one of claims 1 to 7;
and the method comprises the following steps:

A. subjecting the A and a chiral amino acid protected by $N_2$ or a derivative thereof to a condensation reaction to obtain an intermediate C,
B. removing the protective group of the intermediate C to obtain a benzodiazepine intermediate D,
C. subjecting the benzodiazepine intermediate D and Lawesson reagent or phosphorus pentasulfide to a reaction under heating and reflux conditions to obtain an active intermediate E,
D. subjecting the active intermediate E and hydrazine hydrate to a substitution reaction under an ice bath condition or at room temperature or under a solvent reflux condition to obtain an intermediate F,
E. subjecting the intermediate F and thiophosgene to a ring-closure reaction under a basic condition to obtain a target product G,
F. subjecting the intermediate G and a sodium salt to a reaction under a basic condition to obtain a target product H, and
G. subjecting the intermediate H to a substitution reaction to obtain a target product I.

**11.** Use of the compound according to any one of claims 1 to 7 or the composition according to claim 8 for manufacturing a medicament for sedation and anesthesia.

**12.** A method of sedation and anesthesia comprising intravenously administering an effective amount of the compound according to any one of claims 1 to 7 or the pharmaceutical composition according to claim 8.

**INTERNATIONAL SEARCH REPORT**

International application No.
**PCT/CN2021/129655**

**A. CLASSIFICATION OF SUBJECT MATTER**

C07D 487/04(2006.01)i； C07F 9/6561(2006.01)i； A61K 31/5517(2006.01)i； A61K 31/675(2006.01)i； A61P 25/20(2006.01)i； A61P 23/00(2006.01)i

According to International Patent Classification (IPC) or to both national classification and IPC

**B. FIELDS SEARCHED**

Minimum documentation searched (classification system followed by classification symbols)

C07D;C07F;A61K;A61P

Documentation searched other than minimum documentation to the extent that such documents are included in the fields searched

Electronic data base consulted during the international search (name of data base and, where practicable, search terms used)

CNTXT, ENTXT, DWPI, CJFD, CNKI, Caplus(STN), Registry(STN): 中国药科大学, 钱海, 石炜, 黄文龙, 李启飞, 蔡衍, 莫佳贤, 陈斌, 周嘉琪, 邹昱星, 苯, 氮, 杂环, 硫, 麻醉, 镇静, phenyl, aza, heterocycl, sulfur, benzodiazepine, sedation, anesthesia

**C. DOCUMENTS CONSIDERED TO BE RELEVANT**

| Category* | Citation of document, with indication, where appropriate, of the relevant passages | Relevant to claim No. |
|---|---|---|
| PX | CN 112358483 A (CHINA PHARMACEUTICAL UNIVERSITY) 12 February 2021 (2021-02-12)<br>claims 1-12, embodiments 1-113 | 1-12 |
| X | CN 106892924 A (SICHUAN KELUN-BIOTECH BIOPHARMACEUTICA CO., LTD.) 27 June 2017 (2017-06-27)<br>claims 1-13, embodiment 3 | 1-12 |
| X | WO 2018196662 A1 (SICHUAN KELUN-BIOTECH BIOPHARMACEUTICA CO., LTD.) 01 November 2018 (2018-11-01)<br>claims 1-17 | 1-12 |
| A | CN 101501019 A (PAION UK LIMITED) 05 August 2009 (2009-08-05)<br>entire document | 1-12 |
| A | CN 102781943 A (GLAXOSMITHKLINE LLC) 14 November 2012 (2012-11-14)<br>entire document | 1-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 January 2022** | **11 February 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

INTERNATIONAL SEARCH REPORT

International application No.
PCT/CN2021/129655

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **12**
because they relate to subject matter not required to be searched by this Authority, namely:

[1] Claim 12 relates to a method for diagnosing and treating diseases (PCT Rule 39.1(iv)). Therefore, the following search has been carried out on the basis of pharmaceutical uses of said compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2021/129655**

| Patent document cited in search report | Publication date (day/month/year) | Patent family member(s) | Publication date (day/month/year) |
|---|---|---|---|
| CN 112358483 A | 12 February 2021 | None | |
| CN 106892924 A | 27 June 2017 | EP 3363801 A1 | 22 August 2018 |
| | | EP 3363801 A4 | 19 June 2019 |
| | | JP 2018537417 A | 20 December 2018 |
| | | CN 108137606 A | 08 June 2018 |
| | | WO 2017101808 A1 | 22 June 2017 |
| | | US 2018312511 A1 | 01 November 2018 |
| WO 2018196662 A1 | 01 November 2018 | CN 110418791 A | 05 November 2019 |
| CN 101501019 A | 05 August 2009 | CN 101501019 B | 07 May 2014 |
| | | GB 0613692 D0 | 16 August 2006 |
| | | ES 2352360 T3 | 17 February 2011 |
| CN 102781943 A | 14 November 2012 | JP 2013510123 A | 21 March 2013 |
| | | JP 5702396 B2 | 15 April 2015 |
| | | KR 20120097508 A | 04 September 2012 |
| | | DK 2496582 T3 | 21 March 2016 |
| | | EP 2496582 A1 | 12 September 2012 |
| | | EP 2496582 B1 | 27 January 2016 |
| | | PL 2496582 T3 | 31 August 2016 |
| | | IL 239625 D0 | 31 August 2015 |
| | | IL 239625 A | 31 May 2018 |
| | | MX 2012005296 A | 19 June 2012 |
| | | MX 341212 B | 11 August 2016 |
| | | US 2017145021 A1 | 25 May 2017 |
| | | IL 219253 D0 | 28 June 2012 |
| | | IL 219253 A | 30 November 2015 |
| | | HU E026967 T2 | 28 July 2016 |
| | | US 2015299210 A1 | 22 October 2015 |
| | | US 9598420 B2 | 21 March 2017 |
| | | US 2012252781 A1 | 04 October 2012 |
| | | US 9102677 B2 | 11 August 2015 |
| | | JP 2017039738 A | 23 February 2017 |
| | | HK 1221221 A1 | 26 May 2017 |
| | | CY 1117424 T1 | 26 April 2017 |
| | | WO 2011054845 A1 | 12 May 2011 |
| | | BR 112012010705 A2 | 29 March 2016 |
| | | AU 2010317096 A1 | 31 May 2012 |
| | | AU 2010317096 B2 | 26 March 2015 |
| | | HR P20160253 T1 | 08 April 2016 |
| | | EA 201290184 A1 | 28 December 2012 |
| | | EA 023441 B1 | 30 June 2016 |
| | | ME 02356 B | 20 June 2016 |
| | | EP 3050885 A1 | 03 August 2016 |
| | | EP 3050885 B1 | 18 October 2017 |
| | | SI 2496582 T1 | 29 April 2016 |
| | | JP 2015143232 A | 06 August 2015 |
| | | ES 2564318 T3 | 21 March 2016 |
| | | SM T201600104 B | 29 April 2016 |
| | | ES 2654423 T3 | 13 February 2018 |
| | | CA 2779423 A1 | 12 May 2011 |
| | | CA 2779423 C | 14 August 2018 |

Form PCT/ISA/210 (patent family annex) (January 2015)

**INTERNATIONAL SEARCH REPORT**
**Information on patent family members**

International application No.
**PCT/CN2021/129655**

| Patent document cited in search report | Publication date (day/month/year) | Patent family member(s) | Publication date (day/month/year) |
|---|---|---|---|
| | | RS 54645 B1 | 31 August 2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202011237945 A